(19) Europäisches Patentamt
European Patent Office
Office européen des brevets

(11) EP 2 475 020 A1

(12) **EUROPEAN PATENT APPLICATION**
published in accordance with Art. 153(4) EPC

(43) Date of publication:
**11.07.2012 Bulletin 2012/28**

(21) Application number: **10812025.4**

(22) Date of filing: **27.08.2010**

(51) Int Cl.:
***H01L 51/50** (2006.01)* ***C07D 519/00** (2006.01)*
***C09K 11/06** (2006.01)* ***C07F 15/00** (2006.01)*

(86) International application number:
**PCT/JP2010/064648**

(87) International publication number:
**WO 2011/024976 (03.03.2011 Gazette 2011/09)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **31.08.2009 JP 2009201152**
**28.09.2009 JP 2009223452**
**30.03.2010 JP 2010079922**

(71) Applicant: **FUJIFILM Corporation**
**Tokyo 106-0031 (JP)**

(72) Inventor: **FUKUZAKI, Eiji**
**Ashigarakami-gun,**
**Kanagawa (JP)**

(74) Representative: **HOFFMANN EITLE**
**Patent- und Rechtsanwälte**
**Arabellastraße 4**
**81925 München (DE)**

# (54) ORGANIC ELECTROLUMINESCENT ELEMENT

(57) The present invention provides an organic electroluminescence device having high durability, high efficiency, and small misalignment of chromaticity after low driving and deterioration in a device.

Provided is an organic electroluminescence device including at least one organic layer including a light emitting layer containing a light emitting material between a pair of electrodes, in which the organic electroluminescence device contains a compound having at least one specific group and a phosphorescent metal complex having a specific structure.

EP 2 475 020 A1

## Description

### TECHNICAL FIELD

The present invention relates to a luminescence device that converts electric energy into light to emit light, and particularly, to an organic electroluminescence device (a luminescence device or an EL device).

### BACKGROUND ART

Organic electroluminescence (EL) devices are capable of obtaining a light emission with high luminance intensity at low voltage and thus have gained attention as a promising display apparatus. An important characteristic of the organic electroluminescence device is power consumption. The power consumption is expressed as the product of voltage and electric current, and the lower the voltage required to obtain a desired brightness and the smaller the electric current, the lower the power consumption of the device.

As an attempt to lower the current value that flows through the device, a luminescence device utilizing the luminescence from an ortho-metalated iridium complex ($Ir(ppy)_3$: tris-ortho-metalated complex of Iridium (III) with 2-phenylpyridine) has been reported (see, for example, Patent Documents 1 to 3). The phosphorescent luminescence device described therein is greatly enhanced in the external quantum efficiency as compared with single luminescence devices of the related art, and thus succeeded in lowering the value of electric current.

In addition, in the manufacture of an organic electroluminescence device, a method of forming a thin film which is an organic layer formed between a pair of electrodes includes a deposition method such as a vacuum deposition, a wet method such as a spin coating method, a printing method, an inkjet method, and the like.

Among them, if a wet method is used, it is also possible to use polymeric organic compounds, for which it is difficult to form a film in dry processes such as vapor deposition, and when a wet method is used in a flexible display and the like, the method is appropriate from the viewpoint of durability such as flex resistance, film strength and the like, and when the device is manufactured to have a large area, the method is particularly preferable.

However, organic electroluminescence devices obtained by a wet method are problematic in that the devices have low light emission efficiency or device durability.

In addition, devices containing azacarbazole for the purpose of improving light emission efficiency and durability of a phosphorescent luminescence device (see Patent Documents 2 and 3) have been reported, but more improvements have been demanded from the viewpoint of durability and efficiency.

Patent Documents 4 and 5 have reported that luminescence devices, which applies an iridium complex compound having a structure condensed with aromatic rings (an imidazolyl group and the like) as a ligand to a light emitting material, improve the light emission efficiency. However, the luminescence devices are not sufficient from the viewpoint of luminescence quantum efficiency, driving voltage and durability, and, as a result, more improvements thereof have been demanded. Further, the application of the organic electroluminescence (EL) devices to a display apparatus has been put to practical use, but since chromaticity is not easily adjusted due to the bias of a generation position of excitons and the generation of associates, the development of methods for improving chromaticity has been required. For example, the reduction in y values of the CIE coordinate in blue materials has been required.

### RELATED ART

### PATENT DOCUMENTS

Patent Document 1: US Patent Application Publication No. 08/0297033
Patent Document 2: Japanese Patent Application Laid-Open No. 2005-340123
Patent Document 3: Japanese Patent Application Laid-Open No. 2006-120821
Patent Document 4: International Publication No. WO07/095118
Patent Document 5: Japanese Patent Application Laid-Open No. 2007-19462

### SUMMARY OF THE INVENTION

#### Problems to Be Solved by the Invention

Since a compound represented by Formula (1) as described below (for example, a compound having at least one group represented by any one of Formulas (4-1) to (4-10) and Formulas (7-1) to (7-10)) generally does not have

strong durability with respect to a hole, when the compound is used for a host material of a light emitting layer having a hole transporting property, it has been considered that the lifespan of the device is deteriorated.
Further, since a phosphorescent metal complex containing mono-anionic bidentate ligands represented by the following Formulas (A1) to (A4) and a metal having an atomic weight of 40 or more as described below generally has a small Ip value, the phosphorescent metal complex easily traps the holes in the light emitting layer and the recombination site of charges is easily biased toward a hole transporting layer when being used for a light emitting layer, such that it has been difficult to improve the efficiency and control luminescence chromaticity.
An object of the present invention is to provide an organic electroluminescence device having good luminescence chromaticity by improving durability and light emission efficiency.

Means for Solving the Problems

The object of the present invention may be achieved by the following means.

[1] An organic electroluminescence device, comprising; at least one organic layer comprising a light emitting layer comprising a light emitting material between a pair of electrodes, wherein the organic electroluminescence device comprises a compound having at least one group represented by any one of the following Formulas (7-1) to (7-10) and an iridium complex represented by the following Formula (A9-1).

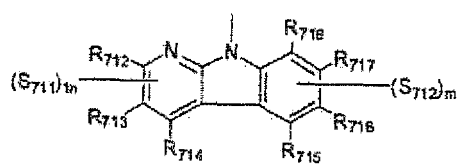

(7-1)

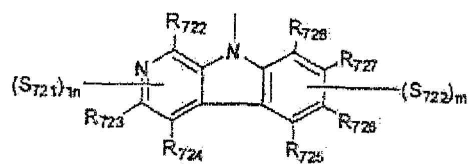

(7-2)

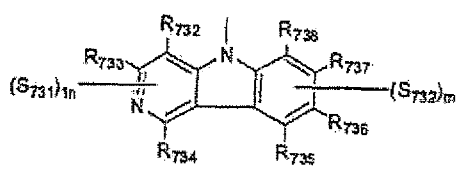

(7-3)

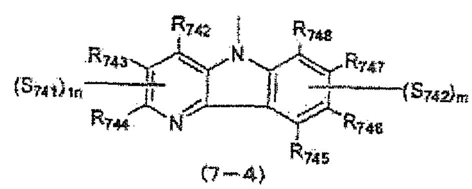

(7-4)

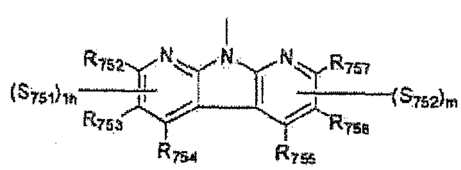

(7-5)

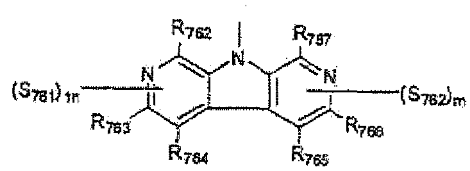

(7-6)

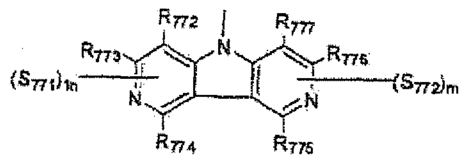

(7−7)

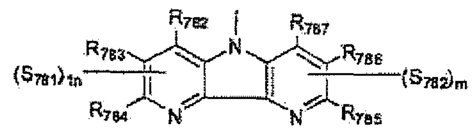

(7−8)

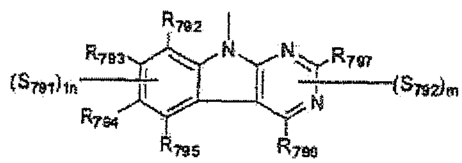

(7−9)

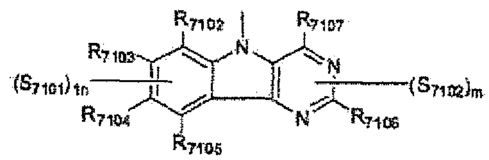

(7−10)

In Formulas (7-1) to (7-10), each of $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ independently represents a hydrogen atom, an alkyl group, or an alicyclic hydrocarbon group which may have an alkyl group. Each of $S_{711}$ to $S_{7101}$ and $S_{712}$ to $S_{7102}$ independently represents the following substituent (S), each of $S_{711}$ to $S_{7101}$ is substituted to a carbon atom as $R_{712}$ to $R_{714}$, $R_{722}$ to $R_{724}$, $R_{732}$ to $R_{734}$, $R_{742}$ to $R_{744}$, $R_{752}$ to $R_{754}$, $R_{762}$ to $R_{764}$, $R_{772}$ to $R_{774}$, $R_{782}$ to $R_{784}$, $R_{792}$ to $R_{795}$, and $R_{7102}$ to $R_{7105}$, and each of $S_{712}$ to $S_{7102}$ is substituted to a carbon atom as $R_{715}$ to $R_{718}$, $R_{725}$ to $R_{728}$, $R_{735}$ to $R_{738}$, $R_{745}$ to $R_{748}$, $R_{755}$ to $R_{757}$, $R_{765}$ to $R_{767}$, $R_{775}$ to $R_{777}$, $R_{785}$ to $R_{787}$, $R_{796}$ to $R_{797}$, and $R_{7106}$ to $R_{7107}$. In and m represent integers of 0 to 4, and In+m is an integer of 1 to 4.

Substituent (S)

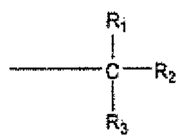

In Substituent (S) $R_1$ represents an alkyl group. $R_2$ represents a hydrogen atom or an alkyl group. $R_3$ represents a hydrogen atom or an alkyl group. R1 to $R_3$ may be linked to each other to form a ring.

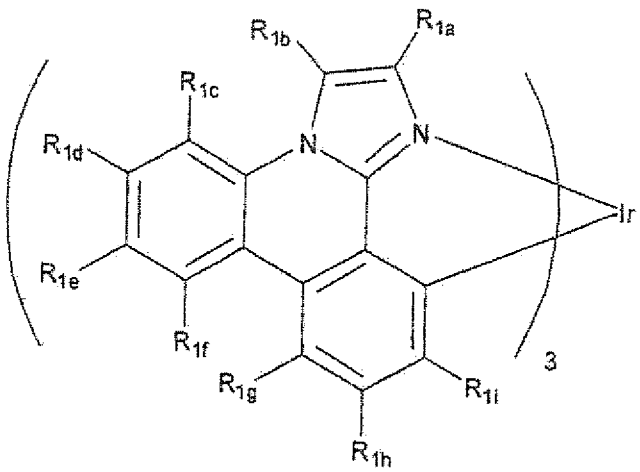

A9-1

In Formula (A9-1), each of $R_{1a}$ to $R_{1i}$ independently represents a hydrogen atom, an alkyl group, an alicyclic hydrocarbon group which may have an alkyl group, or a phenyl group which may have an alkyl group.

[2] The organic electroluminescence device of [1], wherein the compound having at least one group represented by any one of Formulas (7-1) to (7-10) is a compound represented by the following Formula (8).

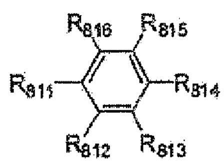

(8)

In Formula (8), each of $R_{811}$ to $R_{816}$ independently represents a hydrogen atom, an alkyl group, an aryl group which may have an alkyl group, a fluorine group, a trifluoromethyl group, or a cyano group, and at least one of $R_{811}$ to $R_{816}$ is a group represented by any one of Formulas (7-1) to (7-10).

[3] The organic electroluminescence device of [1], wherein the compound having at least one group represented by any one of Formulas (7-1) to (7-10) is a compound represented by the following Formula (9).

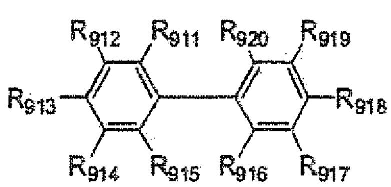

(9)

In Formula. (9), each of $R_{911}$ to $R_{920}$ independently represents a hydrogen atom, an alkyl group, an aryl group which may have an alkyl group, a fluorine group, a trifluoromethyl group, a cyano group, and a silyl group which may have an alkyl group. At least one of $R_{911}$ to $R_{920}$ is a group represented by any one of Formula (7-1) to (7-10).

[4] The organic electroluminescence device of [1] or [3], wherein the compound having at least one group represented by any one of Formulas (7-1) to (7-10) is a compound represented by the following Formula (10).

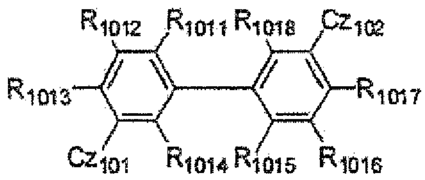

(10)

In Formula (10), each of $R_{1011}$ to $R_{1018}$ independently represents a hydrogen atom, an alkyl group, an aryl group which may have an alkyl group, a fluorine group, a trifluoromethyl group, or a cyano group. Each of $Cz_{101}$ and $Cz_{102}$ independently represents a group represented by any one of Formula (7-1) to (7-10).

[5] The organic electroluminescence device of [1] or [3], wherein the compound having at least one group represented by any one of Formulas (7-1) to (7-10) is a compound represented by the following Formula (11).

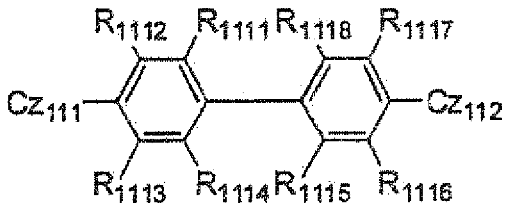

(11)

In Formula (11), each of $R_{1111}$ to $R_{1118}$ independently represents a hydrogen atom, an alkyl group, an aryl group which may have an alkyl group, a fluorine group, a trifluoromethyl group, a cyano group, and a silyl group which may have an alkyl group. Each of $Cz_{111}$ and $Cz_{112}$ independently represents a group represented by any one of Formula (7-1) to (7-10).

[6] The organic electroluminescence device of [1], wherein the compound having at least one group represented by any one of Formulas (7-1) to (7-10) is a compound represented by the following Formula (13).

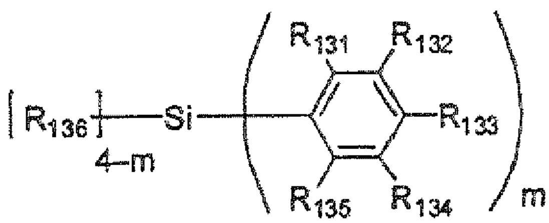

(13)

In Formula (13), each of $R_{131}$ to $R_{135}$ independently represents a hydrogen atom and at least one of $R_{131}$ to $R_{135}$ is a group represented by any one of Formulas (7-1) to (7-10). $R_{136}$ represents a methyl group or a phenyl group which may have an alkyl group. Each $R_{136}$ may be the same as or different from every other $R_{136}$. m represents an integer of 1 to 4.

[7] The organic electroluminescence device of [1], wherein the compound having at least one group represented by any one of Formulas (7-1) to (7-10) is a compound represented by the following Formula (14).

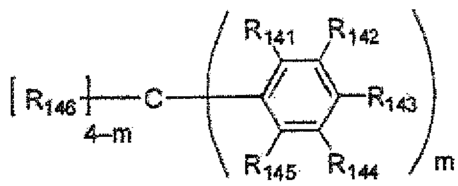

(14)

In Formula (14), each of $R_{141}$ to $R_{145}$ independently represents a hydrogen atom, and at least one of $R_{141}$ to $R_{145}$ is a group represented by any one of Formulas (7-1) to (7-10). $R_{146}$ represents a hydrogen atom, a methyl group,

or a phenyl group which may have an alkyl group, and each $R_{146}$ may be the same as or different from every other $R_{146}$. m represents an integer of 1 to 4.

[8] The organic electroluminescence device of [1], wherein the compound having at least one group represented by any one of Formulas (7-1) to (7-10) is a compound represented by the following Formula (15).

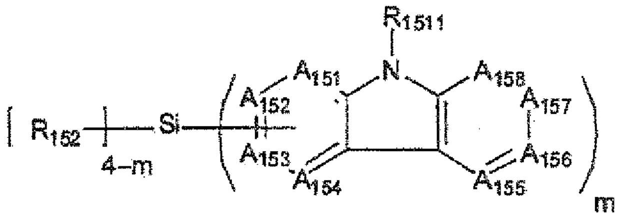
(15)

In Formula (15), $A_{151}$ to $A_{158}$ represent an N atom or C-$R_{153}$, an alkyl group, and $R_{153}$ represents a hydrogen atom, an alkyl group, or an alicyclic hydrocarbon group which may have an alkyl group. $R_{1511}$ represents a phenyl group which may have a substituent, and the substituent which the phenyl group may have is an alkyl group or a phenyl group. Each of $R_{152}$ independently represents a methyl group or a phenyl group which may have an alkyl group, and each $R_{152}$ may be the same as or different from every other $R_{152}$. m represents an integer of 1 to 4. A silicon linking group is linked with a C atom of $A_{151}$ to $A_{158}$.

[9] The organic electroluminescence device of [1], wherein the compound having at least one group represented by any one of Formulas (7-1) to (7-10) is a compound represented by the following Formula (16).

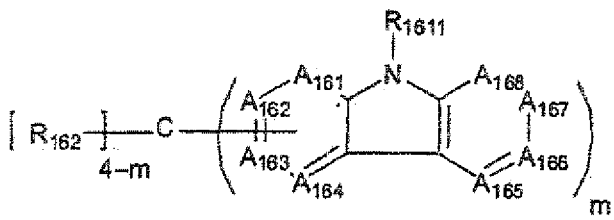
(16)

In Formula (16), $A_{161}$ to $A_{168}$ represent an N atom or C-$R_{163}$ and $R_{163}$ represents a hydrogen atom, an alkyl group, or an alicyclic hydrocarbon group which may have an alkyl group. $R_{1611}$ represents a phenyl group which may have a substituent, and the substituent which the phenyl group may have is an alkyl group or a phenyl group. Each of $R_{162}$ independently represents a methyl group or a phenyl group which may have an alkyl group. Each $R_{162}$ may be the same as or different from every other $R_{162}$. m represents an integer of 1 to 4. A carbon linking group is linked with a C atom of $A_{161}$ to $A_{168}$.

[10] The organic electroluminescence device of [1], wherein the substituent (S) is selected from the following (a) to (e), (i), (1), and (t) to (v).

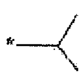
(a)

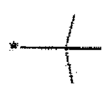
(b)

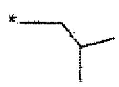
(c)

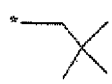
(d)

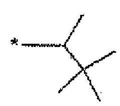
(e)

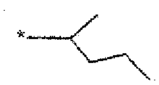
(i)

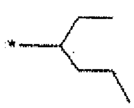
(l)

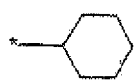
(t)

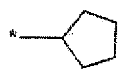
(u)

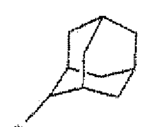
(v)

[11] The organic electroluminescence device of [10], wherein the substituent (S) is selected from the (a) to (e).

[12] An organic electroluminescence device as described in any one of [1] to [11], containing a compound having at least one group represented by any one of Formulas (7-1) to (7-10) in a light emitting layer.
[13] An organic electroluminescence device as described in any one of [1] to [12], containing a compound having at least one group represented by any one of Formulas (7-1) to (7-10) in a layer adjacent to a light emitting layer.
[14] The organic electroluminescence device of [1], wherein at least one kind of the iridium complex (A9-1) is contained in the light emitting layer.
[15] The organic electroluminescence device of [1], wherein at least one kind of the compound having at least one group represented by any one of Formulas (7-1) to (7-10) and at least one kind of the iridium complex (A9-1) are comprised in the light emitting layer.
[16] The organic electroluminescence device of [1], wherein at least one of the organic layers comprising at least one kind of the compound having at least one group represented by any one of Formulas (7-1) to (7-10) and an organic layer comprising at least one kind of the iridium complex (A9-1) is formed by a wet process.
[17] A composition comprising the compound having at least one group represented by any one of Formulas (7-1) to (7-10) of [1] and the iridium complex represented by (A9-1) of claim 1.
[18] A light emitting layer comprising the compound having at least one group represented by any one of Formulas (7-1) to (7-10) of [1] and the iridium complex represented by (A9-1) of claim 1.
[19] A light emission apparatus using the organic electroluminescence device of [1].
[20] A display apparatus using the organic electroluminescence device of [1].
[21] An illumination apparatus using the organic electroluminescence device of [1].

The object of the present invention may also be achieved by the following means.

<1> An organic electroluminescence device, including: at least one organic layer including a light emitting layer containing a light emitting material between a pair of electrodes, in which the organic electroluminescence device contains a compound represented by the following Formula (10) and a phosphorescent metal complex represented by the following Formula (A9-1').

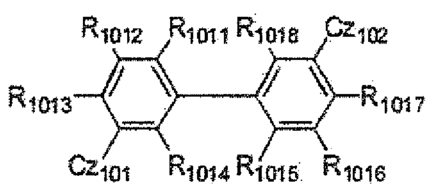

(10)

In Formula (10), each of $R_{1011}$ to $R_{1018}$ represents a hydrogen atom, and each of $Cz_{101}$ and $Cz_{102}$ represents a group represented by the following Formula (4-4).

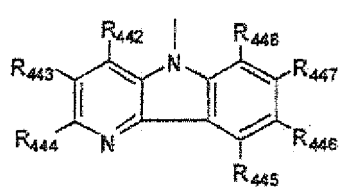

(4-4)

In Formula (4-4), each of $R_{442}$ to $R_{448}$ independently represents a hydrogen atom or any one of the following (a) to (e).

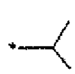 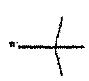 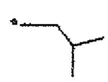 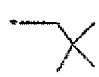 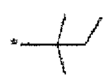

(a) (b) (c) (d) (e)

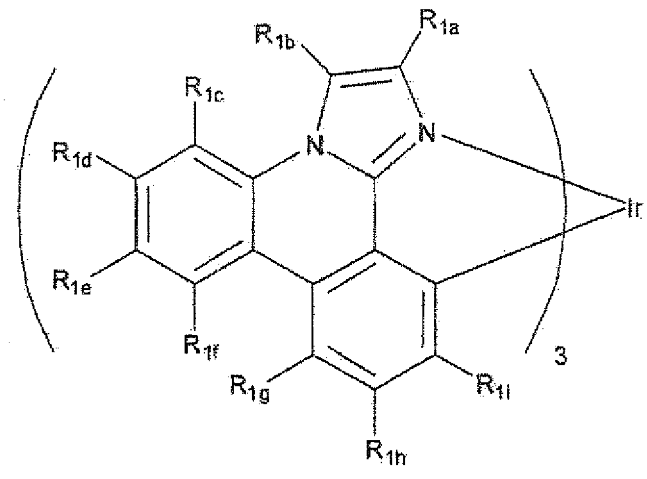

A9-1'

In Formula (A9-1'), each of $R_{1a}$ to $R_{1i}$ independently represents a hydrogen atom, an alkyl group having 1 to 20 carbon atoms, a cycloalkyl group having 3 to 8 carbon atoms, an aryl group having 6 to 18 carbon atoms, or an aryl group having 6 to 18 carbon atoms substituted with an alkyl group having 1 to 20 carbon atoms.

<2> The organic electroluminescence device as described in <1>, in which at least one of $R_{442}$ to $R_{448}$ in Formula (4-4) represents any one of the (a) to (e).

<3> The organic electroluminescence device as described in <1> or <2>, in which a compound represented by Formula (10) is contained in a light emitting layer.

<4> The organic electroluminescence device as described in any one of <1> to <3>, in which a compound represented by Formula (10) is contained in a layer adjacent to a light emitting layer.

<5> The organic electroluminescence device as described in any one of <1> to <4>, in which at least one kind of the phosphorescent metal complex is contained in a light emitting layer.

<6> The organic electroluminescence device as described in any one of <1 > to <5>, in which at least one kind of the compound represented by Formula (10) and at least one kind of the phosphorescent metal complex are contained in a light emitting layer.

<7> The organic electroluminescence device as described in any one of <1> to <6>, in which at least one layer of an organic layer containing at least one kind of the compound represented by Formula (10) and an organic layer containing at least one kind of the phosphorescent metal complex is formed by a wet process.

<8> A composition containing the compound represented by Formula. (10) as described in <1> and the phosphorescent metal complex represented by Formula (A9-1') as described in <1>.

<9> A light emitting layer containing the compound represented by Formula (10) as described in <1> and the phosphorescent metal complex represented by Formula (A9-1') described in <1>.

<10> A light emission apparatus using the organic electroluminescence device as described in any one of <1> to <7>.

<11> A display apparatus using the organic electroluminescence device as described in any one of <1> to <7>.

<12> An illumination apparatus using the organic electroluminescence device as described in any one of <1> to <7>.

Effects of the Invention

The organic electroluminescence device of the present invention includes a compound represented by Formula (1) such as a compound having at least one group represented by any one of Formulas (7-1) to (7-10), a compound represented by Formula (10) or the like (in this specification, used in the same meaning as "a compound of the present invention"), and a phosphorescent metal complex containing mono-anionic bidentate ligands represented by the following Formulas (A1) to (A4) and a metal having an atomic weight of 40 or more such as an iridium complex represented by Formula (A9-1) or the like. Accordingly, it is possible to provide an organic electroluminescence device (in this specification, used in the same meaning as "a device of the present invention") having high light emission efficiency (for example, external quantum efficiency) and high durability. Further, it is possible to provide a device in which each organic electroluminescence device has good luminescence chromaticity.

Brief Description of Drawings

FIG. 1 is a schematic view illustrating an example of a layer configuration of an organic EL device according to the first exemplary embodiment of the present invention.

FIG. 2 is a schematic view illustrating an example of a light emission apparatus according to the second exemplary embodiment of the present invention .

FIG. 3 is a schematic view illustrating an example of an illumination apparatus according to the third embodiment of the present invention.

Embodiments for Carrying Out the Invention

First, an organic electroluminescence device will be described, which includes, between a pair of electrodes, at least one organic layer including a light emitting layer containing a light emitting material, and contains a compound represented by the following Formula (1) and a phosphorescent metal complex (hereinafter, in some cases, referred to as a specific phosphorescent metal complex) containing mono-anionic bidentate ligands represented by the following Formulas (A1] to (A4) and a metal having an atomic weight of 40 or more.

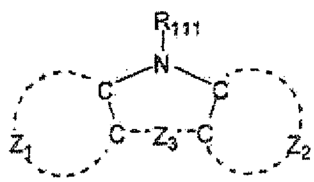

(1)

In Formula (1), $Z_1$ represents an aromatic heterocyclic ring, $Z_2$ represents an aromatic heterocyclic ring or an aromatic hydrocarbon ring, and $Z_3$ represents a divalent linking group or a single bond. $R_{111}$ represents a hydrogen atom or a substituent.

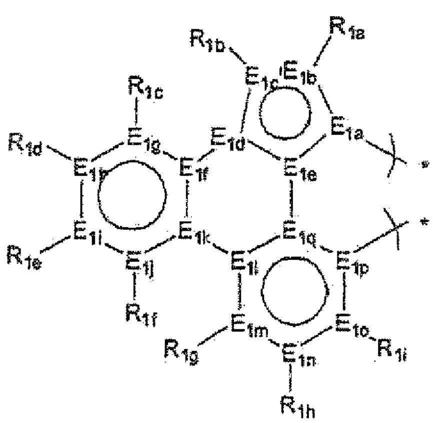

A1

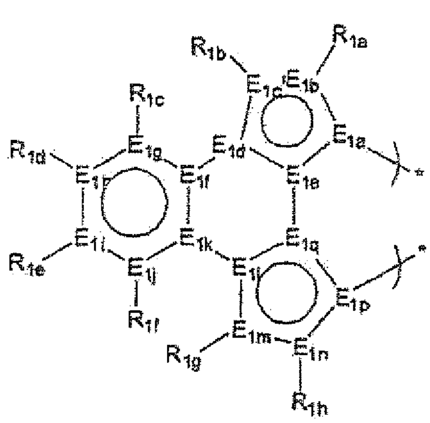

A2

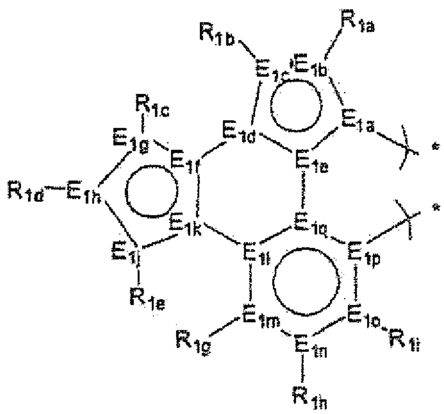

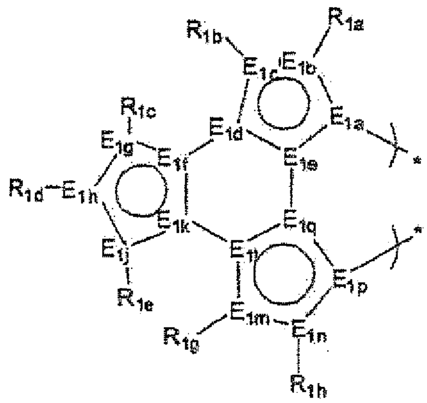

A3 A4

In Formulas (A1) to (A4), each of $E_{1a}$ to $E_{1q}$ independently represents a carbon atom or a heteroatom. Each of $R_{1a}$ to $R_{li}$ independently represents a hydrogen atom or a substituent. Each of structures represented by Formulas (A1) to (A4) totally has an 18π electronic structure.

Durability and light emission efficiency are improved by using a compound represented by Formula (1) in combination with the specific phosphorescent metal complex, thereby improving luminescence chromaticity. As compared with a carbazole derivative generally used in the related art, by combining the compound represented by Formula (1) having excellent electron injection and transporting properties, a charge balance in a charge-poor light emitting layer is improved, thereby improving durability and quantum efficiency. Further, the charge balance is improved and thus the distribution of excitons in the light emitting layer becomes uniform, such that a device having desired chromaticity may be fabricated.

Further, since the compound represented by Formula (1) includes a nitrogen-containing heterocyclic ring such as a pyridine ring, it is considered that tolerance to a hole is lower and durability is lower as compared with a carbazole derivative of a host in the related art, but generally, in the case where the compound combined with a specific phosphorescent metal complex having a low Ip value is used in the light emitting layer, it is considered that a degradation by charges is suppressed by sharing a role of transporting charges with the specific phosphorescent metal complex, thereby improving the durability.

Furthermore, since the compound represented by Formula (1) having an aromatic heterocyclic ring in its structure has higher polarity than a general host material, in the case where the compound is used as the host material, dispersibility to an organic layer made of the light emitting material is improved, thereby preventing interaction with light emitting material molecules. The generation of a quencher and a long-wavelength luminescent component due to the association of the light emitting material in the light emitting layer is reduced due to the improvement in the dispersibility and the prevention of the interaction, and thus, it is considered that the durability and the chromaticity became improved.

In the organic electroluminescence device of the present invention, it is preferred that the compound represented by Formula (1) is a compound having at least one group represented by any one of the following Formulas (4-1) to (4-10). That is, an aspect of the organic electroluminescence device of the present invention provides an organic electroluminescence device including at least one organic layer including a light emitting layer containing a light emitting material between a pair of electrodes, which contains a compound having at least one group represented by any one of the following Formulas (4-1) to (4-10), and a phosphorescent metal complex containing mono-anionic bidentate ligands represented by Formulas (A1) to (A4) and a metal having an atomic weight of 40 or more.

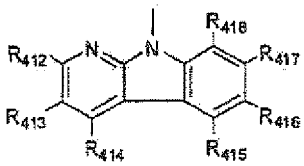

(4-1)

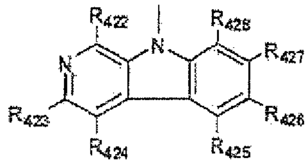

(4-2)

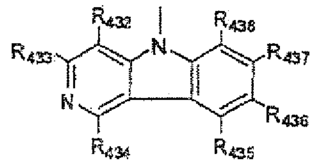

(4-3)

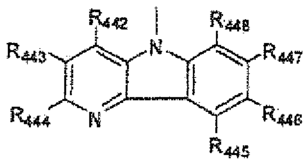

(4-4)

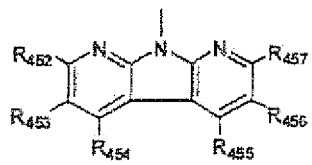

(4-5)

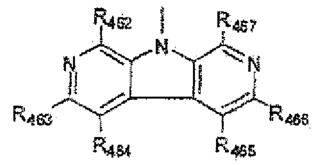

(4-6)

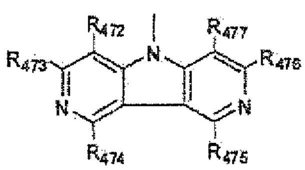

(4-7)

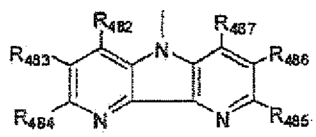

(4-8)

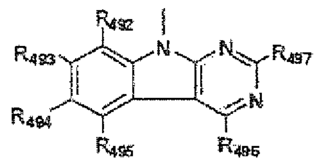

(4-9)

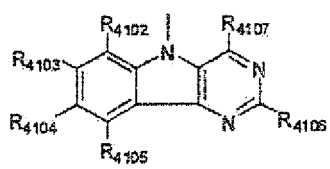

(4-10)

In Formulas (4-1) to (4-10), each of $R_{412}$ to $R_{418}$, $R_{422}$ to $R_{428}$, $R_{432}$ to $R_{438}$, $R_{442}$ to $R_{448}$, $R_{452}$ to $R_{457}$, $R_{462}$ to $R_{467}$, $P_{472}$ to $R_{477}$, $R_{482}$ to $R_{487}$, $R_{492}$ to $R_{497}$, and $R_{4102}$ to $R_{4107}$ independently represents a hydrogen atom and a substituent.

In the organic electroluminescence device of the present invention, particularly, an aspect in which the compound represented by Formula (1) is a compound having at least one group represented by any one of the following Formulas (7-1) to (7-10) having a specific substituent or a compound represented by Formula (10), and a specific phosphorescent metal complex is an iridium complex represented by the following Formula (A9-1) having a specific substituent is preferred in that the durability and the light emission efficiency are improved and the luminescence chromaticity is improved.

That is, another aspect of the organic electroluminescence device of the present invention is an organic electroluminescence device which includes at least one organic layer including a light emitting layer containing a light emitting material between a pair of electrodes, and contains a compound having at least one group represented by any one of the following Formulas (7-1) to (7-10) and an iridium complex represented by the following Formula (A9-1).

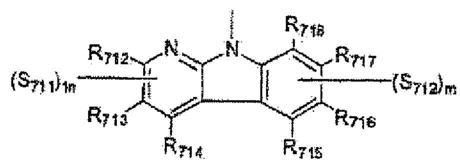

(7-1)

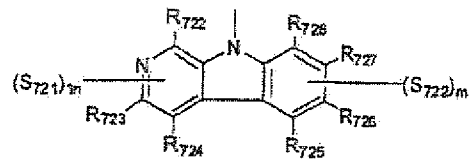

(7-2)

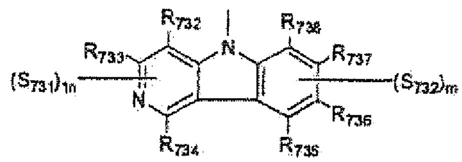

(7-3)

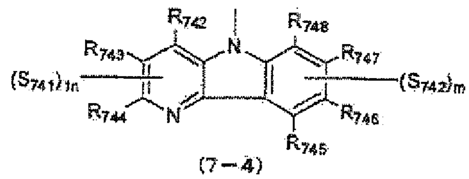

(7-4)

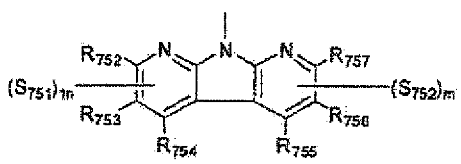

(7-5)

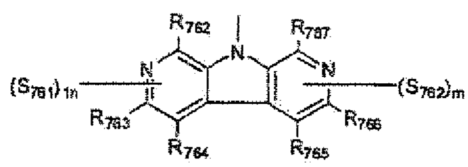

(7-6)

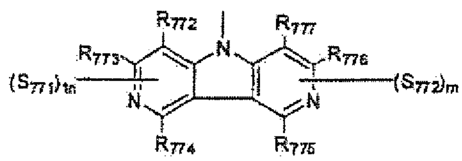

(7-7)

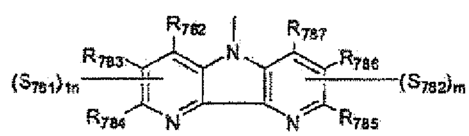

(7-8)

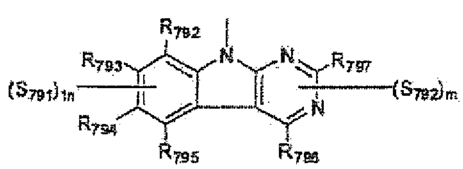

(7-9)

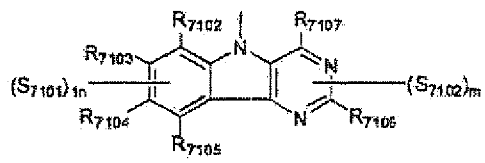

(7-10)

In Formulas (7-1) to (7-10), each of $R_{712}$ to $R_{718}$ $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ independently represents a hydrogen atom, an alkyl group, or an alicyclic hydrocarbon group which may have an alkyl group. Each of $S_{711}$, to $S_{7101}$ and $S_{712}$ to $S_{7102}$ independently represents the following substituent (S), each of $S_{711}$ to $S_{7101}$ is substituted to carbon atoms as $R_{712}$ to $R_{714}$, $R_{722}$ to $R_{724}$, $R_{732}$ to $R_{734}$, $R_{742}$ to $R_{744}$, $R_{752}$ to $R_{754}$, $R_{762}$ to $R_{764}$, $R_{772}$ to $R_{774}$, $R_{782}$ to $R_{784}$, $R_{792}$ to $R_{795}$, and $R_{7102}$ to $R_{7105}$, and each of $S_{712}$ to $S_{7102}$ is substituted to carbon atoms as $R_{715}$ to $R_{718}$, $R_{725}$ to $R_{728}$, $R_{735}$ to $R_{738}$, $R_{745}$ to $R_{748}$, $R_{755}$ to $R_{757}$, $R_{765}$ to $R_{767}$, $R_{775}$ to $R_{777}$, $R_{785}$ to $R_{787}$, $R_{796}$ to $R_{797}$, and $R_{7106}$ to $R_{7107}$. In and m represent

integers of 0 to 4 and n+m is an integer of 1 to 4.

Substituent (S)

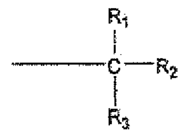

Herein, $R_1$ represents an alkyl group. $R_2$ represents a hydrogen atom or an alkyl group. $R_3$ represents a hydrogen atom or an alkyl group. $R_1$ to $R_3$ may be linked to each other to form a ring.

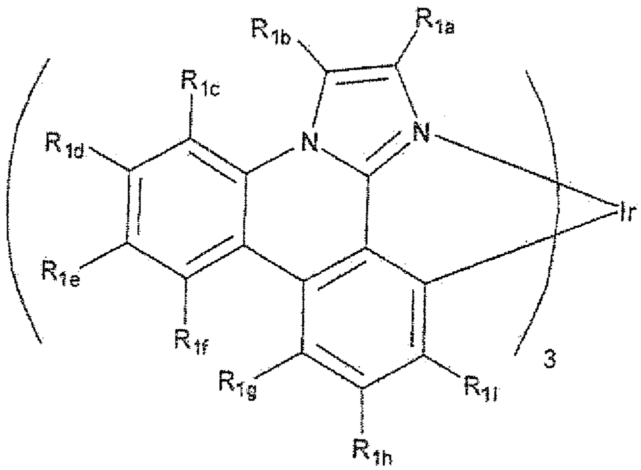

A9-1

In Formula (A9-1), each of $R_{1a}$ to $R_{1i}$ independently represents a hydrogen atom, an alkyl group, an alicyclic hydrocarbon group which may have an alkyl group, or a phenyl group which may have an alkyl group.

Yet another aspect of the organic electroluminescence device according to the present invention is an organic electroluminescence device which includes at least one organic layer including a light emitting layer containing a light emitting material between a pair of electrodes, and contains a compound represented by the following Formula (10) and a phosphorescent metal complex represented by the following Formula (A9-1').

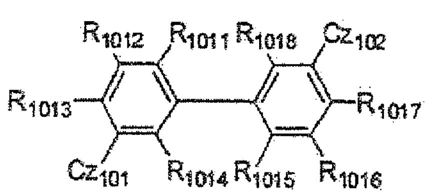

(10)

In Formula (10), each of $R_{1011}$ to $R_{1018}$ represents a hydrogen atom, and each of $Cz_{101}$ and $Cz_{102}$ represents a group represented by the following Formula (4-4).

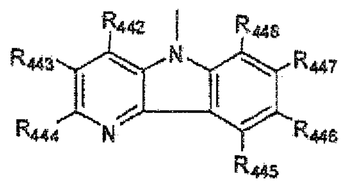

(4-4)

In Formula (4-4), each of $R_{442}$ to $R_{448}$ independently represents a hydrogen atom or any one of the following (a) to (e).

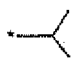 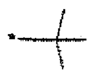 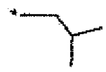 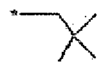 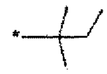

(a) (b) (c) (d) (e)

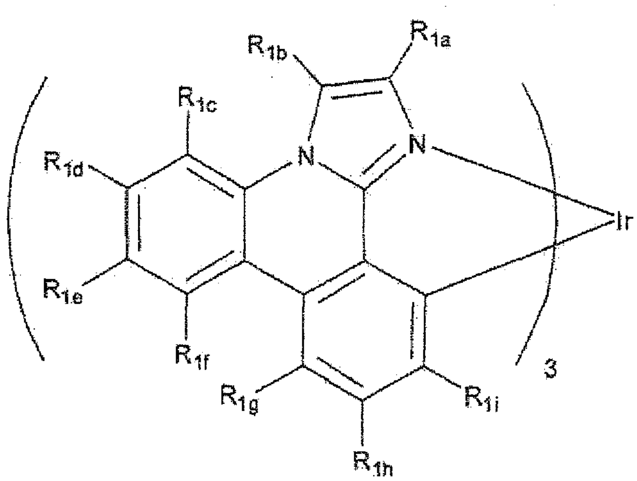

A9-1'

In Formula (A9-1), each of $R_{18}$ to $R_{1i}$ independently represents a hydrogen atom, an alkyl group having 1 to 20 carbon atoms, a cycloalkyl group having 3 to 8 carbon atoms, an aryl group having 6 to 18 carbon atoms, or an aryl group having 6 to 18 carbon atoms substituted with an alkyl group having 1 to 20 carbon atoms.

Hereinafter, a compound represented by Formula (1), including a compound containing groups represented by Formulas (4-1) to (4-10) and Formulas (7-1) to (7-10) or a compound represented by Formula (10), will be described in detail.

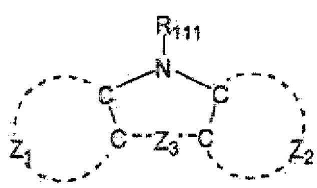

(1)

Formula (1) will be described. $Z_1$ represents an aromatic heterocyclic ring, $Z_2$ represents an aromatic heterocyclic ring or an aromatic hydrocarbon ring, and $Z_3$ represents a divalent linking group or a simple bond. $R_{111}$ represents a hydrogen atom or a substituent. As the substituent represented by $R_{111}$, those exemplified by the following group A of substituents may be applied.

(Group A of substituents)

An alkyl group (having preferably 1 to 30 carbon atoms, more preferably 1 to 20 carbon atoms, and particularly preferably 1 to 10 carbon atoms, and examples thereof include methyl, ethyl, isopropyl, tert-butyl, n-octyl, n-decyl, n-hexadecyl, and the like), an alicyclic hydrocarbon group (having preferably 1 to 30 carbon atoms, more preferably 1 to 20 carbon atoms, and particularly preferably 1 to 10 carbon atoms, and examples thereof include adamantyl, cyclopropyl, cyclopentyl, cyclohexyl, and the like), an alkenyl group (having preferably 2 to 30 carbon atoms, more preferably 2 to 20 carbon atoms, and particularly preferably 2 to 10 carbon atoms, and examples thereof include vinyl, allyl, 2-butenyl, 3-pentenyl, and the like), an alkynyl group (having preferably 2 to 30 carbon atoms, more preferably 2 to 20 carbon atoms, and particularly preferably 2 to 10 carbon atoms, and examples thereof include propargyl, 3-pentynyl, and the like), an aryl group (having preferably 6 to 30 carbon atoms, more preferably 6 to 20 carbon atoms, and particularly preferably 6 to 12 carbon atoms, and examples thereof include phenyl, p-methylphenyl, naphthyl, anthranyl, and the like), an amino group (having preferably 0 to 30 carbon atoms, more preferably 0 to 20 carbon atoms, and particularly preferably 0 to 10 carbon atoms, and examples thereof include amino, methylamino, dimethylamino, diethylamino, dibenzylamine, diphenylamino, ditolylamino, and the like), an alkoxy group (having preferably 1 to 30 carbon atoms, more preferably 1 to 20 carbon atoms, and particularly preferably 1 to 10 carbon atoms, and examples thereof include methoxy, ethoxy, butoxy, 2-ethylhexyloxy, and the like), an aryloxy group (having preferably 6 to 30 carbon atoms, more preferably 6 to 20 carbon atoms, and particularly preferably 6 to 12 carbon atoms, and examples thereof include phenyloxy, 1-naphthyloxy, 2-naphthyloxy, and the like), a heterocyclic oxy group (having preferably 1 to 30 carbon atoms, more preferably 1 to 20 carbon atoms, and particularly preferably 1 to 12 carbon atoms, and examples thereof include pyridyloxy, pyrazyloxy pyrimidyloxy, quinolyloxy, and the like), an acyl group (having preferably 1 to 30 carbon atoms, more preferably 1 to 20 carbon atoms, and particularly preferably 1 to 12 carbon atoms, and examples thereof include acetyl, benzoyl, formyl, pivaloyl, and the like), an alkoxycarbonyl group (having preferably 2 to 30 carbon atoms, more preferably 2 to 20 carbon atoms, and particularly preferably 2 to 12 carbon atoms, and examples thereof include methoxycarbonyl, ethoxycarbonyl, and the like), an aryloxycarbonyl group (having preferably 7 to 30 carbon atoms, more preferably 7 to 20 carbon atoms, and particularly preferably having 7 to 12 carbon atoms, and examples thereof include phenyloxycarbonyl, and the like), an acyloxy group (having preferably 2 to 30 carbon atoms, more preferably 2 to 20 carbon atoms, and particularly preferably 2 to 10 carbon atoms, and examples thereof include acetoxy, benzoyloxy, and the like), an acylamino group (having preferably 2 to 30 carbon atoms, more preferably 2 to 20 carbon atoms, and particularly preferably 2 to 10 carbon atoms, and examples thereof include acetylamino, benzoylamino, and the like), an alkoxycarbonylamino group (having preferably 2 to 30 carbon atoms, more preferably 2 to 20 carbon atoms, and particularly preferably 2 to 12 carbon atoms, and examples thereof include methoxycarbonylamino, and the like), an aryloxycarbonylamino group (having preferably 7 to 30 carbon atoms, more preferably 7 to 20 carbon atoms, and particularly preferably 7 to 12 carbon atoms, and examples thereof include phenyloxycarbonylamino, and the like), a sulfonylamino group (having preferably 1 to 30 carbon atoms, more preferably 1 to 20 carbon atoms, and particularly preferably 1 to 12 carbon atoms, and examples thereof include methanesulfonylammo, benzenesulfonylamino, and the like), a sulfamoyl group (having preferably 0 to 30 carbon atoms, more preferably 0 to 20 carbon atoms, and particularly preferably 0 to 12 carbon atoms, and examples thereof include sulfamoyl, methylsulfamoyl, dimethylsulfamoyl, phenylsulfamoyl, and the like), a carbamoyl group (having preferably 1 to 30 carbon atoms, more preferably 1 to 20 carbon atoms, and particularly preferably 1 to 12 carbon atoms, and examples thereof include carbamoyl, methylcarbamoyl, diethylcarbamoyl, phenylcarbamoyl, and the like), an alkylthio group (having preferably 1 to 30 carbon atoms, more preferably 1 to 20 carbon atoms, and particularly preferably 1 to 12 carbon atoms, and examples thereof include methylthio, ethylthio, and the like), an arylthio group (having preferably 6 to 30 carbon atoms, more preferably 6 to 20 carbon atoms, and particularly preferably 6 to 12 carbon atoms, and examples thereof include phenylthio, and the like), a heterocyclic thio group (having preferably 1 to 30 carbon atoms, more preferably 1 to 20 carbon atoms, and particularly preferably 1 to 12 carbon atoms, and examples thereof include pyridylthio, 2-benzimizolylthio, 2-benzoxazolylthio, 2-benzthiazolylthio, and the like), a sulfonyl group (having preferably 1 to 30 carbon atoms, more preferably 1 to 20 carbon atoms, and particularly preferably 1 to 12 carbon atoms, and examples thereof include mesyl, tosyl, and the like), a sulfinyl group (having preferably 1 to 30 carbon atoms, more preferably 1 to 20 carbon atoms, and particularly preferably 1 to 12 carbon atoms, and examples thereof include methanesulfinyl, benzenesulfinyl, and the like), a ureido group (having preferably 1 to 30 carbon atoms, more preferably 1 to 20 carbon atoms, and particularly preferably 1 to 12 carbon atoms, and examples thereof include ureido, methylureido, phenylureido, and the like), a phosphoric acid amide group (having preferably 1 to 30 carbon atoms, more preferably 1 to 20 carbon atoms, and particularly preferably 1 to 12 carbon atoms, and examples thereof include diethylphosphoric acid amide, phenylphosphoric acid amide, and the like), a hydroxyl group, a mercapto group, a halogen atom (for example, a fluorine atom, a chlorine atom, a bromine atom, and an iodine atom), a cyano group, a sulfo group, a carboxyl group, a nitro group, a hydroxamic acid group, a sulfino group, a hydrazino group, an imino group, a heterocyclic group (having preferably 1 to 30 carbon atoms, and more preferably 1 to 12 carbon atoms, and examples of the heteroatom include a nitrogen atom, an oxygen atom, and

a sulfur atom, and specifically imidazolyl, pyridyl, quinolyl, furyl, thienyl, piperidyl, morpholino, benzoxazolyl, benzimidazolyl, benzothiazolyl, a carbazolyl group, an azepinyl group, and the like), a silyl group (having preferably 3 to 40 carbon atoms, more preferably 3 to 30 carbon atoms, and particularly preferably 3 to 24 carbon atoms, and examples thereof include trimethylsilyl, triphenylsilyl, and the like), and a silyloxy group (having preferably 3 to 40 carbon atoms, more preferably 3 to 30 carbon atoms, and particularly preferably 3 to 24 carbon atoms, and examples thereof include trimethylsilyloxy, triphenylsilyloxy, and the like).

$R_{111}$ may further have a substituent, and as the substituent, those exemplified above for the group A of substituents may be applied. Further, a plurality of substituents may be linked to each other to form a ring.

$R_{111}$ includes preferably a hydrogen atom, an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a cyano group, a silyl group and a heterocyclic group, even more preferably a hydrogen atom, an alkyl group, an alicyclic hydrocarbon group, an aryl group, a fluorine group, a cyano group, a silyl group and a heterocyclic group, and particularly preferably an alkyl group, an alicyclic hydrocarbon group, an aryl group and a heterocyclic group.

In Formula (1), an aromatic heterocyclic ring represented by $Z_1$ and $Z_2$ may include a furan ring, a thiophene ring, a pyridine ring, a pyridazine ring, a pyrimidine ring, a pyrazine ring, a benzoimidazole ring, oxadiazole ring, a triazole ring, an imidazole ring, a pyrazole ring, a thiazole ring, an indole ring, a benzoimidazole ring, a benzotiazole ring, a benzoxazole ring, a quinoxaline ring, a quinazoline ring, a phthalazine ring, a carbazole ring, a carboline ring, a ring in which a carbon atom of a hydrocarbon ring constituting a carboline ring is substituted by a nitrogen atom, and the like. The aromatic heterocyclic ring may have a substituent, and as the substituent, those exemplified above for the group A of substituents may be applied.

In Formula (1), an aromatic hydrocarbon ring represented by $Z_2$ may include a benzene ring, a biphenyl ring, a naphthalene ring, an azulene ring, an anthracene ring, a phenanthrene ring, a pyrene ring, a chrysene ring, a naphthacene ring, a triphenylene ring, o-terphenyl ring, m-terphenyl ring, p-terphenyl ring, an acenaphthene ring, a coronene ring, a fluorine ring, a fluoranthrene ring, a naphthacene ring, a pentacene ring, a perylene ring, a pentaphene ring , a phycene ring, a pyrene ring, a pyranthrene ring, an anthraanthrene ring, and the like. The aromatic hydrocarbon ring may have a substituent, and as the substituent, those exemplified above for the group A of substituents may be applied.

In Formula (1), a divalent linking group represented by $Z_3$ may include, in addition to a hydrocarbon group such as alkylene, alkenylene, alkynylene and arylene, a heteroatom, and also, may be a divalent linking group derived from a compound having an aromatic heterocyclic ring (also referred to as a heteroaromatic compound), such as a thiophene-2,5-diyl group or a pyrazine-2,3-diyl group, and may include a chalcogene atom such as oxygen or sulfur. Further, the divalent linking group may be a linking group through a heteroatom, such as an alkylimino group, a dialkylsilanediyl group and a diarylgermanediyl group.

In Formula (1), a simple bond represented by $Z_3$ means a bond which directly bonds linking substituents to each other.

In the present invention, it is preferred that $Z_1$ of Formula (1) is a 6-membered ring, As a result, the light emission efficiency may be more increased. Furthermore, it is possible to have a longer service life. Specifically, the 6-membered ring includes preferably a pyridine ring, a pyridazine ring, a pyrimidine ring and a pyrazine ring, and more preferably a pyridine ring and a pyrimidine ring.

In the present invention, it is preferred that $Z_2$ of Formula (1) is a 6-membered ring. As a result, the light emission efficiency may be more increased. Furthermore, it is possible to have a longer service life. In detail, the 6-membered ring includes preferably a benzene ring, a pyridine ring, a pyridazine ring, a pyrimidine ring and a pyrazine ring, and more preferably a benzene ring, a pyridine ring and a pyrimidine ring.

Further, in the present invention, it is more preferred that both $Z_1$ and $Z_2$ of Formula (1) are 6-membered rings. It is preferred because the light emission efficiency may be more increased when both $Z_1$ and $Z_2$ are 6-membered rings. Further, it is preferred because it is possible to have a much longer service life.

In the present invention, it is preferred that $Z_3$ of Formula (1) is a simple bond.

As the compound represented by Formula (1), one of the preferable forms is a compound represented by the following Formula (2). Since $Z_3$ does not include a linking group, a dissociation reaction of $Z_1$ is suppressed in the luminescence device, and thus, a long service life in the device is expected, such that it is preferred to use a compound of the following Formula (2).

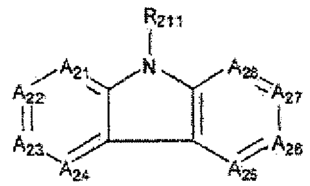

(2)

Formula (2) will be described. $R_{211}$ represents a hydrogen atom or a substituent. $R_{211}$ has the same meaning as $R_{111}$ in Formula (1), and preferred ranges thereof are also the same. $A_{21}$ to $A_{24}$ represent an N atom or C-$R_{222}$. Among $A_{21}$ to $A_{24}$, the number of the N atoms is an integer of 1 to 2. $A_{25}$ to $A_{28}$ represent an N atom or C-$R_{222}$. In $A_{25}$ to $A_{28}$, the number of the N atoms is an integer of 0 to 2. $R_{222}$ represents a hydrogen atom or a substituent.

As $R_{222}$, those exemplified above for the group A of substituents may be applied. Each $R_{222}$ may be the same as or different from every other $R_{222}$.

A group represented by $R_{222}$ includes preferably a hydrogen atom, an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a cyano group, a silyl group and a heterocyclic group, and even more preferably a hydrogen atom, an alkyl group, an alicyclic hydrocarbon group, an aryl group, a fluorine group, a cyano group, a silyl group and a heterocyclic group.

A substituent represented by $R_{222}$ may further have a substituent, and as the substituent, those exemplified above for the group A of the substituents may be applied. Further, a plurality of the substituents may be linked to each other to form a ring.

As the compound represented by Formula (2), one of the preferable forms is a compound represented by the following Formula (3-1).

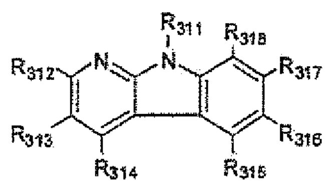

(3-1)

Formula (3-1) will be described. $R_{311}$ represents a hydrogen atom or a substituent. $R_{311}$ has the same meaning as $R_{211}$ in Formula (2), and preferred ranges thereof are also the same. Each of $R_{312}$ to $R_{318}$ independently represents a hydrogen atom or a substituent, and has the same meaning as $R_{222}$ to $R_{610}$ in Formula (2), and preferred ranges thereof are also the same.

As the compound represented by Formula (2), one of the preferable forms is a compound represented by the following Formula (3-2).

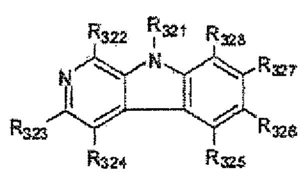

(3-2)

Formula (3-2) will be described. $R_{321}$ represents a hydrogen atom or a substituent.
$R_{321}$ has the same meaning as $R_{211}$ in Formula (2), and preferred ranges thereof are also the same. Each of $R_{322}$ to $R_{328}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-3).

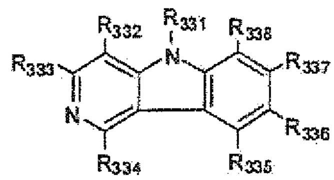

(3-3)

Formula (3-3) will be described. $R_{331}$ represents a hydrogen atom or a substituent. $R_{331}$ has the same meaning as $R_{211}$ in Formula (2), and preferred ranges thereof are also the same. Each of $R_{332}$ to $R_{338}$ independently represents a hydrogen atom or a substituent, and has the same meaning as $R_{222}$ to $R_{610}$ in Formula (2), and preferred ranges thereof are also the same.

As the compound represented by Formula (2), one of the preferable forms is a compound represented by the following Formula (3-4).

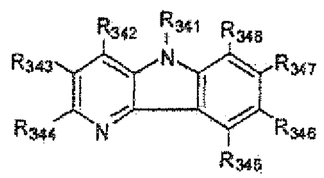

(3-4)

Formula (3-4) will be described. $R_{341}$ represents a hydrogen atom or a substituent. $R_{341}$ has the same meaning as $R_{211}$ in Formula (2), and preferred ranges thereof are also the same. Each of $R_{342}$ to $R_{348}$ independently represents a hydrogen atom or a substituent, and has the same meaning as $R_{222}$ to $R_{610}$ in Formula (2), and preferred ranges thereof are also the same.

As the compound represented by Formula (2), one of the preferable forms is a compound represented by the following Formula (3-5).

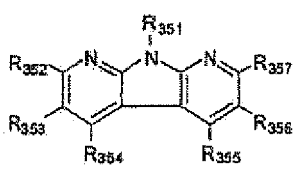

(3-5)

Formula (3-5) will be described. $R_{351}$ represents a hydrogen atom or a substituent. $R_{351}$ has the same meaning as $R_{211}$ in Formula (2), and preferred ranges thereof are also the same. Each of $R_{352}$ to $R_{357}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-6).

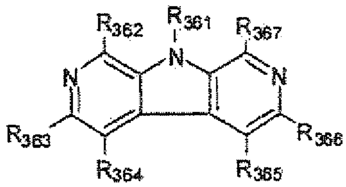

(3-6)

Formula (3-6) will be described. $R_{361}$ represents a hydrogen atom or a substituent. $R_{361}$ has the same meaning as $R_{211}$ in Formula (2), and preferred ranges thereof are also the same. Each of $R_{362}$ to $R_{367}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-7).

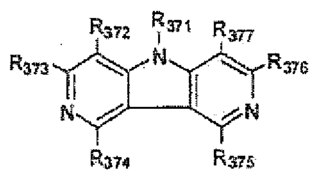

(3-7)

Formula (3-7) will be described. $R_{371}$ represents a hydrogen atom or a substituent. $R_{371}$ has the same meaning as $R_{211}$ in Formula (2), and preferred ranges thereof are also the same. Each of $R_{372}$ to $R_{377}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-8).

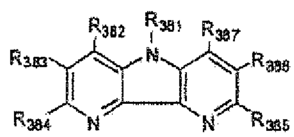

(3-8)

Formula (3-8) will be described. $R_{381}$ represents a hydrogen atom or a substituent. $R_{381}$ has the same meaning as $R_{211}$ in Formula (2), and preferred ranges thereof are also the same. Each of $R_{382}$ to $R_{387}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-9).

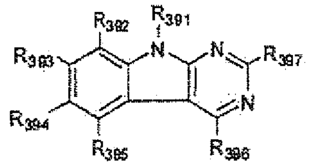

(3-9)

Formula (3-9) will be described. $R_{391}$ represents a hydrogen atom or a substituent. $R_{391}$ has the same meaning as $R_{211}$ in Formula (2), and preferred ranges thereof are also the same. Each of $R_{392}$ to $R_{397}$ independently represents a hydrogen atom or a substituent, and has the same meaning as $R_{222}$ in Formula (2), and preferred ranges thereof are also the same.

As the compound represented by Formula (2), one of the preferable forms is a compound represented by the following Formula (3-10).

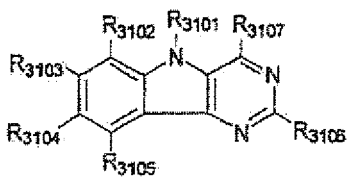

(3-10)

Formula (3-10) will be described. $R_{3101}$ represents a hydrogen atom or a substituent. $R_{3101}$ has the same meaning as $R_{211}$ in Formula (2), and preferred ranges thereof are also the same. Each of $R_{3102}$ to $R_{3107}$ independently represents a hydrogen atom or a substituent, and has the same meaning as $R_{222}$ in Formula (2), and preferred ranges thereof are also the same.

As the compound represented by Formula (2), one of the preferable forms is a compound having at least one group represented by any one of Formulas (4-1) to (4-10). Particularly, it is more preferred to have two to four groups represented by any one of Formula (4-1) to (4-10) in its molecule. In this case, the structure represented by Formula (2) includes the case where a portion except for $R_{211}$ is replaced by Formulas (4-1) to (4-10).

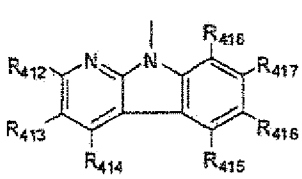

(4-1)

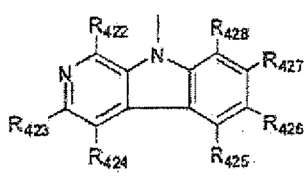

(4-2)

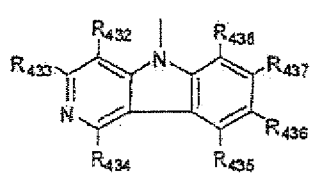

(4-3)

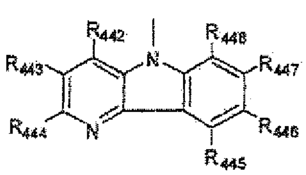

(4-4)

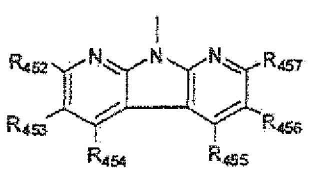

(4-5)

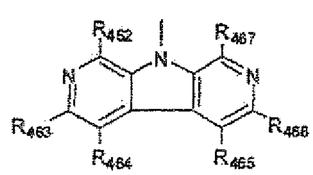

(4-6)

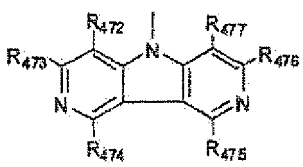

(4−7)

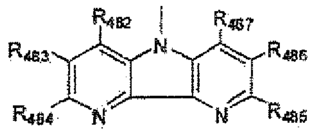

(4−8)

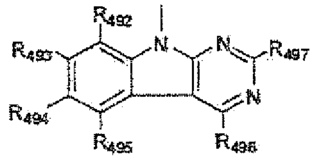

(4−9)

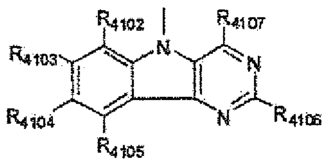

(4−10)

Formulas (4-1) to (4-10) will be described. Each of $R_{412}$ to $R_{418}$, $R_{422}$ to $R_{428}$, $R_{432}$ to $R_{438}$, $R_{442}$ to $R_{448}$, $R_{452}$ to $R_{457}$, $R_{462}$ to $R_{467}$, $R_{472}$ to $R_{477}$, $R_{482}$ to $R_{487}$, $R_{492}$ to $P_{497}$, and $R_{4102}$ to $R_{4107}$ independently represents a hydrogen atom or a substituent, and has the same meaning as $R_{222}$ in Formula (2), and preferred ranges thereof are also the same. A molecular weight of the compound having at least one group represented by any one of Formulas (4-1) to (4-10) is preferably 400 or more to 1000 or less, more preferably 450 or more to 800 or less, and even more preferably 500 or more to 700 or less.

Among Formulas (4-1) to (4-10), it is preferred that each of at least one of $R_{412}$ to $R_{418}$, at least one of $R_{422}$ to $R_{428}$, at least one of $R_{432}$ to $R_{438}$, at least one of $R_{442}$ to $R_{448}$, at least one of $R_{452}$ to $R_{457}$, at least one of $F_{462}$ to $R_{467}$, at least one of $R_{472}$ to $R_{477}$, at least one of $R_{482}$ to $R_{487}$, at least one of $R_{492}$ to $R_{497}$, and at least one of $R_{4102}$ to $R_{4107}$ is a substituent (S) as described below.

As the compound represented by Formula (2), one of the preferable forms is a compound represented by the following Formula (5).

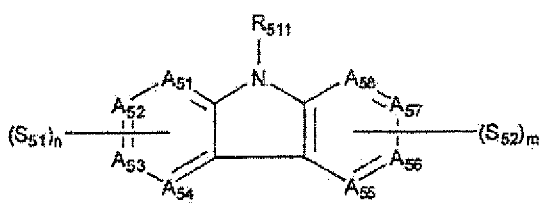

(5)

Formula (5) will be described. $R_{511}$ represents a hydrogen atom or a substituent. $A_{51}$ to $A_{54}$ represent N atoms or C-$R_{522}$. Among $A_{51}$ to $A_{54}$, the number of N atoms is an integer of 1 to 2. $A_{55}$ to $A_{58}$ represent N atoms or C-$R_{522}$. In $A_{55}$ to $A_{58}$, the number of N atoms is an integer of 0 to 2. $R_{522}$ represents a hydrogen atom or a substituent. Each of $S_{51}$ and $S_{52}$ independently represents a substituent (S), $S_{51}$ is substituted to a carbon atom of $A_{51}$ to $A_{54}$, and $S_{52}$ is substituted to a carbon atom of $A_{55}$ to $A_{58}$. In this case, $S_{51}$ and $S_{52}$ have the same meaning as $R_{522}$. $R_{511}$ and $R_{522}$ have the same meaning as $R_{211}$ and $R_{222}$ in Formula (2), and preferred ranges thereof are also the same.

Substituent (S)

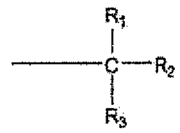

$R_1$ represents an alkyl group. $R_1$ includes preferably a methyl group, an ethyl group, a propyl group, an isopropyl group, a butyl group and a tert-butyl group, more preferably a methyl group, an ethyl group, an isopropyl group and a tert-butyl group, even more preferably a methyl group, an ethyl group, an isopropyl group and a tert-butyl group, and particularly, preferably a methyl group and a tert-butyl group.

$R_2$ represents a hydrogen atom or an alkyl group. $R_2$ includes preferably a hydrogen atom, a methyl group, an ethyl group, a propyl group, an isopropyl group, a butyl group and a tert-butyl group, more preferably a hydrogen atom, a methyl group, an ethyl group and a propyl group, more preferably a hydrogen atom and a methyl group, and more preferably a methyl group.

$R_3$ represents a hydrogen atom or an alkyl group. $R_3$ includes preferably a hydrogen atom and a methyl group, and more preferably a methyl group.

Further, each of $R_1$ to $R_3$ may be linked to each other to form a ring. When the ring is formed, the number of members is not particularly limited, but preferably a 5- or 6-membered ring and more preferably a 6-membered ring.
The substituent (S) may include preferably the following (a) to (x) and more preferably (a) to (e).

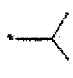
(a)
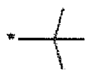
(b)
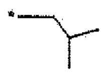
(c)
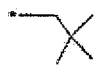
(d)
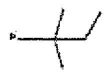
(e)
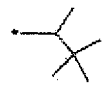
(f)
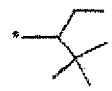
(g)

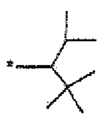
(h)
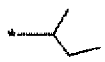
(i)
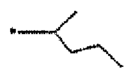
(j)
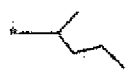
(k)
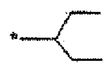
(l)

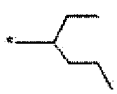
(m)
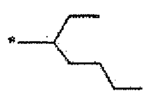
(n)
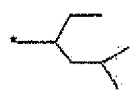
(o)
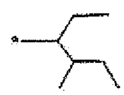
(p)
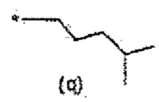
(q)

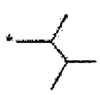
(r)
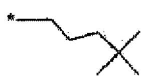
(s)
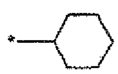
(t)
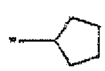
(u)
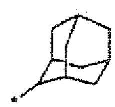
(v)
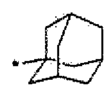
(w)

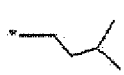
(x)

In the case where the compound represented by Formula (1) has a substituent (S), if the substituent is a substituent having a large volume such as the aforementioned substituents (a) to (x), it is preferred in that a reaction active site is protected and chemical stability in a state of cation and anion is improved. From this viewpoint, the substituent (S) is more preferably (a) to (e), (i), (1), and (t) to (v), and even more preferably (a) to (e).
In the case where the compound represented by Formula (1) has a substituent (S), it is preferred that a substituent included in a specific phosphorescent metal complex as described below is a branched alkyl group. Since compatibility with Formula (1) is increased in the light emitting layer, aggregation and association hardly occur, and as a result, it is considered that device deterioration due to generation of a quencher and misalignment of chromaticity due to association and light emission are suppressed.

In Formula (5), n and m represent integers of 0 to 4, and n+m is an integer of 1 to 4. n+m is preferably 1 and 2.

As the compound containing a group represented by Formula (4-1) and the compound represented by Formula (5), one of the preferable forms is a compound represented by the following Formula (6-1).

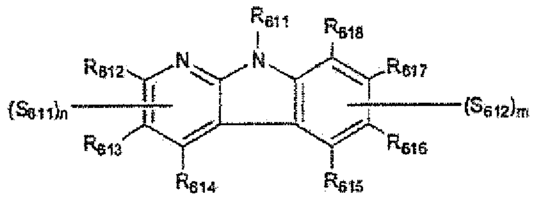

(6-1)

Formula (6-1) will be described. $R_{611}$ represents a hydrogen atom or a substituent. $R_{611}$ has the same meaning as $R_{51l}$ in Formula (5), and preferred ranges thereof are also the same. Each of $R_{612}$ to $R_{618}$ independently represents a hydrogen atom or a substituent, and has the same meaning as $R_{522}$ in Formula (5), and preferred ranges thereof are also the same. Each of $S_{611}$ and $S_{612}$ independently represents the substituent (S), $S_{611}$ is substituted to carbon atoms as $R_{612}$ to $R_{614}$, and $S_{612}$ is substituted to carbon atoms as $R_{615}$ to $R_{618}$.

n represents an integer of 0 to 3, and m represents an integer of 0 to 4. n+m is an integer of 1 to 4. n+m is preferably 1 and 2.

As the compound containing a group represented by Formula (4-2) and the compound represented by Formula (5), one of the preferable forms is a compound represented by the following Formula (6-2).

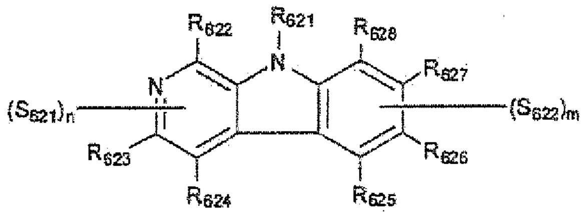

(6-2)

Formula (6-2) will be described. $R_{621}$ represents a hydrogen atom or a. substituent. $R_{621}$ has the same meaning as $R_{511}$ in Formula (5), and preferred ranges thereof are also the same. Each of $R_{622}$ to $R_{628}$ independently represents a hydrogen atom or a substituent, and has the same meaning as $R_{522}$ in Formula (5), and preferred ranges thereof are also the same. Each of $S_{621}$ and $S_{622}$ independently represents the substituent (S), $S_{621}$ is substituted to carbon atoms as $R_{622}$ to $R_{624}$, and $S_{622}$ is substituted to carbon atoms as $R_{625}$ to $R_{628}$.

n represents an integer of 0 to 3, and m represents an integer of 0 to 4. n+m is an integer of 1 to 4. n+m is preferably 1 and 2.

As the compound containing a group represented by Formula (4-3) and the compound represented by Formula (5), one of the preferable forms is a compound represented by the following Formula (6-3).

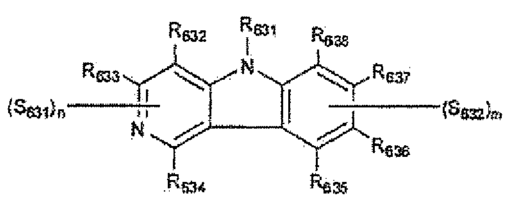

(6-3)

Formula (6-3) will be described. $R_{631}$ represents a hydrogen atom or a substituent. $R_{631}$ has the same meaning as $R_{511}$ in Formula (5), and preferred ranges thereof are also the same. Each of $R_{632}$ to $R_{638}$ independently represents a hydrogen atom or a substituent, and has the same meaning as $R_{522}$ in Formula (5), and preferred ranges thereof are also the same. Each of $S_{631}$ and $S_{632}$ independently represents the substituent (S), $S_{631}$ is substituted to carbon atoms as $R_{632}$ to $R_{634}$, and $S_{632}$ is substituted to carbon atoms as $R_{635}$ to $R_{638}$.

n represents an integer of 0 to 3, and m represents an integer of 0 to 4. n+m is an integer of 1 to 4. n+m is preferably 1 and 2.

As the compound containing a group represented by Formula (4-4) and the compound represented by Formula (5), one of the preferable forms is a compound represented by the following Formula (6-4).

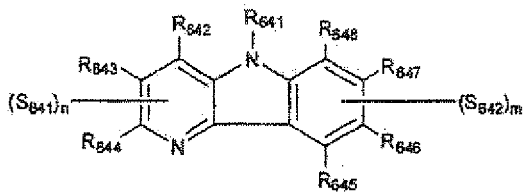

(6−4)

Formula (6-4) will be described. $P_{641}$ represents a hydrogen atom or a substituent. $R_{641}$ has the same meaning as $R_{511}$ in Formula (5), and preferred ranges thereof are also the same. Each of $B_{642}$ to $R6_{48}$ independently represents a hydrogen atom or a substituent, and has the same meaning as $R_{522}$ in Formula (5), and preferred ranges thereof are also the same. Each of $S_{641}$ and $S_{642}$ independently represents the substituent (S), $S_{641}$ is substituted to carbon atoms as $R_{642}$ to $R_{644}$, and $S_{642}$ is substituted to carbon atoms as $R_{645}$ to $R_{648}$.

n represents an integer of 0 to 3, and m represents an integer of 0 to 4. n+m is an integer of 1 to 4. n+m is preferably 1 and 2.

As the compound containing a group represented by Formula (4-5) and the compound represented by Formula (5), one of the preferable forms is a compound represented by the following Formula (6-5).

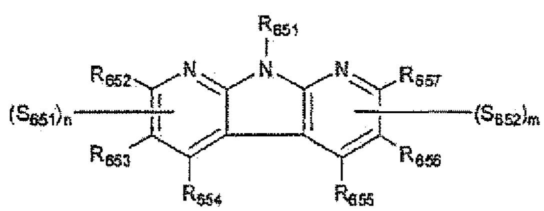

(6−5)

Formula (6-5) will be described. $R_{651}$ represents a hydrogen atom or a substituent. $R_{651}$ has the same meaning as $R_{511}$ in Formula (5), and preferred ranges thereof are also the same. Each of $R_{652}$ to $R_{657}$ independently represents a hydrogen atom or a substituent, and has the same meaning as $R_{522}$ in Formula (5), and preferred ranges thereof are also the same. Each of $S_{651}$ and $S_{652}$ independently represents the substituent (S), $S_{651}$ is substituted to carbon atoms as $R_{652}$ to $R_{654}$, and $S_{652}$ is substituted to carbon atoms as $R_{655}$ to $R_{657}$.

Each of n and m represents an integer of 0 to 3. n+m is an integer of 1 to 4. n+m is preferably 1 and 2.

As the compound containing a group represented by Formula (4-6) and the compound represented by Formula (5), one of the preferable forms is a compound represented by the following Formula (6-6).

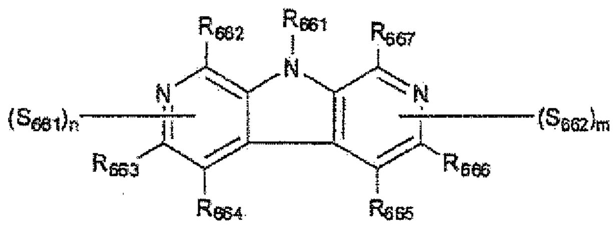

(6-6)

Formula (6-6) will be described. $R_{661}$ represents a hydrogen atom or a substituent. $R_{661}$ has the same meaning as $R_{511}$ in Formula (5), and preferred ranges thereof are also the same. Each of $R_{662}$ to $R_{667}$ independently represents a hydrogen atom or a substituent, and has the same meaning as $R_{522}$ in Formula (5), and preferred ranges thereof are also the same. Each of $S_{661}$ and $S_{662}$ independently represents the substituent (S), $S_{661}$ is substituted to carbon atoms as $R_{662}$ to $R_{664}$, and $S_{662}$ is substituted to carbon atoms as $R_{665}$ to $R_{667}$.

Each of n and m represents an integer of 0 to 3. n+m is an integer of 1 to 4. n+m is preferably 1 and 2.

As the compound containing a group represented by Formula (4-7) and the compound represented by Formula (5), one of the preferable forms is a compound represented by the following Formula (6-7).

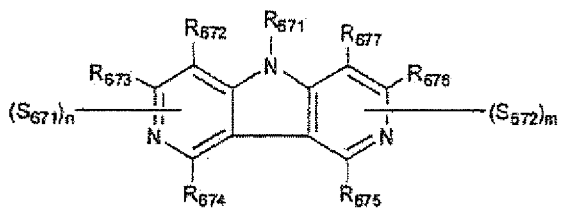

(6-7)

Formula (6-7) will be described. $R_{671}$ represents a hydrogen atom or a substituent. $R_{671}$ has the same meaning as $R_{511}$ in Formula (5), and preferred ranges thereof are also the same. Each of $R_{672}$ to $R_{677}$ independently represents a hydrogen atom or a substituent, and has the same meaning as $R_{522}$ in Formula (5), and preferred ranges thereof are also the same. Each of $S_{671}$ and $S_{672}$ independently represents the substituent (S), $S_{671}$ is substituted to carbon atoms as $R_{672}$ to $R_{674}$, and $S_{672}$ is substituted to carbon atoms as $R_{675}$ to $R_{677}$.

Each of n and m represents an integer of 0 to 3. n+m is an integer of 1 to 4. n+m is preferably 1 and 2.

As the compound containing a group represented by Formula (4-8) and the compound represented by Formula (5), one of the preferable forms is a compound represented by the following Formula (6-8).

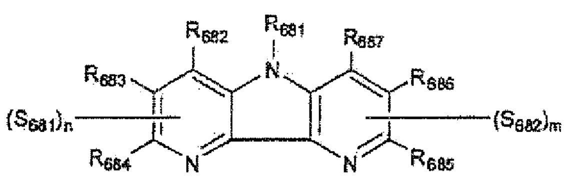

(6-8)

Formula (6-8) will be described. $R_{681}$ represents a hydrogen atom or a substituent. $R_{681}$ has the same meaning as $R_{511}$ in Formula (5), and preferred ranges thereof are also the same. Each of $R_{682}$ to $R_{687}$ independently represents a hydrogen atom or a substituent, and has the same meaning as $R_{522}$ in Formula (5), and preferred ranges thereof are also the same. Each of $S_{681}$ and $S_{682}$ independently represents the substituent (S), $S_{681}$ is substituted to carbon atoms

as $R_{682}$ to $R_{684}$, and $S_{622}$ is substituted to carbon atoms as $R_{685}$ to $P_{687}$.

Each of n and m represents an integer of 0 to 3. n+m is an integer of 1 to 4. n+m is preferably 1 and 2.

As the compound containing a group represented by Formula (4-9) and the compound represented by Formula (5), one of the preferable forms is a compound represented by the following Formula (6-9).

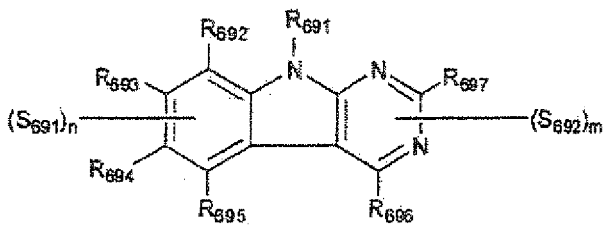

(6−9)

Formula (6-9) will be described. $R_{691}$ represents a hydrogen atom or a substituent. $R_{691}$ has the same meaning as $R_{511}$ in Formula (5), and preferred ranges thereof are also the same. Each of $R_{692}$ to $R_{697}$ independently represents a hydrogen atom or a substituent, and has the same meaning as $R_{522}$ in Formula (5), and preferred ranges thereof are also the same. Each of $S_{691}$ and $S_{692}$ independently represents the substituent (S), $S_{691}$ is substituted to carbon atoms as $R_{692}$ to $R_{695}$, and $S_{692}$ is substituted to carbon atoms as $R_{696}$ to $R_{697}$.

n represents an integer of 0 to 4. m represents an integer of 0 to 2. n+m is an integer of 1 to 4. n+m is preferably 1 and 2.

As the compound containing a group represented by Formula (4-10) and the compound represented by Formula (5), one of the preferable forms is a compound represented by the following Formula (6-10).

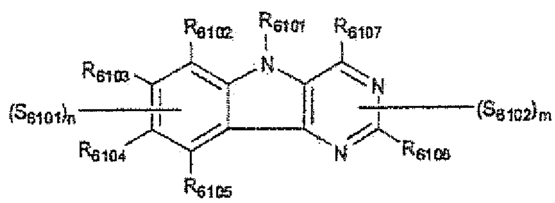

(6−10)

Formula (6-10) will be described. $R_{6101}$ represents a hydrogen atom or a substituent. $R_{6101}$ has the same meaning as $R_{511}$ in Formula (5), and preferred ranges thereof are also the same. Each of $R_{6102}$ to $R_{6107}$ independently represents a hydrogen atom or a substituent, and has the same meaning as $R_{522}$ in Formula (5), and preferred ranges thereof are also the same. Each of $S_{6101}$ and $S_{6102}$ independently represents the substituent (S), $S_{6101}$ is substituted to carbon atoms as $R_{6102}$ to $R_{6105}$, and $S_{6102}$ is substituted to carbon atoms as $R_{6106}$ to $P_{6107}$.

n represents an integer of 0 to 4. m represents an integer of 0 to 2. n+m is an integer of 1 to 4. n+m is preferably 1 and 2.

As the compound containing a group represented by Formulas (4-1) to (4-10) and the compound represented by Formula (5), one of the preferable forms is a compound containing a group represented by any one of Formulas (7-1) to (7-10). Particularly, in the present invention, as the compound containing at least one group represented by any one of Formulas (7-1) to (7-10), it is more preferred to have two to four groups represented by any one of Formulas (7-1) to (7-10) in its molecule. Particularly, it is preferred to have two groups represented by any one of Formulas (7-1) to (7-10) in its molecule. In this case, the structure represented by Formula (5) includes the case where a portion except for $R_{511}$ is replaced by Formulas (7-1) to (7-10).

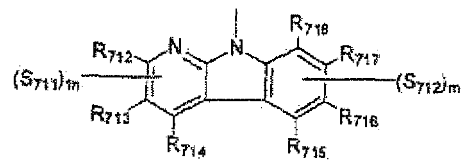

(7-1)

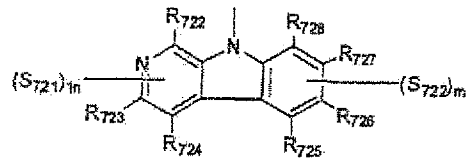

(7-2)

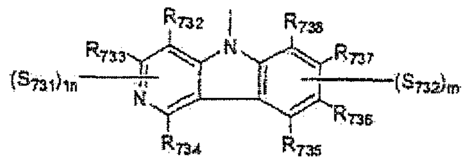

(7-3)

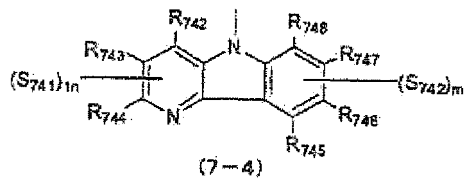

(7-4)

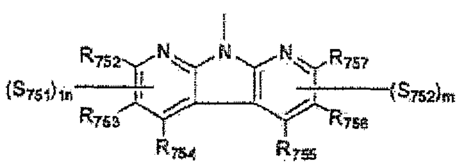

(7-5)

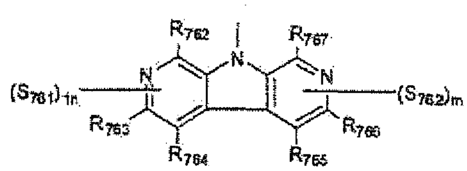

(7-6)

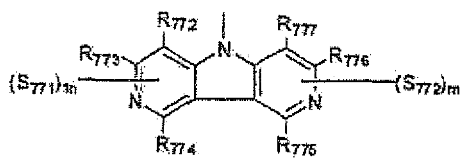

(7-7)

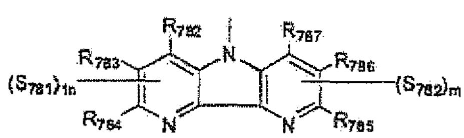

(7-8)

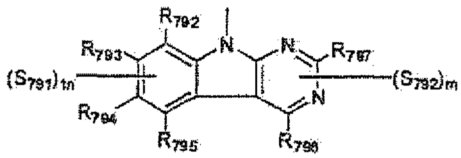

(7-9)

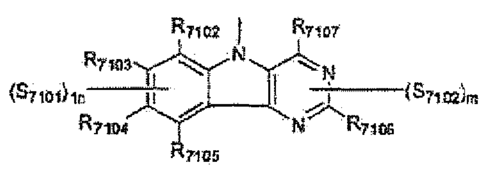

(7-10)

In Formula (7-1) to (7-10), each of $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ independently represents a hydrogen atom or a substituent, each of $S_{711}$ to $S_{7101}$ and $S_{712}$ to $S_{7102}$ independently represents the substituent (S), each of $S_{711}$ to $S_{7101}$ is substituted to a carbon atom as $R_{712}$ to $R_{714}$, $R_{722}$ to $R_{724}$, $R_{732}$ to $R_{734}$, $R_{742}$ to $R_{744}$, $R_{752}$ to $R_{754}$, $R_{762}$ to $R_{764}$, $R_{772}$ to $R_{774}$, $R_{782}$ to $R_{784}$, $R_{792}$ to $R_{795}$, and $R_{7102}$ to $R_{7105}$, and each of $S_{712}$ to $S_{7102}$ is substituted to a carbon atom as $R_{715}$ to $R_{718}$, $R_{725}$ to $R_{728}$, $R_{735}$ to $R_{738}$, $R_{745}$ to $R_{748}$, $R_{755}$ to $R_{757}$, $R_{765}$ to $R_{767}$, $R_{775}$ to $R_{777}$, $R_{785}$ to $R_{787}$, $R_{796}$ to $R_{797}$, and $R_{7106}$ to $R_{7107}$. In and m represent integers of 0 to 4, and 1n+m is an integer of 1 to 4.

Formulas (7-1) to (7-10) will be described.
Each of $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ independently represents a hydrogen atom and a substituent. As the substituent, those exemplified for the group A of substituents may be applied. Each of $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ may be the same as or different from every other $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$.

Groups represented by $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ include preferably a hydrogen atom, an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a cyano group, a silyl group and a heterocyclic group, even more preferably a hydrogen atom, an alkyl group, an alicyclic hydrocarbon group, an aryl group, a fluorine group, a cyano group, a silyl group and a heterocyclic group, particularly preferably a hydrogen atom, an alkyl group, an alicyclic hydrocarbon group, an aryl group and a fluorine group, and most preferably a hydrogen atom, an alkyl group (preferably an alkyl group having 1 to 6 carbon atoms and more preferably a methyl group, an ethyl group, a propyl group, an isopropyl group, a butyl group, a tert-butyl group and the like) and an alicyclic hydrocarbon group (preferably a cycloalkyl group having 5 to 6 carbon atoms, and more preferably a cycloheptyl group, a cyclohexyl group, an adamantyl group and the like).

A substituent represented by $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ may further have a substituent, and as the substituent, those exemplified above for the group A of the substituents may be applied. The substituent include preferably an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably an alkyl group, an alicyclic hydrocarbon group, an aryl group, a fluorine group, a cyano group, a silyl group and a heterocyclic group, even more preferably a hydrogen atom, an alkyl group, an alicyclic hydrocarbon group, an aryl group, a fluorine group, a cyano group, a silyl group and a heterocyclic group, particularly preferably an alkyl group, an alicyclic hydrocarbon group, an aryl group and a fluorine group, more preferably an alkyl group and an alicyclic hydrocarbon group (preferably a cycloalkyl group having 5 to 6 carbon atoms and more preferably a cycloheptyl group, a cyclohexyl group, an adamantyl group and the like), and particularly preferably an alkyl group (preferably an alkyl group having 1 to 6 carbon atoms and more preferably a methyl group, an ethyl group, a propyl group, an isopropyl group, a butyl group, a tert-butyl group and the like).
Further, a plurality of the substituents may be linked to each other to form a ring.

Each of $S_{711}$ to $S_{7101}$, and $S_{712}$ to $S_{7102}$ independently represents the substituent (S), each of $S_{711}$ to $S_{7101}$ is substituted to a carbon atom as $R_{712}$ to $R_{714}$, $R_{722}$ to $R_{724}$, $R_{732}$ to $R_{734}$, $R_{742}$ to $R_{744}$, $R_{752}$ to $R_{754}$, $R_{762}$ to $R_{764}$, $R_{772}$ to $R_{774}$, $R_{782}$ to $R_{784}$, $R_{792}$ to $R_{795}$, and $R_{7102}$ to $R_{7105}$, and each of $S_{712}$ to $S_{7102}$ is substituted to a carbon atom as $R_{715}$ to $R_{718}$, $R_{725}$ to $R_{728}$, $R_{735}$ to $R_{738}$, $R_{745}$ to $R_{748}$, $R_{755}$ to $R_{757}$, $R_{765}$ to $R_{767}$, $R_{775}$ to $R_{777}$, $R_{785}$ to $R_{787}$, $R_{796}$ to $R_{797}$, and $R_{7106}$ to $R_{7107}$.
The substituent (S) may be the (a) to (x) and among them, preferably (a) to (e), (i), (l), and (t) to (v), and more preferably (a) to (e).
In the case where the groups represented by Formulas (7-1) to (7-10) have a substituent (S), if the substituent has a large volume, such as the aforementioned substituents (a) to (x), it is preferred in that a reaction active site is protected and chemical stability in a state of cation and anion is improved.
In the case where the groups represented by Formulas (7-1) to (7-10) have a substituent (S), a substituent included in a specific phosphorescent metal complex A9 as described below is preferably a branched alkyl group. Since compatibility with the compound having the groups represented by Formulas (7-1) to (7-10) is increased in the light emitting layer, aggregation and association hardly occur, and as a result, it is considered that device deterioration due to generation of a quencher and misalignment of chromaticity due to association light emission are suppressed.

In and m represent integers of 0 to 4, and 1n+m is an integer of 1 to 4. 1n+m is preferably 1 and 2.

Among the groups represented by Formulas (7-1) to (7-4), the groups represented by Formulas (7-1) to (7-4), Formula (7-5) and Formula (7-8) are more preferred, and the groups represented by Formulas (7-1), Formula (7-4) and Formula (7-5) are even more preferred.

As the compound represented by Formula (2) and the compound containing at least one group represented by any one of Formulas (7-1) to (7-10), one of the preferable forms is a compound represented by the following Formula (8).

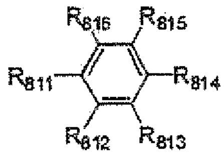

(8)

Each of $R_{811}$ to $R_{816}$ independently represents a hydrogen atom or a substituent and those exemplified above for the group A of substituents may be applied, but at least one of $R_{811}$ to $R_{816}$ is a group represented by any one of Formulas (4-1) to (4-10) and Formulas (7-1) to (7-10). More preferably, a group substituting at least one of $R_{811}$ to $R_{816}$ is (7-1) to (7-10).

Groups represented by $R_{811}$ to $R_{816}$ include preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a cyano group, a trifluoromethyl group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group (preferably an alkyl group having 1 to 6 carbon atoms, more preferably a methyl group, an ethyl group, a propyl group, an isopropyl group, a butyl group, a tert-butyl group and the like, and particularly preferably a tert-butyl group), and an aryl group which may have a substituent (The aryl group is preferably an aryl group having 6 to 12 carbon atoms, more preferably phenyl, p-methylphenyl, o-methylphenyl, naphthyl and anthranyl, and particularly preferably a phenyl group. The substituent may be the following substituent represented by $R_{811}$ to $R_{816}$ which may further have a substituent.), a fluorine group, a trifluoromethyl group and a cyano group, and among them, preferably a hydrogen atom and an alkyl group and most preferably a hydrogen atom.

A substituent represented by $R_{811}$ to $R_{816}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group).

Further, a plurality of the substituents may be linked to each other to form a ring.

As the compound represented by Formula (2) and the compound containing at least one group represented by any one of Formulas (7-1) to (7-10), one of the preferable forms is a compound represented by the following Formula (9).

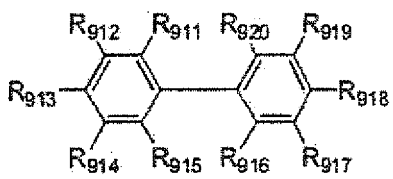

(9)

Formula (9) will be described. Each of $R_{911}$ to $R_{920}$ independently represents a hydrogen atom or a substituent and as the substituent, those exemplified above for the group A of substituents may be applied, but at least one of $R_{911}$ to $R_{920}$ is a group represented by any one of Formulas (4-1) to (4-10) and Formulas (7-1) to (7-10). A group substituting at least one of $R_{811}$ to $R_{816}$ is more preferably (7-1) to (7-10).

Groups represented by $R_{911}$ to $R_{920}$ include preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group (preferably an alkyl group having 1 to 6 carbon atoms, more preferably a methyl group, an ethyl group, a propyl group, an isopropyl group, a butyl group, a tert-butyl group and the like, and particularly preferably a tert-butyl group), and an aryl group which may have a. substituent (The aryl group is preferably an aryl group having 6 to 12 carbon atoms, more preferably phenyl, p-methylphenyl, o-methylphenyl, naphthyl and anthranyl and particularly preferably a phenyl group. The substituent may be the following substituent represented by $R_{911}$ to $R_{920}$ which may further have a substituent.), a fluorine group, a trifluoromethyl group, a cyano group, and a silyl group which may have a substituent (The substituent may be the following substituent represented by $R_{911}$ to $R_{920}$ which may further have a substituent.), and among them, preferably a hydrogen atom, an alkyl group, an aryl group, and a fluorine group, and most preferably a hydrogen atom.

A substituent represented by $R_{911}$ to $R_{920}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group).
Further, a plurality of the substituents may be linked to each other to form a ring.

As the compound represented by Formula (9), one of the preferable forms is a compound represented by the following Formula (10).

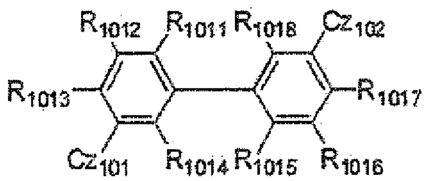

(10)

Formula (10) will be described. Each of $R_{1011}$ to $R_{1018}$ independently represents a hydrogen atom or a substituent, and those exemplified above for the group A of substituents may be applied. Each of $Cz_{101}$ and $Cz_{102}$ independently is a group represented by any one of Formulas (4-1) to (4-10) and Formulas (7-1) to (7-10), and more preferably Formulas (7-1) to (7-10).

Groups represented by $R_{1011}$ to $R_{1018}$ include preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group (preferably an alkyl group having 1 to 6 carbon atoms, more preferably a methyl group, an ethyl group, a propyl group, an isopropyl group, a butyl group, a tert-butyl group, and the like, and particularly preferably a tert-butyl group), and an aryl group which may have a substituent (The aryl group is preferably an aryl group having 6 to 12 carbon atoms, more preferably phenyl, p-methylphenyl, o-methylphenyl, naphthyl and anthranyl, and particularly preferably a phenyl group. The substituent may be the following substituent represented by $R_{1011}$ to $R_{1018}$ which may further have a substituent.), a fluorine group, a trifluoromethyl group and a cyano group, and among them, preferably a hydrogen atom, an alkyl group, an aryl group and a fluorine group, and most preferably a hydrogen atom.

A substituent represented by $R_{1011}$ to $R_{1018}$ may further have a substituent and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group).
Further, a plurality of the substituents may be linked to each other to form a ring.

As one of preferable aspects of the compound represented by Formula (10), each of $R_{1011}$ to $R_{1018}$ may represent a hydrogen atom, and each of $Cz_{101}$ and $Cz_{102}$ may represent a group represented by Formula (4-4). Further, in the aspect, it is preferred that each of $R_{442}$ to $R_{448}$ of Formula (4-4) independently represents a hydrogen atom or any one of the (a) to (e) of the substituents S.

As the compound represented by Formula (9) and the compound containing at least one group represented by any one of Formulas (7-1) to (7-10), one of the preferable aspects is a compound represented by the following Formula (11).

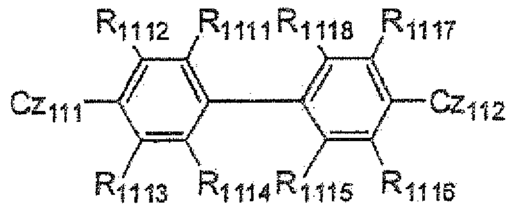

(11)

Formula (11) will be described. Each of $R_{1111}$ to $R_{1118}$ independently represents a hydrogen atom or a substituent, and those exemplified above for the group A of substituents may be applied. Each of $Cz_{111}$ and $Cz_{112}$ independently is a group represented by any one of Formulas (4-1) to (4-10) and Formulas (7-1) to (7-10) and more preferably Formulas (7-1) to (7-10).

Groups represented by $R_{1111}$ to $R_{1118}$ include preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group), and an aryl group which may have a substituent (The aryl group is preferably an aryl group having 6 to 12 carbon atoms, more preferably phenyl, p-methylphenyl, o-methylphenyl, naphthyl and anthranyl, and particularly preferably a phenyl group. The substituent may be the following substituent represented by $R_{1111}$ to $R_{1118}$ which may further have a substituent.), a fluorine group, a trifluoromethyl group, a cyano group and a silyl group which may have a substituent (The substituent may be the following substituent represented by $R_{1111}$ to $R_{1118}$ which may further have a substituent.), and among them, preferably a hydrogen atom, an alkyl group, an aryl group and a fluorine group, and most preferably a hydrogen atom.

A substituent represented by $R_{1111}$ to $R_{1118}$ may further have a substituent and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group, and the like, and particularly preferably a methyl group and a tert-butyl group).

Further, a plurality of the substituents may be linked to each other to form a ring.

As the compound represented by Formula (2) and the compound containing at least one group represented by any one of Formulas (7-1) to (7-10), one of the preferable aspects is a compound represented by the following Formula (12).

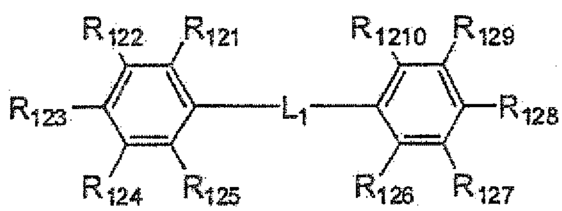

(12)

Formula (12) will be described. Each of $R_{121}$ to $R_{1210}$ independently represents a hydrogen atom or a substituent, and those exemplified above for the group A of substituents may be applied, but at least one of $R_{121}$ to $R_{1210}$ is a group represented by any one of Formulas (4-1) to (4-10) and Formulas (7-1) to (7-10), and more preferably Formulas (7-1) to (7-10). $L_1$ represents a divalent linking group.

The divalent linking group represented by $L_1$ may include a group including a heteroatom, (for example, a divalent group including a chalcogen atom such as -O-, -S- and the like, and an -N(R)- group, herein, R represents a hydrogen atom or an alkyl group, and the alkyl group has the same meaning as the alkyl group represented by $R_{111}$ in Formula (1)) and the like, in addition to a hydrocarbon group such as an alkylene group (for example, an ethylene group,

a trimethylene group, a tetramethylene group, a propylene group, an ethylethylene group, a pentamethylene group, a hexamethylene group, a 2,2,4-trimethylhexamethylene group, a heptamethylene group, an octamethylene group, a nonamethylene group, a decamethylene group, an undecamethylene group, a dodecamethylene group, a cyclohexylene group (for example, an 1,6-cyclohexanediyl group and the like), a cyclopentylene group (for example, an 1,5-cyclopentanediyl group and the like), and the like), an alkenylene group (for example, a vinylene group, a propenylene group and the like), an alkynylene group (for example, an ethynylene group, a 3-pentinylene group and the like), an arylene group, and the like.

Further, in each of the akylene group, the alkenylene group, the alkynylene group, and the arylene group, at least one of carbon atoms, which constitute a divalent linking group, may be substituted with a chalcogen atom (such as oxygen, sulfur, and the like) or the -N(R)- group, or the like.

In addition, as the divalent linking group represented by $L_1$, for example, a group having a divalent heterocyclic group is used, and examples thereof include an oxazolediyl group, a pyrimidinediyl group, a pyridazinediyl group, a pyrandiyl group, a pyrrolinediyl group, an imidazolinediyl group, an imidazolidinediyl group, a pyrazolidinediyl group, a pyrazolinediyl group, a piperidinediyl group, a piperazinediyl group, a morpholinediyl group, a quinuclidinediyl group and the like, and examples thereof may also be a divalent linking group derived from a compound having an aromatic heterocyclic ring (also referred to as a heteroaromatic compound) such as a thiophene-2,5-diyl group or a pyrazine-2,3-diyl group.

Further, the divalent linking group may be a linking group through a heteroatom, such as an alkylimino group, a dialkylsilanediyl group or a diarylgermanediyl group.

The divalent linking group represented by $L_1$ includes preferably a methylene group, an ethylene group, a cyclohexylene group, a cyclopentylene group, a substituted silicon atom, a substituted germanium atom, an oxygen atom, a sulfur atom, a 5- or 6-membered aromatic hydrocarbon ring group and an aromatic heterocyclic group, more preferably a methylene group, an ethylene group, a cyclohexylene group, a substituted or unsubstituted nitrogen atom, a substituted silicon atom, a substituted germanium atom and a 5- or 6-membered aromatic hydrocarbon ring group, even more preferably a methylene group, an ethylene group, a substituted silicon atom, a substituted nitrogen atom and a substituted germanium atom, and particularly preferably a methylene group substituted with an alkyl group or a phenyl group, a silicon atom, a germanium atom and a nitrogen atom.
These linking groups may further have a substituent, if possible, and as a substituent which may be introduced, those exemplified for the group A of substituents may be applied. When the aromatic hydrocarbon ring group or the aromatic heterocyclic group is used as the linking group, the size of the ring is a 5- or 6-membered ring.

As the compound represented by Formula (2) and the compound containing at least one group represented by any one of Formulas (7-1) to (7-10), one of the preferable aspects is a compound represented by the following Formula (13).

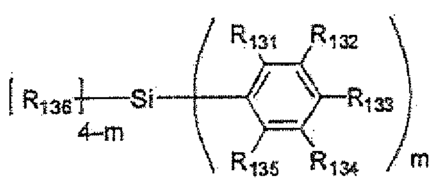

(13)

Formula (13) will be described. Each of $R_{131}$ to $R_{135}$ independently represents a hydrogen atom or a substituent, and those exemplified above for the group A of substituents may be applied, but at least one of $R_{131}$ to $R_{135}$ is a group represented by any one of Formulas (4-1) to (4-10) and Formulas (7-1) to (7-10), and more preferably Formulas (7-1) to (7-10).

$R_{136}$represents a hydrogen atom or a substituent. Each $R_{136}$ may be the same as or different from every other $R_{136}$. $R_{136}$ represents a hydrogen atom or a substituent. Each $R_{136}$ may be the same as or different from every other $R_{136}$.

As the substituent represented by $R_{136}$, those exemplified above for the group A of substituents may be applied. $R_{136}$ includes preferably a hydrogen atom, an alkyl group, an aromatic hydrocarbon ring group, an amino group, an alkoxy group, an aryloxy group, an aromatic heterocyclic oxy group, an alkylthio group, an arylthio group, a heterocyclic thio group, a cyano group, an aromatic heterocyclic group, a silyl group and a silyloxy group, more preferably an alkyl group, an aromatic hydrocarbon ring group, an amino group, a cyano group and an aromatic heterocyclic group, even more preferably an alkyl group, an aromatic hydrocarbon ring group, a cyano group, and an aromatic heterocyclic group, particularly preferably an alkyl group and an aromatic hydrocarbon ring group (preferably an aryl group having 6 to 12 carbon atoms, more preferably phenyl, p-methylphenyl, o-methylphenyl, naphthyl and anthranyl, and particularly preferably a phenyl group), and most preferably a methyl group and a phenyl group which may have a substituent (the

substituent may be the following substituent represented by $R_{136}$ which may further have a substituent).

A substituent represented by $R_{136}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group). Further, a plurality of the substituents may be linked to each other to form a ring.

A group represented by $R_{131}$ to $R_{135}$ includes preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group, and a silyloxy group, more preferably a hydrogen atom, an alkyl group, an alicyclic hydrocarbon group, an aryl group, a fluorine group, a trifluoromethyl group, a cyano group, a silyl group, and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorin group and among them, particularly preferably a hydrogen atom, an alkyl group and an aryl group, and most preferably a hydrogen atom.

A substituent represented by $R_{131}$ to $R_{135}$ may further have a substituent and as the substituent, those exemplified above for the group A of the substituents may be applied. Further, a plurality of the substituents may be linked to each other to form a ring.

m represents an integer of 1 to 4, preferably 1 to 3, and more preferably 2.

As the compound represented by Formula (2) and the compound containing at least one group represented by any one of Formulas (7-1) to (7-10), one of the preferable forms is a compound represented by the following Formula (14).

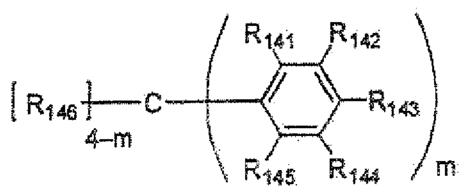

(14)

Formula (14) will be described. Each of $R_{141}$ to $R_{145}$ independently represents a hydrogen atom or a substituent, and as the substituent, those exemplified above for the group A of substituents may be applied, but at least one of $R_{141}$ to $R_{145}$ is a group represented by any one of Formulas (4-1) to (4-10) and Formulas (7-1) to (7-10), and more preferably Formulas (7-1) to (7-10).

$R_{146}$ represents a hydrogen atom or a substituent. Each $R_{146}$ may be the same as or different from every other $R_{146}$. $R_{146}$ represents a hydrogen atom or a substituent. Each $R_{146}$ may be the same as or different from every other $R_{146}$.

As the substituent represented by $R_{146}$, those exemplified above for the group A of substituents may be applied. $R_{146}$ includes preferably a hydrogen atom, an alkyl group, an aromatic hydrocarbon ring group, an amino group, an alkoxy group, an aryloxy group, an aromatic heterocyclic oxy group, an alkylthio group, an arylthio group, a heterocyclic thio group, a cyano group, an aromatic heterocyclic group, a silyl group, and a silyloxy group, more preferably an alkyl group, an aromatic hydrocarbon ring group, an amino group, a cyano group and an aromatic heterocyclic group, even more preferably an alkyl group, an aromatic hydrocarbon ring group, a cyano group, and an aromatic heterocyclic group, particularly preferably an alkyl group and an aromatic hydrocarbon ring group (preferably an aryl group having 6 to 12 carbon atoms, more preferably phenyl, p-methylphenyl, o-methylphenyl, naphthyl and anthranyl, and particularly preferably a phenyl group), and most preferably a methyl group and a phenyl group which may have a substituent (the substituent may be the following substituent represented by $R_{146}$ which may further have a substituent).

A substituent represented by $R_{146}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group). Further, a plurality of the substituents may be linked to each other to form a ring.

A substituent represented by $R_{141}$ to $R_{145}$ includes preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a

heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group, an alicyclic hydrocarbon group, an aryl group and a fluorine group, and among them, preferably a hydrogen atom, an alkyl group, and an aryl group, and most preferably a hydrogen atom.

A substituent represented by $R_{141}$ to $R_{145}$ may further have a substituent and as the substituent, those exemplified above for the group A of the substituents may be applied. Further, a plurality of the substituents may be linked to each other to form a ring.

m represents an integer of 1 to 4, preferably 1 to 3, and more preferably 2.

As the compound represented by Formula (2) and the compound containing at least one group represented by any one of Formulas (7-1) to (7-10), one of the preferable forms is a compound represented by the following Formula (15).

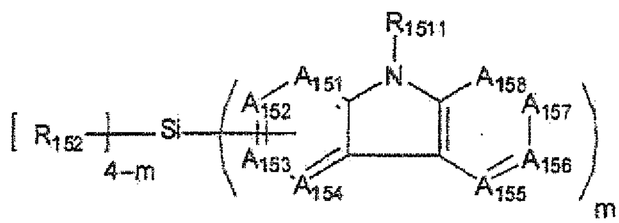

(15)

Formulas (15) will be described. $A_{151}$ to $A_{158}$ and $R_{1511}$ have the same meaning as $A_{21}$to $A_{28}$ and $R_{211}$ in Formula (2), and preferred ranges thereof are also the same. $R_{152}$ represents a hydrogen atom or a substituent. Each $R_{152}$ may be the same as or different from every other $R_{152}$. $R_{1511}$ represents a hydrogen atom or a substituent. m represents an integer of 1 to 4. A silicon linking group is linked with a C atom of $A_{151}$ to $A_{158}$.

As the substituent represented by $R_{1511}$, those exemplified for the following group A of substituents may be applied. $R_{1511}$ includes preferably a hydrogen atom, an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group, and a silyloxy group, more preferably a hydrogen atom, an alkyl group, an alicyclic hydrocarbon group, an aryl group, a fluorine group, a cyano group, a silyl group and a heterocyclic group, even more preferably a hydrogen atom, an alkyl group, an alicyclic hydrocarbon group, an aryl group, a fluorine group, a cyano group, a silyl group and a heterocyclic group, particularly preferably an alkyl group, an alicyclic hydrocarbon group, an aryl group, and a heterocyclic group, and among them, an aryl group (preferably an aryl group having 6 to 12 carbon atoms, more preferably phenyl, p-methylphenyl, o-methylphenyl, naphthyl and anthranyl, and particularly preferably a phenyl group), and most preferably a phenyl group which may have a substituent (the substituent may be the following substituent represented by $R_{1511}$ which may further have a substituent).

$R_{1511}$ may have a substituent and as the substituent, those exemplified above for the group A may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group) and a phenyl group.
Further, a plurality of the substituents may be linked to each other to form a ring.

As the substituent represented by $R_{152}$, those exemplified for the following group A of substituents may be applied. $R_{92}$ includes preferably a hydrogen atom, an alkyl group, an aromatic hydrocarbon ring group, an amino group, an alkoxy group, an aryloxy group, an aromatic heterocyclic oxy group, an alkylthio group, an arylthio group, a heterocyclic thio group, a cyano group, an aromatic heterocyclic group, a silyl group, and a silyloxy group, more preferably an alkyl group, an aromatic hydrocarbon ring group, an amino group, a cyano group and an aromatic heterocyclic group, even more preferably an alkyl group, an aromatic hydrocarbon ring group, a cyano group, and an aromatic heterocyclic group, particularly preferably an alkyl group and an aromatic hydrocarbon ring group (preferably an aryl group having 6 to 12 carbon atoms, more preferably phenyl, p-methylphenyl, o-methylphenyl, naphthyl and anthranyl, and particularly preferably a phenyl group), and most preferably a methyl group and a phenyl group which may have a substituent (the substituent may be the following substituent represented by $R_{152}$ which may further have a substituent).

A substituent represented by $R_{152}$ may further have a substituent and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl

group, a butyl group, a tert-butyl group and the like, and particularly preferably a methyl group and a tert-butyl group). Further, a plurality of the substituents may be linked to each other to form a ring.

A group represented by $R_{153}$ includes preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, particularly preferably a hydrogen atom, an alkyl group, an alicyclic hydrocarbon group, an aryl group and a fluorine group, and among them, preferably a hydrogen atom, an alkyl group (preferably an alkyl group having 1 to 6 carbon atoms, and more preferably a methyl group, an ethyl group, a propyl group, an isopropyl group, a butyl group, a tert-butyl group and the like), and an alicyclic hydrocarbon group which may have a substituent (The alicyclic hydrocarbon group is preferably a cycloalkyl group having 5 to 6 carbon atoms, and more preferably a cycloheptyl group, a cyclohexyl group, an adamantyl group and the like. The substituent may be the following substituent represented by $R_{153}$ which may further have a substituent.), and most preferably a hydrogen atom.

A substituent represented by $R_{153}$ may further have a substituent and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group). Further, a plurality of the substituents may be linked to each other to form a ring.

As the compound represented by Formula (2) and the compound containing at least one group represented by any one of Formulas (7-1) to (7-10), one of the preferable forms is a compound represented by the following Formula (16).

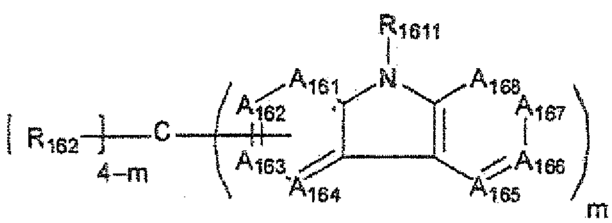

(16)

Formula (16) will be described. $A_{161}$ to $A_{168}$ and $R_{1611}$ have the same meaning as $A_{21}$to $A_{28}$ and $R_{211}$ in Formula (2), and preferred ranges thereof are also the same. $R_{162}$ represents a hydrogen atom or a substituent. Each $R_{162}$ may be the same as or different from every other $R_{162}$. $R_{1611}$ represents a hydrogen atom or a substituent. m represents an integer of 1 to 4. A silicon linking group is linked with a C atom of $A_{161}$ to $A_{168}$.

As the substituent represented by $R_{1611}$, those exemplified for the following group A of substituents may be applied. $R_{1611}$ includes preferably a hydrogen atom, an alkyl group, an alicyclic hydrocarbon group, an aryl group, a fluorine group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a cyano group, a silyl group and a heterocyclic group, even more preferably a hydrogen atom, an alkyl group, an alicyclic hydrocarbon group, an aryl group, a fluorine group, a cyano group, a silyl group and a heterocyclic group, particularly preferably an alkyl group, an alicyclic hydrocarbon group, an aryl group and a heterocyclic group, and among them, preferably an aryl group (preferably an aryl group having 6 to 12 carbon atoms, more preferably phenyl, p-methylphenyl, o-methylphenyl, naphthyl and anthranyl, and particularly preferably a phenyl group), and most preferably a phenyl group which may have a substituent (the substituent may be the following substituent represented by $R_{1611}$ which may further have a substituent).

A substituent represented by $R_{1611}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group) and a phenyl group.

Further, a plurality of the substituents may be linked to each other to form a ring.

As the substituent represented by $R_{162}$, those exemplified above for the group A of substituents may be applied.

$R_{92}$ includes preferably a hydrogen atom, an alkyl group, an aromatic hydrocarbon ring group, an amino group, an alkoxy group, an aryloxy group, an aromatic heterocyclic oxy group, an alkylthio group, an arylthio group, a heterocyclic thio group, a cyano group, an aromatic heterocyclic group, a silyl group and a silyloxy group, more preferably an alkyl group, an aromatic hydrocarbon ring group, an amino group, a cyano group and an aromatic heterocyclic group, even more preferably an alkyl group, an aromatic hydrocarbon ring group, a cyano group, and an aromatic heterocyclic group, particularly preferably an alkyl group and an aromatic hydrocarbon ring group (preferably an aryl group having 6 to 12 carbon atoms, more preferably phenyl, p-methylphenyl, o-methylphenyl, naphthyl and anthranyl, and particularly preferably a. phenyl group), and most preferably a methyl group and a phenyl group which may have a substituent (the substituent may be the following substituent represented by $R_{162}$ which may further have a substituent).

A substituent represented by $R_{162}$ may further have a substituent, and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group). Further, a plurality of the substituents may be linked to each other to form a ring.

A group represented by $R_{163}$ includes preferably a hydrogen atom, an alkyl group, an alicyclic hydrocarbon group, an aryl group, a fluorine group, a trifluoromethyl group, an amino group, an alkoxy group, an aryloxy group, a heterocyclic oxy group, an alkylthio group, an arylthio group, a heterocyclic thio group, a cyano group, a heterocyclic group, a silyl group and a silyloxy group, more preferably a hydrogen atom, an alkyl group, an alicyclic hydrocarbon group, an aryl group, a fluorine group, a trifluoromethyl group, a cyano group, a silyl group and a heterocyclic group, even more preferably a hydrogen atom, an alkyl group, an alicyclic hydrocarbon group, an aryl group, a fluorine group, a trifluoromethyl group, a cyano group, a silyl group and a heterocyclic group, and particularly preferably a hydrogen atom, an alkyl group, an alicyclic hydrocarbon group, an aryl group and a fluorine group, and among them, preferably a hydrogen atom, an alkyl group (preferably an alkyl group having 1 to 6 carbon atoms, and more preferably a methyl group, an ethyl group, a propyl group, an isopropyl group, a butyl group, a tert-butyl group and the like), and an alicyclic hydrocarbon group which may have a substituent (The alicyclic hydrocarbon group, preferably a cycloalkyl group having 5 to 6 carbon atoms, and more preferably a cycloheptyl group, a cyclohexyl group, an adamantyl group and the like. The substituent may be the following substituent represented by $R_{163}$ which may further have a substituent), and most preferably a hydrogen atom.

A substituent represented by $R_{163}$ may further have a substituent and as the substituent, those exemplified above for the group A of the substituents may be applied. The substituent includes preferably an alkyl group (preferably an alkyl group having 1 to 6 carbon atoms, more preferably a methyl group, an ethyl group, a propyl group, an isopropyl group, a butyl group, a tert-butyl group and the like, and particularly preferably a methyl group and a tert-butyl group).

One of the preferable aspects of the compounds represented by Formulas (1) to (16) may include those which may not have a substituent. In the case where the compounds represented by Formulas (1) to (16) are used on the same layer as the aforementioned specific phosphorescent light emitting material, it is preferred that the compounds represented by Formulas (1) to (16) do not have a substituent in order to suppress increase in driving voltage.

Examples of the compounds represented by Formulas (1) to (16) are represented below, but the present invention is not limited thereto.

| Compound | Central Structure | A |
|---|---|---|
| 1 | 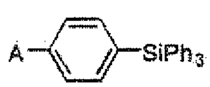 | 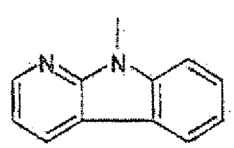 |
| 2 | 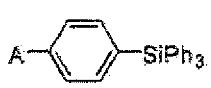 | 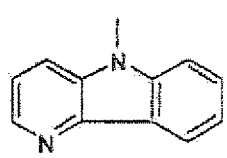 |

(continued)

| Compound | Central Structure | A |
|---|---|---|
| 3 | 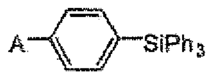 | 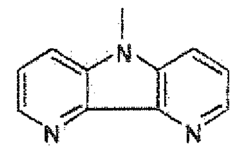 |
| 4 | 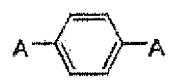 | 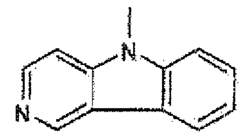 |
| 5 | 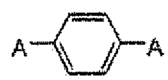 | 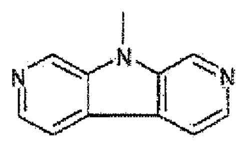 |
| 6 | 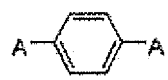 | 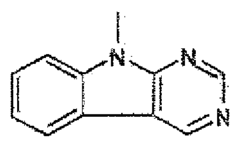 |
| 7 | 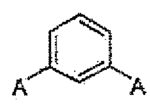 | 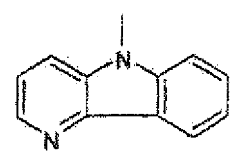 |
| 8 | 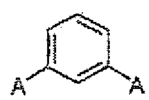 | 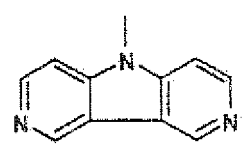 |
| 9 | 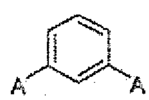 | 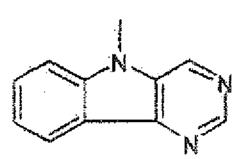 |

| Compound | Central Structure | A |
|---|---|---|
| 10 | 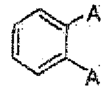 | 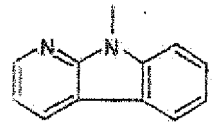 |

(continued)

| Compound | Central Structure | A |
|---|---|---|
| 11 | 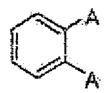 | 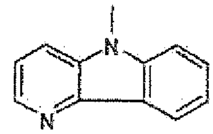 |
| 12 | 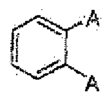 | 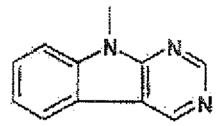 |
| 13 | 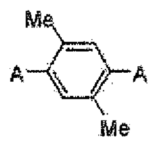 | 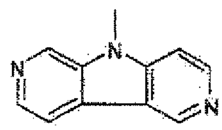 |
| 14 | 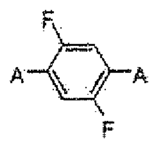 | 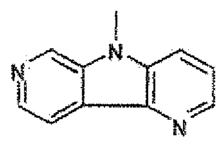 |
| 15 | 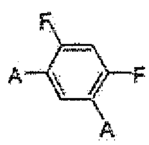 | 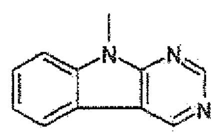 |
| 16 | 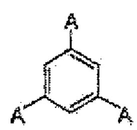 | 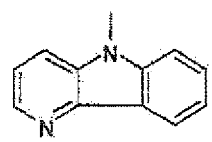 |
| 17 | 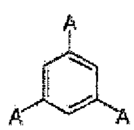 | 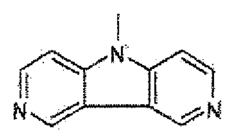 |
| 18 | 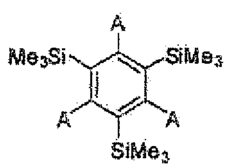 | 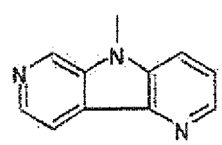 |

| Compound | Central Structure | A |
|---|---|---|
| 19 | 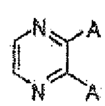 | 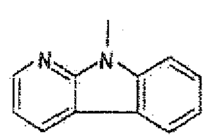 |

(continued)

| Compound | Central Structure | A |
|---|---|---|
| 20 | 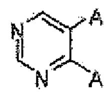 | 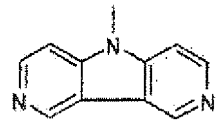 |
| 21 | 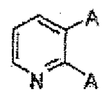 | 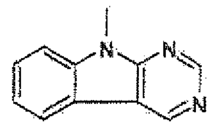 |
| 22 | 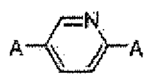 | 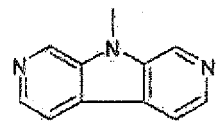 |
| 23 | 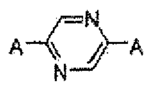 | 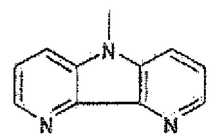 |
| 24 | 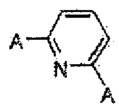 | 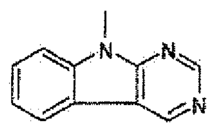 |
| 25 | 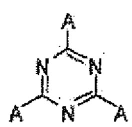 | 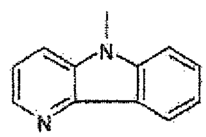 |
| 26 | 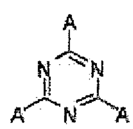 | 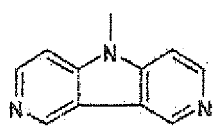 |
| 27 | 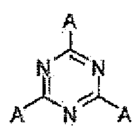 | 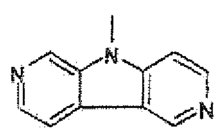 |

| Compound | Central Structure | A |
|---|---|---|
| 28 | 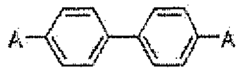 | 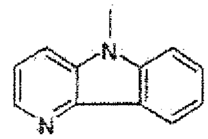 |

(continued)

| Compound | Central Structure | A |
|---|---|---|
| 29 | 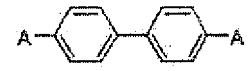 | 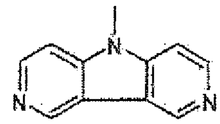 |
| 30 | 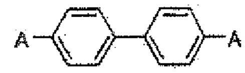 | 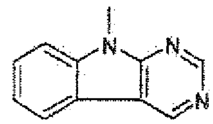 |
| 31 | 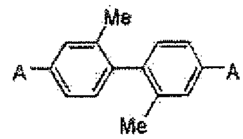 | 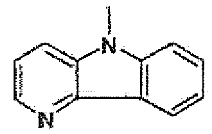 |
| 32 | 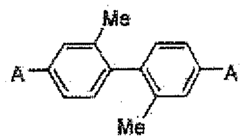 | 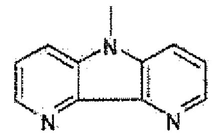 |
| 33 | 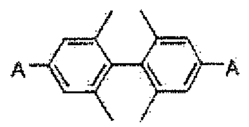 | 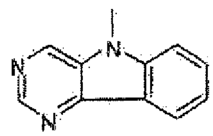 |
| 34 | 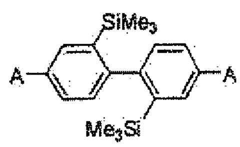 | 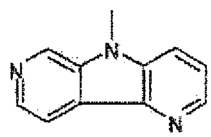 |
| 35 | 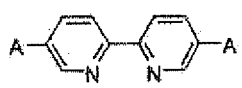 | 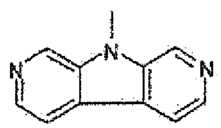 |
| 36 | 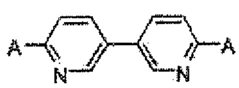 | 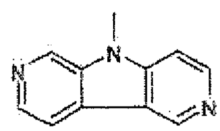 |

| Compound | Central Structure | A |
|---|---|---|
| 37 | 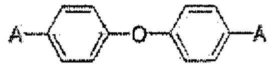 | 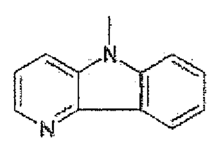 |

(continued)

| Compound | Central Structure | A |
|---|---|---|
| 38 | 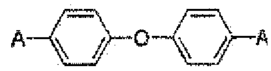 | 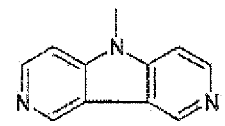 |
| 39 | 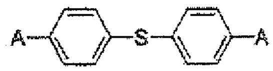 | 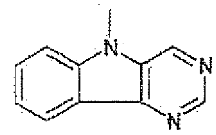 |
| 40 | 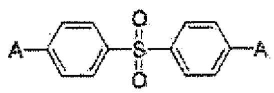 | 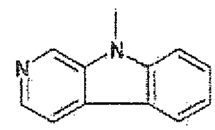 |
| 41 | 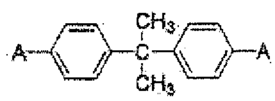 | 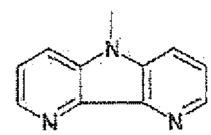 |
| 42 | 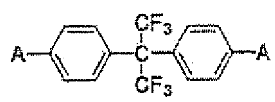 | 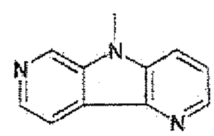 |
| 43 | 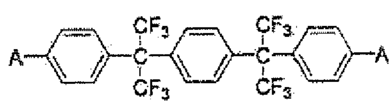 | 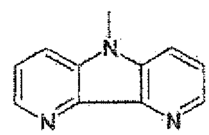 |
| 44 | 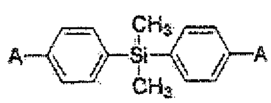 | 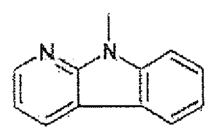 |
| 45 | 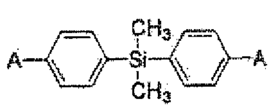 | 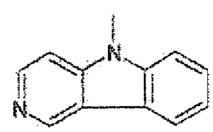 |

| Compound | Central Structure | A |
|---|---|---|
| 46 | 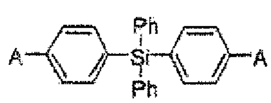 | 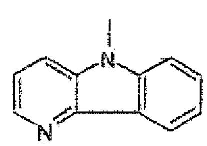 |

(continued)

| Compound | Central Structure | A |
|---|---|---|
| 47 | 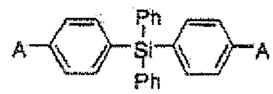 | 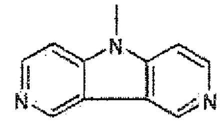 |
| 48 | 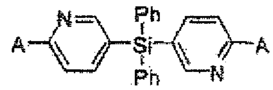 | 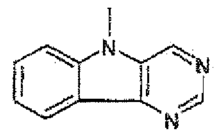 |
| 49 | 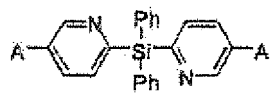 | 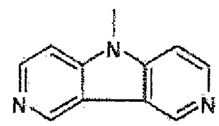 |
| 50 | 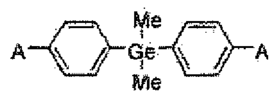 | 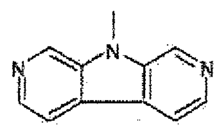 |
| 51 | 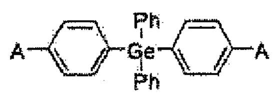 | 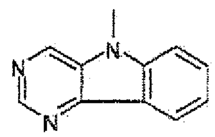 |
| 52 | 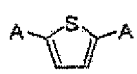 | 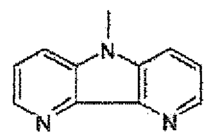 |
| 53 | 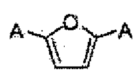 | 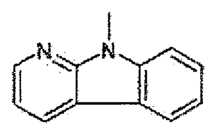 |
| 54 | 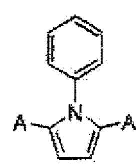 | 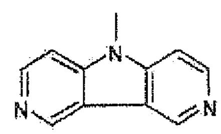 |

| Compound | Central Structure | A |
|---|---|---|
| 55 | 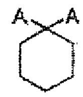 | 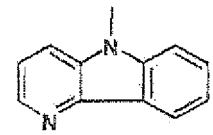 |
| 56 | 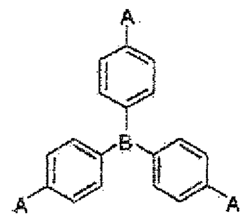 | 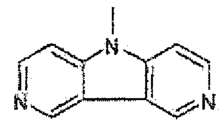 |
| 57 | 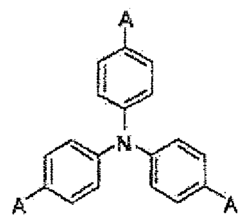 | 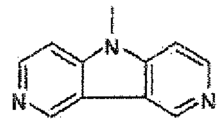 |
| 58 | 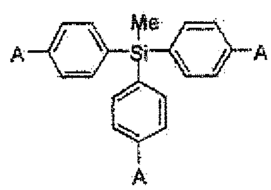 | 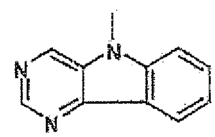 |
| 59 | 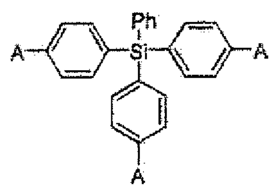 | 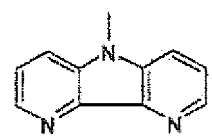 |
| 60 | 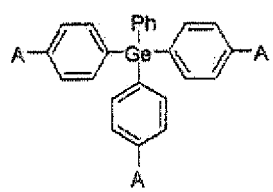 | 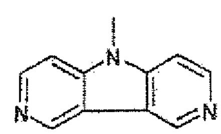 |

| Compound | Central Structure | A |
|---|---|---|
| 61 | 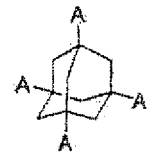 | 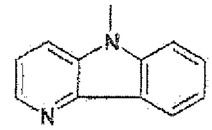 |
| 62 | 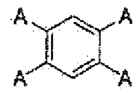 | 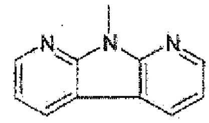 |
| 63 | 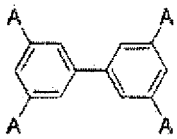 | 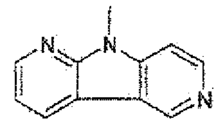 |
| 64 | 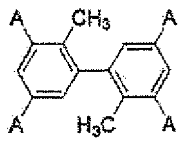 | 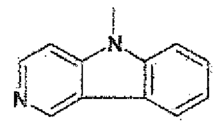 |
| 65 | 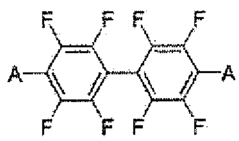 | 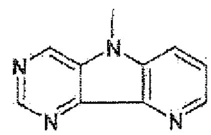 |
| 66 | 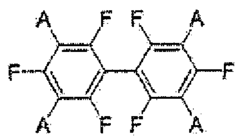 | 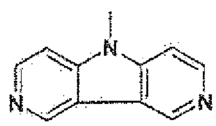 |
| 67 | 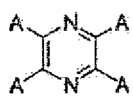 | 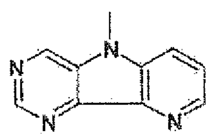 |
| 68 | 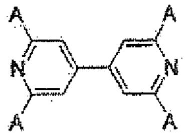 | 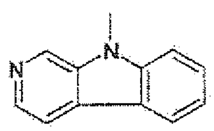 |
| 69 | 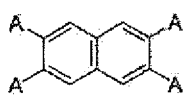 | 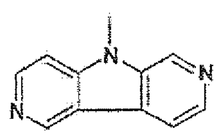 |

| Compound | Central Structure | A |
|---|---|---|
| 70 | 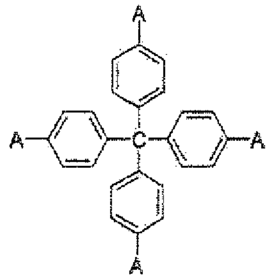 | 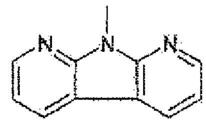 |
| 71 | 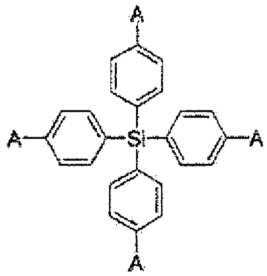 | 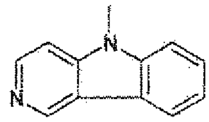 |
| 72 | 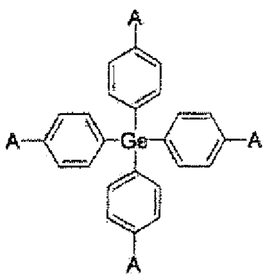 | 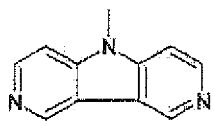 |
| 75 | 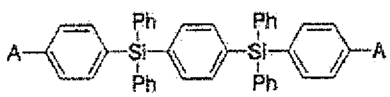 | 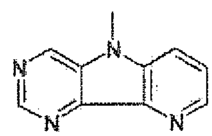 |
| 76 | 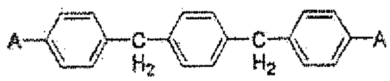 | 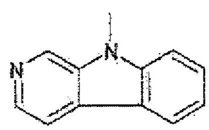 |
| 77 | 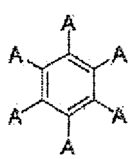 | 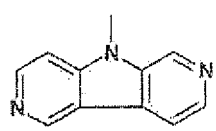 |

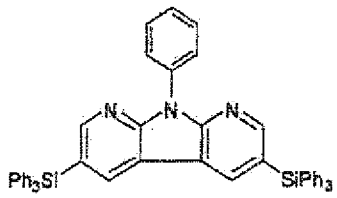

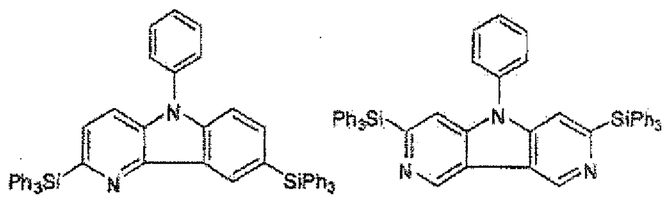

78 80 90

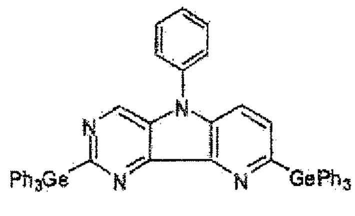

91

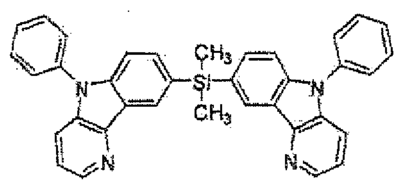

92

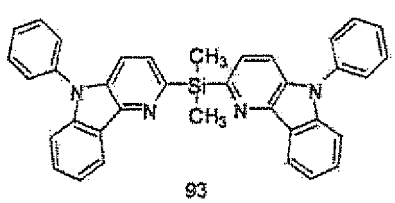

93

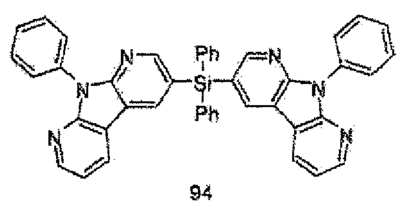

94

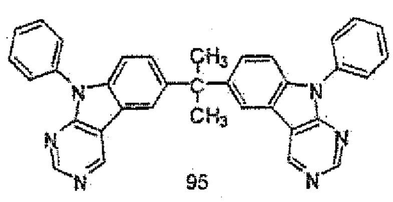

95

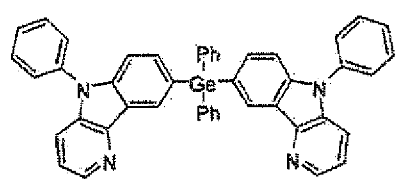

96

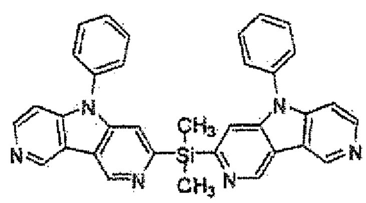

97

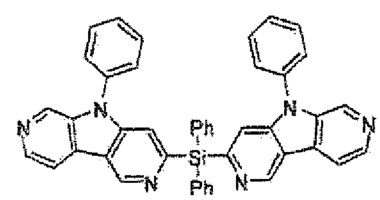

98

| Compound | Central Structure | A |
|---|---|---|
| 99 | 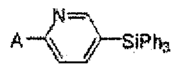 | 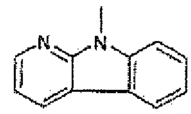 |
| 100 | 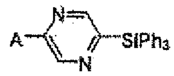 | 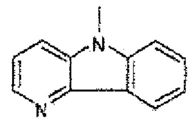 |
| 101 | 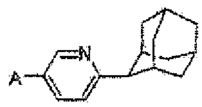 | 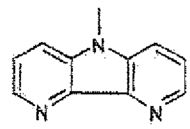 |
| 102 | 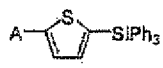 | 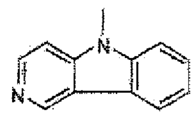 |
| 103 | 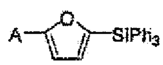 | 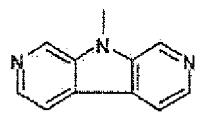 |
| 104 | 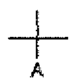 | 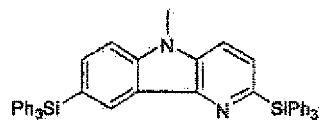 |
| 105 | 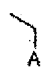 | 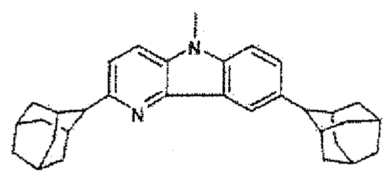 |
| 106 | 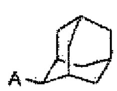 | 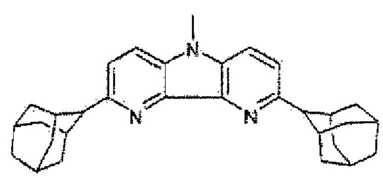 |

| Compound | Central Structure | A |
|---|---|---|
| 107 | 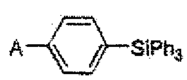 | 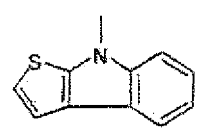 |

(continued)

| Compound | Central Structure | A |
|---|---|---|
| 108 | 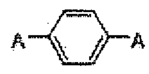 | 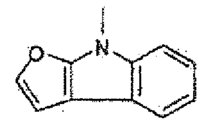 |
| 109 | 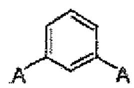 | 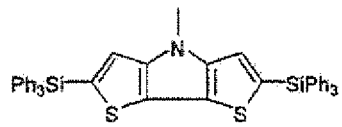 |
| 110 | 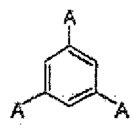 | 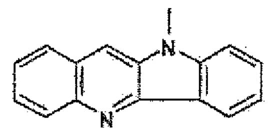 |
| 111 | 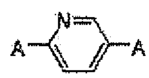 | 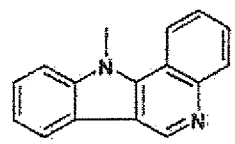 |
| 112 | 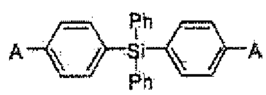 | 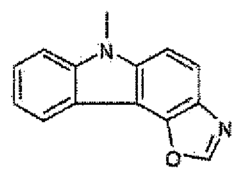 |
| 113 | 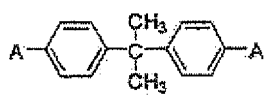 | 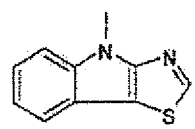 |
| 114 | 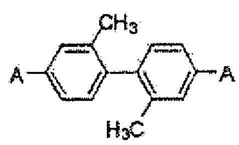 | 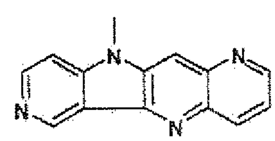 |
| 115 | 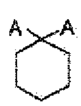 | 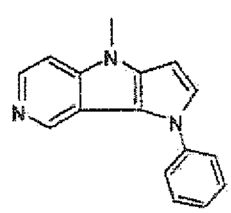 |

| Compound | Central Structure | A |
|---|---|---|
| 116 | 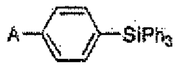 | 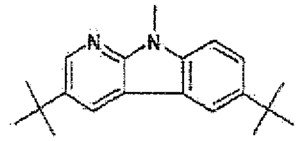 |
| 117 | 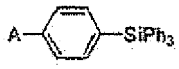 | 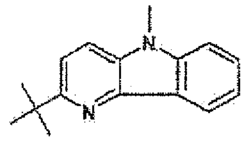 |
| 118 | 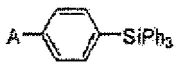 | 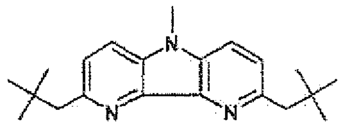 |
| 119 | 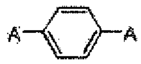 | 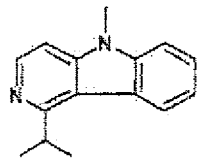 |
| 120 | 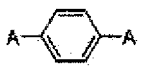 | 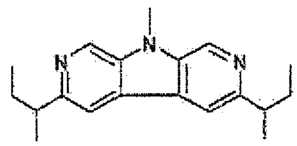 |
| 121 | 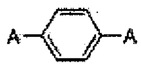 | 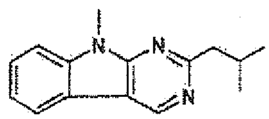 |
| 122 | 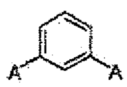 | 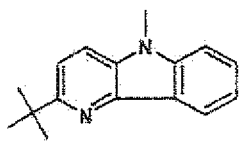 |
| 123 | 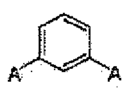 | 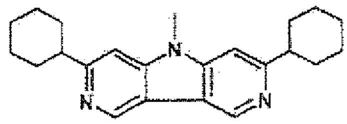 |
| 124 | 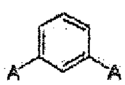 | 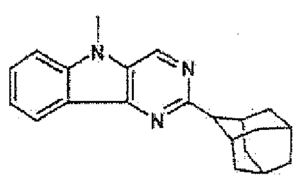 |

| Compound | Central Structure | A |
|---|---|---|
| 125 | 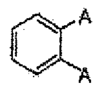 | 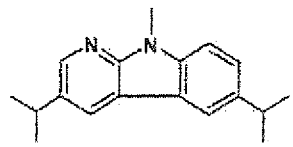 |
| 126 | 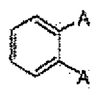 | 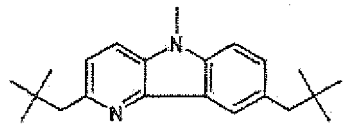 |
| 127 | 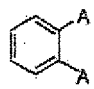 | 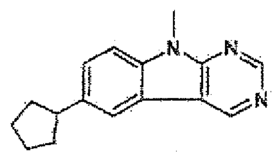 |
| 128 | 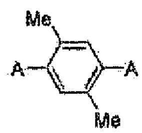 | 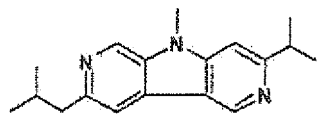 |
| 129 | 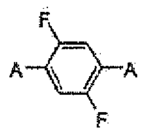 | 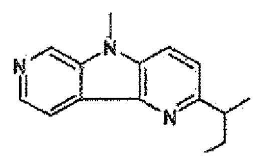 |
| 130 | 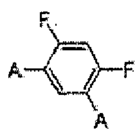 | 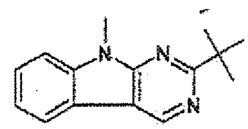 |
| 131 | 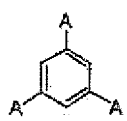 | 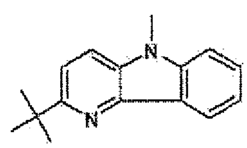 |
| 132 | 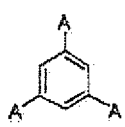 | 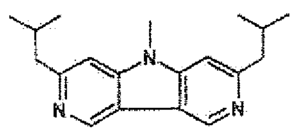 |
| 133 | 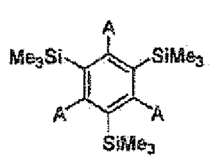 | 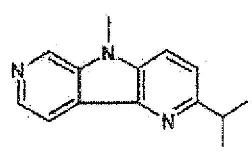 |

| Compound | Central Structure | A |
|---|---|---|
| 134 | 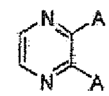 | 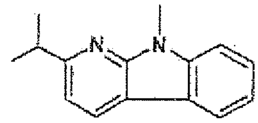 |
| 135 | 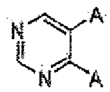 | 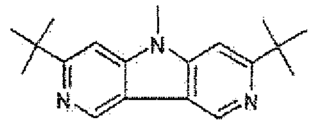 |
| 136 | 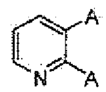 | 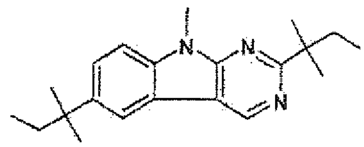 |
| 137 | 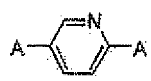 | 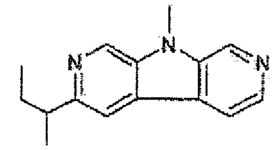 |
| 138 | 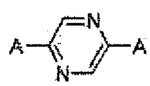 | 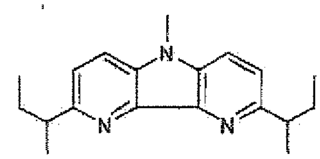 |
| 139 | 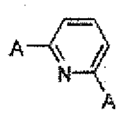 | 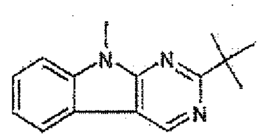 |
| 140 | 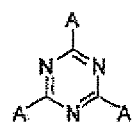 | 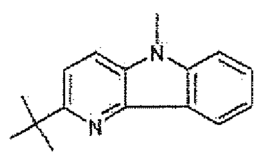 |
| 141 | 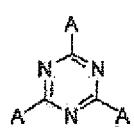 | 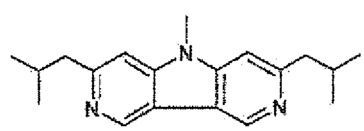 |
| 142 | 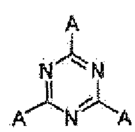 | 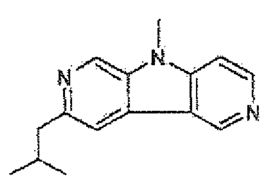 |

| Compound | Central Structure | A |
|---|---|---|
| 143 | 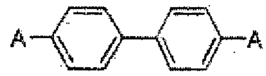 | 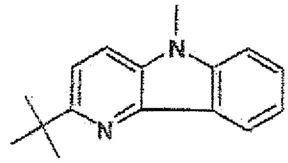 |
| 144 | 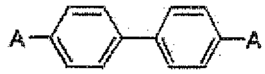 | 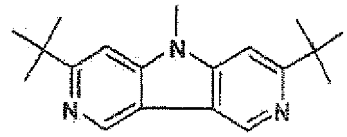 |
| 145 | 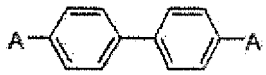 | 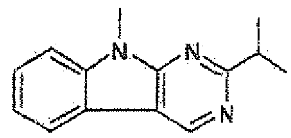 |
| 146 | 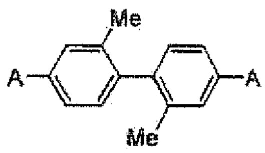 | 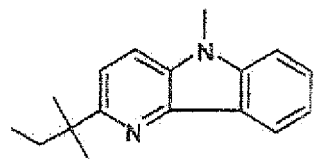 |
| 147 | 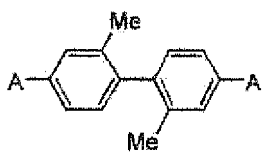 | 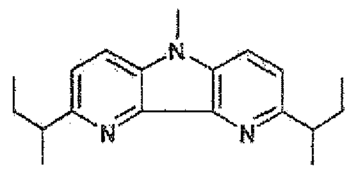 |
| 148 | 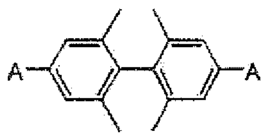 | 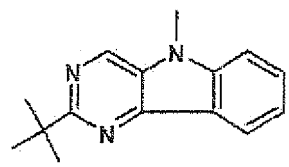 |
| 149 | 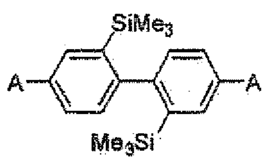 | 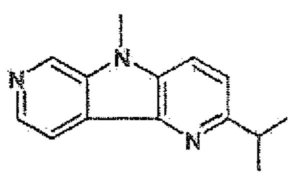 |
| 150 | 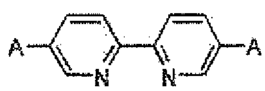 | 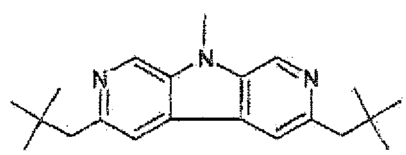 |
| 151 | 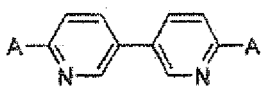 | 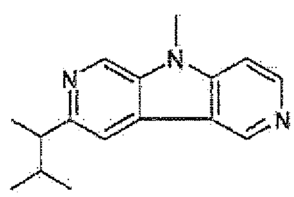 |

| Compound | Central Structure | A |
|---|---|---|
| 152 | 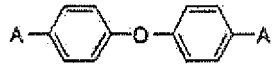 | 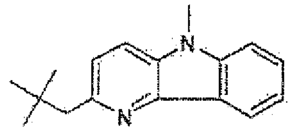 |
| 153 | 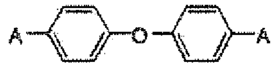 | 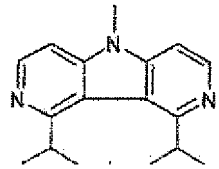 |
| 154 | 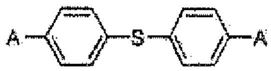 | 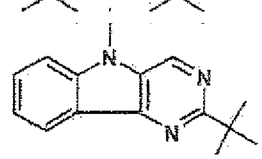 |
| 155 | 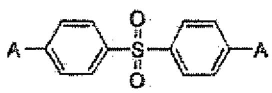 | 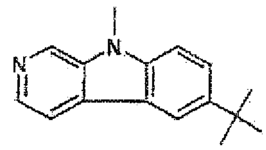 |
| 156 | 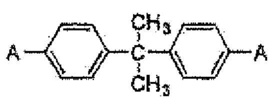 | 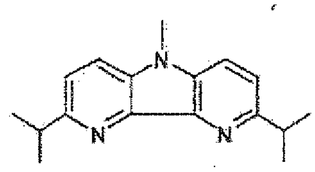 |
| 157 | 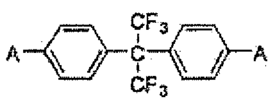 | 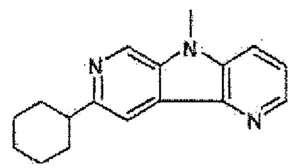 |
| 158 | 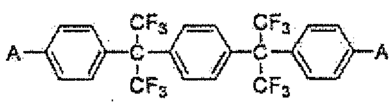 | 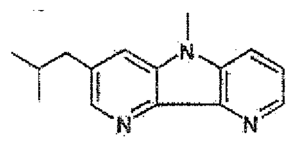 |
| 159 | 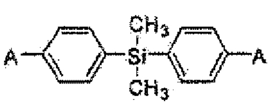 | 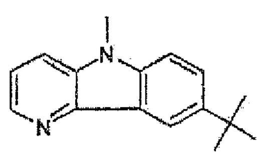 |
| 160 | 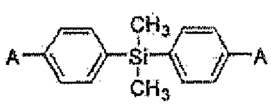 | 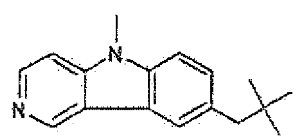 |

| Compound | Central Structure | A |
| --- | --- | --- |
| 161 | 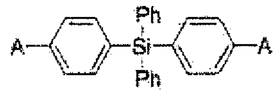 | 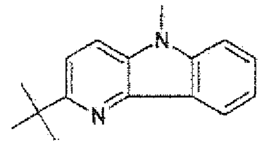 |
| 162 | 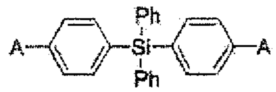 | 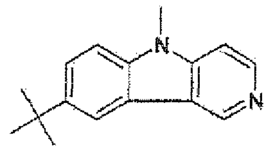 |
| 163 | 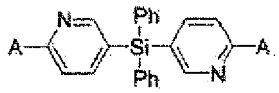 | 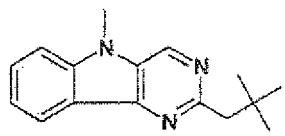 |
| 164 | 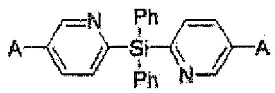 | 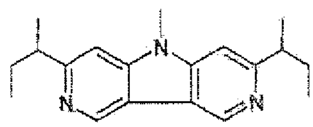 |
| 165 | 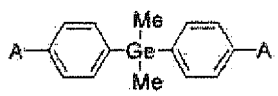 | 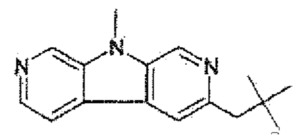 |
| 166 | 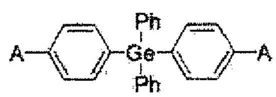 | 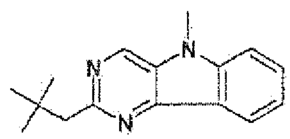 |
| 167 | 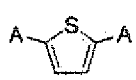 | 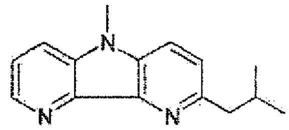 |
| 168 | 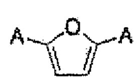 | 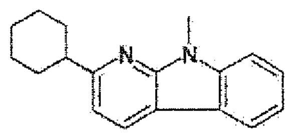 |
| 169 | 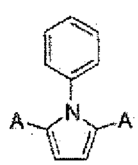 | 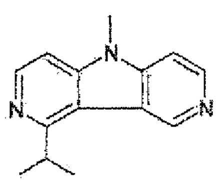 |

| Compound | Central Structure | A |
|---|---|---|
| 170 | 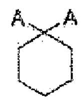 | 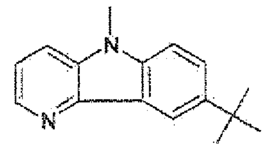 |
| 171 | 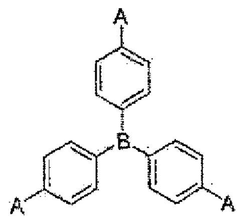 | 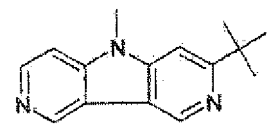 |
| 172 | 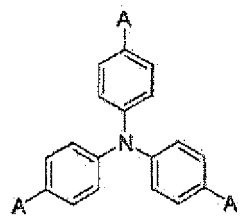 | 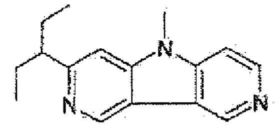 |
| 173 | 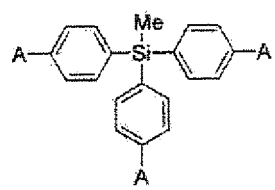 | 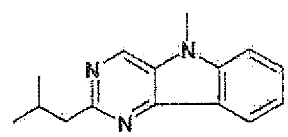 |
| 174 | 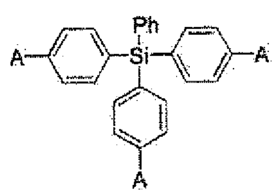 | 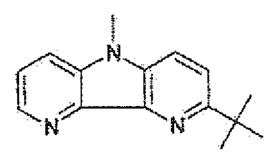 |
| 175 | 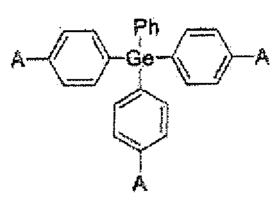 | 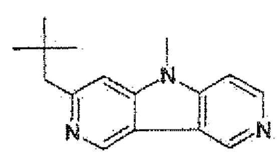 |

| Compound | Central Structure | A |
|---|---|---|
| 176 | 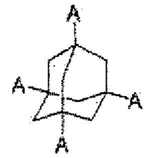 | 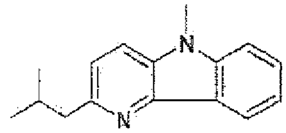 |
| 177 | 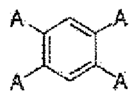 | 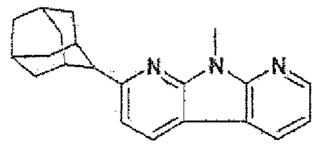 |
| 178 | 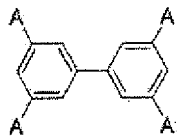 | 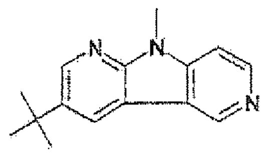 |
| 179 | 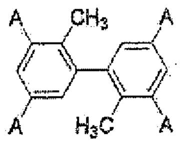 | 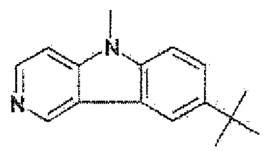 |
| 180 | 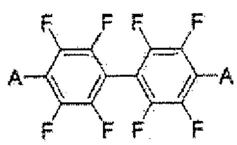 | 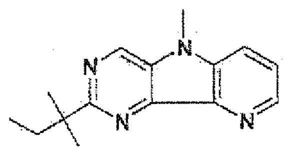 |
| 181 | 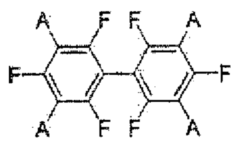 | 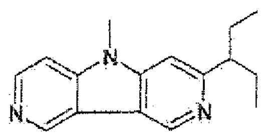 |
| 182 | 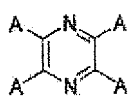 | 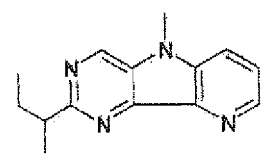 |
| 183 | 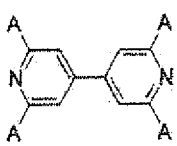 | 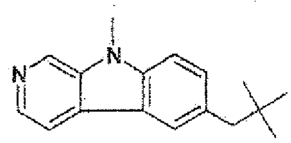 |
| 184 | 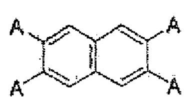 | 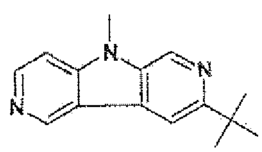 |

| Compound | Central Structure | A |
|---|---|---|
| 185 | 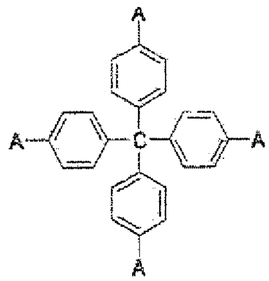 | 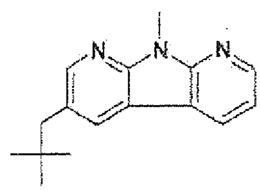 |
| 186 | 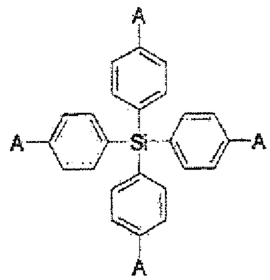 | 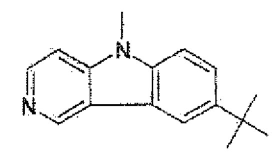 |
| 187 | 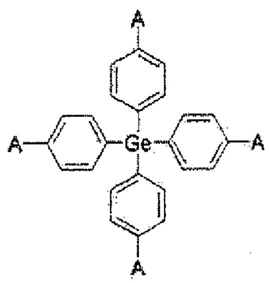 | 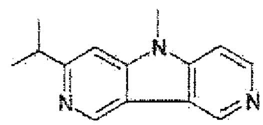 |
| 188 | 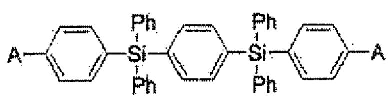 | 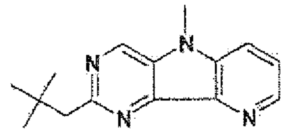 |
| 189 | 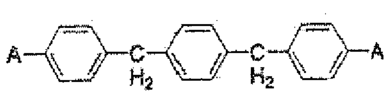 | 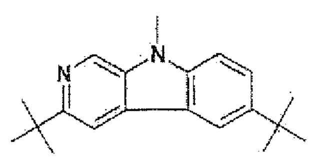 |
| 190 | 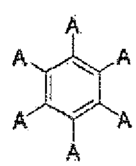 | 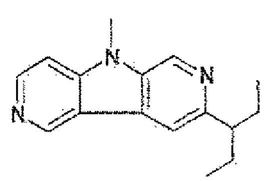 |

| Compound | Central Structure | A |
|---|---|---|
| 191 | 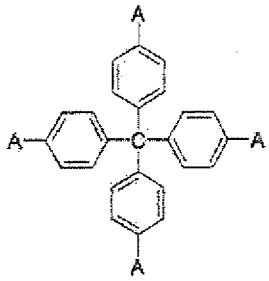 | 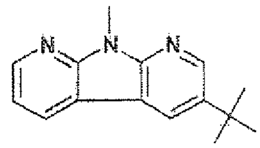 |
| 192 | 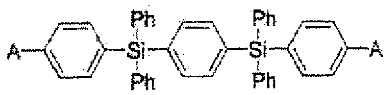 | 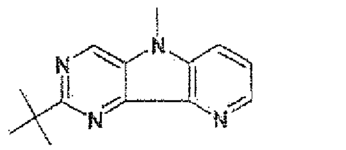 |
| 193 | 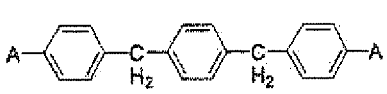 | 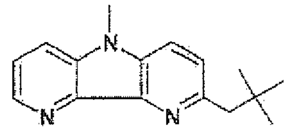 |
| 194 | 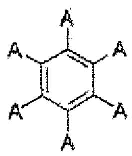 | 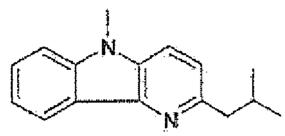 |

| Compound | Central Structure | A |
|---|---|---|
| 195 | 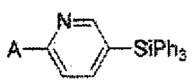 | 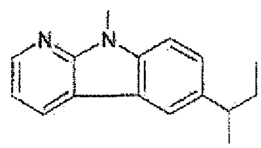 |
| 196 | 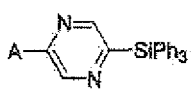 | 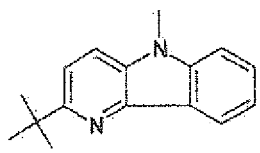 |
| 197 | 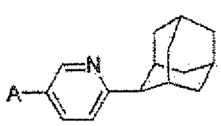 | 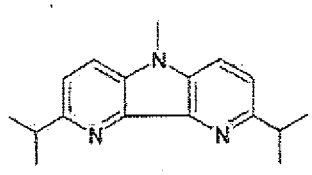 |
| 198 | 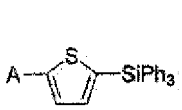 | 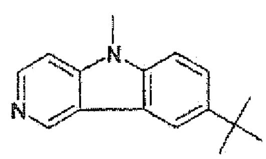 |

(continued)

| Compound | Central Structure | A |
|---|---|---|
| 199 | 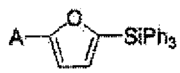 | 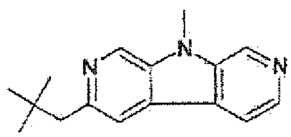 |
| 200 | 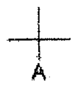 | 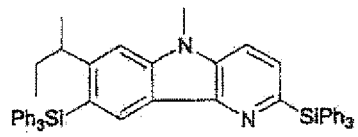 |
| 201 | 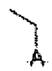 | 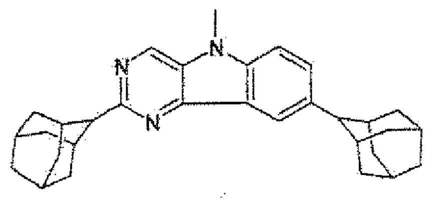 |
| 202 | 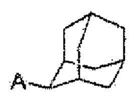 | 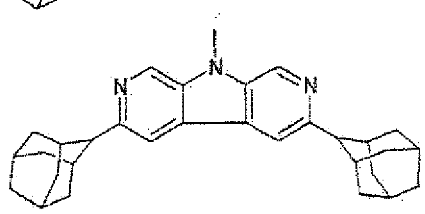 |

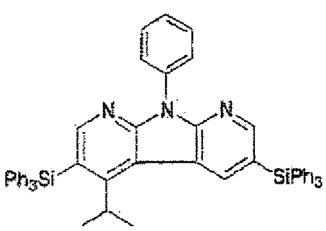

203

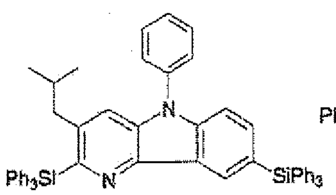

204

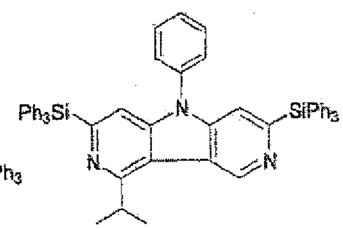

205

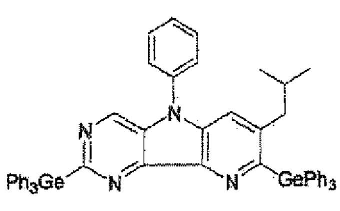

206

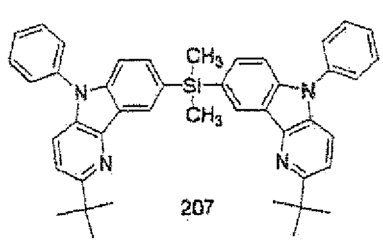

207

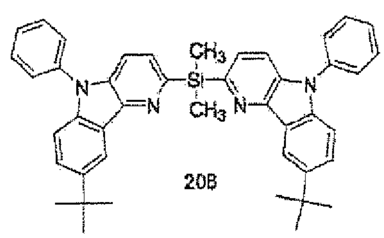

208

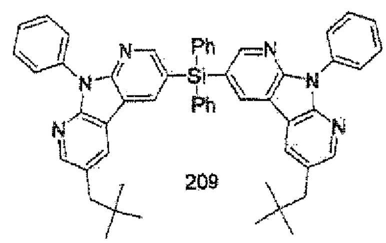

209

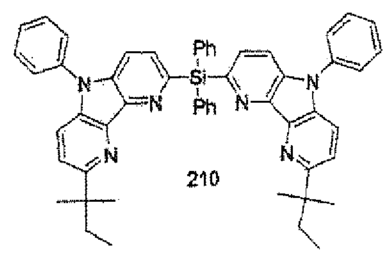

210

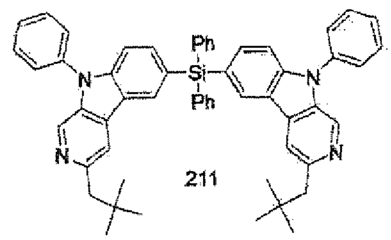

211

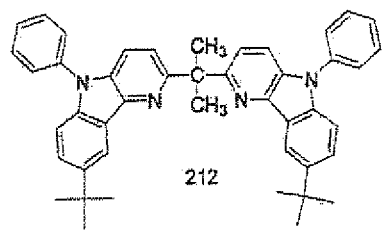

212

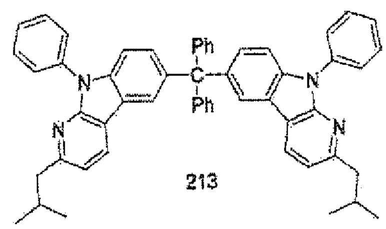

213

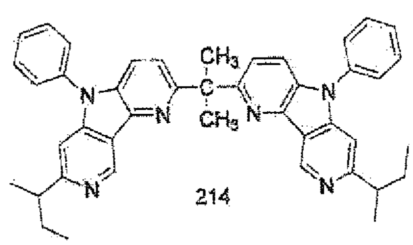

214

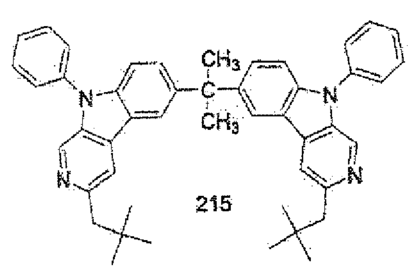

215

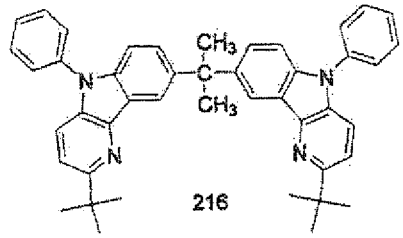

216

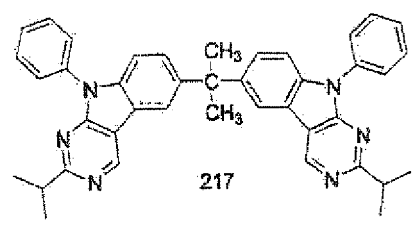

217

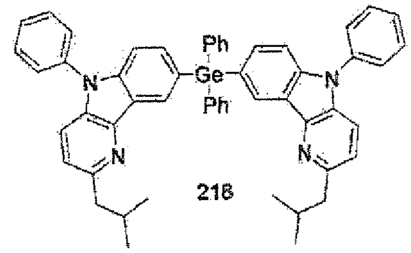

218

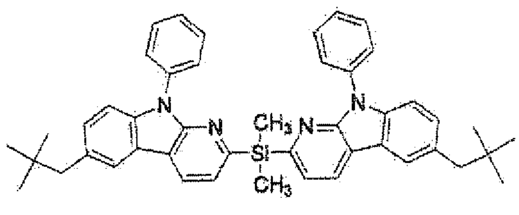

219

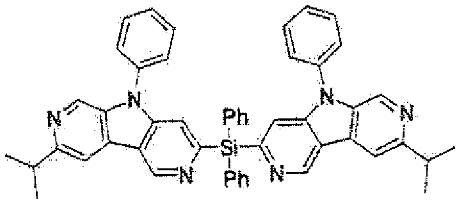

220

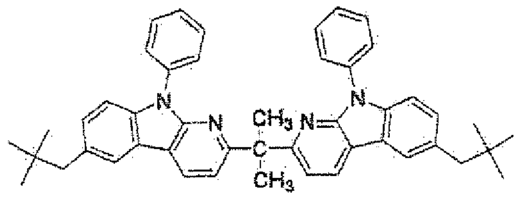

221

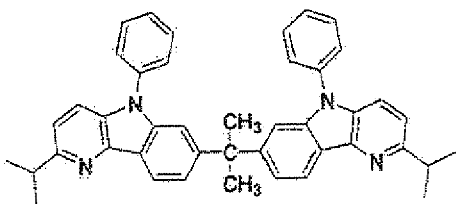

222

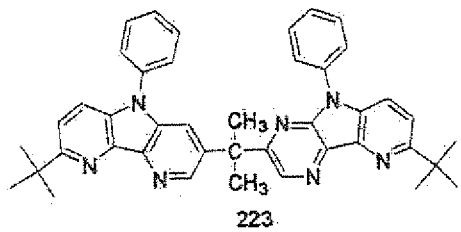

223

| Compound | Central Structure | A |
|---|---|---|
| 224 | 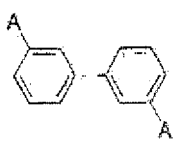 | 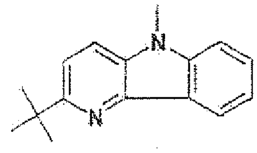 |
| 225 | 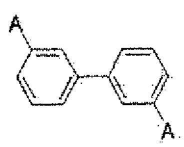 | 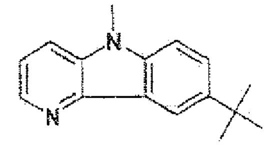 |
| 226 | 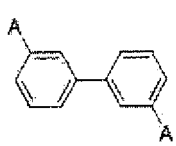 | 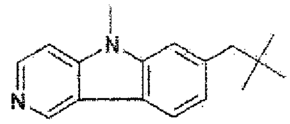 |

(continued)

| Compound | Central Structure | A |
|---|---|---|
| 227 | 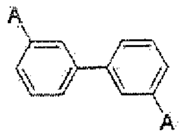 | 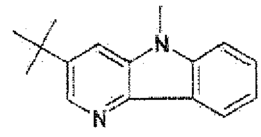 |
| 228 | 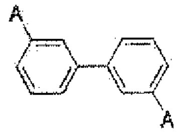 | 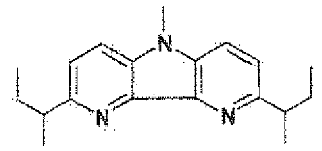 |
| 229 | 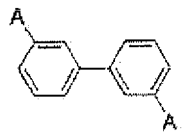 | 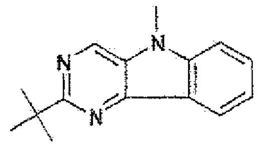 |
| 230 | 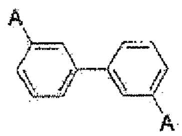 | 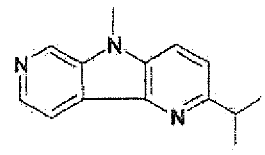 |
| 231 | 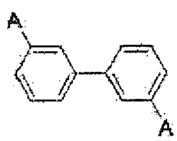 | 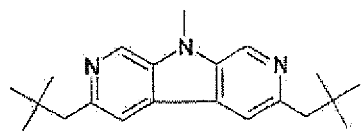 |
| 232 | 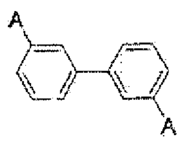 | 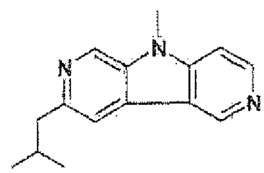 |

| Compound | Central Strtucture | A | B |
|---|---|---|---|
| 233 | 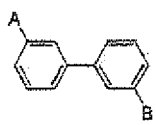 | 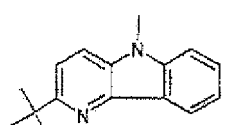 | 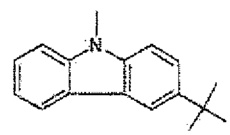 |
| 234 | 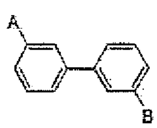 | 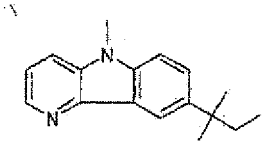 | 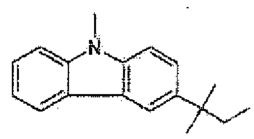 |
| 235 | 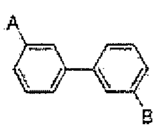 | 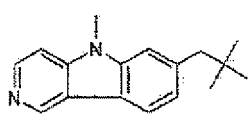 | 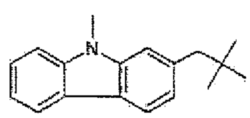 |

(continued)

| Compound | Central Strtucture | A | B |
|---|---|---|---|
| 236 | 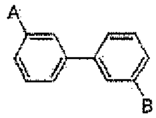 | 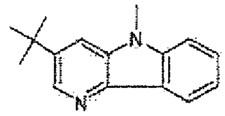 | 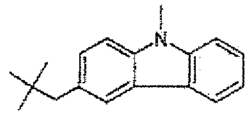 |
| 237 | 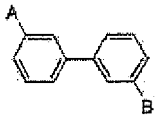 | 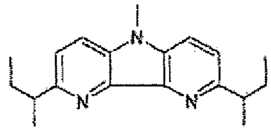 | 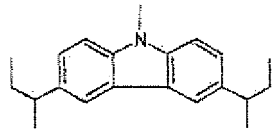 |
| 238 | 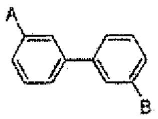 | 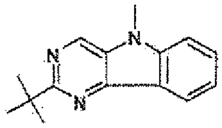 | 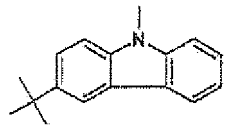 |
| 239 | 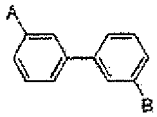 | 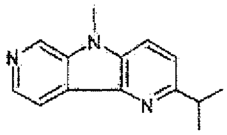 | 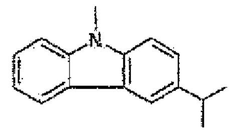 |
| 240 | 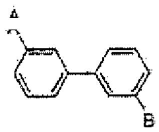 | 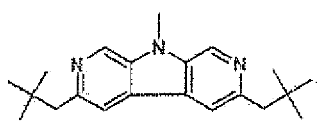 | 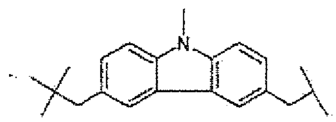 |
| 241 | 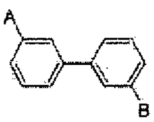 | 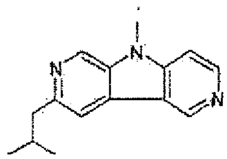 | 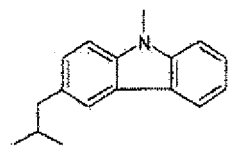 |

The compounds represented by Formulas (1) to (16) may be synthesized by various known synthesis methods such as, for example, J. Chem. Soc., Perkin Trains. 1, 1505-1510(1999), J. Org. Chem., 7832-7838(1933), Tetrahedron, 49-64(1993), and the like.

A glass transition temperature of the compound represented by Formula. (1) (the compound having at least one group represented by any one of Formulas (4-1) to (4-10) and Formulas (7-1) to (7-10) and the like) is preferably 130˚C or more to 450˚C or less, more preferably 140˚C or more to 450˚C or less, and even more preferably 160˚C or more to 450˚C or less. If the glass transition temperature is within the range, it is preferred in that thermal resistance and durability of the device may be improved.

In the present invention, the compound represented by Formula (1) (for example, the compound having at least one group represented by any one of Formulas (4-1) to (4-10)) is not limited from the viewpoint of a use, and may be contained in any layer in the organic layer. It is preferred that a introduction layer of the compound represented by Formula (1) may be included in any one of a light emitting layer, a hole injection layer, a hole transporting layer, an electron transporting layer, an electron injection layer, an exciton blocking layer, and a charge blocking layer, or a plurality thereof

In the present invention, in order to further suppress a change in chromaticity when driving at a high temperature, it is preferred that the compound represented by Formula (1) (for example, the compound having at least one group represented by any one of Formulas (4-1) to (4-10) or the compound having at least one group represented by any one of Formulas (7-1) to (7-10)) is contained in any of the light emitting layer or layers adjacent to the light emitting layer. Further, the compound represented by Formula (1) may be contained in both layers of the light emitting layer and the layer adjacent to the light emitting layer.

When the compound represented by Formula (1) is contained in the light emitting layer, the compound represented by Formula (1) has preferably 0.1 to 99% by mass, more preferably 1 to 95% by mass, and even more preferably 10 to

95% by mass based on the total mass of the light emitting layer.

When the compound represented by Formula (1) is contained in the layer adjacent to the light emitting layer, the compound represented by Formula (1) has preferably 0.1 to 100% by mass, more preferably 1 to 95% by mass, even more preferably 10 to 100% by mass, and particularly preferably 50 to 100% by mass based on the total mass of the light emitting layer.

The organic electroluminescent device including the phosphorescent metal complex containing mono-anionic bidentate ligands represented by Formulas (A1) to (A4) and a metal having an atom weight of 40 or more and the compound represented by Formula (1) (for example, the compound having at least one group represented by any one of Formulas (4-1) to (4-10) or the compound having at least one group represented by any one of Formulas (7-1) to (7-10)) may include a laminated film laminating a thin film containing the phosphorescent metal complex containing mono-anionic bidentate ligands represented by Formulas (A1) to (A4) and a metal having a atom weight of 40 or more and a thin film containing the compound represented by Formula (1). By the laminated film, it is possible to provide an organic electroluminescent device having excellent light emission efficiency and small dependence on light emission efficiency due to a film thickness.

Meanwhile, among Formulas of ligands in the present invention, * means a coordinating site to a metal and each of a bond of $E_{1a}$ and a metal and a bond of $E_{1p}$ and a metal independently may be a covalent bond or a coordinate bond.

Hereinafter, bidentate ligands represented by Formulas (A1) to (A4) will be described.

[Bidentate ligands represented by Formulas (A1) to (A4)]

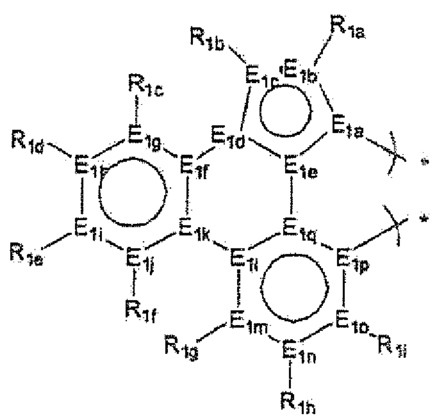

A1

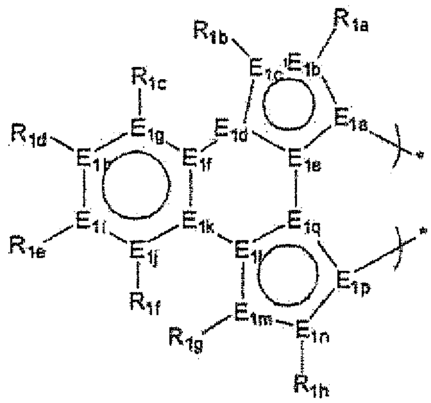

A2

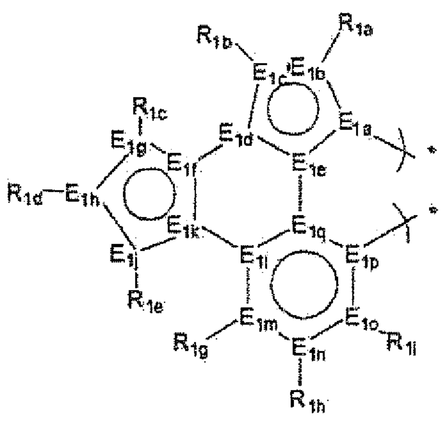

A3

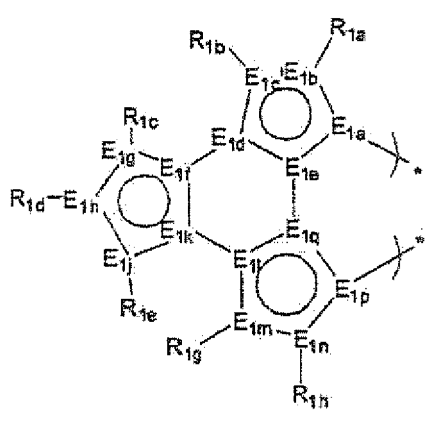

A4

In Formulas (A1) to (A4), each of $E_{1a}$ to $E_{1q}$ independently represents a carbon atom or a heteroatom. Each of $R_{1a}$ to $R_{1i}$ independently represents a hydrogen atom or a substituent. Each of structures represented by Formulas (A1) to (A4) totally has an 18π electron structure.

The bidentate ligands may form tridentate, tetradentate, pentadentate and hexadentate ligands by bonding with other ligands.

A molecular weight of the phosphorescent metal complex containing the mono-anionic bidentate ligands represented by Formulas (A1) to (A4) is preferably 400 or more to 1000 or less, more preferably 450 or more to 800 or less, and even more preferably 500 or more to 700 or less.

$E_{1a}$ to $E_{1q}$ are selected from carbon atoms or heteroatoms, and preferably carbon atoms or nitrogen atoms. Further, $E_{1a}$ and $E_{1q}$ are preferably different atoms. Further, the metal complex has an 18π electron structure.

Rings formed by $E_{1a}$ to $E_{1e}$ represent 5-membered heterocyclic rings and particularly, include oxazole, thiazole, isoxazole, isothiazole, pyrrole, imidazole, pyrazole, triazole, tetrazole and the like. The ring includes preferably imidazole or pyrazole, and more preferably imidazole.

Each of rings formed by $E_{1f}$ to $E_{1k}$ and. $E_{1l}$ to $E_{1q}$ is independently selected from a 6-membered aromatic hydrocarbon ring and a 5- or 6-membered heterocyclic ring, and may include, for example, benzene, oxazole, thiazole, isoxazole, isothiazole, oxadiazole, thiadiazole, furan, thiophene, pyrrole, imidazole, pyrazole, triazole, pyridine, pyrazine, pyrimidine, pyridazine, triazine and the like.

Each of $R_{1a}$ to $R_{1i}$ independently selected from a group Z of substituents as described below, and preferably a hydrogen atom, a hydrocarbon substituent, a cyano group, a fluoro group, $OR_{2a}$, $SR_{2a}$, $NR_{2a}R_{2b}$, $BR_{2a}R_{2b}$ or $SiR_{2a}R_{2b}R_{2c}$. Each of $R_{2a}$ to $R_{2c}$ independently represents a hydrocarbon substituent or a hydrocarbon substituent substituted with a heteroatom, and may form a saturated or unsaturated aromatic ring or non-aromatic ring by bonding two of $R_{1a}$ to $R_{1i}$ and $R_{2a}$ to $R_{2c}$ with each other. In the case of bonding with a nitrogen atom, $R_{1a}$ to $R_{1i}$ are not hydrogen atoms.

The heteroatom represents an atom other than a carbon atom or a hydrogen atom. An example of the heteroatom may include oxygen, nitrogen, phosphorus, sulfur, selenium, arsenic, chlorine, bromine, silicon or fluorine.

Particular examples of the group Z of substituents may particularly include an alkyl group, an alkenyl group, an alkynyl group, an aryl group, a heteroaryl group, an amino group, an alkoxy group, an aryloxy group, a heterocyclic oxy group, an acyl group, an alkoxycarbonyl group, an aryloxycarbonyl group, an aryloxy group, an acylamino group, an alkoxycarbonylamino group, an aryloxycarbonylamino group, an sulfonylamino group, a sulfamoyl group, a carbamoyl group, an alkylthio group, an arylthio group, a heteroarylthio group, a sulfonyl group, a sulfinyl group, an ureido group, a phosphoric acid amide group, hydroxyl group, a mercapto group, a halogen atom, a cyano group, a sulfo group, a carboxyl group, a nitro group, a hydroxamic acid group, a sulfino group, a hydrazino group, an imino group, a heterocyclic group other than a heteroaryl group, a silyl group, a silyloxy group, a heavy hydrogen atom and the like. Those substituents may further be substituted with other substituents.

Herein, the alkyl group may have preferably 1 to 30 carbon atoms, more preferably 1 to 20 carbon atoms, and particularly preferably 1 to 10 carbon atoms, and for example, may include methyl, ethyl, n-propyl, isopropyl, n-butyl, tert-butyl, n-octyl, n-nonyl, n-decyl, n-dodecyl, n-octadecyl, n-hexadecyl, cyclopropyl, cyclobutyl, cyclopentyl, cyclohexyl, cyclooctyl, 1-adamantyl, trifluoromethyl and the like.

Further, the alkenyl group may have preferably 2 to 30 carbon atoms, more preferably 2 to 20 carbon atoms, and particularly preferably 2 to 10 carbon atoms, and for example, may include vinyl, aryl, 1-prophenyl, 1-isoprophenyl, 1-butenyl, 2-butenyl, 3-pentenyl and the like.

Further, the alkynyl group may have preferably 2 to 30 carbon atoms, more preferably 2 to 20 carbon atoms, and particularly preferably 2 to 10 carbon atoms, and for example, may include ethynyl, propargyl, 1-propynyl, 3-pentynyl and the like.

The aryl group represents an aromatic hydrocarbon monoradical. In the case where the aryl group is substituted, the substituent may include preferably a fluoro group, a hydrocarbon substituent, a hydrocarbon substituent substituted with a heteroatom, a cyano group, and the like. The aryl group may have preferably 6 to 30 carbon atoms, more preferably 6 to 20 carbon atoms, and particularly preferably 6 to 12 carbon atoms, and for example, may include phenyl, o-methylphenyl, m-methylphenyl, p-methylphenyl, 2,6-xylyl, p-cumenyl, mesityl, naphthyl, anthranyl and the like.

The hetero aryl group represents an aromatic heterocyclic monoradical. In the case where the hetero aryl group is substituted, the substituent may include preferably a fluoro group, a hydrocarbon substituent, a hydrocarbon substituent substituted with a heteroatom, a cyano group and the like. The heterocyclic group may include, for example, imidazolyl, pyrazolyl, pyridyl, pyrazyl, pyrimidyl, triazynyl, quinolyl, isoquinolinyl, pyrrolyl, indolyl, furyl, thienyl, benzoxazolyl, benzoimidazolyl, benzothiazolyl, carbazolyl, azepinyl and the like.

Further, the amino group may have preferably 0 to 30 carbon atoms, more preferably 0 to 20 carbon atoms, and particularly preferably 0 to 10 carbon atoms, and for example, may include amino, methylamino, dimethylamino, diethylammo, dibenzylamino, diphenylamino, ditolyiamino and the like.

Further, the alkoxy group may have preferably 1 to 30 carbon atoms, more preferably 1 to 20 carbon atoms, and particularly preferably 1 to 10 carbon atoms, and for example, may include methoxy, ethoxy, butoxy, 2-ethylhexyloxy and the like.

Further, the aryloxy group may have preferably 6 to 30 carbon atoms, more preferably 6 to 20 carbon atoms, and particularly preferably 6 to 12 carbon atoms, and for example, may include phenyloxy, 1-naphthyloxy, 2-naphthyloxy

and the like.

Further, the heterocyclic oxy group may have preferably 1 to 30 carbon atoms, more preferably 1 to 20 carbon atoms, and particularly preferably 1 to 12 carbon atoms, and for example, may include pyridyloxy, pyrazyloxy, pyrimidyloxy, quinolyloxy and the like.

Further, the acyl group may have preferably 2 to 30 carbon atoms, more preferably 2 to 20 carbon atoms, and particularly preferably 2 to 12 carbon atoms, and for example, may include acetyl, benzoyl, formyl, pivaloyl and the like.

Further, the alkoxylcarbonyl group may have preferably 2 to 30 carbon atoms, more preferably 2 to 20 carbon atoms, and particularly preferably 2 to 12 carbon atoms, and for example, may include methoxycarbonyl, ethoxycarbonyl and the like.

Further, the aryloxycarbonyl group may have preferably 7 to 30 carbon atoms, more preferably 7 to 20 carbon atoms, and particularly preferably 7 to 12 carbon atoms, and for example, may include phenyloxycarbonyl and the like.

Further, the acyloxy group may have preferably 2 to 30 carbon atoms, more preferably 2 to 20 carbon atoms, and particularly preferably 2 to 10 carbon atoms, and for example, may include acetoxy, benzoyloxy and the like.

Further, the acylamion group may have preferably 2 to 30 carbon atoms, more preferably 2 to 20 carbon atoms, and particularly preferably 2 to 10 carbon atoms, and for example, may include acetylamino, benzoylamino and the like.

Further, the alkoxycarbonylamino group may have preferably 2 to 30 carbon atoms, more preferably 2 to 20 carbon atoms, and particularly preferably 2 to 12 carbon atoms, and for example, may include methoxycarbonylamino and the like.

Further, the aryloxycarbonylamino group may have preferably 7 to 30 carbon atoms, more preferably 7 to 20 carbon atoms, and particularly preferably 7 to 12 carbon atoms, and for example, may include phenyloxycarbonylamino and the like.

Further, the sulfonylamino group may have preferably 1 to 30 carbon atoms, more preferably 1 to 20 carbon atoms, and particularly preferably 1 to 12 carbon atoms, and for example, may include methansulfonylamino, benzenesulfonylamino and the like.

Further, the sulfamoyl group may have preferably 0 to 30 carbon atoms, more preferably 0 to 20 carbon atoms, and particularly preferably 0 to 12 carbon atoms, and for example, may include sulfamoyl, methylsulfamoyl, dimethylsulfamoyl, phenylsulfamoyl and the like.

Further, the carbamoyl group may have preferably 1 to 30 carbon atoms, more preferably 1 to 20 carbon atoms, and particularly preferably 1 to 12 carbon atoms, and for example, may include carbamoyl, methylcarbamoyl, diethylcarbamoyl, phenylcarbamoyl and the like.

Further, the alkylthio group may have preferably 1 to 30 carbon atoms, more preferably 1 to 20 carbon atoms, and particularly preferably 1 to 12 carbon atoms, and for example, may include methylthio, ethylthio and the like.

Further, the arylthio group may have preferably 6 to 30 carbon atoms, more preferably 6 to 20 carbon atoms, and particularly preferably 6 to 12 carbon atoms, and for example, may include phenylthio and the like.

Further, the heteroarylthio group may have preferably 1 to 30 carbon atoms, more preferably 1 to 20 carbon atoms, and particularly preferably 1 to 12 carbon atoms, and for example, may include pyridylthio, 2-benzoimidazolylthio, 2-benzooxazolylthio, 2-benzothiazolylthio and the like.

Further, the sulfonyl group may have preferably 1 to 30 carbon atoms, more preferably 1 to 20 carbon atoms, and particularly preferably 1 to 12 carbon atoms, and for example, may include mesyl, tosyl, trifluoromethanesulfonyl and the like.

Further, the sulfinyl group may have preferably 1 to 30 carbon atoms, more preferably 1 to 20 carbon atoms, and particularly preferably 1 to 12 carbon atoms, and for example, may include methanesulfinyl, benzenesulfinyl and the like.

Further, the ureido group may have preferably 1 to 30 carbon atoms, more preferably 1 to 20 carbon atoms, and particularly preferably 1 to 12 carbon atoms, and for example, may include ureido, methylureido, phenylureido and the like.

Further, the phosphoric acid amide group may have preferably 1 to 30 carbon atoms, more preferably 1 to 20 carbon atoms, and particularly preferably 1 to 12 carbon atoms, and for example, may include diethylphosphoric acid amide, phenylphosphoric acid amide and the like.

Further, the halogen atom may include, for example, a fluorine atom, a chlorine atom, a bromine atom, an iodine atom and the like.

Further, the heterocyclic group other than the hetero aryl group may have preferably 1 to 30 carbon atoms and more preferably 1 to 12 carbon atoms, and the heteroatom may include, for example, a nitrogen atom, an oxygen atom, a sulfur atom, and particularly, for example, piperidyl, morpholino, pyrrolidyl and the like.

Further, the silyl group may have preferably 3 to 40 carbon atoms, more preferably 3 to 30 carbon atoms, and particularly preferably 3 to 24 carbon atoms and for example, may include trimethylsilyl, triethylsilyl, triisopropylsilyl, dimethyl-tert-butylsilyl, dimethylphenylsilyl, diphenyl-tert-butylsilyl, triphenylsilyl, tri-1-naphthylsilyl, tri-2-naphthylsilyl and the like.

Further, the silyloxy group may have preferably 3 to 40 carbon atoms, more preferably 3 to 30 carbon atoms, and particularly preferably 3 to 24 carbon atoms, and for example, may include trimethylsilyloxy, triphenylsilyloxy and the like.

At least one of $R_{1a}$ to $R_{1i}$ is preferably an aryl group which has a dihedron of 70 degrees or more with respect to a parent structure, more preferably a substituent represented by the following Formula ss-1, and even more preferably a 2,6-disubstituted aryl group, and most preferably, $R_{1b}$ is a 2,6-disubstituted aryl group.

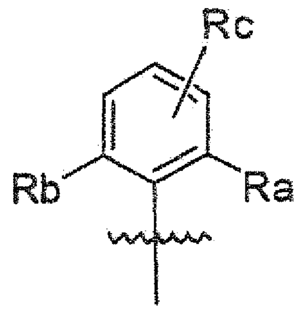

ss-1

In Formula ss-1, each of Ra, Rb and Rc independently represents any one of a hydrogen atom, an alkyl group and an aryl group.

Alkyl groups represented by Ra, Rb and Rc may have preferably 1 to 30 carbon atoms, more preferably 1 to 20 carbon atoms, and particularly preferably 1 to 10 carbon atoms, and for example, may include methyl, ethyl, n-propyl, isopropyl, n-butyl, tert-butyl, n-octyl, n-nonyl, n-decyl, n-dodecyl, n-octadecyl, n-hexadecyl, cyclopropyl, cyclobutyl, cyclopentyl, cyclohexyl, cyclooctyl, 1-adamantyl, trifluoromethyl and the like, and preferably a methyl group or an isopropyl group.

Aryl groups represented by Ra, Rb and Rc may have preferably 6 to 30 carbon atoms, more preferably 6 to 20 carbon atoms, and particularly preferably 6 to 12 carbon atoms, and for example, may include phenyl, o-methylphenyl, m-methylphenyl, p-methylphenyl, 2,6-xylyl, p-cumenyl, mesityl, naphthyl, anthranyl and the like, and preferably a phenyl group.

At least one of Ra and Rb is selected from an aLkyl group or an aryl group, at least one of Ra and Rb is preferably selected from an alkyl group, both of Ra and Rb are preferably alkyl groups, and both of Ra and Rb are most preferably methyl groups or isopropyl groups.

The 2,6-disubstituted aryl group includes preferably, a 2,6-dimethylphenyl group, a 2,4,6-trimethylphenyl group, a 2,6-diisopropylphenyl group, a 2,4,6-triisopropylphenyl group, a 2,6-dirraethyl-4-phenylphenyl group, a 2,6-dimethyl-4-(2,6-dimethylpyridine-4-yl)phenyl group, a 2,6-diphenylphenyl group, a 2,6-diphenyl-4-isopropylphenyl group, a 2,4,6-triphenylphenyl group, a 2,6-diisopropyl-4-(4-isopropylphenyl)phenyl group, a 2,6-diisopropyl-4-(3,5-dimethylphenyl)phenyl group, a 2,6-diisopropyl-4-(pyridine-4-yl)phenyl group or a 2,6-di-(3,5-dimethylphenyl)phenyl group.

Meanwhile, at least one of $R_{1a}$ to $R_{1i}$ is preferably an alkyl group, and $R_{1e}$ is more preferably an alkyl group. It is preferred that the alkyl group is an alkyl group branched in a moiety distanced from a benzyl moiety, which is composed of 4 or more carbon atoms.

Meanwhile, it is preferred that at least one of $R_{1a}$ and $R_{1b}$ is an alkyl group.

Meanwhile, $R_{1a}$ is preferably an electron donating substituent, and more preferably a methyl group.

The hydrocarbon substituent means a monovalent or divalent and chained, branched or cyclic substituent, and represents a substituent composed only of a carbon atom and a hydrogen atom. Examples of the monovalent hydrocarbon substituent may include an alkyl group having 1 to 20 carbon atoms; an alkyl group having 1 to 20 carbon atoms substituted with one or more group selected from an alkyl group having 1 to 20 carbon atoms, a cycloalkyl group having 3 to 8 carbon atoms, and an aryl group; a cycloalkyl group having 3 to 8 carbon atoms; a cycloalkyl group having 3 to 8 carbon atoms substituted with one or more group selected from an alkyl group having 1 to 20 carbon atoms, a cycloalkyl group having 3 to 8 carbon atoms, and an aryl group; an aryl group having 6 to 18 carbon atoms; an aryl group atoms substituted with one or more group selected from an alkyl group having 1 to 20 carbon atoms, a cycloalkyl group having 3 to 8 carbon atoms, and an aryl group; and the like.

The divalent hydrocarbon group may be, for example, a $-CH_2-$, $-CH_2CH_2-$, $-CH_2CH_2CH_2-$, an 1,2-phenylene group and the like.

A metal is selected from metals having nonradioactivity and also an atom weight of 40 or more, and includes

preferably any one of Re, Ru, Os, Rh, Ir, Pd, Pt, Cu or Au, more preferably Os, Ir or Pt, even more preferably Ir or Pt, and most preferably Ir, from the viewpoint of high light emission efficiency, high complex stability, and carrier balance control of transport of holes and electrons in the light emitting layer.

In the present invention, the metal complex composed of a ligand in Formula may be constituted by a combination of a main ligand and a tautomer thereof and a sub-ligand or a tautomer thereof, or may also be constituted by only a partial structure in which when n is 0, that is, all the ligands of the metal complex are represented by the main ligands or the tautomers thereof.

Further, a ligand (also referred to as a coordinated compound) known by those skilled in the art as a called ligand which is used for forming a known metal complex in the related art may be included as a sub-ligand, as necessary.

From the viewpoint of acquiring effects disclosed in the present invention, ligands in the complex is constituted by preferably 1 to 2 kinds, and more preferably one kind. When a reactive group is introduced in the complex molecule, from the viewpoint of easy synthesis, it is preferred that the ligand is also composed of two kinds.

The ligand used in the known metal complex in the related art includes various known ligands, and may include, for example, ligands (for example, a halogen ligand (preferably a chlorine ligand), a nitrogen-containing heteroaryl ligand (for example, bipyridyl, phenanthroline and the like), and a diketone ligand (for example, acetylacetone and the like)) which are disclosed in "Photochemistry and Photophysics of Coordination Compounds" copyrighted by Springer-Verlag Co., Ltd., written by H Yersin, and published in 1987, "Organic Metal Chemisty - Basis and Application -" copyrighted by SHOKABO PUBLISHING Co., Ltd., written by Yamamoto Archio, and published in 1982, and the like. The ligand is preferably diketones or picoline acid derivatives.

Hereinafter, examples of the sub-ligand are described in detail, but the present invention is not limited thereto.

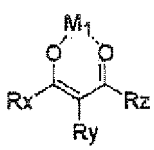
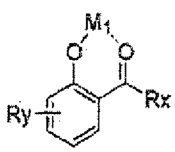
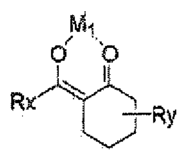
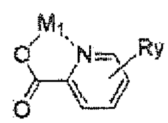
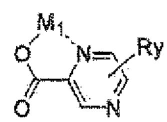

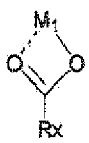
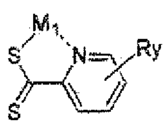
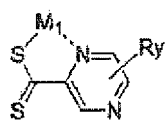
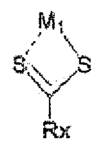
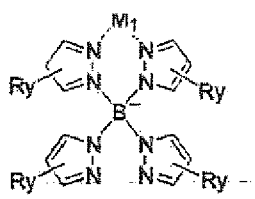

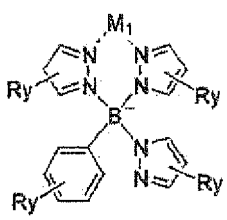
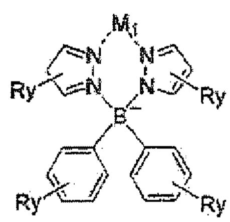
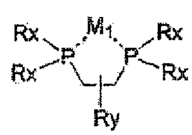
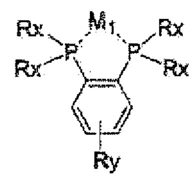

In the example of the sub-ligand, $M_1$ represents a metal atom having an atom weight of 40 or more. Each of Rx, Ry and Rz independently represents a hydrogen atom or a substituent. As the substituent, those exemplified above for the group Z of substituents may be included.

It is preferred that the mono-anionic bidentate ligands represented by Formulas (A1) to (A4) are a mono-anionic bidentate ligand represented by Formula (A1) or Formula (A3).

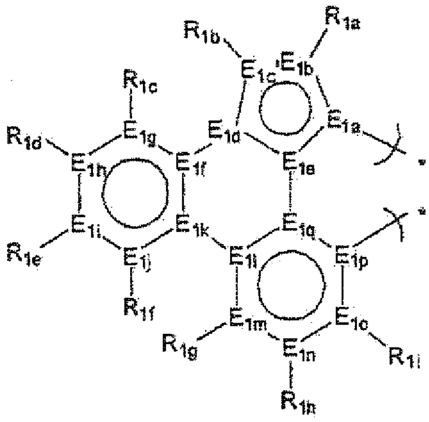

(A1)

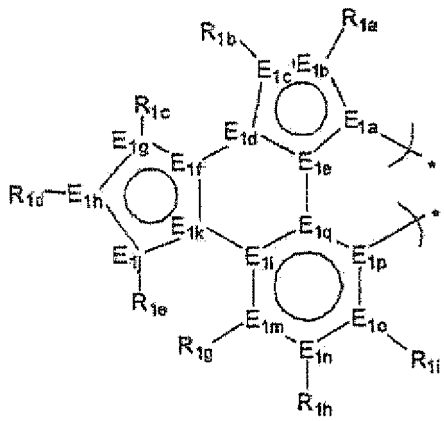

(A3)

In Formulas (A1) and (A3), each of $E_{1a}$ to $E_{1q}$ independently represents a carbon atom or a heteroatom. Each of $R_{1a}$ to $R_{1i}$ independently represents a hydrogen atom or a substituent. Each of structures represented by Formulas (A1) and (A3) totally has an 18π electron structure.

It is preferred that the mono-anionic bidentate ligands represented by Formulas (A1) and (A3) are a mono-anionic bidentate ligand represented by Formula (A1-1) or Formula (A3-1).

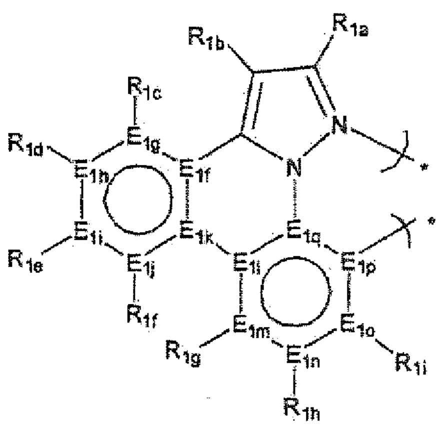

(A1-1)

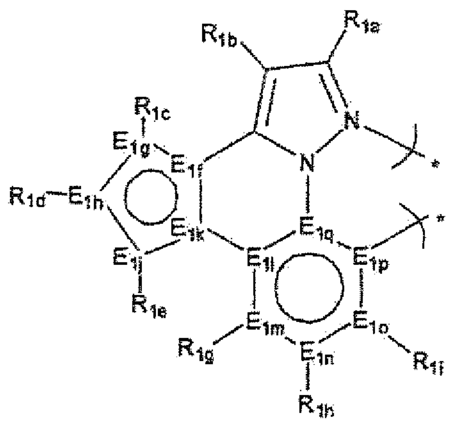

(A3-1)

In Formulas (A1-1) and (A3-1), each of $E_{1f}$ to $E_{1q}$ independently represents a carbon atom or a heteroatom. Each of $A_{1a}$ to $R_{1i}$ independently represents a hydrogen atom or a substituent. Each of structures represented by Formulas (A1-1) and (A3-1) totally has an 18π electron structure.

In Formulas (A1-1) and (A3-1), the definitions of $E_{1f}$ to $E_{1q}$ and $R_{1a}$ to $R_{1i}$ are the same as those of $E_{1f}$ to $E_{1q}$ and $R_{1a}$ to $R_{1i}$ in Formulas (A1) and (A3), and preferable values thereof are the same.

It is preferred that the mono-anionic bidentate ligands represented by Formulas (A1-1) and (A3-1) are a mono-anionic bidentate ligand represented by Formula (A1-2) or Formula (A3-2).

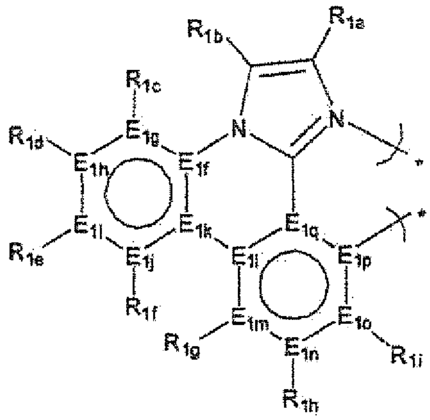

(A1-2)

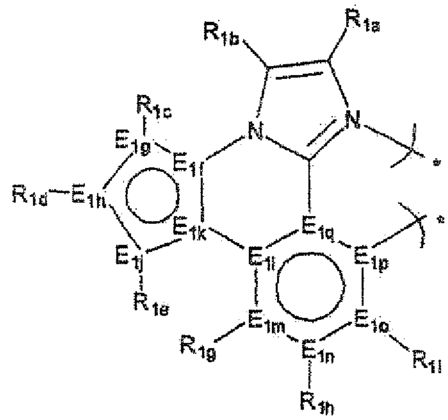

(A3-2)

In Formulas (A1-2) and (A3-2), each of $E_{1f}$ to $E_{1q}$ independently represents a carbon atom or a heteroatom. Each of $R_{1a}$ to $R_{1i}$ independently represents a hydrogen atom or a substituent. Each of structures represented by Formulas (A1-2) and (A3-2) totally has an 18$\pi$ electron structure.

In Formulas (A1-2) and (A3-2), the definitions of $E_{1f}$ to $E_{1q}$ and $R_{1a}$ to $R_{1i}$ are the same as those of $E_{1f}$ to $E_{1q}$ and $R_{1a}$ to $R_{1i}$ in Formulas (A1-1) and (A3-1), and preferable values thereof are the same.

It is preferred that the mono-anionic bidentate ligands represented by Formulas (A1-1) and (A3-1) are a mono-anionic bidentate ligand represented by Formula (A1-3) or Formula (A3-3).

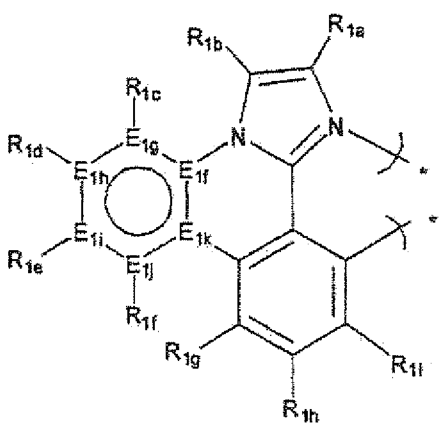

(A1-3)

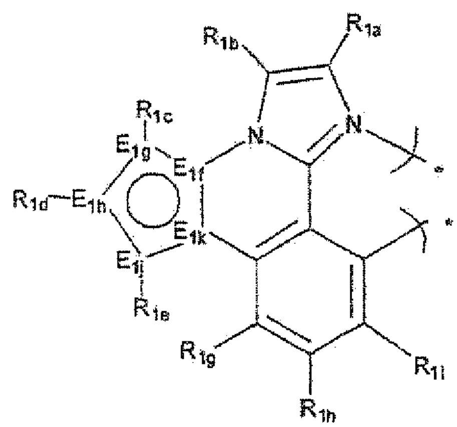

(A3-3)

In Formulas (A1-3) and (A3-3), each of $E_{1f}$ to $E_{1k}$ independently represents a carbon atom or a heteroatom. Each of $R_{1a}$ to $R_{1i}$ independently represents a hydrogen atom or a substituent. Each of structures represented by Formulas (A1-3) and (A3-3) totally has an 18$\pi$ electron structure.

In Formulas (A1-3) and (A3-3), the definitions of $E_{1f}$ to $E_{1k}$ and $R_{1a}$ to $R_{1i}$ are the same as those of $E_{1f}$ to $E_{1k}$ and $R_{1a}$ to $R_{1i}$ in Formulas (A1-1) and (A3-1), and preferable values thereof are the same.

The mono-anionic bidentate ligand represented by Formula (A1-3) or Formula (A3-3) is preferably a mono-anionic bidentate ligand represented by Formula (A1-3) and more preferably a mono-anionic bidentate ligand represented by Formula (A1-4).

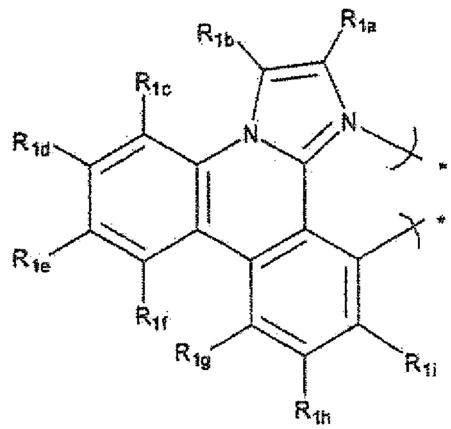

(A1-4)

In Formula (A1-4), each of $R_{1a}$ to $R_{1i}$ independently represents a hydrogen atom or a substituent.

In Formula (A1-4), the definitions of $R_{1a}$ to $R_{1i}$ are the same as those of $R_{1a}$ to $R_{1i}$ in Formula (A1-1), and preferable values thereof are the same.

The phosphorescent metal complex containing the mono-anionic bidentate ligands represented by Formulas (A1-3) and (A3-3) and the metal having an atom weight of 40 or more is preferably an iridium complex represented by Formula (A9).

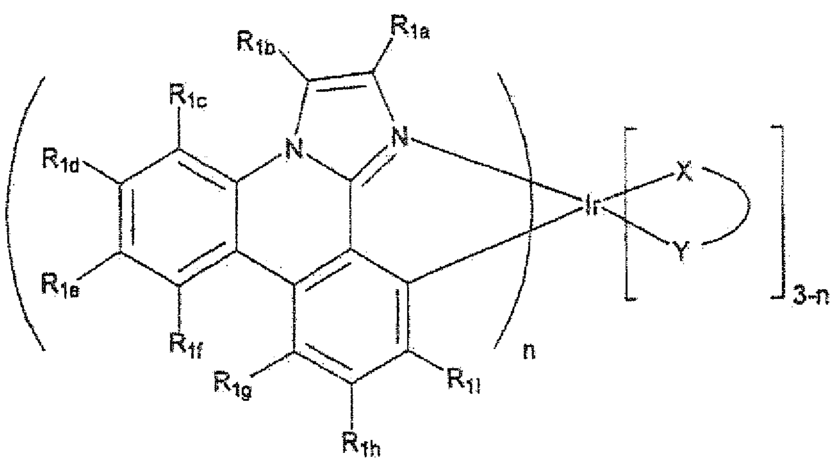

In Formula (A9), each of $R_{1a}$ to $R_{1i}$ independently represents a hydrogen atom or a substituent. X-Y represents a mono-anionic bidendate ligand. n represents an integer of 1 to 3.

In Formula (A9), the definition of $R_{1a}$ to $R_{1i}$ are the same as those of $R_{1a}$ to $R_{1i}$ in Formulas (A1-3) and (A3-3), each of $R_{1a}$ to $R_{1i}$ is independently selected from the aforementioned group Z of substituents, and may include preferably a hydrogen atom, a hydrocarbon substituent, a cyano group, a fluoro group, $OR_{2a}$, $SR_{2a}$, $NR_{2a}R_{2b}$, $BR_{2a}R_{2b}$ or $SiR_{2a}R_{2b}R_{2c}$, more preferably a hydrogen atom, an alkyl group, an alicyclic hydrocarbon group, an aryl group and a fluoro group, even more preferably a hydrogen atom, an alkyl group, an alicyclic hydrocarbon group and an aryl group, and particularly preferably a hydrogen atom, an alkyl group, an alicyclic hydrocarbon group and a phenyl group. Each of $R_{2a}$ to $R_{2c}$ independently represents a hydrocarbon substituent or a hydrocarbon substituent substituted with a heteroatom, and may form a saturated or unsaturated aromatic ring or non-aromatic ring by bonding two of $R_{1a}$ to $R_{1i}$ and $R_{2a}$ to $R_{2c}$ with each other.

Herein, the heteroatom represents an atom other than a carbon atom or a hydrogen atom. Examples of the heteroatom may include, for example, oxygen, nitrogen, phosphorus, sulfur, selenium, arsenic, chlorine, bromine, silicon or fluorine. Further, the alicyclic hydrocarbon group may include preferably a cycloalkyl group having 5 to 6 carbon atoms, and more

preferably a cycloheptyl group, a cycloshexyl group, an adamantyl group and the like.

A substituent represented by $R_{1a}$ to $R_{1i}$ may further have a substituent, and as the substituent, those exemplified above for the group Z of the substituents may be applied. The substituent includes preferably an alkyl group, more preferably an alkyl group having 1 to 6 carbon atoms, even more preferably a methyl group, an ethyl group, a propyl group, an isopropyl group, a butyl group, a tert-butyl group and the like, and particularly preferably a tert-butyl group. At least one of $R_{1a}$ to $R_{1i}$ is preferably an aryl group which has a dihedron of 70 degrees or more with respect to a main structure, more preferably a substituent represented by Formula ss-1, even more preferably a 2,6-disubstituted aryl group, and most preferably, $R_{1b}$ is a 2,6-disubstituted aryl group.

X-Y represents a sub-ligand and n represents an integer of 1 to 3 and preferably n=3. Particularly, as the sub-ligand, the ligands such as the above examples may be appropriately used, and the sub-ligand includes preferably an acetylacetonato ligand or a substituted acetylacetonato ligand analog.

It is preferred that the indium complex represented by Formula (A9) is an iridium complex represented by Formula (A9-1).

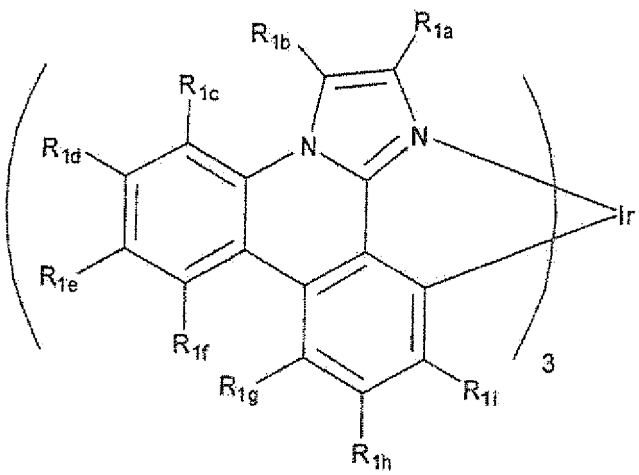

A9-1

In Formula (A9-1), each of $R_{1a}$ to $R_{1i}$ independently represents a hydrogen atom or a substituent.

In Formula (A9-1), $R_{1a}$ to $R_{1i}$ have the same meaning as $R_{1a}$ to $R_{1i}$ in Formula (A9) and preferable values thereof are the same. Further, it is preferred that each of $R_{1a}$ to $R_{1i}$ independently represents a hydrogen atom, an alkyl group, an alicyclic hydrocarbon group which may have an alkyl group, or a phenyl group which may have an alkyl group.

It is preferred that the iridium complex represented by Formula (A9) is an iridium complex represented by Formula A9-1').

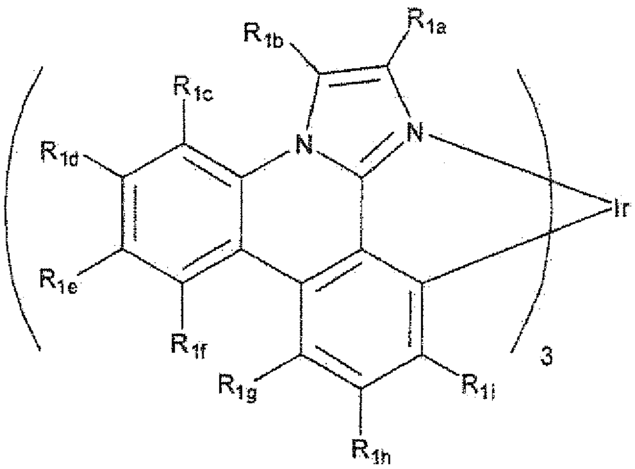

A9-1'

In Formula (A9-1'), each of $R_{1a}$ to $R_{1i}$ independently represents a hydrogen atom, an alkyl group having 1 to 20 carbon atoms, a cycloalkyl group having 3 to 8 carbon atoms, an aryl group having 6 to 18 carbon atoms, and an aryl group having 6 to 18 carbon atoms substituted by an alkyl group having 1 to 20 carbon atoms.

It is preferred that the iridium complex represented by Formula (A9), Formula (A9-1) or Formula (A9-1') is an iridium complex represented by Formula (A10).

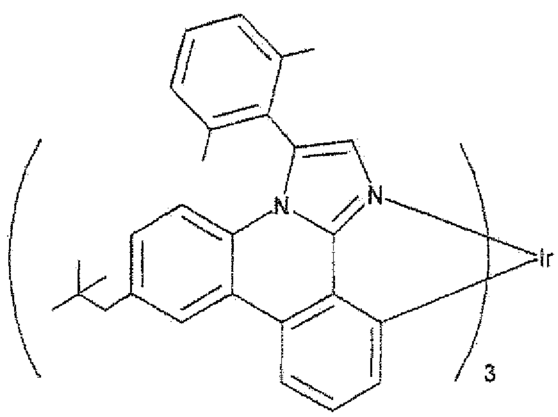

A10

In another aspect of the present invention, it is preferred that any one of $R_{1a}$ to $R_{1i}$ in Formulas (A1) to (A4), Formula (A1-1) or (A3-1), Formula (A1-2) or (A3-2), Formula (A1-3) or (A3-3), Formula (A9), and Formula (A9-1) or (A9-1') represents the following substituent (S).

Substituent (S):

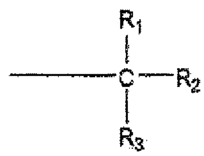

$R_1$ represents an alkyl group. $R_1$ includes preferably a methyl group, an ethyl group, a propyl group, an isopropyl group, a butyl group and a tert-butyl group, more preferably a methyl group, an ethyl group, an isopropyl group and a tert-butyl group, even more preferably a methyl group, an ethyl group, an isopropyl group and a tert-butyl group, and particularly preferably a methyl group and a tert-butyl group.

$R_2$ represents a hydrogen atom or an alkyl group. $R_2$ includes preferably a hydrogen atom, a methyl group, an ethyl group, a propyl group, an isopropyl group, a butyl group and a tert-butyl group, more preferably a hydrogen atom, a methyl group, an ethyl group and a propyl group, even more preferably a hydrogen atom and a methyl group, and particularly preferably a methyl group.

$R_3$ represents a hydrogen atom or an alkyl group. $R_3$ includes preferably a hydrogen atom and a methyl group, and more preferably a methyl group.

Further, each of $R_1$ to $R_3$ may be linked to each other to form a ring. When the ring is formed, the number of members is not particularly limited, but the ring is preferably a 5- or 6-membered ring, and more preferably a 6-membered ring.

The substituent (S) in the specific phosphorescent metal complex may include preferably the following substituent (a) to (x), preferably the substituents (a) to (e), (i), (l), and (t) to (v), more preferably the substituents (a) to (e), and even more preferably the substituent (c) or (d).

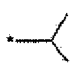
(a)
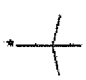
(b)
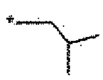
(c)
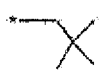
(d)
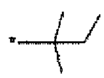
(e)
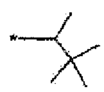
(f)
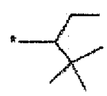
(g)

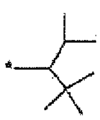
(h)
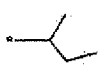
(i)
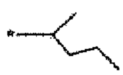
(j)
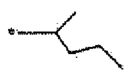
(k)
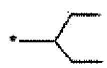
(l)

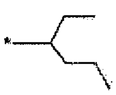
(m)
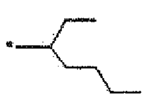
(n)
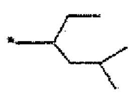
(o)
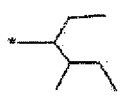
(p)
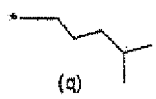
(q)

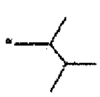
(r)
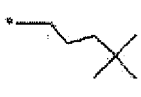
(s)
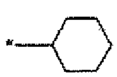
(t)
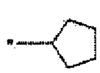
(u)
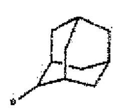
(v)
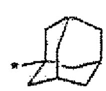
(w)

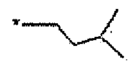

(x)

In the case where any one of $R_{1a}$ to $R_{1i}$ in the specific phosphorescent metal complex has the substituent (S), if the substituent has a large volume like the above substituents (a) to (x), it is considered that a quencher can be suppressed from being generated due to the reaction between the metal complexes.

In the case where any one of $R_{1a}$ to $R_{1i}$ in the specific phosphorescent metal complex has the substituent (S), it is preferred that the substituent included in the compound represented by Formula (1) is an alkyl group and particularly a branched alkyl group. Since compatibility with Formula (1) is increased, aggregation and association hardly occur, and as a result, it is considered that device deterioration due to generation of the quencher and misalignment of chromaticity due to association light emission are suppressed.

More particularly, the bidentate ligands represented by Formulas (A1) and (A3) have preferably the following structures and among them, most preferably (X-64) to (X-68).

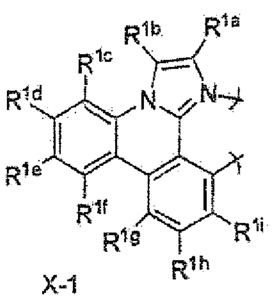

X-1

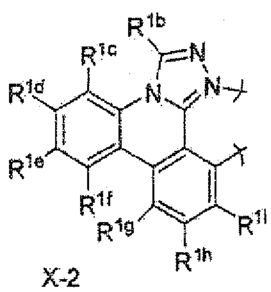

X-2

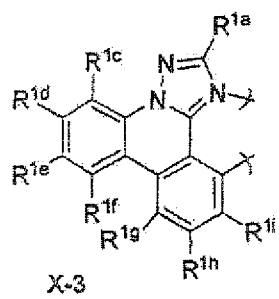

X-3

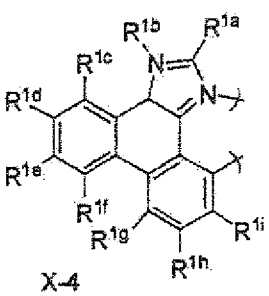

X-4

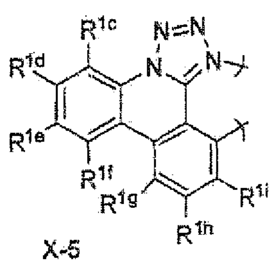

X-5

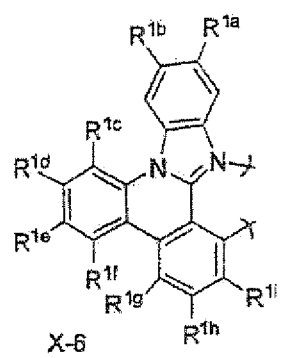

X-6

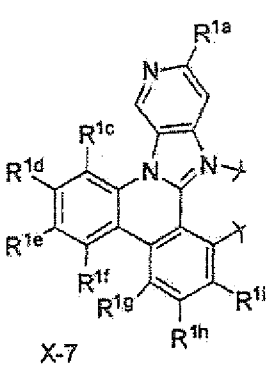

X-7

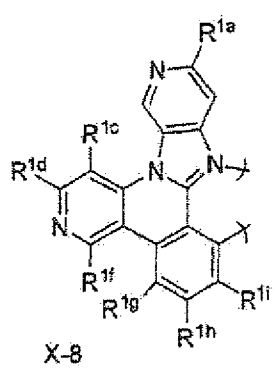

X-8

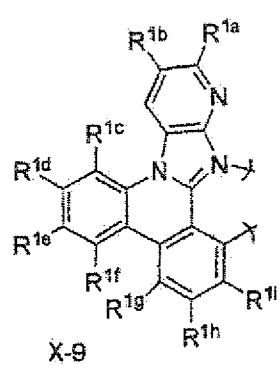

X-9

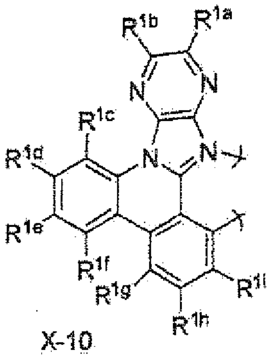
X-10

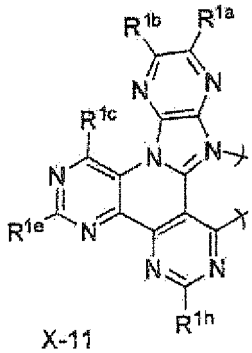
X-11

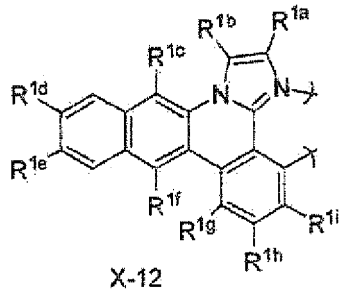
X-12

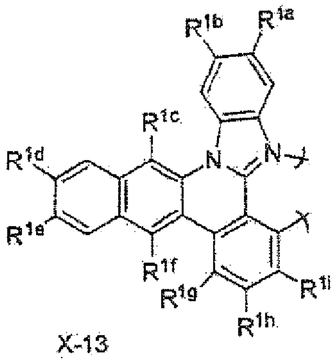
X-13

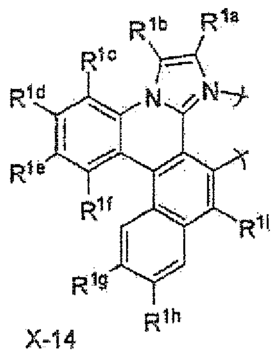
X-14

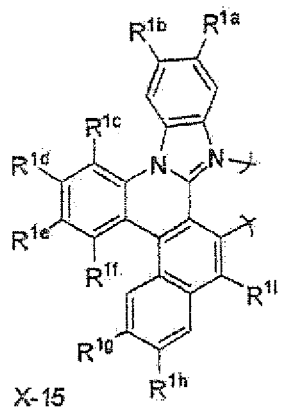
X-15

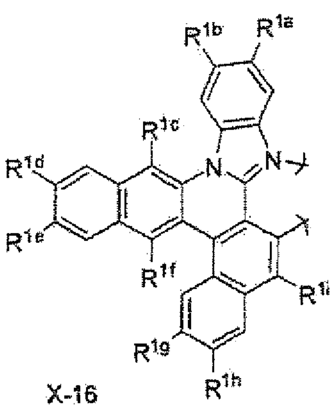
X-16

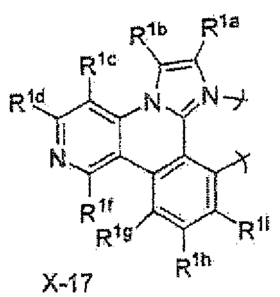
X-17

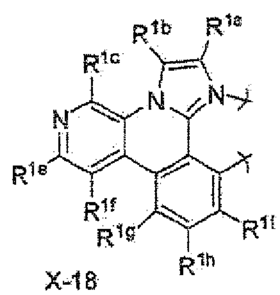
X-18

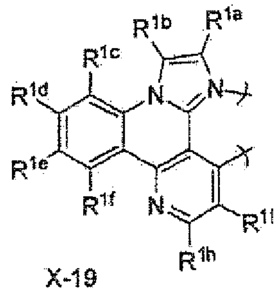
X-19

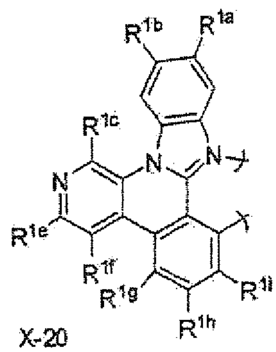
X-20

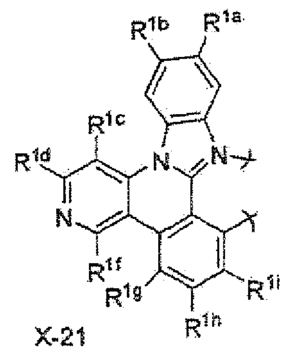
X-21

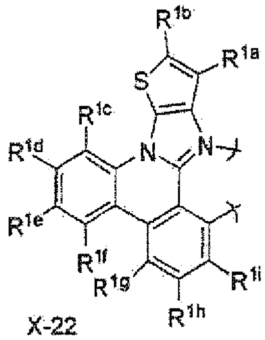
X-22

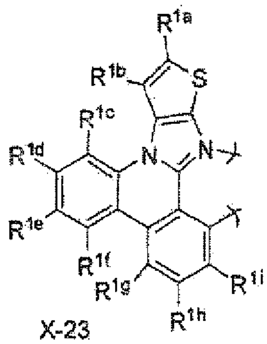
X-23

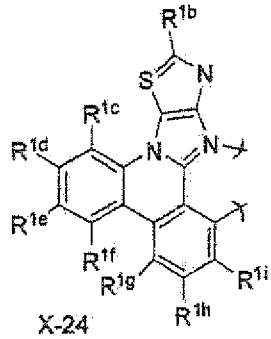
X-24

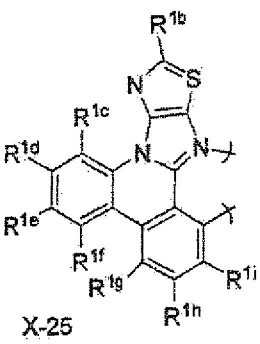
X-25

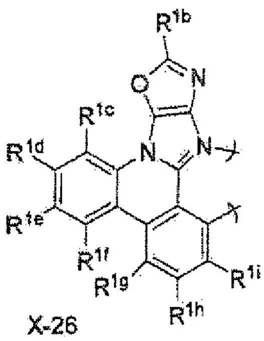
X-26

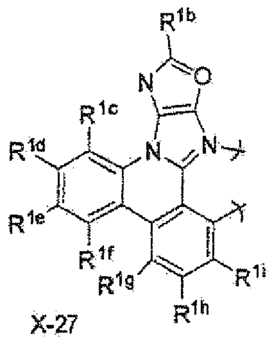
X-27

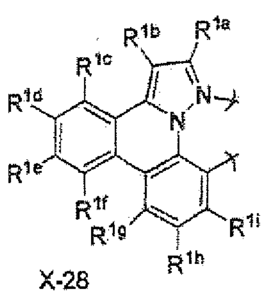
X-28

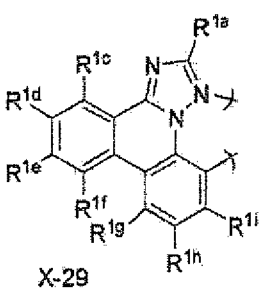
X-29

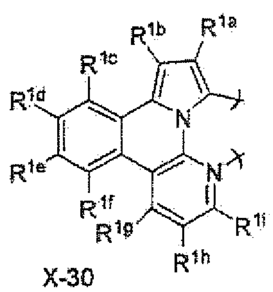
X-30

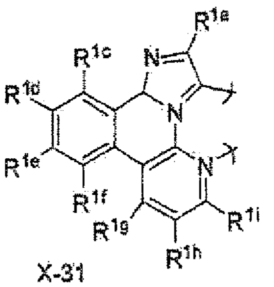

X-31

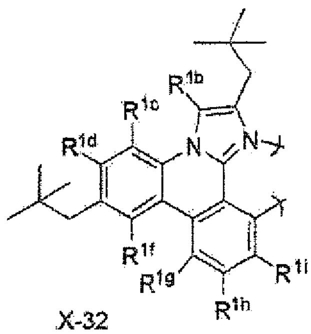

X-32

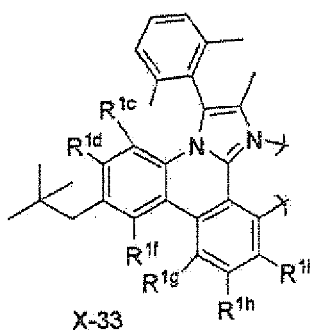

X-33

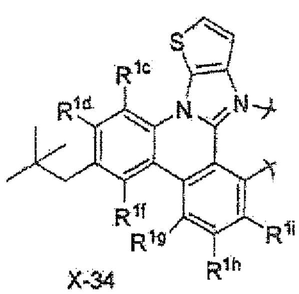

X-34

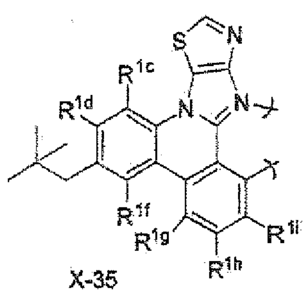

X-35

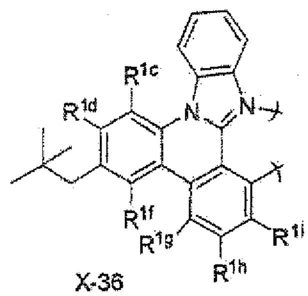

X-36

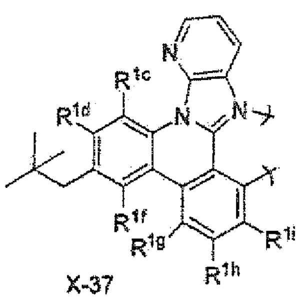

X-37

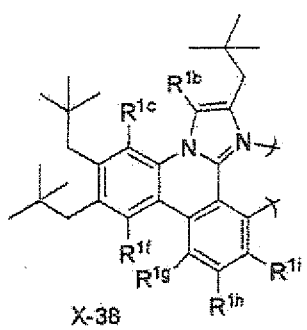

X-38

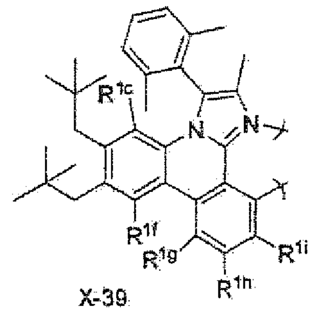

X-39

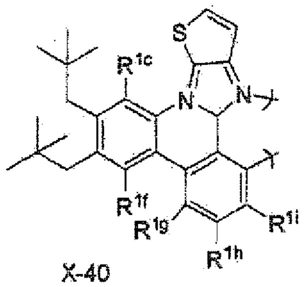

X-40

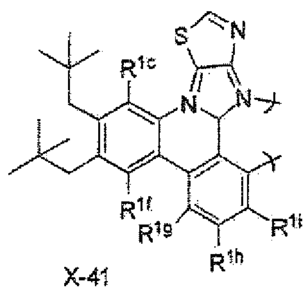

X-41

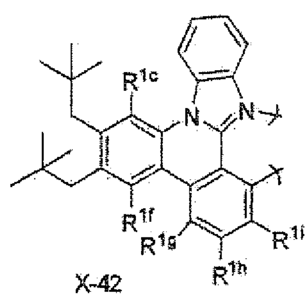

X-42

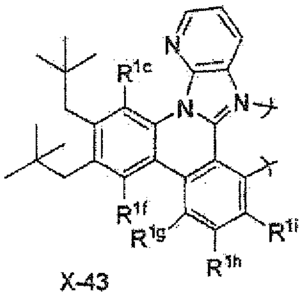
X-43

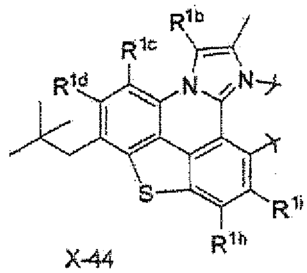
X-44

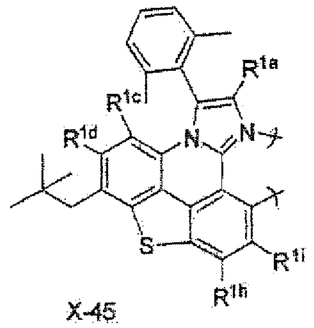
X-45

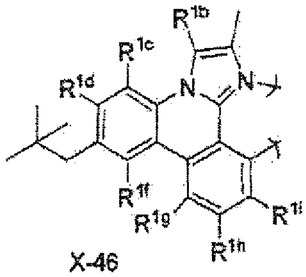
X-46

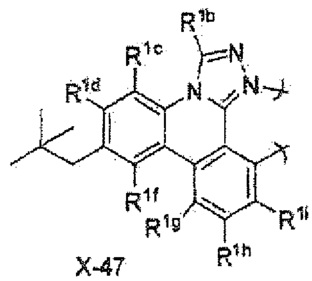
X-47

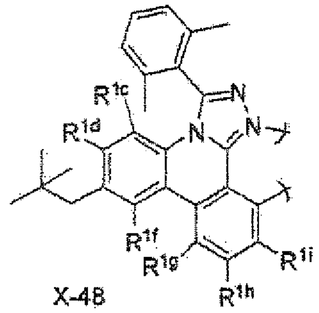
X-48

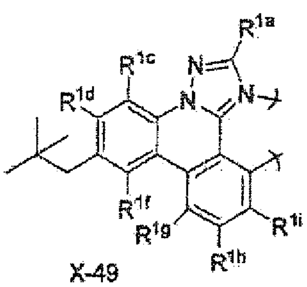
X-49

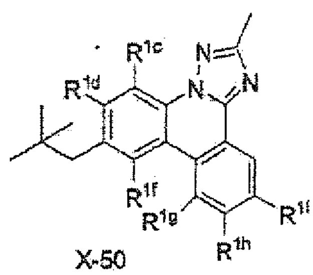
X-50

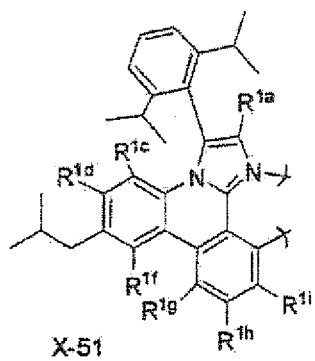
X-51

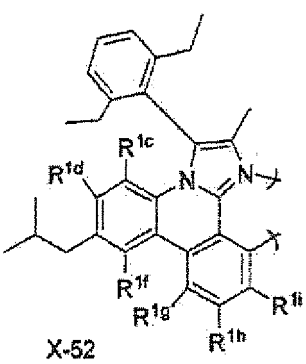
X-52

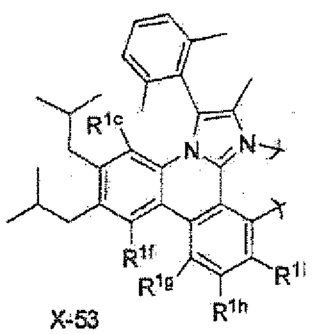
X-53

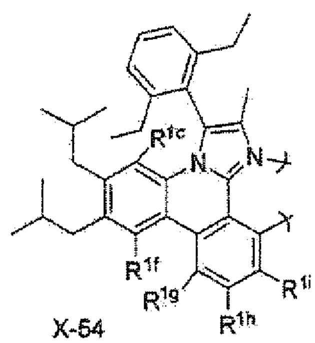
X-54

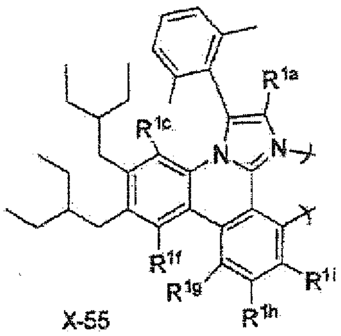
X-55

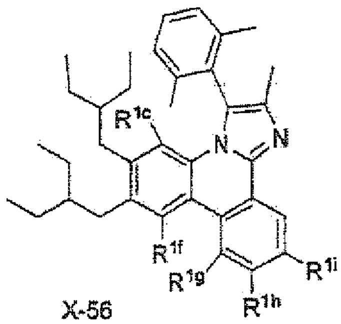
X-56

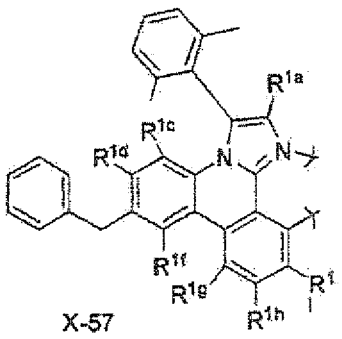
X-57

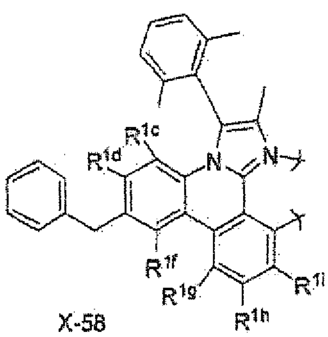
X-58

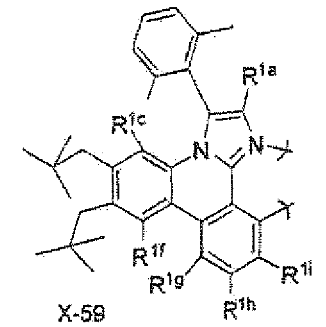
X-59

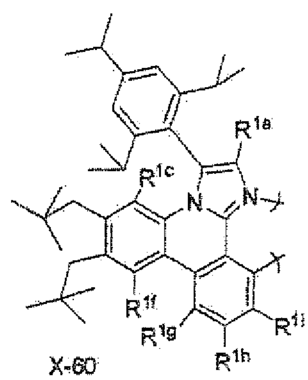
X-60

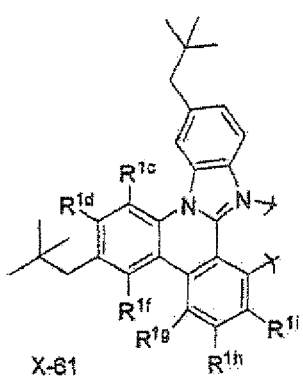
X-61

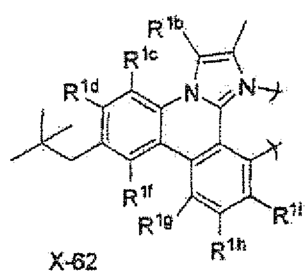
X-62

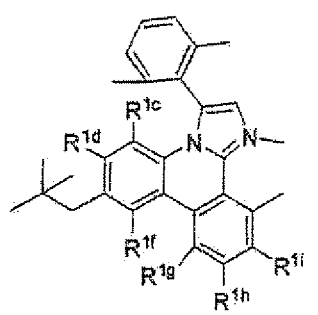
X-63

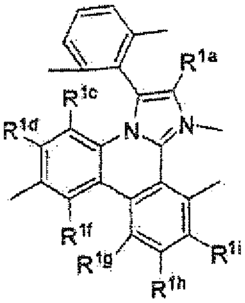
X-64

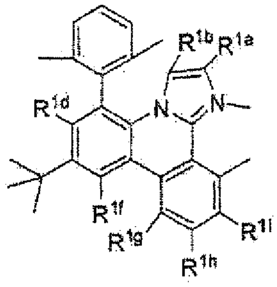
X-65

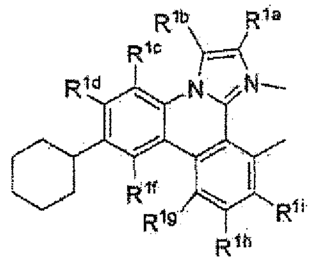
X-66

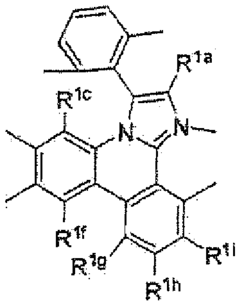
X-67

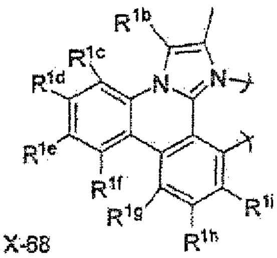
X-68

$R_{1a}$ to $R_{1i}$ have the same meaning as Formula (A1) and it is preferred that all of $R_{1a}$ to $R_{1i}$ are hydrogen atoms.

The phosphorescent metal complex containing the mono-anionic bidentate ligands represented by Formulas (A1) to (A4) and the metal having an atom weight of 40 or more may be synthesized by various methods such as, for example, methods described in US2007/0190359 and US2008/0297033, and the like.

For example, the phosphorescent metal complex may be obtained by treating a ligand or a dissociation material thereof and a metal compound in the presence or absence of a solvent (for example, a halogen-based solvent, an alcohol-based solvent, an ether-based solvent, an ester-based solvent, a ketone-based solvent, a nitrile-based solvent, an amide-based solvent, a sulfone-based solvent, a sulfoxide-based solvent, water and the like) and in the presence or absence of a base (various inorganic or organic bases, for example, sodium methoxide, t-butoxy potassium, triethylamine, potassium carbonate and the like) at room temperature or a lower temperature or by heating (in addition to typical heating, a technique for achieving heating by microwaves is also effective). Particularly, XM-64 may be synthesized by a synthesis method disclosed in [0132] to [0134] of US2007/0190359 by using 7-methylimidazophenanthridine as a starting material. Further, XM-63 may be synthesized by a synthesis method disclosed in [0281] to [0287] of US2008/0297033.

The present invention also relates to a composition containing Formula (1) and the phosphorescent metal complex containing the mono-anionic bidentate ligands represented by Formulas (A1) to (A4) and the metal having an atom weight of 40 or more. More particularly, the present invention also relates to a composition containing the compound having at least one group represented by any one of Formulas (4-1) to (4-10) and the phosphorescent metal complex containing the mono-anionic bidentate ligands represented by Formulas (A1) to (A4) and the metal having an atom weight of 40 or more, a composition containing the compound having at least one group represented by any one of Formulas (7-1) to (7-10) and the iridium complex represented by Formula (A9-1), and a composition containing the compound represented by Formula (10) and the iridium complex represented by Formula (A9-1').

The specific phosphorescent metal complex is used in combination with the compound represented by Formula (1) (for example, the compound having at least one group represented by any one of Formulas (4-1) to (4-10), the compound having at least one group represented by any one of Formulas (7-1) to (7-10), or the compound represented by Formula (10)), thereby acquiring an organic electroluminescence device having more excellent external quantum efficiency.

Further, in the composition of the present invention, a host material other than the compound represented by Formula (1) (the compound having at least one group represented by any one of Formulas (7-1) to (7-10)), a light emitting material other than the specific phosphorescent metal complex, or the like, may be added.

Further, it is preferred that at least one kind of the specific phosphorescent metal complex is contained in the light emitting layer. The content of the specific phosphorescent metal complex in the light emitting layer of the present

invention is preferably 1 to 40% by mass, and more preferably 5 to 30% by mass in the light emitting layer.

The present invention also relates to a light emitting layer containing Formula (1) and the phosphorescent metal complex containing the mono-anionic bidentate ligands represented by Formulas (A1) to (A4) and the metal having an atom weight of 40 or more. More particularly, the present invention also relates to a light emitting layer containing the compound having at least one group represented by any one of Formulas (4-1) to (4-10) and the phosphorescent metal complex containing the mono-anionic bidentate ligands represented by Formulas (A1) to (A4) and the metal having an atom weight of 40 or more, a light emitting layer containing the compound having at least one group represented by any one of Formulas (7-1) to (7-10) and the iridium complex represented by Formula (A9-1), and a light emitting layer containing the compound represented by Formula (10) and the iridium complex represented by Formula (A9-1').

In the composition or the light emitting layer of the present invention, with respect to the compound represented by Formula (1) (for example, the compound having at least one group represented by any one of Formulas (4-1) to (4-10) or the compound having at least one group represented by any one of Formulas (7-1) to (7-10)), a use ratio of the phosphorescent metal complex is preferably in the range of 0.1% by mass or more to 50% by mass or less, more preferably in the range of 1% by mass or more to 40% by mass or less, and most preferably in the range of 5% by mass or more to 30% by mass or less.

In the present invention, it is preferred that at least one kind of the compound represented by Formula (1) (for example, the compound having at least one group represented by any one of Formulas (4-1) to (4-10) or the compound having at least one group represented by any one of Formulas (7-1) to (7-10)) and at least one kind of the phosphorescent metal complex are contained in the light emitting layer.

As a preferable combination of the compound represented by Formula (1) (for example, the compound having at least one group represented by any one of Formulas (4-1) to (4-10) or the compound having at least one group represented by any one of Formulas (7-1) to (7-10)) and the specific phosphorescent metal complex, when the compound having at least one group represented by any one of Formulas (4-1) to (4-10) and the compound having at least one group represented by any one of Formulas (7-1) to (7-10) have the substituent (S), $R_{1a}$ to $R_{1i}$ of the specific phosphorescent metal complex are preferably a hydrogen atom and an alkyl group, more preferably a methyl group, an isopropyl group, tert-butyl group, an isobutyl group and a neophentyl group, even more preferably a methyl group, an isobutyl group and a neophentyl group, and particularly preferably a neophentyl group.

Further, when $R_{1a}$ to $R_{1i}$ of the specific phosphorescent metal complex have the substituent (S), the substituent of the compound having at least one group represented by any one of Formulas (4-1) to (4-10) or the compound having at least one group represented by any one of Formulas (7-1) to (7-10) is preferably a hydrogen atom, tert-butyl group, an isobutyl group, an amyl group and a neophentyl group. Since compatibility with the specific phosphorescent metal complex is increased in the light emitting layer, aggregation and association hardly occur, and as a result, it is considered that device deterioration due to generation of a quencher and misalignment of chromaticity due to association light emission are suppressed.

In one aspect, when any one of $R_{1a}$ to $P_{1i}$ of the specific phosphorescent metal complex is a substituent having a large volume represented by (a), (b), (e), (f), (g), (h), (o), (p), (r), and (t) to (w), it is preferred that the compound having at least one group represented by any one of Formulas (4-1) to (4-10) or the compound having at least one group represented by any one of Formulas (7-1) to (7-10) is unsubstituted or has a substituent represented by (c), (d), (i) to (n), (q), (s) and (x). When any one of $R_{1a}$ to $R_{1i}$ of the specific phosphorescent metal complex is (a) and (b), it is more preferred that the compound having at least one group represented by any one of Formulas (4-1) to (4-10) or the compound having at least one group represented by any one of Formulas (7-1) to (7-10) is unsubstituted or has a substituent represented by (c), (d) or (q), (s), (x). When $R_{1b}$ of the specific phosphorescent metal complex is a 2,6-disubstituted aryl group and any one of $R_{1a}$ to $R_{1i}$ is (a) and (b), it is even more preferred that the compound having at least one group represented by any one of Formulas (4-1) to (4-10) or the compound having at least one group represented by any one of Formulas (7-1) to (7-10) is unsubstituted or has one or two substituents represented by (c) or (d).

In another aspect of the present invention, when any one of $R_{1a}$ to $R_{1i}$ of the specific phosphorescent metal complex is a branched alkyl substituent represented by (c), (d), (i) to (n), (p), (q), (s), (x), it is preferred that the compound having at least one group represented by any one of Formulas (4-1) to (4-10) or the compound having at least one group represented by any one of Formulas (7-1) to (7-10) is unsubstituted or has a substituent represented by (a), (b), (d), (e). When any one of $R_{1a}$ to $R_{1i}$ of the specific phosphorescent metal complex is (c), (d), (q), (s), (x), it is more preferred that the compound having at least one group represented by any one of Formulas (4-1) to (4-10) or the compound having at least one group represented by any one of Formulas (7-1) to (7-10) is unsubstituted or has a substituent represented by (b), (d), (e). When $R_{1b}$ of the specific phosphorescent metal complex is a 2,6-disubstituted aryl group and any one of $R_{1a}$ to $R_{1i}$ of the specific phosphorescent metal complex is (c), (d), it is even more preferred that the compound having at least one group represented by any one of Formulas (4-1) to (4-10) or the compound having at least one group represented by any one of Formulas (7-1) to (7-10) is unsubstituted or has one or two substituents represented by (b), (d).

In yet another aspect of the present invention, when $R_{1a}$ to $R_{1i}$ of the specific phosphorescent metal complex

do not have the substituent (S), it is preferred that the compound having at least one group represented by any one of Formulas (4-1) to (4-10) or the compound having at least one group represented by any one of Formulas (7-1) to (7-10) has a substituent represented by (a), (b), (d), (e). When $R_{1b}$ of the specific phosphorescent metal complex is a 2,6-disubstituted aryl group, it is more preferred that the compound having at least one group represented by any one of Formulas (4-1) to (4-10) or the compound having at least one group represented by any one of Formulas (7-1) to (7-10) has a substituent represented by (b), (d), (e). When $R_{1b}$ of the specific phosphorescent metal complex is a 2,6-disubstituted aryl group and $R_{1e}$ is a methyl group, it is even more preferred that the compound having at least one group represented by any one of Formulas (4-1) to (4-10) or the compound having at least one group represented by any one of Formulas (7-1) to (7-10) has a substituent represented by (b), (d).

When numbers of alkyl substituents of the compound having at least one group represented by any one of Formulas (4-1) to (4-10) or the compound having at least one group represented by any one of Formulas (7-1) to (7-10) and the specific phosphorescent metal complex are contained in the light emitting layer, a ratio (mass ratio of alkyl groups in the light emitting layer) of a mass of the alkyl substituents contained in the light emitting layer to the total mass of the light emitting layer (the total mass of the light emitting material and the host material included in the light emitting layer) is preferably 0 to 0.3, more preferably 0.02 to 0.27, and particularly preferably 0.05 to 0.23. When the mass ratio of the alkyl groups in the light emitting layer is in the range, the compatibility with the compound having at least one group represented by any one of Formulas (4-1) to (4-10) or the compound having at least one group represented by any one of Formulas (7-1) to (7-10) and the specific phosphorescent metal complex is increased while the driving voltage does not increase, such that it is considered that durability of the device is improved and the misalignment of chromaticity is suppressed.

Meanwhile, the mass ratio of the alkyl groups in the light emitting layer is calculated from the following Equation.

[Equation 1]

$$\text{Mass ratio of alkyl groups} = \frac{\begin{array}{c}\text{Ratio of light emitting material in the light emitting layer (mass ratio)} \times \text{Mass number of alkyl groups substituted in the light emitting material} \\ + \text{Ratio of host material in the light emitting layer (mass ratio)} \times \text{Mass number of alkyl groups substituted in the host material}\end{array}}{\begin{array}{c}\text{Ratio of light emitting material in the light emitting layer (mass ratio)} \times \text{Mass number of the light emitting material} \\ + \text{Ratio of host material in the light emitting layer (mass ratio)} \times \text{Mass number of the host material}\end{array}}$$

[Organic Electroluminescence Device]

The device of the present invention will be described in detail.

The organic electroluminescence device of the present invention includes a pair of electrodes and at least one organic layer including a light emitting layer between the electrodes on a substrate, in which the device contains a specific phosphorescent metal complex and a compound represented by Formula (1) (for example, a compound having at least one group represented by any one of Formulas (4-1) to (4-10) or a compound having at least one group represented by any one of Formulas (7-1) to (7-10)).

In the organic electroluminescence device of the present invention, the light emitting layer may be an organic layer and a plurality of organic layers in addition to the at least one organic layer may also be included between the light emitting layer and a cathode.

Due to properties of the luminescence device, at least one of positive and cathodes is preferably transparent or semi-transparent.

FIG. 1 illustrates an example of the configuration of an organic electroluminescence device according to the present invention. An organic electroluminescence device 10 according to the present invention, which is illustrated in FIG. 1, is disposed on a supporting substrate 2, and a light emitting layer 6 is interposed between a anode 3 and a cathode 9. Specifically, a hole injection layer 4, a hole transporting layer 5, the light emitting layer 6, a hole blocking layer 7, and an electron transporting layer 8 are stacked in this order between the anode 3 and the cathode 9.

<Configuration of an organic layer>

The layer configuration of the organic layer is not particularly limited, and may be appropriately selected according to the use and purpose of the organic electroluminescence device, but is preferably formed on the transparent

electrode or on the rear electrode. In this case, the organic layer is formed on the front surface or one surface on the transparent electrode or the rear electrode.
The shape, size, thickness and the like of the organic layer are not particularly limited and may be appropriately selected according to the purpose.

The specific layer configuration may include the followings, but the present invention is not limited to the configurations.
Anode/hole transporting layer/light emitting layer/electron transporting layer/cathode,
Anode/hole transporting layer/light emitting layer/blocking layer/electron transporting layer/cathode,
Anode/hole transporting layer/light emitting layer/blocking layer/electron transporting layer/electron injection layer/cathode,
Anode/hole injection layer/hole transporting layer/light emitting layer/blocking layer/electron transporting layer/cathode, and
Anode/hole injection layer/hole transporting layer/light emitting layer/blocking layer/electron transporting layer/electron injection layer/cathode.
The device configuration, substrate, cathode, and anode of the organic electroluminescence device are described in detail in, for example, Japanese Patent Application Laid-Open No. 2008-270736, and the subject matters described in the publication may be applied to the present invention.

<Substrate>

It is preferred that the substrate which is used in the present invention is a substrate which does not scatter or decay light generated from the organic layer. In the case of an organic material, it is preferred that the organic material is excellent in heat resistance, dimensional stability, solvent resistance, electrical insulation properties and processibility.

<Anode>

Typically, the anode may have a function as an electrode for supplying holes into the organic layer, is not particularly limited with respect to shape, structure, size, and the like and may be appropriately selected among the known electrode materials depending upon a use or purpose of the luminescence device. As described above, the anode is usually provided as a transparent anode.

<Cathode>

Typically, the cathode may have a function as an electrode for injecting electrons into the organic layer, is not particularly limited with respect to shape, structure, size and the like, and may be appropriately selected among the known electrode materials depending upon a use or purpose of the luminescence device.

With respect to the substrate, the anode, and the cathode, subject matters described in paragraph Nos. [0070] to [0089] of Japanese Patent Application Laid-Open No. 2008-270736 may be applied to the present invention.

<Organic layer>

An organic layer in the present invention will be described.

-Formation of Organic Layer-

In the organic electroluminescence device of the present invention, each organic layer may be appropriately formed by any one of dry film-forming methods such as a vapor deposition method, a sputtering method, and the like, and wet film-forming methods (wet process) such as a transfer method, a printing method, a spin-coat method, and the like. It is preferred that at least one of the organic layers containing at least one compound represented by Formula (1) and the organic layer containing at least one specific phosphorescent metal complex is formed by the wet process. For example, it is preferred that at least one layer of the organic layer containing at least one compound containing at least one group represented by any one of Formulas (4-1) to (4-10) and the organic layer containing at least one specific phosphorescent metal complex is formed by the wet process. Further, it is preferred that at least one layer of the organic layer containing at least one compound containing at least one group represented by any one of Formulas (7-1) to (7-10) and the organic layer containing at least one indium complex represented by Formula (A9-1) is formed by the wet process. Furthermore, It is preferred that at least one layer of the organic layer containing at least one compound represented by Formula (10) and the organic layer containing at least one iridium complex represented by Formula (A9-1') is formed by the wet process.

(Light Emitting Layer)

<Light Emitting Material>

A light emitting material in the present invention is preferably the specific phosphorescent metal complex.

The light emitting material in the light emitting layer is contained in an amount of 0.1% by mass to 50% by mass, preferably 1% by mass to 50% by mass, and more preferably 2% by mass to 40% by mass from the viewpoint of durability and external quantum efficiency, based on the mass of the total compound which generally forms the light emitting layer in the light emitting layer.

A thickness of the light emitting layer is not particularly limited, but typically, the thickness is preferably 2 nm to 500 nm. Among the numbers of thicknesses, from the viewpoint of external quantum efficiency, the thickness of the light emitting layer is more preferably 3 nm to 200 nm, and even more preferably 5 nm to 100 nm.

The light emitting layer in the device of the present invention may be configured by only the light emitting material, and may also have a configuration of a mixed layer of a host material and a light emitting material. The light emitting material may be a fluorescent light emitting material or a phosphorescent light emitting material, and a dopant may be used either alone or two or more kinds. The host material is preferably a charge transporting material. The host material may be either alone or in combination of two or more kinds, and may have, for example, a configuration of a mixture of an electron transporting host material and a hole transporting host material. Further, the host material may include a material which does not have a charge transporting property in the light emitting layer and does not emit light. In addition, the light emitting layer may be either a single layer or a multilayer of two or more layers. Furthermore, each light emitting layer may emit light with different light emission colors.

<Host Material>

The host material used in the present invention may include the following compounds. Examples of the host material include pyrrole, indole, carbazole (for example, CBP (4,4'-di(9-carbazoyl)biphenyl)), azaindole, azacarbazole, triazole, oxazole, oxadiazole, pyrazole, imidazole, thiophene, polyarylalkane, pyrazoline, pyrazolone, phenylenediamine, arylamine, amino substituted chalcone, styrylanthracene, fluorenone, hydrazone, stilbene, silazane, aromatic tertiary amine compounds, styrylamine compounds, porphyrin-based compounds, polysilane-based compounds, poly(N-vinylcarbazole), aniline-based copolymers, electrically conductive high-molecular oligomers such as thiophene oligomers, polythiophene and the like, organosilanes, carbon films, pyridine, pyrimidine, triazine, imidazole, pyrazole, triazole, oxazole, oxadiazole, fluorenone, anthraquinodimethane, anthrone, diphenylquinone, thiopyran dioxide, carbodiimide, fluorenylidenemethane, distyrylpyrazine, fluorine- substituted aromatic compounds, heterocyclic tetracarboxylic anhydrides such as naphthalene perylene and the like, phthalocyanine, a variety of metal complexes represented by metal complexes of a 8-quinolinol derivative, metal phthalocyanine, and metal complexes having benzoxazole or benzothiazole as the ligand thereof, and derivatives thereof (which may have a substituent or a condensed ring), and the like.

In the light emitting layer in the present invention, it is preferred that the lowest triplet excitation energy ($T_1$ energy) of the host material is higher than the $T_1$ energy of the phosphorescent light emitting material from the viewpoint of color purity, light emission efficiency and driving durability.

Further, although the content of the host compound included in the present invention is not particularly limited, the content is preferably 15% by mass to 95% by mass with respect to the total mass of the compounds forming the light emitting layer, from the viewpoint of light emission efficiency and driving voltage.

(Fluorescent light emitting material)

Examples of the fluorescent light emitting material which may be used in the present invention include, for example, benzoxazole derivatives, benzoimidazole derivatives, benzothiazole derivatives, styrylbenzene derivatives, polyphenyl derivatives, diphenylbutadiene derivatives, tetraphenylbutadiene derivatives, naphthalimide derivatives, coumarin derivatives, condensed aromatic compounds, perynone derivatives, oxadiazole derivatives, oxazine derivatives, aldazine derivatives, pyralidine derivatives, cyclopentadiene derivative, bisstyrylanthracene derivatives, quinacridone derivatives, pyrrolopyridine derivatives, thiadiazolopyridine derivatives, cyclopentadiene derivatives, styrylamine derivatives, diketopyrrolopyrole derivatives, aromatic dimethylidine compounds, various complexes represented by a complex of 8-quinolinol derivatives or a complex of pyromethene derivatives and the like, polymer compounds such as polythiophene, polyphenylene, polyphenylenevinylene, and the like, compounds such as organic silane derivatives, and the like.

(Phosphorescent Light Emitting Material)

Examples of the phosphorescent light emitting material which may be used in the present invention include phosphorescent light emitting compounds and the like as disclosed in patent documents such as US 6,303,238B1, US 6,097,147, WO00/57676, WO00/70655, WO01/8230, WO01/39234A2, WO01/41512A1, WO02/02714A

WO02/15645A1, WO02/44189A1, WO05/19373A2, Japanese Patent Application Laid-Open No. 2001-247859, Japanese Patent Application Laid-Open No. 2002-302671, Japanese Patent Application Laid-Open No. 2002-117978, Japanese Patent Application Laid-Open No. 2003- 133074, Japanese Patent Application Laid-Open No. 2002-235076, Japanese Patent Application Laid-Open No. 2003-123982, Japanese Patent Application Laid-Open No. 2002-170684, EP1211257, Japanese Patent Application Laid-Open No. 2002-226495, Japanese Patent Application Laid-Open No. 2002-234894, Japanese Patent Application Laid-Open No. 2001-247859, Japanese Patent Application Laid-Open No. 2001-298470, Japanese Patent Application Laid-Open No. 2002-173674, Japanese Patent Application Laid-Open No. 2002-203678, Japanese Patent Application Laid-Open No. 2002-203679, Japanese Patent Application Laid-Open No. 2004-357791, Japanese Patent Application Laid-Open No. 2006-256999, Japanese Patent Application Laid-Open No. 2007-19462, Japanese Patent Application Laid-Open No. 2007-84635, Japanese Patent Application Laid-Open No. 2007-96259, and the like, in addition to the compound represented by Formula (1). Among them, as the light emitting dopant, an Ir complex, a Pt complex, a Cu complex, a Re complex, a W complex, a Rh complex, a Ru complex, a Pd complex, an Os complex, an Eu complex, a Tb complex, a Gd complex, a Dy complex and a Ce complex are even more preferable. An Ir complex, a Pt complex, or a Re complex are particularly preferable, and among them, an Ir complex, a Pt complex, or a Re complex containing at least one coordination mode of a metal-carbon bond, a metal-nitrogen bond, a metal-oxygen bond, and a metal-sulfur bond are preferable. Further, from the viewpoint of light emission efficiency, driving durability, chromaticity and the like, an Ir complex, a Pt complex, or a Re complex containing a tridentate or higher polydentate ligand are particularly preferable.

The content of the phosphorescent light emitting material included in the light emitting layer is preferably 0.1% by mass to 50% by mass, more preferably 0.2% by mass to 50% by mass, even more preferably 0.3% by mass to 40% by mass, and most preferably 20% by mass to 30% by mass, with respect to the total mass of the light emitting layer.

The content of the phosphorescent light emitting material (specific phosphorescent metal complex and/or phosphorescent light emitting material used in combination) which may be used in the present invention is preferably 0.In/% by mass to 50% by mass, more preferably 1% by mass to 40% by mass, and most preferably 5% by mass to 30% by mass, with respect to the total mass of the light emitting layer. In particular, within the range of 5% by mass to 30% by mass, the luminescence chromaticity of the organic electroluminescence device is small in the dependency on the added concentration of the phosphorescent light emitting material.

In the organic electroluminescence device of the present invention, it is most preferred that at least one specific phosphorescent metal complex is contained by 5 to 30% by mass with respect to the total mass of the light emitting layer.

-Hole Injection Layer and Hole Transporting Layer-

Each of the hole injection layer and the hole transporting layer is a layer having a function of accepting a hole from the anode or the anode side to transport the hole into the cathode side.

For the present invention, a hole injection layer or hole transporting layer containing an electron accepting dopant is preferably included as an organic layer.

-Electron Injection Layer and Electron Transporting Layer-

Each of the electron injection layer and the electron transporting layer is a layer having a function of accepting an electron from the cathode or the cathode side to transport the electron into the anode side.

It is preferred that at least one of the electron injection layer and the electron transporting layer in the present invention contains the compound represented by Formula (1) (the compound having at least one group represented by any one of Formulas (4-1) to (4-10) or the compound having at least one group represented by any one of Formulas (7-1) to (7-10)). The content of the compound represented by Formula (1) in the electron injection layer and the electron transporting layer is preferably 30% by mass to 100% by mass, more preferably 50% by mass to 100% by mass, and still more preferably 70% by mass to 100% by mass.

By using a combination of the light emitting layer containing the phosphorescent metal complex having a ligand represented by Formulas (A1) to (A4) (particularly, the iridium complex represented by Formula (A9)) and the compound represented by Formula (1) (for example, the compound having at one group represented by any one of Formulas (4-1) to (4-10) or the compound having at one group represented by any one of Formulas (7-1) to (7-10)) , the excitation energy generated in the light emitting layer prevented due to the high lowest triplet excitation energy ($T_1$ energy) of the compound represented by the Formula (1) from diffusing, thereby improving efficiency. Further, in the light emitting layer containing the phosphorescent metal complex having ligands represented by Formulas ($A_1$) to (A2), it is inferred that the balance of a hole and an electron is improved when an electron is injected into the light emitting layer from a specific energy level. Therefore, the electron conduction level of the adjacent layer injected into the light emitting layer is important to control the balance of carrier. Meanwhile, the emission spectrum depends largely on the balance of carrier since the wavelenght enhanced by the interference differs according to the point of light emission. Therefore, it is known that

when the spectrum varies with change in the film thickness of the electron transporting layer. However, it is inferred that the energy level suitable for effective injection of an electron of the compound represented by Formula (1) (for example, the compound having at one group represented by any one of Formulas (4-1) to (4-10) or the compound having at one group represented by any one of Formulas (7-1) to (7-10)) meets the level for controlling the balance of carrier of the light emitting layer in the present invention, and as a result, the spectrum hardly changes even if the film thickness of the electron transporting layer is changed.
With respect to the hole injection layer, the hole transporting layer, the electron injection layer and the electron transporting layer, the subject matters described in paragraph Nos. [0165] to [0167] of Japanese Patent Application Laid-Open No. 2008-270736 may be applied to the present invention.

-Hole Blocking Layer-

The hole blocking layer is a layer having a function of preventing a hole transported to the light emitting layer from the anode side from penetrating to the cathode side. In the present invention, the hole blocking layer may be provided as an organic layer adjacent to the light emitting layer on the cathode side.
Examples of the organic compounds constituting the hole blocking layer include an aluminum complex such as aluminumx (III)bis(2-methyl-8-quinolinato)4-phenylphenolate (simply referred to as BAlq), and the like, triazole derivatives, and phenanthroline derivatives such as 2,9-dimethyl-4,7-diphenyl-1,10-phenanthroline (simply referred to as BCP), and the like, in addition to the compound represented by Formula (1) (for example, the compound having at least one group represented by any one of Formulas (4-1) to (4-10) or the compound having at least one group represented by any one of Formulas (7-1) to (7-10)) in the present invention.
The thickness of the hole blocking layer is preferably 1 nm to 500 nm, more preferably 5 nm to 200 nm, and even more preferably 10 nm to 100 nm.
The hole blocking layer may have a single layer structure composed of one or two or more kinds of the above-described materials or may have a multilayer structure composed of a plurality of layers of the same or different compositions.

-Electron Blocking Layer-

The electron blocking layer is a layer having a function of preventing an electron transported to the light emitting layer from the cathode side from penetrating to the anode side.
In the present invention, the electron blocking layer may be provided, as an organic layer adjacent to the light emitting layer on the anode side.
Examples of the organic compound constituting the electron blocking layer include those described above as the hole transporting material.
The thickness of the electron blocking layer is preferably 1 nm to 500 nm, more preferably 5 nm to 200 nm, and even more preferably 10 nm to 100 nm.
The electron blocking layer may have a single layer structure composed of one or two or more kinds of the above-described materials or may have a multilayer structure composed of a plurality of layers of the same or different compositions.

<protective Layer>

In the present invention, the entire organic EL device may be protected by a protective layer.
With respect to the protective layer, the subject matters described in paragraph Nos. [0169] and [0170] of Japanese Patent Application Laid-Open No. 2008-270736, may be applied to the present invention.

<Sealing Vessel>

In the device of the present invention, the entire device may be sealed using a sealing vessel.
With respect to the sealing vessel, the subject matters described in paragraph No. [0171] of of Japanese Patent Application Laid-Open No. 2008-270736 may be applied to the present invention.

(Driving)

In the organic electroluminescence device of the present invention, light emission may be obtained by applying a voltage (typically 2 volts to 15 volts) of direct current (may include an alternating current component if necessary) or a current of direct current between the anode and the cathode.
With respect to the driving method of the organic electroluminescence device of the present invention, driving methods

described in each official gazette of Japanese Patent Application Laid-Open No. Hei 2-148687, Japanese Patent Application Laid-Open No. Hei 6-301355, Japanese Patent Application Laid-Open No. Hei 5-29080, Japanese Patent Application Laid-Open No. Hei 7-134558, Japanese Patent Application Laid-Open No. Hei 8-234685, and Japanese Patent Application Laid-Open No. Hei 8-241047, and Japanese Patent No. 2784615, US Patent No. 5,828,429, and US Patent No. 6,023,308, and the like may be applied.

The light collection efficiency of the luminescence device of the present invention may be enhanced by various known devices. For example, the light collection efficiency may be enhanced to enhance the external quantum efficiency by processing the substrate surface shape (for examples, forming a fine uneven pattern), by controlling the refractive index of the substrate, ITO layer or organic layer, by controlling the film thickness of the substrate, ITO layer organic layer, and the like.

The luminescence device of the present invention may be in a so-called top emission mode of collecting light emission from the anode side.

The organic EL device in the present invention may have a resonator structure. For example, a multilayer mirror composed of a plurality of laminated films having the different refractive index, a transparent or semi-transparent electrode, a light emitting layer, and a metal electrode are superimposed on a transparent substrate. Light generated in the light emitting layer is repeatedly reflected and resonated between the multilayer film mirror and the metal electrode as a reflection plate.

In another preferred aspect, each of a transparent or semi-transparent electrode and a metal electrode functions as a reflecting plate on a transparent substrate, and light generated in the light emitting layer repeats reflection and resonates therebetween.

In order to form a resonance structure, the effective refractive index of two reflecting plates and the optical path length determined from the refractive index and thickness of each layer between the reflecting plates may be adjusted to be optimal values to obtain a desired resonance wavelength. The calculating formula in the case of the first aspect is described in Japanese Patent Application Laid-Open No. Hei 9-180883. The calculating formula in the case of the second aspect is described in Japanese Patent Application Laid-Open No. 2004-127795.

The external quantum efficiency of the organic electroluminescence device of the present invention is preferably 5% or more, and more preferably 7% or more. As values of external quantum efficiency, a maximum value of external quantum efficiency, or a value of external quantum efficiency near 100 to 300 cd/m$^2$ may be used when driving the device at 20˚C.

The internal quantum efficiency of the organic electroluminescence device of the present invention is preferably 30% or more, more preferably 50% or more, and even more preferably 70% or more. The internal quantum efficiency of the device is calculated by dividing the external quantum efficiency by the light collection efficiency. Although typical organic EL devices have an light collection efficiency of about 20%, it is possible to achieve a light collection efficiency of 20% or more by studying the shape of the substrate, the shape of the electrode, the film thickness of the organic layer, the film thickness of the inorganic layer, the refractive index of the organic layer, the refractive index of the inorganic layer and the life.

The organic electroluminescence device of the present invention has ultra-high power emission wavelength (maximum strength wavelength of the emission spectrum) of preferably 350 nm or more and 700 nm or less, more preferably 350 nm or more and 600 nm or less, even more preferably 400 nm or more and 520 nm or less, and particularly preferably 400 nm or more and 465 nm or less.

(Use of Luminescence Device of the Present Invention)

The luminescence device of the present invention may be suitably used for light emission apparatuses, pixels, display devices, displays, backlights, electrophotography, illumination light sources, recording light sources, exposure light sources, reading light sources, indicators, signboards, interiors, optical communication or the like. In particular, the luminescence device of the present invention is preferably used for a device that is driven in a region with high luminescence luminance intensity, such as an illumination apparatus, a display apparatus, and the like.

Next, the light emission apparatus of the present invention will be described by referring to FIG. 2.

The light emission apparatus of the present invention is configured by using the above-described organic electroluminescence device.

FIG. 2 is a cross-sectional view schematically illustrating an example of a light emission apparatus of the present invention. The light emission apparatus 20 of FIG. 2 is composed of a transparent substrate (supporting substrate) 2, an organic electroluminescence device 10, a sealing vessel 16, and the like.

The organic electroluminescence device 10 is configured by sequentially laminating an anode (first electrode) 3, an organic layer 11, and a cathode (second electrode) 9 on the substrate 2. Further, a protective layer 12 is laminated on the cathode 9, and the sealing vessel 16 is further provided on the protective layer 12 through an adhesive layer 14. In addition, a part of each of electrodes 3 and 9, a partition wall, an insulating layer, and the like are omitted.

Here, as the adhesive layer 14, a photocurable or thermosetting adhesive such as an epoxy resin, and the like may be used and, for example, a thermosetting adhesive sheet may also be used.

The use of the light emission apparatus of the present invention is not particularly limited and, for example, the light emission apparatus may be used not only for an illumination apparatus but also as a display apparatus such as a television set, a personal computer, a cellular phone, an electronic paper and the like.

(Illumination apparatus)

Next, an illumination apparatus according to an embodiment of the present invention will be described by referring to FIG. 3.
FIG. 3 is a cross-sectional view schematically illustrating an example of the illumination apparatus according to the embodiment of the present invention.
The illumination apparatus 40 according to the embodiment of the present invention includes, as illustrated in FIG. 3, the above-described organic EL device 10 and a light scattering member 30. More specifically, the illumination apparatus 40 is configured such that the substrate 2 of the organic EL device 10 and the light scattering member 30 are in contact with each other.
The light scattering member 30 is not particularly limited so long as the member may scatter light, but in FIG. 3, a member obtained by dispersing microparticulates 32 in a transparent substrate 31 is used. Suitable examples of the transparent substrate 31 include a glass substrate. Suitable examples of the microparticulate 32 include a transparent resin microparticulate. As the glass substrate and the transparent resin microparticulate, products known in the art may be used.
In such an illumination apparatus 40, when light emitted from the organic electroluminescence device 10 is incident on a light incident surface 30A of the scattering member 30, the incident light is scattered by the light scattering member 30 and the scattered light is reflected as illuminating light from a light reflecting surface 30B.

[Example]

Hereinafter, the present invention will be described in detail with reference to Examples, but the present invention is not limited thereto. Further, the following exemplified compounds also include other compounds which do not correspond to the compound having at least one group represented by any one of Formulas (7-1) to (7-10) and the iridium complex compound represented by Formula (A9-1), but other compounds were disclosed for reference.

(Synthesis of Exemplary Compound 1)

Exemplary Compound 1 was synthesized according to the following scheme.

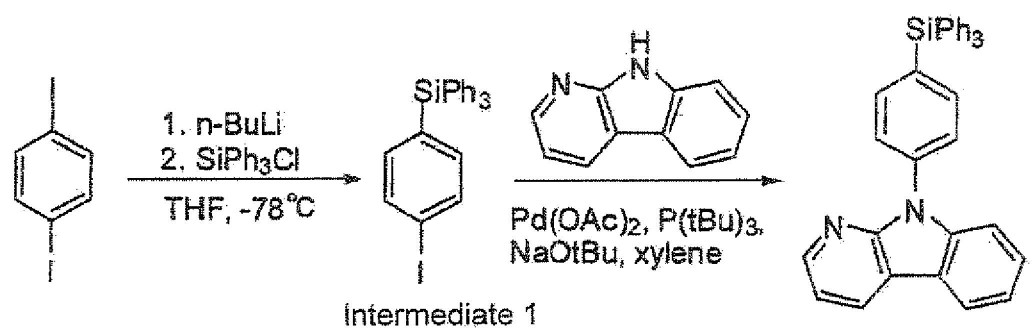


1.1 equivalents of normal butyl lithium was added to p-diiodobenzene in tetrahydrofuran at -78°C, and was reacted for 30 min. 1.2 equivalents of chlorotriphenylsilane was added thereto and reacted at room temperature for about 1 hr to obtain intermediate 1 in a yield of 72%. Intermediate 1, 0.05 equivalents of palladium acetate, 0.15 equivalents of tri(t-butyl)phosphine, 2.4 equivalents of sodium-tert-butoxide and 1 equivalent of a-carboline were dissolved in xylene, and reacted at a reflux temperature for 10 hr. Ethyl acetate and water were added to the reaction mixture to separate an organic phase, the organic phase was washed with water and saturated saline solution and then concentrated under reduced pressure, and the obtained residue was purified by silica gel column chromatography, recrystallization, purification by sublimation and the like to obtain exemplary compound 1 in a yield of 55%.

(Synthesis of Exemplary Compound 122)

Exemplary Compound 122 was synthesized according to the following scheme.

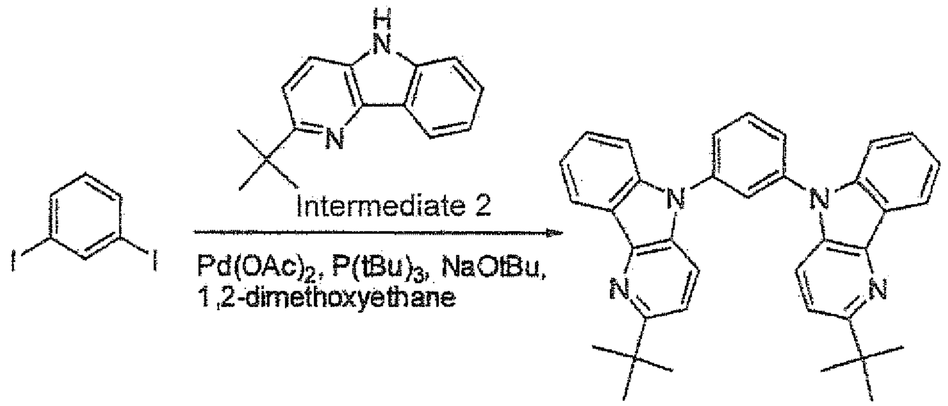


m-diiodobenzene. 0.05 equivalents of palladium acetate, 0.15 equivalents of tri(t-butyl)phosphine, 4 equivalents of sodium-tert-butoxide and 2 equivalents of Intermediate 2 were dissolved in xylene and reacted at a reflux temperature for 12 hr. Ethyl acetate and water were added to the reaction mixture to separate an organic phase, the organic phase was washed with water and saturated saline solution and then concentrated under reduced pressure, and the obtained residue was purified by silica gel column chromatography, recrystallization, purification by sublimation and the like to obtain exemplary compound 122 in a yield of 43%.

(Synthesis of Exemplary Compound 160)

Exemplary Compound 160 was synthesized according to the following scheme.

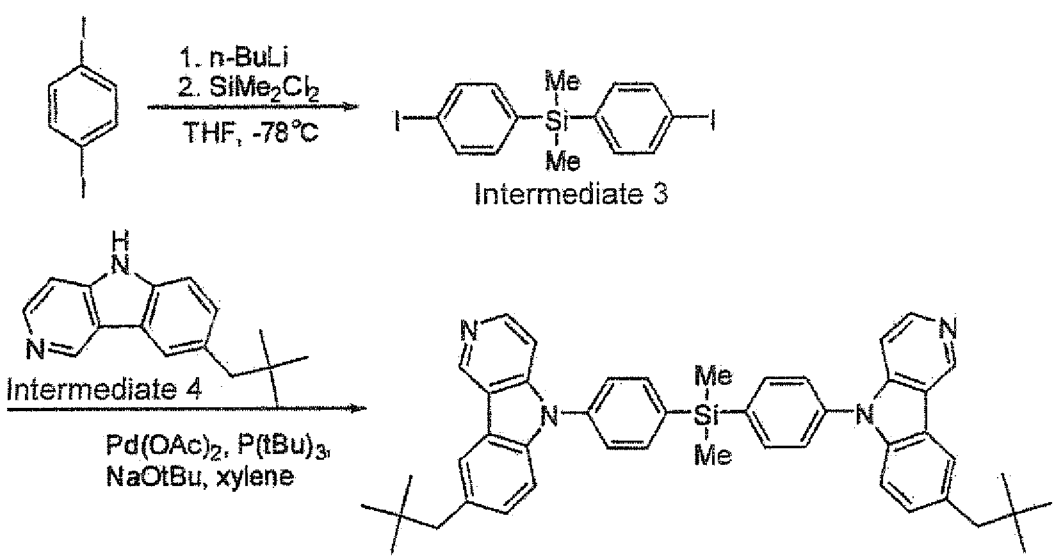


1 equivalent of normal butyl lithium was added to p-diiodobenzene in tetrahydrofuran at -78°C and reacted for 30 min. 0.5 equivalents of dichlorotriphenylsilane was added thereto and reacted at room temperature for about 1 hr to obtain intermediate 3 in a yield of 55%. Intermediate 3, 0.1 equivalents of palladium acetate, 0.3 equivalents of tri(t-butyl)phosphine, 4 equivalents of sodium-tert-butoxide and 2.1 equivalents of Intermediate 4 were dissolved in xylene and reacted at a reflux temperature for 15 hr. Ethyl acetate and water were added to the reaction mixture to separate an organic phase, the organic phase was washed with water and saturated saline solution and then concentrated under

reduced pressure, and the obtained residue was purified by silica gel column chromatography, recrystallization, purification by sublimation and the like to obtain exemplary compound 160 in a yield of 55%.

(Synthesis of Exemplary Compound 156)

Exemplary Compound 156 was synthesized according to the following scheme.

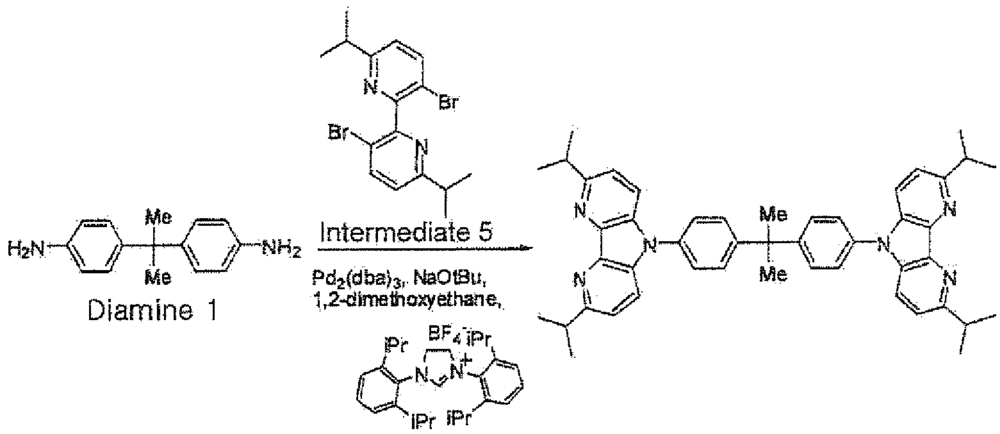


Diamine 1, 0.05 equivalents of tris(dibenzylideneacetone)dipalladium, 0.1 equivalents of imidazolium salt, 4 equivalents of sodimn-tert-butoxide and 2 equivalents of Intermediate 5 were dissolved in xylene and reacted at a reflux temperature for 20 hr. Ethyl acetate and water were added to the reaction mixture to separate an organic phase, the organic phase was washed with water and saturated saline solution and then concentrated under reduced pressure, and the obtained residue was purified by silica gel column chromatography, recrystallization, purification by sublimation and the like to obtain exemplary compound 156 in a yield of 24%.

[Manufacture of Organic Electrolysis Luminescence Device]

[Comparative Example 1-1]

A washed ITO substrate was put in a vapor deposition apparatus to vapor-deposit copper phthalocyanine to a thickness of 10 nm, and N,N'-di-α-naphthyl-N,N'-diphenyl)benzidine (NPD) was vapor-deposited thereon to a thickness of 40 nm (hole transporting layer). Thereon, A-2 and H-1 were vapor-deposited at a ratio of 9:91 (mass ratio) to a thickness of 30 nm (light emitting layer), and H-1 was vapor-deposited thereon to a thickness of 5 nm (adjacent layer). Thereon, Alq(tris(8-hydroxyquinolin)aluminum complex) was vapor-deposited to a thickness of 30 nm (electron transporting layer). Thereon, lithium fluoride was vapor-depasited to a thickness of 3 nm, and aluminum was vapor-deposited thereon to a thickness of 60 nm. This was placed in a glove box substituted with argon gas so as not to be contacted with the atmosphere and sealed using a stainless steel-made sealing can and a UV-curable adhesive (XNR5516HV, manufactured by Nagase-CHIBA Ltd.) to obtain an organic electroluminescence device in Comparative Example 1-1. A direct current constant voltage was applied to the EL device to emit light by means of a source measure unit Model 2400 manufactured by TOYO TECHNIKA INC., and as a result, the phosphorescent light emission derived from A-2 could be obtained.

[Examples 1-1 to 1-169 and Comparative Examples 1-2 to 1-9]

A device was manufactured in the same manner as in Comparative Example 1-1, except that the compounds used for the light emitting material and the host material were changed into those described in Table 1. The obtained devices of Examples 1-1 to 1-169 of the present invention and the obtained comparative devices of Comparative Examples 1-1 to 1-9 were evaluated as follows. Meanwhile, in all of the devices, phosphorescent light emission derived from a light emitting material used could be obtained. The obtained results were incorporated into Table 1.

(Measurement of Driving Voltage)

The organic electroluminescence devices of Examples 1-1 to 1-169 and Comparative Examples 1-1 to 1-9 were set on an emission spectrum measurement system (ELS1500), manufactured by Shimadzu Corporation, and applied voltages at the time when the luminance intensity thereof was 1,000 cd/m$^2$ were measured.

(Evaluation of Driving Durability)

The organic electroluminescence devices of Examples 1-1 to 1-169 and Comparative Examples 1-1 to 1-9 were set on an OLED test system, MODEL ST-D, manufactured by Tokyo Systems Development Co., Ltd. and driven in a constant current mode under a condition of an initial luminance intensity of 1,000 cd/cm$^2$, thereby measuring a half-luminance intensity time. Meanwhile, a value of the device in Comparative Example 1-1 was made to be 100, and with reference to the value, the half-luminance intensity time was represented by a relative value.

(Evaluation of External Quantum Efficiency)

The organic electroluminescence devices of Examples 1-1 to 1-169 and Comparative Examples 1-1 to 1-9 emitted light by applying a direct current constant voltage to the EL device by using a Source Measure Unit 2400, manufactured by TOYO TECHNIKA INC. The external quantum efficiency (%) was calculated from the front luminance intensity at the time of 1,000 cd/m$^2$.

(Evaluation of Chromaticity)

The chromaticity (CIE chromaticity) was calculated by applying a direct current voltage so as to have luminance intensity of 1,000 cd/m$^2$, and measuring an emission spectrum by an emission spectrum measurement system (ELS1500), manufactured by Shimadzu Corporation.

[Table 1]

Table 1

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Comparative example 1-1 | A-2 | H-1 | H-1 | 12.1 | 6.2 | 100 | (0.16, 0.28) |
| Comparative example 1-2 | A-1 | H-1 | H-1 | 12.6 | 4.8 | 13 | (0.18, 0.33) |
| Comparative example 1-3 | A-3 | H-1 | H-1 | 12.3 | 6.6 | 211 | (0.16, 0.27) |
| Comparative example 1-4 | A-4 | H-1 | H-1 | 13.6 | 5.8 | 68 | (0.16, 0.26) |
| Comparative example 1-5 | A-5 | H-1 | H-1 | 12.1 | 6.5 | 54 | (0.17, 0.30) |
| Comparative example 1-6 | A-6 | H-1 | H-1 | 11.7 | 6.8 | 127 | (0.16, 0.28) |
| Comparative example 1-7 | A-7 | H-1 | H-1 | 12.3 | 6.1 | 32 | (0.16, 0.27) |
| Comparative example 1-8 | A-1 | C-1 | C-1 | 12.3 | 4.9 | 15 | (0.18, 0.32) |
| Comparative example 1-9 | A-1 | C-10 | C-10 | 12.4 | 4.7 | 16 | (0.17, 0.32) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-1 | A-2 | C-1 | C-1 | 10.5 | 8.1 | 142 | (0.16, 0.23) |
| Example 1-2 | A-2 | C-3 | C-3 | 10.2 | 7.4 | 118 | (0.16, 0.24) |
| Example 1-3 | A-2 | C-4 | C-4 | 11.7 | 7.1 | 125 | (0.16, 0.23) |
| Example 1-4 | A-2 | C-5 | C-5 | 10.8 | 7.8 | 136 | (0.16, 0.24) |
| Example 1-5 | A-2 | C-6 | C-6 | 10.9 | 7.6 | 140 | (0.15, 0.23) |
| Example 1-6 | A-2 | C-9 | C-9 | 11.1 | 7.2 | 129 | (0.15, 0.22) |
| Example 1-7 | A-2 | C-12 | C-12 | 10.4 | 8.1 | 144 | (0.16, 0.23) |
| Example 1-8 | A-2 | C-30 | C-30 | 10.7 | 7.8 | 133 | (0.16, 0.22) |
| Example 1-9 | A-2 | C-36 | C-36 | 11.2 | 7.2 | 113 | (0.15, 0.21) |
| Example 1-10 | A-2 | C-45 | C-45 | 11.7 | 7.5 | 121 | (0.15, 0.22) |
| Example 1-11 | A-2 | C-48 | C-48 | 10.4 | 8.3 | 204 | (0.16, 0.23) |
| Example 1-12 | A-2 | C-57 | C-57 | 10.6 | 7.8 | 136 | (0.15, 0.23) |
| Example 1-13 | A-2 | C-66 | C-66 | 10.7 | 7.9 | 168 | (0,16, 0.23) |
| Example 1-14 | A-2 | C-74 | C-74 | 10.8 | 7.9 | 148 | (0.16, 0.24) |
| Example 1-15 | A-2 | C-77 | C-77 | 10.9 | 7.7 | 139 | (0.16, 0.23) |
| Example 1-16 | A-2 | C-83 | C-83 | 10.9 | 7.6 | 130 | (0.16, 0.23) |
| Example 1-17 | A-2 | C-77 | C-88 | 10.7 | 7.9 | 132 | (0.15, 0.23) |

[Table 2]

Table 1 (Continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-18 | A-3 | C-23 | C-23 | 11.2 | 7.6 | 369 | (0.16, 0.21) |
| Example 1-19 | A-3 | C-24 | C-24 | 11.0 | 7.8 | 325 | (0.16, 0.21) |

(continued)

Table 1 (Continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-20 | A-3 | C-25 | C-24 | 10.8 | 7.8 | 311 | (0.16, 0.22) |
| Example 1-21 | A-3 | C-1 | C-1 | 10.7 | 7.9 | 326 | (0.16, 0.22) |
| Example 1-22 | A-3 | C-26 | C-26 | 10.4 | 7.6 | 268 | (0.15, 0.21) |
| Example 1-23 | A-3 | C-27 | C-27 | 10.5 | 7.4 | 261 | (0.15, 0.21) |
| Example 1-24 | A-3 | C-3 | C-3 | 10.1 | 7.6 | 291 | (0.15, 0.20) |
| Example 1-25 | A-3 | C-28 | C-28 | 10.3 | 7.5 | 281 | (0.15, 0.21) |
| Example 1-26 | A-3 | C-29 | C-29 | 11.7 | 7.1 | 258 | (0.15, 0.21) |
| Example 1-27 | A-3 | C-4 | C-4 | 11.5 | 7.3 | 284 | (0.15, 0.21) |
| Example 1-28 | A-3 | C-30 | C-30 | 10.8 | 7.7 | 295 | (0.15, 0.21) |
| Example 1-29 | A-3 | C-31 | C-31 | 10.7 | 8.0 | 333 | (0.15, 0.21) |
| Example 1-30 | A-3 | C-32 | C-32 | 10.8 | 7.8 | 281 | (0.16, 0.22) |
| Example 1-31 | A-3 | C-33 | C-33 | 10.1 | 7.6 | 304 | (0.16, 0.21) |
| Example 1-32 | A-3 | C-34 | C-34 | 10.6 | 7.4 | 243 | (0.15, 0.21) |
| Example 1-33 | A-3 | C-35 | C-35 | 10.6 | 7.2 | 235 | (0.15, 0.21) |
| Example 1-34 | A-3 | C-36 | C-36 | 11.3 | 7.4 | 282 | (0.15, 0.20) |
| Example 1-35 | A-3 | C-37 | C-37 | 10.6 | 7.8 | 288 | (0.16, 0.21) |
| Example 1-36 | A-3 | C-6 | C-6 | 10.7 | 7.9 | 318 | (0.15, 0.22) |
| Example 1-37 | A-3 | C-38 | C-38 | 10.5 | 7.8 | 317 | (0.15, 0.22) |
| Example 1-38 | A-3 | C-39 | C-39 | 10.5 | 7.9 | 326 | (0.15, 0.21) |

(continued)

Table 1 (Continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-39 | A-3 | C-40 | C-40 | 10.3 | 7.7 | 273 | (0.15, 0.21) |
| Example 1-40 | A-3 | C-41 | C-41 | 10.1 | 8.0 | 252 | (0.15, 0.20) |
| Example 1-41 | A-3 | C-42 | C-42 | 10.4 | 7.7 | 255 | (0.15, 0.21) |
| Example 1-42 | A-3 | C-43 | C-43 | 10.4 | 7.8 | 270 | (0.15, 0.21) |
| Example 1-43 | A-3 | C-44 | C-44 | 11.2 | 7.4 | 298 | (0.15, 0.20) |
| Example 1-44 | A-3 | C-45 | C-45 | 11.3 | 7.3 | 254 | (0.15, 0.20) |
| Example 1-45 | A-3 | C-46 | C-46 | 10.3 | 8.2 | 484 | (0.15, 0.22) |
| Example 1-46 | A-3 | C-47 | C-47 | 10.4 | 7.8 | 449 | (0.16, 0.22) |
| Example 1-47 | A-3 | C-48 | C-48 | 10.2 | 8.5 | 481 | (0.15, 0.21) |
| Example 1-48 | A-3 | C-16 | C-16 | 9.9 | 8.7 | 537 | (0.15, 0.22) |
| Example 1-49 | A-3 | C-49 | C-49 | 10.1 | 7.8 | 289 | (0.15, 0.21) |
| Example 1-50 | A-3 | C-50 | C-50 | 10.7 | 7.8 | 277 | (0.15, 0.20) |
| Example 1-51 | A-3 | C-9 | C-9 | 11.3 | 7.1 | 269 | (0.15, 0.21) |
| Example 1-52 | A-3 | C-51 | C-51 | 10.5 | 7.6 | 266 | (0.15, 0.20) |
| Example 1-53 | A-3 | C-52 | C-52 | 10.8 | 7.6 | 262 | (0.15, 0.20) |
| Example 1-54 | A-3 | C-53 | C-53 | 11.0 | 7.4 | 277 | (0.15, 0.20) |
| Example 1-55 | A-3 | C-54 | C-54 | 10.4 | 8.2 | 311 | (0.16, 0.21) |
| Example 1-56 | A-3 | C-55 | C-55 | 10.4 | 8.1 | 339 | (0.16, 0.21) |

[Table 3]

| Table 1 (Continued) | | | | | | | |
|---|---|---|---|---|---|---|---|
| | Light emitting layer | | Adjacent layer material | Driving voltage (V) | External quantum efficiency (%) | Half-luminance intensity time (Relative value) | Initial chromaticity |
| | Light emitting material | Host material | | | | | |
| Example 1-57 | A-3 | C-12 | C-12 | 10.2 | 8.5 | 315 | (0.16, 0.21) |
| Example 1-58 | A-3 | C-56 | C-56 | 10.2 | 8.6 | 387 | (0.16, 0.20) |
| Example 1-59 | A-3 | C-57 | C-57 | 10,4 | 8.0 | 301 | (0.15, 0.21) |
| Example 1-60 | A-3 | C-58 | C-58 | 10.5 | 8.2 | 318 | (0.15, 0.21) |
| Example 1-61 | A-3 | C-59 | C-59 | 10.6 | 7.9 | 305 | (0.15, 0.20) |
| Example 1-62 | A-3 | C-60 | C-60 | 10.7 | 7.8 | 287 | (0.15, 0.20) |
| Example 1-63 | A-3 | C-61 | C-61 | 10.9 | 7.6 | 279 | (0.15, 0.21) |
| Example 1-64 | A-3 | C-62 | C-62 | 11.1 | 7.5 | 273 | (0.16, 0.21) |
| Example 1-65 | A-3 | C-63 | C-63 | 10.4 | 8.1 | 337 | (0.16, 0.20) |
| Example 1-66 | A-3 | C-64 | C-64 | 10.3 | 8.2 | 346 | (0.16, 0.21) |
| Example 1-67 | A-3 | C-65 | C-65 | 10.3 | 8.2 | 364 | (0.16, 0.20) |
| Example 1-68 | A-3 | C-66 | C-66 | 10.2 | 8.3 | 390 | (0.16, 0.20) |
| Example 1-69 | A-3 | C-67 | C-67 | 10.5 | 7.8 | 286 | (0.16, 0.21) |
| Example 1-70 | A-3 | C-68 | C-68 | 10.5 | 7.9 | 303 | (0.15, 0.21) |
| Example 1-71 | A-3 | C-69 | C-69 | 10.6 | 7.7 | 281 | (0.15, 0.20) |
| Example 1-72 | A-3 | C-5 | C-5 | 10.6 | 8.1 | 305 | (0.15, 0.20) |
| Example 1-73 | A-3 | C-70 | C-70 | 10.8 | 7.8 | 293 | (0.15, 0.20) |
| Example 1-74 | A-3 | C-71 | C-71 | 11.0 | 7.5 | 272 | (0.15, 0.21) |
| Example 1-75 | A-3 | C-72 | C-72 | 10.4 | 8.1 | 322 | (0.16, 0.20) |

(continued)

Table 1 (Continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-76 | A-3 | C-73 | C-73 | 10.2 | 8.2 | 368 | (0.16, 0.20) |
| Example 1-77 | A-3 | C-74 | C-74 | 10.4 | 8.1 | 329 | (0.15, 0.21) |
| Example 1-78 | A-3 | C-75 | C-75 | 10.6 | 7.9 | 297 | (0.16, 0.21) |
| Example 1-79 | A-3 | C-76 | C-76 | 10.5 | 8.0 | 302 | (0.16, 0.20) |
| Example 1-80 | A-3 | C-77 | C-77 | 10.5 | 8.0 | 314 | (0.16, 0.21) |
| Example 1-81 | A-3 | C-78 | C-78 | 10.6 | 7.9 | 291 | (0.15, 0.21) |
| Example 1-82 | A-3 | C-79 | C-79 | 10.7 | 7.8 | 282 | (0.16, 0.20) |
| Example 1-83 | A-3 | C-80 | C-80 | 10.8 | 7.9 | 293 | (0.15, 0.21) |
| Example 1-84 | A-3 | C-81 | C-81 | 10.7 | 7.8 | 280 | (0.15, 0.20) |
| Example 1-85 | A-3 | C-82 | C-82 | 10.6 | 8.0 | 310 | (0.16, 0.20) |
| Example 1-86 | A-3 | C-83 | C-83 | 10.6 | 7.9 | 291 | (0.16, 0.21) |
| Example 1-87 | A-3 | C-84 | C-84 | 10.4 | 8.1 | 309 | (0.15, 0.21) |
| Example 1-88 | A-3 | C-85 | C-85 | 10.2 | 8.2 | 358 | (0.16, 0.20) |
| Example 1-89 | A-3 | C-86 | C-86 | 10.6 | 7.9 | 284 | (0.15, 0.21) |
| Example 1-90 | A-3 | C-87 | C-87 | 10.7 | 7.8 | 281 | (0.16, 0.20) |
| Example 1-91 | A-3 | C-88 | C-88 | 10.6 | 8.1 | 302 | (0.16, 0.20) |
| Example 1-92 | A-3 | C-89 | C-89 | 10.7 | 7.8 | 294 | (0.15, 0.21) |
| Example 1-93 | A-3 | C-90 | C-90 | 10.4 | 7.9 | 291 | (0.15, 0.21) |
| Example 1-94 | A-3 | C-91 | C-91 | 10.7 | 7.8 | 290 | (0.15, 0.20) |

[Table 4]

| Table 1 (Continued) | | | | | | | |
|---|---|---|---|---|---|---|---|
| | Light emitting layer | | Adjacent layer material | Driving voltage (V) | External quartum efficiency (%) | Half-luminance intensity time (Relative value) | Initial chromaticity |
| | Light emitting material | Host material | | | | | |
| Example 1-95 | A-4 | C-1 | C-1 | 13.0 | 6.3 | 79 | (0.16, 0.24) |
| Example 1-96 | A-4 | C-5 | C-5 | 12.5 | 6.2 | 83 | (0.16, 0.24) |
| Example 1-97 | A-4 | C-6 | C-6 | 12.2 | 6.8 | 94 | (0.16, 0.23) |
| Example 1-98 | A-4 | C-12 | C-12 | 12.3 | 6.7 | 91 | (0.16, 0.23) |
| Example 1-99 | A-4 | C-27 | C-27 | 12.9 | 6.1 | 74 | (0.16, 0.23) |
| Example 1-100 | A-4 | C-31 | C-31 | 12.8 | 6.3 | 80 | (0.16, 0.24) |
| Example 1-101 | A-4 | C-35 | C-35 | 13.1 | 6.1 | 70 | (0.16, 0.24) |
| Example 1-102 | A-4 | C-38 | C-38 | 12.1 | 6.8 | 95 | (0.16, 0.23) |
| Example 1-103 | A-4 | C-43 | C-43 | 12.1 | 6.2 | 82 | (0.16, 0.24) |
| Example 1-104 | A-4 | C-46 | C-46 | 11.8 | 7.0 | 114 | (0.16, 0.23) |
| Example 1-105 | A-4 | C-52 | C-52 | 12.0 | 6.4 | 71 | (0.16, 0.23) |
| Example 1-106 | A-4 | C-56 | C-56 | 12.2 | 6.8 | 98 | (0.16, 0.24) |
| Example 1-107 | A-4 | C-62 | C-62 | 12.8 | 6.1 | 73 | (0.16, 0.25) |
| Examples 1-108 | A-4 | C-63 | C-63 | 12.2 | 6.7 | 89 | (0.16, 0.23) |
| Example 1-109 | A-4 | C-67 | C-67 | 12.5 | 6.4 | 88 | (0.16, 0.24) |
| Example 1-110 | A-4 | C-56 | C-73 | 12.1 | 6.8 | 95 | (0.16, 0.23) |
| Example 1-111 | A-4 | C-62 | C-76 | 12.4 | 6.3 | 81 | (0.16, 0.24) |
| Example 1-112 | A-4 | C-84 | C-84 | 12.4 | 6.2 | 82 | (0.16, 0.24) |
| Example 1-113 | A-4 | C-87 | C-87 | 12.6 | 6.0 | 72 | (0.16, 0.24) |

(continued)

| Table 1 (Continued) | | | | | | | |
|---|---|---|---|---|---|---|---|
| | Light emitting layer | | Adjacent layer material | Driving voltage (V) | External quartum efficiency (%) | Half-luminance intensity time (Relative value) | Initial chromaticity |
| | Light emitting material | Host material | | | | | |
| Example 1-114 | A-5 | C-1 | C-1 | 11.9 | 6.5 | 58 | (0.17, 0.28) |
| Example 1-115 | A-5 | C-5 | C-5 | 12.0 | 6.5 | 60 | (0.17, 0.29) |
| Example 1-116 | A-5 | C-6 | C-6 | 11.4 | 7.1 | 81 | (0.16, 0.26) |
| Example 1-117 | A-5 | C-23 | C-23 | 11.9 | 6.6 | 68 | (0.17, 0.28) |
| Example 1-118 | A-5 | C-28 | C-28 | 11.5 | 6.5 | 59 | (0.16, 0.27) |
| Example 1-119 | A-5 | C-30 | C-31 | 11.8 | 6.6 | 64 | (0.16, 0.27) |
| Example 1-120 | A-5 | C-34 | C-34 | 11.9 | 6.6 | 59 | (0.16, 0.26) |
| Example 1-121 | A-5 | C-39 | C-39 | 11,6 | 6.7 | 62 | (0.17, 0.27) |
| Example 1-222 | A-5 | C-44 | C-44 | 11.8 | 6.5 | 61 | (0.17, 0.28) |
| Example 1-123 | A-5 | C-47 | C-47 | 11.4 | 6.9 | 91 | (0.16, 0.26) |
| Example 1-124 | A-5 | C-49 | C-49 | 11.5 | 6.8 | 61 | (0.17, 0.27) |
| Example 1-125 | A-5 | C-55 | C-55 | 11.5 | 6.8 | 67 | (0.17, 0.27) |
| Example 1-126 | A-5 | C-58 | C-58 | 11.6 | 6.8 | 62 | (0.17, 0.28) |
| Example 1-127 | A-5 | C-65 | C-65 | 11.4 | 6.9 | 68 | (0.17, 0.27) |
| Example 1-128 | A-5 | C-70 | C-70 | 11.8 | 6.6 | 60 | (0.17, 0.28) |
| Example 1-129 | A-5 | C-72 | C-72 | 11.5 | 6.7 | 65 | (0.17, 0.27) |
| Example 1-130 | A-5 | C-78 | C-78 | 11.2 | 6.6 | 61 | (0,16, 0.26) |
| Example 1-131 | A-5 | C-85 | C-85 | 11.4 | 6.9 | 74 | (0.17, 0.28) |
| Example 1-132 | A-5 | C-89 | C-89 | 11.8 | 6.6 | 60 | (0.16, 0.27) |

[Table 5]

| Table 1 (Continued) | | | | | | | |
|---|---|---|---|---|---|---|---|
| | Light emitting layer | | Adjacent layer material | Driving voltage (V) | External quantum efficiency (%) | Half-luminance intensity time (Relative value) | Initial chromaticity |
| | Light emitting material | Host material | | | | | |
| Example 1-133 | A-6 | C-6 | C-6 | 10.4 | 8.1 | 163 | (0.15, 0.23) |
| Example 1-134 | A-6 | C-12 | C-12 | 10.2 | 8.4 | 170 | (0.16, 0.23) |
| Example 1-135 | A-6 | C-1 | C-1 | 10.5 | 8.2 | 171 | (0.15, 0.23) |
| Example 1-136 | A-6 | C-26 | C-26 | 10.6 | 7.9 | 137 | (0.16, 0.24) |
| Example 1-137 | A-6 | C-32 | C-32 | 10.5 | 8.0 | 154 | (0.16, 0.23) |
| Example 1-138 | A-6 | C-36 | C-36 | 10.6 | 8.0 | 152 | (0.16, 0.23) |
| Example 1-139 | A-6 | C-37 | C-37 | 10.7 | 7.9 | 156 | (0.16, 0.24) |
| Example 1-140 | A-6 | C-42 | C-42 | 10.8 | 7.9 | 139 | (0.16, 0.25) |
| Example 1-141 | A-6 | C-16 | C-16 | 9.9 | 8.9 | 274 | (0.15, 0.23) |
| Example 1-142 | A-6 | C-53 | C-53 | 11.0 | 7.7 | 144 | (0.16, 0.24) |
| Example 1-143 | A-6 | C-54 | C-54 | 10.2 | 8.4 | 173 | (0.16, 0.23) |
| Example 1-144 | A-6 | C-61 | C-61 | 10.6 | 7.9 | 150 | (0.16, 0.24) |
| Example 1-145 | A-6 | C-64 | C-64 | 10.3 | 8.5 | 175 | (0.16, 0.23) |
| Example 1-146 | A-6 | C-71 | C-71 | 10.8 | 7.5 | 149 | (0.16, 0.24) |
| Example 1-147 | A-6 | C-75 | C-75 | 10.6 | 7.9 | 161 | (0.16, 0.24) |
| Example 1-148 | A-6 | C-81 | C-81 | 10.7 | 7.9 | 155 | (0.16, 0.24) |
| Example 1-149 | A-6 | C-86 | C-86 | 10.7 | 7.9 | 158 | (0.16, 0.24) |
| Example 1-150 | A-6 | C-91 | C-91 | 10.6 | 7.8 | 155 | (0.16, 0.23) |
| Example 1-151 | A-7 | C-1 | C-1 | 11.5 | 8.0 | 51 | (0.15, 0.23) |

(continued)

Table 1 (Continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 1-152 | A-7 | C-5 | C-5 | 11.4 | 8.1 | 63 | (0.15, 0.23) |
| Example 1-153 | A-7 | C-6 | C-6 | 11.4 | 8.0 | 52 | (0.15, 0.23) |
| Example 1-154 | A-7 | C-12 | C-12 | 11.2 | 8.0 | 55 | (0.15, 0.23) |
| Example 1-155 | A-7 | C-24 | C-24 | 11.7 | 7.9 | 55 | (0.16, 0.24) |
| Example 1-156 | A-7 | C-25 | C-25 | 11.8 | 7.9 | 51 | (0.16, 0.24) |
| Example 1-157 | A-7 | C-33 | C-33 | 11.0 | 7.8 | 50 | (0.15, 0.24) |
| Example 1-158 | A-7 | C-37 | C-37 | 11.9 | 7.7 | 47 | (0.16, 0.24) |
| Example 1-159 | A-7 | C-40 | C-40 | 12.0 | 7.7 | 43 | (0.16, 0.25) |
| Example 1-160 | A-7 | C-16 | C-16 | 10.6 | 8.8 | 82 | (0.15, 0.23) |
| Example 1-161 | A-7 | C-51 | C-51 | 11.4 | 7.8 | 44 | (0.16, 0.24) |
| Example 1-162 | A-7 | C-59 | C-59 | 11.7 | 8.0 | 52 | (0.15, 0.24) |
| Example 1-163 | A-7 | C-60 | C-60 | 11.6 | 8.0 | 51 | (0.15, 0.24) |
| Example 1-164 | A-7 | C-65 | C-65 | 11.1 | 8.3 | 66 | (0.15, 0.24) |
| Example 1-165 | A-7 | C-69 | C-69 | 11.6 | 7.8 | 46 | (0.16, 0.25) |
| Example 1-166 | | A-7 C-73 | C-73 | 11.2 | 8.2 | 61 | (0.15, 0.24) |
| Example 1-167 | A-7 | C-79 | C-79 | 11.4 | 7.9 | 48 | (0.15, 0.23) |
| Example 1-168 | A-7 | C-83 | C-83 | 11.3 | 7.8 | 47 | (0.16, 0.24) |
| Example 1-169 | A-7 | C-90 | C-90 | 11.4 | 7.8 | 50 | (0.15, 0.23) |

As apparent form the above results, the device of the present invention had higher external quantum efficiency, lower driving voltage, and more improved durability than the comparative devices. Further, color purity was improved, and thus, preferable chromaticity could be used as a blue luminescence device. Particularly, when comparing each of

the devices of the present invention in Examples 1-1 to 1-17 and the comparative devices in Comparative Example 1-1, the devices of the present invention in Examples 1-18 to 1-94 and the comparative devices in Comparative Example 1-3, the devices of the present invention in Examples 1-95 to 1-113 and the comparative devices in Comparative Example 1-4, the devices of the present invention in Examples 1-114 to 132 and the comparative devices in Comparative Example 1-5, the devices of the present invention in Examples 1-136 to 1-150 and the comparative devices in Comparative Example 1-6, the devices of the present invention in Examples 1-151 to 1-169 and the comparative devices in Comparative Example 1-7, the organic electroluminescence device having high external quantum efficiency, low driving voltage, high durability, and good color purity could be obtained by using the compound having at least one group represented by any one of Formulas (7-1) to (7-10) and the iridium complex represented by Formula (A9).

(Example 2)

(Comparative Example 2-1)

A glass substrate having an ITO film having a thickness of 0.5 mm and each side of 2.5 cm in square (manufactured by GEOMATEC Co., Ltd., and surface resistance 10 Ω/□) was put into a washing container, ultrasonically washed in 2-propanol, followed by UV-ozone treatment for 30 min. A solution of poly(3,4-ethylenedioxythiophene)-polystyrenesulfonate (PEDOT/PSS) diluted with pure water to 70% was coated thereon by using a spin coater, to form a hole transporting layer having a thickness of 50 nm. A methylene chloride solution in which H-1 and A-2 were dissolved in a ratio of 96/5 (mass ratio) was coated by using a spin coater to obtain a light emitting layer having a thickness of 30 nm. Thereon, BAlq [bis-(2-methyl-8-quinolinolate)-4-(phenylphenolate)aluminum] was vapor-deposited to a thickness of 40 nm. On the organic compound layer, lithium fluoride as a cathode buffer layer and aluminum as a cathode were vapor-deposited to thicknesses of 0.5 nm. and 150 nm, respectively, in a vapor deposition apparatus. This was placed in a glove box substituted with argon gas so as not to be exposed to the atmosphere and sealed using a stainless steel-made sealing can and a UV-curable adhesive (XNR5516HV, manufactured by Nagase-CHIBA Ltd.) to manufacture an organic EL device in Comparative Example 2-1. A direct current constant voltage was applied to the organic EL device to emit light by using a Source Measure Unit 2400 type manufactured by TOYO TECHNIKA INC., and as a result, the light emission derived from A-2 could be obtained.

(Examples 2-1 to 2-94 and Comparative Examples 2-2 to 2-9)

Devices in Examples 2-1 to 2-94 and Comparative Examples 2-2 to 2-9 were manufactured in the same manner as in Comparative Example 2-1, except that the materials used in Comparative Example 2-1 were changed into the materials described in Table 2. A direct current constant voltage was applied to the organic EL device to emit light by using the Source Measure Unit 2400 type manufactured by TOYO TECHNIKA INC., and as a result, light emission of a color derived from each light emitting material could be obtained. The obtained devices of the present invention of Examples 2-1 to 2-94 and the obtained comparative devices of Comparative Examples 2-1 to 2-9 were evaluated in the same manner as Example 1. The results thereof were shown in Table 2. Meanwhile, a value of the device in Comparative Example 2-1 was made to be 100, and with reference to the value, the half-luminance intensity time was represented by a relative value.

[Table 6]

Table 2

| | Light emitting layer | | Driving volage (V) | External quantum efficiency (%) | Half-luminance intensity time (Relative value) | Initial chromaticity |
|---|---|---|---|---|---|---|
| | Light emitting material | Host material | | | | |
| Comparative example 2-1 | A-2 | H-1 | 14.6 | 4.2 | 100 | (0.16, 0.27) |
| Comparative example 2-2 | A-1 | H-1 | 15.1 | 2.8 | 11 | (0.18, 0.32) |
| Comparative example 2-3 | A-3 | H-1 | 14.8 | 4.6 | 235 | (0.16, 0.27) |

(continued)

| | Light emitting layer | | Driving volage (V) | External quantum efficiency (%) | Half-luminance intensity time (Relative value) | Initial chromaticity |
|---|---|---|---|---|---|---|
| | Light emitting material | Host material | | | | |
| Comparative example 2-4 | A-4 | H-1 | 15.3 | 3.8 | 63 | (0.16, 0.27) |
| Comparative example 2-5 | A-5 | H-1 | 14.2 | 4.3 | 48 | (0.18, 0.31) |
| Comparative example 2-6 | A-6 | H-1 | 13.9 | 4.5 | 111 | (0.17, 0.28) |
| Comparative example 2-7 | A-7 | H-1 | 14.5 | 4.1 | 35 | (0.16, 0.27) |
| Comparative example 2-8 | A-1 | C-6 | 15.8 | 2.9 | 13 | (0-18, 0.31) |
| Comparative example 2-9 | A-1 | C-10 | 15.9 | 2.7 | 12 | (0.17, 0.32) |
| Example 2-1 | A-2 | C-5 | 14.3 | 3.1 | 114 | (0.16, 0.26) |
| Example 2-2 | A-2 | C-6 | 14.4 | 5.6 | 165 | (0.15, 0.22) |
| Example 2-3 | A-2 | C-12 | 13.9 | 5.6 | 154 | (0.15, 0.22) |
| Example 2-4 | A-2 | C-25 | 14.3 | 5.4 | 148 | (0.15, 0.24) |
| Example 2-5 | A-2 | C-27 | 14.2 | 5.0 | 122 | (0.16, 0.24) |
| Example 2-6 | A-2 | C-30 | 14.4 | 4.9 | 120 | (0.16,0.25) |
| Example 2-7 | A-2 | C-37 | 14.4 | 5.1 | 135 | (0.15, 0.24) |
| Example 2-8 | A-2 | C-44 | 14.6 | 5.0 | 124 | (0.16, 0.25) |
| Example 2-9 | A-2 | C-48 | 13.9 | 5.8 | 241 | (0.15, 0.22) |
| Example 2-10 | A-2 | C-49 | 14.0 | 5.1 | 133 | (0-15, 0,23) |
| Example 2-11 | A-2 | C-56 | 14.2 | 5.1 | 162 | (0.16, 0.24) |
| Example 2-12 | A-2 | C-62 | 14.5 | 4.7 | 117 | (0.16, 0.25) |
| Example 2-13 | A-2 | C-63 | 14.5 | 4.9 | 139 | (0.16, 0.24) |
| Example 2-14 | A-2 | C-69 | 14.4 | 5.0 | 138 | (0.15, 0.24) |
| Example 2-15 | A-2 | C-73 | 14.2 | 5.7 | 179 | (0.15, 0.22) |
| Example 2-16 | A-2 | C-79 | 14.4 | 5.2 | 141 | (0.15, 0.23) |
| Example 2-17 | A-2 | C-84 | 14.1 | 5.6 | 162 | (0.15, 0.22) |
| Example 2-18 | A-2 | C-90 | 14.6 | 5.0 | 118 | (0.15, 0.24) |
| Example 2-19 | A-3 | C-5 | 14.1 | 5.4 | 285 | (0.15, 0.23) |
| Example 2-20 | A-3 | C-6 | 14.2 | 5.8 | 364 | (0.15, 0.21) |
| Example 2-21 | A-3 | C-12 | 13.7 | 6.2 | 386 | (0.15, 0.20) |
| Example 2-22 | A-3 | C-23 | 14.3 | 5.8 | 419 | (0.15, 0.23) |
| Example 2-23 | A-3 | C-4 | 14.4 | 5.3 | 283 | (0.15, 0.23) |
| Example 2-24 | A-3 | C-36 | 14.4 | 5.4 | 314 | (0.15, 0.23) |
| Example 2-25 | A-3 | C-38 | 14.1 | 6.0 | 375 | (0.15, 0.22) |

(continued)

| | Light emitting layer | | Driving volage (V) | External quantum efficiency (%) | Half-luminance intensity time (Relative value) | Initial chromaticity |
|---|---|---|---|---|---|---|
| | Light emitting material | Host material | | | | |
| Example 2-26 | A-3 | C-43 | 14.2 | 5.7 | 300 | (0.15, 0.23) |
| Example 2-27 | A-3 | C-16 | 13.5 | 6.5 | 529 | (3.15, 0.21) |
| Example 2-28 | A-3 | C-9 | 14.4 | 5.3 | 311 | (0.15, 0.22) |
| Example 2-29 | A-3 | C-55 | 13.9 | 6.0 | 390 | (0.15, 0.22) |
| Example 2-30 | A-3 | C-61 | 14.5 | 5.5 | 288 | (0.15, 0.23) |
| Example 2-31 | A-3 | C-64 | 14.3 | 5.7 | 363 | (0.15, 0.23) |
| Example 2-32 | A-3 | C-71 | 14.7 | 5.1 | 257 | (0.16, 0.24) |
| Example 2-33 | A-3 | C-74 | 14.2 | 6.0 | 371 | (0.15, 0.22) |
| Example 2-34 | A-3 | C-80 | 14.5 | 5.1 | 265 | (0.15, 0.24) |
| Example 2-35 | A-3 | C-85 | 14.2 | 5.6 | 334 | (0.15, 0.23) |
| Example 2-36 | A-3 | C-89 | 14.5 | 5.3 | 277 | (0.15, 0.24) |

[Table 7]

Table 2 (Continued)

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Example 2-37 | A-4 | C-5 | 15.3 | 4.1 | 65 | (0.16, 0.25) |
| Example 2-38 | A-4 | C-6 | 14.8 | 4.8 | 111 | (0.15. 0.22) |
| Example 2-39 | A-4 | C-12 | 14.7 | 5.3 | 99 | (0.16, 0.23) |
| Example 2-40 | A-4 | C-24 | 14.9 | 4.7 | 95 | (0.16, 0.24) |
| Example 2-41 | A-4 | C-3 | 14.8 | 4.6 | 96 | (0.16, 0.23) |
| Example 2-42 | A-4 | C-31 | 14.8 | 5.0 | 118 | (0.16, 0.23) |
| Example 2-43 | A-4 | C-39 | 15.0 | 4.9 | 106 | (0.16, 0.23) |
| Example 2-44 | A-4 | C-40 | 15.1 | 4.6 | 74 | (0.16, 0.25) |
| Example 2-45 | A-4 | C-46 | 14.6 | 5.5 | 173 | (0.16, 0.23) |
| Example 2-46 | A-4 | C-52 | 14.9 | 4.6 | 80 | (0,16, 0.23) |
| Example 2-47 | A-4 | C-54 | 14.8 | 4.8 | 101 | (0.16, 0.24) |
| Example 2-48 | A-4 | C-60 | 14.9 | 4.7 | 95 | (0.16, 0.23) |
| Example 2-49 | A-4 | C-65 | 14.7 | 5.3 | 133 | (0.16, 0.22) |
| Example 2-50 | A-4 | C-70 | 15.0 | 4.8 | 91 | (0.15, 0.22) |
| Example 2-51 | A-4 | C-75 | 14.8 | 4.8 | 105 | (0.16, 0.23) |
| Example 2-52 | A-4 | C-78 | 14.9 | 4.7 | 98 | (0.16, 0.23) |
| Example 2-53 | A-4 | C-82 | 14.9 | 4.8 | 108 | (0.15, 0.23) |

(continued)

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Example 2-54 | A-4 | C-86 | 15.0 | 4.5 | 91 | (0.15, 0.23) |
| Example 2-55 | A-5 | C-5 | 14.2 | 4.4 | 53 | (0.17, 0.28) |
| Example 2-56 | A-5 | C-6 | 13.5 | 5.2 | 93 | (0.17, 0.27) |
| Example 2-57 | A-5 | C-12 | 13.2 | 5.7 | 77 | (0.17, 0.28) |
| Example 2-58 | A-5 | C-1 | 14.0 | 4.8 | 70 | (0.17, 0.27) |
| Example 2-59 | A-5 | C-32 | 13.9 | 4.9 | 81 | (0.17, 0.27) |
| Example 2-60 | A-5 | C-45 | 14.1 | 4.5 | 56 | (0.16, 0.26) |
| Example 2-61 | A-5 | C-47 | 13.2 | 5.5 | 133 | (0.16, 0.27) |
| Example 2-62 | A-5 | C-56 | 14.0 | 5.3 | 107 | (0.17, 0,28) |
| Example 2-63 | A-5 | C-66 | 13.4 | 5.5 | 122 | (0.16, 0.27) |
| Example 2-64 | A-5 | C-72 | 13.6 | 5.1 | 88 | (0.17, 0.28) |
| Example 2-65 | A-5 | C-83 | 14.3 | 4.6 | 73 | (0.17, 0.28) |

[Table 8]

Table 2 (Continued)

| | Light emitting layer | | Diving voltage (V) | External quantum efficiency (%) | Half-luminance intensity time (Relative value) | Initial chromaticity |
|---|---|---|---|---|---|---|
| | Light emitting material | Host material | | | | |
| Example 2-66 | A-6 | C-5 | 13-6 | 5.2 | 128 | (0.16, 0.24) |
| Example 2-67 | A-6 | C-6 | 13.5 | 5.5 | 188 | (0.16, 0.23) |
| Example 2-68 | A-6 | C-12 | 13.6 | 5.3 | 165 | (0.15, 0.22) |
| Example 2-69 | A-6 | C-25 | 13.6 | 5.4 | 173 | (0.16, 0.23) |
| Example 2-70 | A-6 | C-26 | 13.4 | 5.2 | 154 | (0-15, 0,23) |
| Example 2-71 | A-6 | C-34 | 13.6 | 5.0 | 130 | (0.15, 0.23) |
| Example 2-72 | A-6 | C-37 | 13.7 | 5.3 | 159 | (0.16, 0.24) |
| Example 2-73 | A-6 | C-42 | 13.8 | 5.1 | 138 | (0.16, 0.23) |
| Example 2-74 | A-6 | C-16 | 13.2 | 5.8 | 315 | (0.15, 0.23) |
| Example 2-75 | A-6 | C-50 | 13.6 | 5.2 | 163 | (0.15, 0.23) |
| Example 2-76 | A-6 | C-55 | 13.4 | 5.6 | 137 | (0.16, 0.23) |
| Example 2-77 | A-6 | C-57 | 13.6 | 5.5 | 167 | (0.16, 0.23) |
| Example 2-78 | A-6 | C-65 | 13.3 | 5.7 | 201 | (0.16, 0.23) |
| Example 2-79 | A-6 | C-68 | 13.7 | 5.5 | 174 | (0.15, 0.23) |
| Example 2-80 | A-6 | C-75 | 13.6 | 5.4 | 160 | (0.16, 0.23) |
| Example 2-81 | A-6 | C-81 | 14.0 | 5.0 | 128 | (0.16, 0.23) |

(continued)

| | Light emitting layer | | Diving voltage (V) | External quantum efficiency (%) | Half-luminance intensity time (Relative value) | Initial chromaticity |
|---|---|---|---|---|---|---|
| | Light emitting material | Host material | | | | |
| Example 2-82 | A-6 | C-82 | 13.6 | 5.3 | 177 | (0.16, 0.23) |
| Example 2-83 | A-6 | C-87 | 13.8 | 5.4 | 172 | (0.15, 0.22) |
| Example 2-84 | A-7 | C-5 | 13.9 | 4.8 | 54 | (0.16, 0.23) |
| Example 2-85 | A-7 | C-6 | 13.8 | 5.0 | 61 | (0.16, 0.23) |
| Example 2-86 | A-7 | C-12 | 13.6 | 5.3 | 56 | (0.15, 0.23) |
| Example 2-87 | A-7 | C-28 | 14.2 | 4.6 | 50 | (0.16, 0.24) |
| Example 2-88 | A-7 | C-31 | 13.6 | 5.2 | 65 | (0.16, 0.23) |
| Example 2-89 | A-7 | C-41 | 14.2 | 4.5 | 44 | (0.16, 0.23) |
| Example 2-90 | A-7 | C-48 | 13.5 | 5.3 | 98 | (0.16, 0.23) |
| Example 2-91 | A-7 | C-59 | 13.8 | 4.9 | 52 | (0.16, 0,24) |
| Example 2-92 | A-7 | C-67 | 13.9 | 5.0 | 47 | (0.16, 0.23) |
| Example 2-93 | A-7 | C-76 | 14.1 | 4.9 | 54 | (0.15, 0,23) |
| Example 2-94 | A-7 | C-91 | 14.3 | 4.7 | 41 | (0.15, 0.23) |

As apparent from the above results, although the devices were manufactured by using the wet process, the device of the present invention had higher external quantum efficiency, lower driving voltage, and more improved durability than the comparative devices. Further, the color purity was improved, and thus, preferable chromaticity could be used as a blue luminescence device.

<Example 3>

[Examples 3-1 to 3-12 and Reference Examples 3-1 to 3-4]

A device was manufactured in the same manner as in Comparative Example 1-1and evaluated in the same manner as in Example 1, except that the compounds used for the light emitting material, the host material, and the adjacent layer material were changed into those described in Table 3. In all of the devices, phosphorescent light emission derived from the light emitting material used could be obtained. The evaluation results are shown in Table 3.

[Table 9]

Table 3

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 3-1 | A-2 | C-10 | C-10 | 9.8 | 8.9 | 176 | (0.16,0.23) |
| Example 3-1 | A-2 | C-15 | C-15 | 9.9 | 8.8 | 216 | (0.15, 0.23) |
| Example 3-2 | A-2 | C-16 | C-16 | 9.8 | 8.9 | 201 | (0.15, 0.23) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Example 3-3 | A-2 | C-17 | C-17 | 9.8 | 8.8 | 210 | (0.16, 0.23) |
| Reference Example 3-2 | A-3 | C-10 | C-10 | 9.9 | 8.8 | 438 | (0.16, 0.22) |
| Example 3-4 | A-3 | C-15 | C-15 | 10.1 | 8.7 | 492 | (0.16, 0,23) |
| Example 3-5 | A-3 | C-16 | C-16 | 9.9 | 8.7 | 537 | (0.15, 0.22) |
| Example 3-6 | A-3 | C-17 | C-17 | 9.9 | 8.8 | 509 | (0.16, 0.23) |
| Reference Example 3-3 | A-5 | C-10 | C-10 | 10.7 | 7.6 | 108 | (0,16, 0,26) |
| Example 3-7 | A-5 | C-15 | C-15 | 11.2 | 7.1 | 84 | (0.18, 0.29) |
| Example 3-8 | A-5 | C-16 | C-16 | 10.7 | 7.4 | 129 | (0.16, 0,26) |
| Example 3-9 | A-5 | C-17 | C-17 | 11.2 | 7.2 | 85 | (0.17, 0.29) |
| Reference Example 3-4 | A-7 | C-10 | C-10 | 10.8 | 8.3 | 65 | (0.15, 0-21) |
| Example 3-10 | A-7 | C-15 | C-15 | 10.7 | 8.3 | 113 | (0,15, 0.22) |
| Example 3-11 | A-7 | C-16 | C-16 | 10.8 | 8.4 | 94 | (0.15, 0.22) |
| Example 3-12 | A-7 | C-17 | C-17 | 10.8 | 8.4 | 105 | (0.15, 0.22) |

As apparent from the above results, when the substituent had a large volume in the specific phosphorescent metal complex used in the light emitting layer, if the host material was unsubstituted or a branched alkyl group, the manufactured device had improved durability. Meanwhile, when the substituent of the specific phosphorescent metal complex did not have a large volume, if the host material was a substituent having a large volume or a branched alkyl group, the manufactured device had more improved durability and good purity.

Example 4 [Examples 4-1 to 4-19, Comparative Example 4-1, and Reference Examples 4-1 to 4-3]

A device was manufactured in the same manner as in Comparative Example 1-1 and evaluated in the same manner as in Example 1, except that the compounds used for the light emitting material, the host material, and the adjacent layer material were changed into those described in Table 4. A mass ratio of alkyl substituents contained in the light emitting layer to the total mass of the light emitting layer (the total mass of the light emitting material and the host material contained in the light emitting layer) was disclosed as "a mass ratio of alkyl groups in the light emitting layer". The phosphorescent light emission derived from the light emitting material using all of the devices could be obtained. The obtained results were incorporated into Table 4. Meanwhile, a value of the device in Comparative Example 1-1 was made to be 100, and with reference to the value, the half-luminance intensity time was represented by a relative value.

Meanwhile, the mass ratio of alkyl groups in the light emitting layer was calculated from the following Equation.

[Equation 2]

$$\text{Mass ratio of alkyl groups} = \frac{\text{Ratio of light emitting material in the light emitting layer (mass ratio)} \times \text{Mass number of alkyl groups substituted in the light emitting material} + \text{Ratio of host material in the light emitting layer (mass ratio)} \times \text{Mass number of alkyl groups substituted in the host material}}{\text{Ratio of light emitting material in the light emitting layer (mass ratio)} \times \text{Mass number of the light emitting material} + \text{Ratio of host material in the light emitting layer (mass ratio)} \times \text{Mass number of the host material}}$$

[Table 10]

Table 4

| | Light emitting layer | | | Adjacent layer material | Driving voltage (V) | External quantum efficiency (%) | Half-luminance intensity time (Relative value) | Initial chromaticity |
|---|---|---|---|---|---|---|---|---|
| | Light emitting material | Host material | Alkyl group in light emitting layer (mass ratio) | | | | | |
| Comparative example 1-1 | A-2 | H-1 | 0.02 | H-1 | 12.1 | 6.2 | 100 | (0.16, 0.28) |
| Reference Example 4-1 | A-2 | C-10 | 0.02 | C-10 | 9.8 | 8.9 | 176 | (0.16, 0.23) |
| Example 4-1 | A-2 | C-18 | 0.13 | C-18 | 9.7 | 8.9 | 187 | (0.16, 0.23) |
| Example 4-2 | A-2 | C-16 | 0.21 | C-16 | 9.8 | 8.9 | 201 | (0.15, 0.23) |
| Example 4-3 | A-2 | C-19 | 0.28 | C-19 | 10.8 | 7.9 | 140 | (0.16, 0.24) |
| Example 4-4 | A-2 | C-20 | 0.34 | C-20 | 13.1 | 6.0 | 108 | (0.16, 0.27) |
| Example 4-5 | A-2 | C-21 | 0.12 | C-21 | 10.3 | 8.1 | 129 | (0.16, 0.23) |
| Example 1-1 | A-2 | C-1 | 0.20 | C-1 | 10.5 | 8.1 | 142 | (0.16, 0.23) |
| Example 4-6 | A-2 | C-22 | 0.26 | C-22 | 10.9 | 7.8 | 125 | (0.16, 0.23) |
| Comparative example 1-7 | A-7 | H-1 | 0.01 | H-1 | 12.3 | 6.1 | 32 | (0.16, 0.27) |
| Reference Example 4-2 | A-7 | C-10 | 0.01 | C-10 | 10.8 | 8.3 | 65 | (0.15,0.21) |
| Example 4-8 | A-7 | C-18 | 0.12 | C-18 | 10.8 | 8.3 | 78 | (0.15,0.22) |
| Example 3-11 | A-7 | C-16 | 0.21 | C-16 | 10.8 | 8.4 | 94 | (0.15,0.22) |
| Example 4-9 | A-7 | C-19 | 0.28 | C-19 | 11.6 | 7.5 | 60 | (0.16,0.24) |
| Example 4-10 | A-7 | C-20 | 0.34 | C-20 | 12.8 | 6.2 | 41 | (0.16,0.26) |
| Example 4-11 | A-7 | C-21 | 0.11 | C-21 | 11.4 | 7.9 | 37 | (0.16,0.23) |

(continued)

| | Light emitting layer | | | Adjacent layer material | Driving voltage (V) | External quantum efficiency (%) | Half-luminance intensity time (Relative value) | Initial chromaticity |
|---|---|---|---|---|---|---|---|---|
| | Light emitting material | Host material | Alkyl group in light emitting layer (mass ratio) | | | | | |
| Example 1-15 | A-7 | C-1 | 0.19 | C-1 | 11.5 | 8.0 | 51 | (0.15,0.23) |
| Example 4-12 | A-7 | C-22 | 0.26 | C-22 | 11.9 | 7.7 | 35 | (0.16,0.23) |
| Comparative example 4-1 | A-8 | H-1 | 0.07 | H-1 | 12.4 | 5.9 | 78 | (0.16,0.27) |
| Reference Example 4-3 | A-8 | C-10 | 0.07 | C-10 | 10.5 | 8.1 | 138 | (0.15,0.22) |
| Example 4-13 | A-8 | C-18 | 0.17 | C-18 | 10.6 | 8.1 | 155 | (0.15,0.22) |
| Example 4-14 | A-8 | C-16 | 0.24 | C-16 | 10.8 | 8.0 | 152 | (0.15,0.23) |
| Example 4-15 | A-8 | C-19 | 0.31 | C-19 | 12.7 | 6.3 | 102 | (0.16,0.24) |
| Example 4-16 | A-8 | C-20 | 0.36 | C-20 | 14.6 | 5.9 | 82 | (0.16,0.26) |
| Example 4-17 | A-8 | C-21 | 0.17 | C-21 | 11.1 | 7.9 | 103 | (0.15,0.23) |
| Example 4-18 | A-8 | C-1 | 0.23 | C-1 | 11.0 | 7.9 | 111 | (0.15,0.23) |
| Example 4-19 | A-8 | C-22 | 0.30 | C-22 | 12.4 | 6.2 | 87 | (0.16,0.24) |

As apparent from the above results, when the mass ratio of alkyl groups in the light emitting layer was within the appropriated range, the manufactured device had high external quantum efficiency, low driving voltage and improved durability. Further, the color purity was improved and thus preferable chromaticity could be used as a blue luminescence device. If the number of carbon atoms of the substituents of the compound (host material) represented by Formula (1) and the mass ratio of alkyl groups in the light emitting layer were small, the durability in the device was deteriorated, and if being excessively large, the driving voltage increased and the durability was deteriorated.

As apparent from the above results, when the substituent had a large volume in the specific phosphorescent metal complex used in the light emitting layer, if the host material was unsubstituted, the manufactured device had high external quantum efficiency, low driving voltage, and improved durability. Further, the color purity was improved and thus preferable chromaticity could be used as a blue luminescence device.

<Reference Example>

[Reference Examples 5-1 to 5-31]

A device was manufactured in the same manner as in Comparative Example 1-1 and evaluated in the same manner as in Example 1, except that the compounds used for the light emitting material, the host material, and the adjacent layer material were changed into those described in Table 5. In all of the devices, tphosphorescent light emission derived from the light emitting material used could be obtained. The evaluation results are shown in Table 5.

[Table 11]

[Table 5]

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 5-1 | A-9 | C-1 | C-1 | 10.7 | 7.8 | 121 | (0.16, 0.23) |
| Reference Example 5-2 | A-9 | C-4 | C-4 | 12.0 | 6.9 | 103 | (0.16, 0.22) |
| Reference Example 5-3 | A-9 | C-9 | C-9 | 11.3 | 7.0 | 107 | (0.15, 0.22) |
| Reference Example 5-4 | A-9 | C-12 | C-12 | 10.7 | 7.8 | 122 | (0.16, 0.24) |
| Reference Example 5-5 | A-9 | C-36 | C-36 | 11.5 | 7.0 | 100 | (0.15, 0.22) |
| Reference Example 5-6 | A-9 | C-45 | C-45 | 11.7 | 7.5 | 121 | (0.16, 0.23) |
| Reference Example 5-7 | A-9 | C-48 | C-48 | 10.8 | 8.0 | 181 | (0.16, 0.23) |
| Reference Example 5-8 | A-9 | C-66 | C-66 | 11.0 | 7.7 | 143 | (0.16, 0.23) |
| Reference Example 5-9 | A-9 | C-74 | C-74 | 11.2 | 7.5 | 125 | (0.16, 0.25) |
| Reference Example 5-10 | A-9 | C-77 | C-77 | 11.2 | 7.4 | 117 | (0.16, 0.23) |
| Reference Example 5-11 | A.10 | C-23 | C-23 | 11.2 | 7.6 | 369 | (0.16, 0.21) |
| Reference Example 5-12 | A-10 | C-45 | C-45 | 11.6 | 7.0 | 229 | (0.15, 0.21) |
| Reference Example 5-13 | A-10 | C-48 | C-48 | 10.6 | 8.2 | 444 | (0.15,0.21) |
| Reference Example 5-14 | A-10 | C-16 | C-16 | 10.4 | 8.3 | 476 | (0.15, 0:22) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 5-15 | A-10 | C-12 | C-12 | 10.6 | 8.0 | 281 | (0.16, 0.22) |
| Reference Example 5-16 | A-10 | C-56 | C-56 | 10.5 | 8.0 | 343 | (0.16, 0.22) |
| Reference Example 5-17 | A-10 | C-65 | C-65 | 10.7 | 7.9 | 330 | (0.16, 0.22) |
| Reference Example 5-18 | A-10 | C-66 | C-66 | 10.6 | 8.0 | 343 | (0.16, 0.22) |
| Reference Example 5-19 | A-10 | C-74 | C-74 | 10.7 | 7.8 | 295 | (0.16, 0.22) |
| Reference Example 5-20 | A-10 | C-84 | C-84 | 10.8 | 7.7 | 278 | (0.16, 0.21) |
| Reference Example 5-21 | A-10 | C-91 | C-91 | 11.2 | 7.5 | 249 | (0.16, 0.21) |
| Reference Example 5-22 | A-11 | C-6 | C-6 | 10.6 | 7.7 | 138 | (0.16, 0.24) |
| Reference Example 5-23 | A-11 | C-12 | C-12 | 10.5 | 8.0 | 142 | (0.16, 0.24) |
| Reference Example 5-24 | A-11 | C-26 | C-26 | 10.9 | 7.5 | 104 | (0.16, 0.23) |
| Reference Example 5-25 | A-11 | C-36 | C-36 | 11.0 | 7.7 | 130 | (0.16, 0.24) |
| Reference Example 5-26 | A-11 | C-16 | C-16 | 10.3 | 8.4 | 241 | (0.15, 0.23) |
| Reference Example 5-27 | A-11 | C-53 | C-53 | 11.3 | 7.3 | 122 | (0.16,0.23) |
| Reference Example 5-29 | A-11 | C-61 | C-61 | 11.0 | 7.5 | 133 | (0.16,0.24) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 5-29 | A-11 | C-71 | C-71 | 11.2 | 7.3 | 125 | (0.16,0.25) |
| Reference Example 5-30 | A-11 | C-86 | C-86 | 11.0 | 7.7 | 132 | (0.16,0.24) |
| Reference Example 5-31 | A-11 | C-91 | C-91 | 10.9 | 7.4 | 137 | (0.16, 0.23) |

[Reference Examples 6-1 to 6-30]

A device was manufactured in the same manner as in Comparative Example 2-1 and evaluated in the same manner as in Example 1, except that the compounds used for the light emitting material and the host material were changed into those described in Table 6. In all of the devices, phosphorescent light emission derived from the light emitting material used could be obtained. The evaluation results are shown in Table 6.

[Table 12]

Table 6

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Reference Example 6-1 | A-9 | C-6 | 15.1 | 5.4 | 143 | (0.16, 0.23) |
| Reference Example 6-2 | A-9 | C-12 | 14.5 | 5.2 | 121 | (0.16, 0.22) |
| Reference Example 6-3 | A-9 | C-27 | 14.8 | 4.7 | 109 | (0.16, 0.25) |
| Reference Example 6-4 | A-9 | C-37 | 14.9 | 4.5 | 127 | (0.16, 0.24) |
| Reference Example 6-5 | A-9 | C-48 | 14.4 | 5.2 | 216 | (0.15, 0.23) |
| Reference Example 6-6 | A-9 | C-56 | 14.7 | 4.5 | 239 | (0.16, 0.25) |
| Reference Example 6-7 | A-9 | C-63 | 15.0 | 4.5 | 117 | (0.15, 0.23) |
| Reference Example 6-8 | A-9 | C-79 | 15.1 | 4.5 | 122 | (0.16, 0.25) |
| Reference Example 6-9 | A-9 | C-90 | 15.3 | 4.4 | 98 | (0.16, 0.25) |

(continued)

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Reference Example 6-10 | A-10 | C-6 | 14.9 | 5.4 | 333 | (0.15, 0.23) |
| Reference Example 6-11 | A-10 | C-12 | 14.2 | 5.7 | 351 | (0.15, 0.22) |
| Reference Example 6-12 | A-10 | C-23 | 14.7 | 5.4 | 380 | (0.16, 0.24) |
| Reference Example 6-13 | A-10 | C-36 | 14.8 | 5.0 | 286 | (0.15, 0.24) |
| Reference Example 6-14 | A-10 | C-43 | 14.9 | 5.1 | 272 | (0.16, 0.24) |
| Reference Example 6-15 | A-10 | C-16 | 14.2 | 6.0 | 469 | (0.15, 0.23) |
| Reference Example 6-16 | A-10 | C-55 | 14.5 | 5.6 | 334 | (0.15, 0.23) |
| Reference Example 6-17 | A-10 | C-61 | 15.1 | 5.0 | 255 | (0.16, 0.24) |
| Reference Example 6-18 | A-10 | C-71 | 15.2 | 4.6 | 225 | (0.16, 0.25) |
| Reference Example 6-19 | A-10 | C-80 | 15.1 | 4.4 | 239 | (0.16, 0.25) |
| Reference Example 6-20 | A-10 | C-89 | 15.2 | 4.9 | 247 | (0.16, 0.24) |
| Reference Example 6-21 | A-11 | C-6 | 14.1 | 5.0 | 165 | (0.16, 0.24) |
| Reference Example 6-22 | A-11 | C-12 | 14.2 | 4.8 | 144 | (0.15, 0.24) |
| Reference Example 6-23 | A-11 | C-25 | 14.2 | 4.9 | 148 | (0.16, 0.24) |

(continued)

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Reference Example 6-24 | A-11 | C-34 | 14.7 | 4.1 | 102 | (0.15, 0.24) |
| Reference Example 6-25 | A-11 | C-42 | 14.2 | 4.5 | 117 | (0.16, 0.23) |
| Reference Example 6-26 | A-11 | C-16 | 13.9 | 5.5 | 283 | (0.15, 0.24) |
| Reference Example 6-27 | A-11 | C-55 | 14.0 | 5.2 | 176 | (0.16, 0.23) |
| Reference Example 6-28 | A-11 | C-65 | 13.9 | 5.3 | 179 | (0.16, 0.24) |
| Reference Example 6-29 | A-11 | C-75 | 14.2 | 5.0 | 140 | (0.16, 0.23) |
| Reference Example 6-30 | A-11 | C-87 | 14.5 | 4.9 | 151 | (0.15, 0.23) |

[Reference Examples 7-1 to 7-26]

A device was manufactured in the same manner as in Comparative Example 1-1 and evaluated in the same manner as in Example 1, except that the compounds used for the light emitting material, the host material, and the adjacent layer material were changed into those described in Table 7. In all of the devices, phosphorescent light emission derived from the light emitting material used could be obtained. The evaluation results are shown in Table 7.
[Table 13]

Table 7

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 7-1 | A-2 | C-2 | C-2 | 11.3 | 7.3 | 131 | (0.16,0.23) |
| Reference Example 7-2 | A-2 | C-7 | C-7 | 10.6 | 8-4 | 163 | (0.15,0.23) |
| Reference Example 7-3 | A-2 | C-8 | C-8 | 10.3 | 8.5 | 132 | (0.15,0.22) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 7-4 | A-2 | C-11 | C-11 | 10.1 | 8.7 | 122 | (0.15, 0.21) |
| Reference Example 7-5 | A-2 | C-13 | C-13 | 10.9 | 8.1 | 148 | (0.16, 0.23) |
| Reference Example 7-6 | A-2 | C-14 | C-14 | 11.1 | 8.7 | 159 | (0.16, 0.24) |
| Reference Example 7-7 | A-3 | C-2 | C-2 | 11.1 | 7.5 | 308 | (0.16, 0.22) |
| Reference Example 7-8 | A-3 | C-7 | C-7 | 10.0 | 8.4 | 372 | (0.15, 0.21) |
| Reference Example 7-9 | A-3 | C-8 | C-8 | 10.3 | 8.2 | 298 | (0.15, 0.19) |
| Reference Example 7-10 | A-3 | C-11 | C-11 | 10.3 | 8.5 | 273 | (0.15, 0.18) |
| Reference Example 7-11 | A-3 | C-13 | C-13 | 10.7 | 7.8 | 336 | (0.15, 0.21) |
| Reference Example 7-12 | A-3 | C-14 | C-14 | 11.0 | 7.7 | 377 | (0.15, 0.21) |
| Reference Example 7-13 | A-4 | C-2 | C-2 | 12.6 | 6.5 | 81 | (0.16, 0.23) |
| Reference Example 7-14 | A-4 | C-7 | C-7 | 11.7 | 7.3 | 126 | (0.16, 0.21) |
| Reference Example 7-15 | A-4 | C-10 | C-10 | 11.5 | 7.2 | 133 | (0.15, 0.21) |
| Reference Example 7-16 | A-4 | C-13 | C-13 | 12.1 | 7.0 | 103 | (0.15, 0.21) |
| Reference Example 7-17 | A-5 | C-2 | C-2 | 11.8 | 6.6 | 63 | (0.16, 0.28) |

(continued)

| | Light emitting layer | | Adjacent layer material | Driving voltage (V) | External quantum efficiency (%) | Half-luminance intensity time (Relative value) | Initial chromaticity |
|---|---|---|---|---|---|---|---|
| | Light emitting material | Host material | | | | | |
| Reference Example 7-18 | A-5 | C-7 | C-7 | 10.8 | 7.5 | 94 | (0.16, 0.26) |
| Reference Example 7-19 | A-5 | C-13 | C-13 | 11.0 | 7.2 | 88 | (0.17, 0.29) |
| Reference Example 7-20 | A-6 | C-2 | C-2 | 11.1 | 7.4 | 156 | (0.16, 0.24) |
| Reference Example 7-21 | A-6 | C-7 | C-7 | 10.2 | 8.6 | 193 | (0.15, 0.21) |
| Reference Example 7-22 | A-6 | C-10 | C-10 | 9.9 | 8.9 | 211 | (0.15, 0.22) |
| Reference Example 7-23 | A-6 | C-13 | C-13 | 10.7 | 8.3 | 176 | (0.16, 0.23) |
| Reference Example 7-24 | A-7 | C-2 | C-2 | 11.7 | 7.9 | 48 | (0.16, 0.24) |
| Reference Example 7-25 | A-7 | C-7 | C-7 | 11.0 | 8.2 | 58 | (0.15, 0.22) |
| Reference Example 7-26 | A-7 | C-13 | C-13 | 11.2 | 7.7 | 48 | (0.16, 0.24) |

[Reference Examples 8-1 to 8-16]

A device was manufactured in the same manner as in Comparative Example 2-1 and evaluated in the same manner as in Example 1, except that the compounds used for the light emitting material and the host material were changed into those described in Table 8. In all of the devices, phosphorescent light emission derived from the light emitting material used could be obtained. The evaluation results are shown in Table 8.

[Table 14]

Table 8

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Reference Example 8-1 | A-2 | C-8 | 13.8 | 6.3 | 129 | (0.15, 0.23) |

(continued)

| | Light emitting layer | | Driving voltage (V) | External quantum efficiency (%) | Half-luminance intensity time (Relative value) | Initial chromaticity |
|---|---|---|---|---|---|---|
| | Light emitting material | Host material | | | | |
| Reference Example 8-2 | A-2 | C-14 | 14.5 | 5.1 | 134 | (0.16, 0.25) |
| Reference Example 8-3 | A-3 | C-8 | 13.8 | 5.6 | 286 | (0.15, 0.20) |
| Reference Example 8-4 | A-3 | C-10 | 13.4 | 6.2 | 397 | (0.16, 0.22) |
| Reference Example 8-5 | A-3 | C-14 | 14.5 | 5.3 | 312 | (0.16, 0.23) |
| Reference Example 8-6 | A-4 | C-8 | 14.4 | 5.5 | 83 | (0.15, 0.21) |
| Reference Example 8-7 | A-4 | C-10 | 14.2 | 5.7 | 128 | (0.15, 0.22) |
| Reference Example 8-8 | A-4 | C-14 | 14.9 | 5.0 | 86 | (0.16, 0.23) |
| Reference Example 8-9 | A-5 | C-8 | 12.8 | 5.8 | 64 | (0.16, 0.27) |
| Reference Example 8-10 | A-5 | C-10 | 12.7 | 6.0 | 81 | (0.16, 0.27) |
| Reference Example 8-11 | A-5 | C-14 | 13.6 | 5,5 | 65 | (0.17, 0.28) |
| Reference Example 8-12 | A-6 | C-8 | 130 | 6.0 | 139 | (0.15, 0.21) |
| Reference Example 8-13 | A-6 | C-10 | 13.2 | 6.2 | 182 | (0.15,0.22) |
| Reference Example 8-14 | A-6 | C-1.4 | 13.9 | 5.3 | 150 | (0.16, 0.23) |
| Reference Example 8-15 | A-7 | C-8 | 13.2 | 5.6 | 38 | (0.15, 0.20) |
| Reference Example 8-16 | A-7 | C-14 | 13.8 | 5.1 | 44 | (0.16, 0,24) |

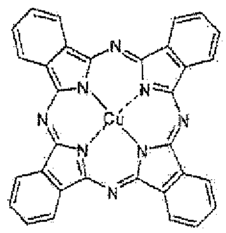
Copper phthalocyanine

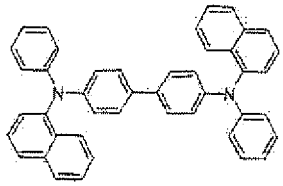
NPD

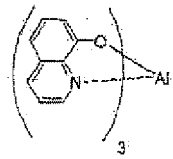
Alq

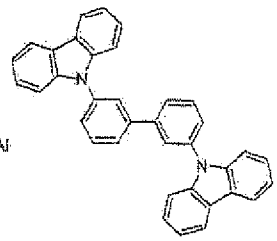
H-1

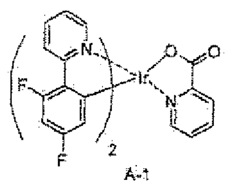
A-1

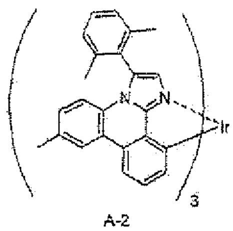
A-2

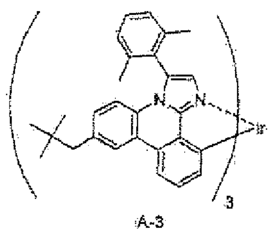
A-3

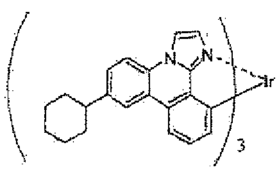
A-4

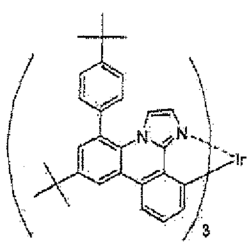
A-5

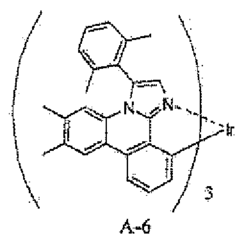
A-6

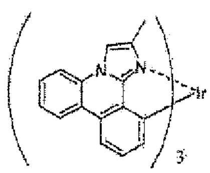
A-7

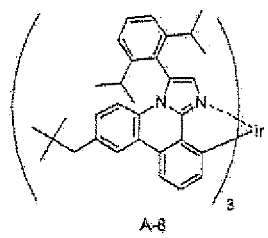
A-8

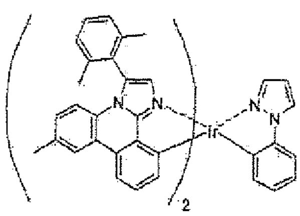
A-9

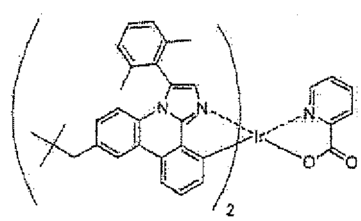
A-10

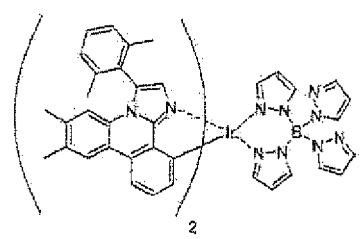
A-11

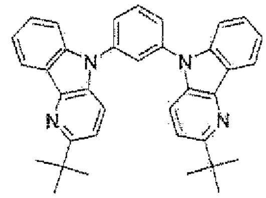
C-1

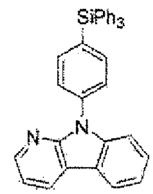
C-2

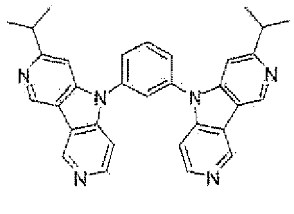
C-3

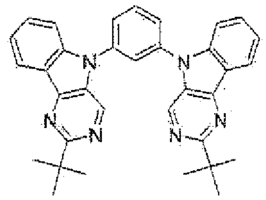
C-4

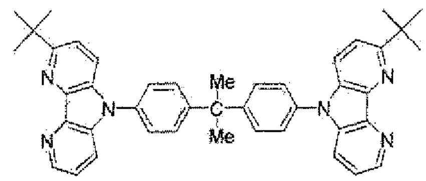
C-5

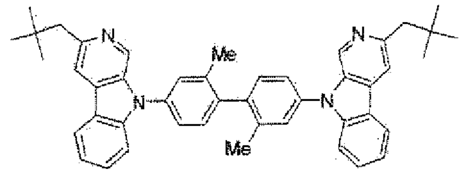
C-6

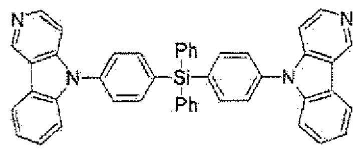
C-7

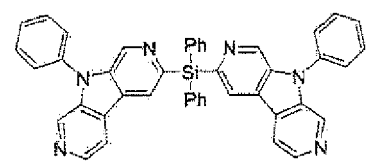
C-8

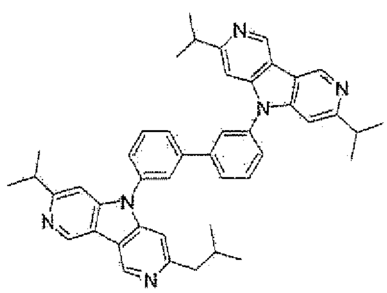
C-9

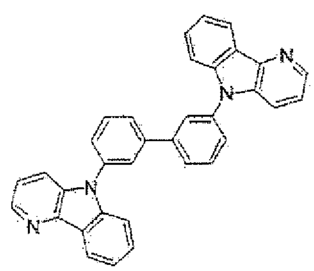
C-10

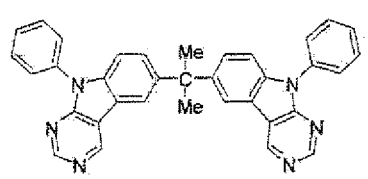
C-11

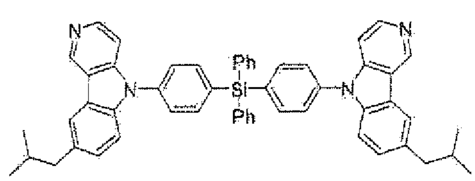
C-12

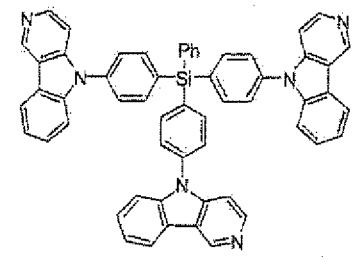

C-13

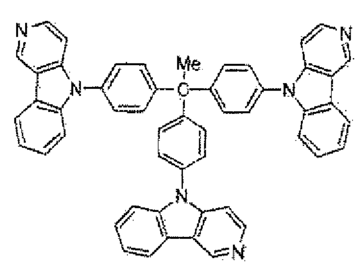

C-14

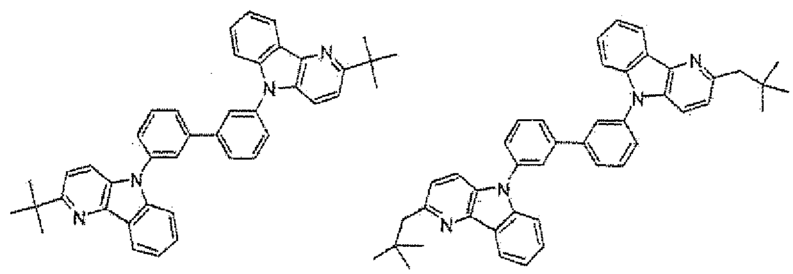

C-15

C-16

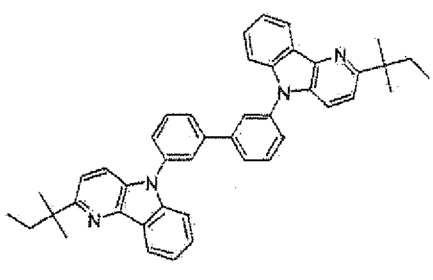

C-17

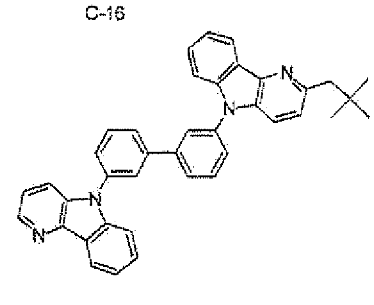

C-18

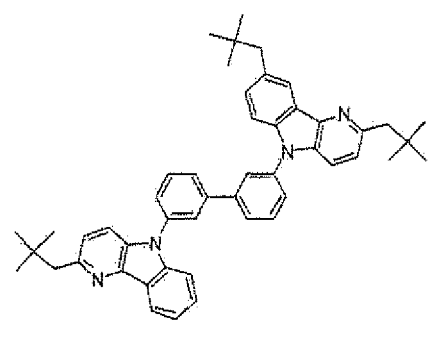

C-19

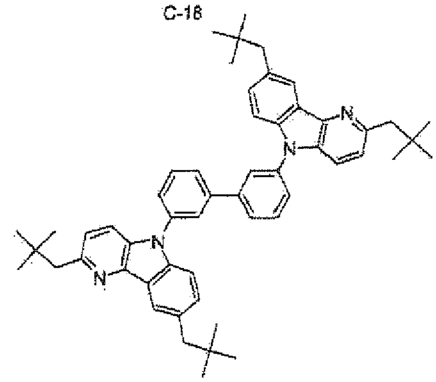

C-20

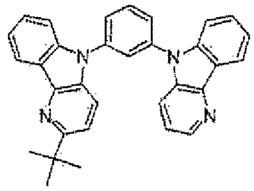

C-21

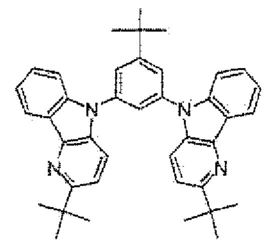

C-22

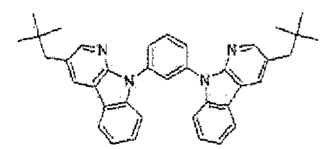

C-23

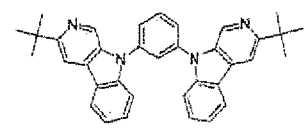

C-24

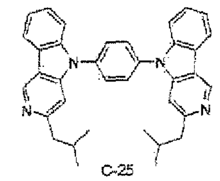

C-25

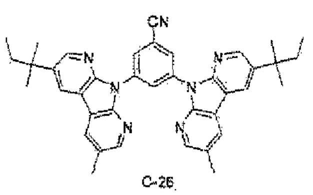

C-26

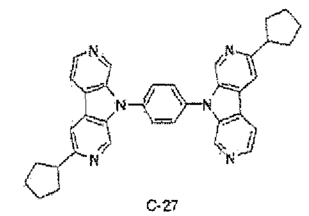

C-27

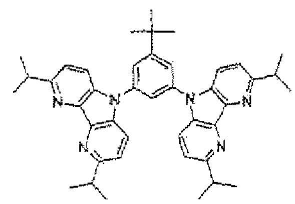

C-28

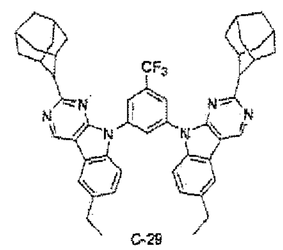

C-29

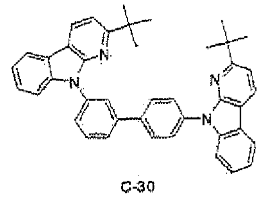

C-30

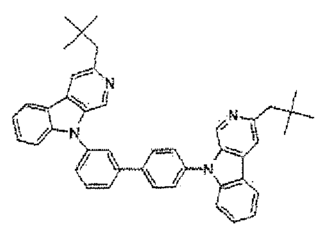

C-31

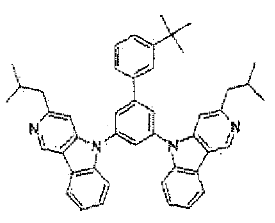

C-32

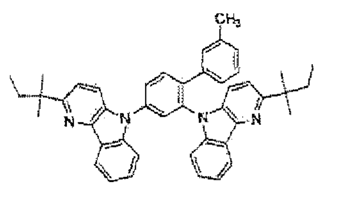

C-33

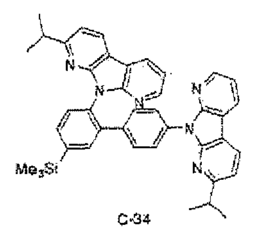

C-34

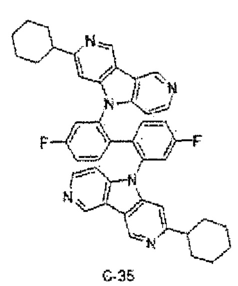

C-35

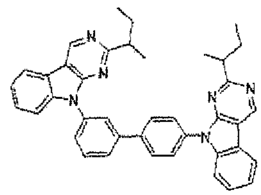

C-36

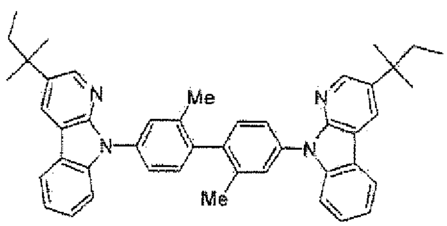

C-37

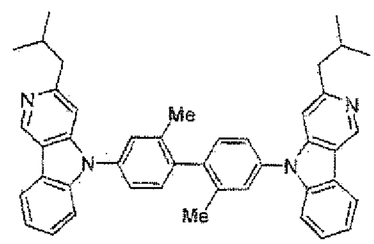

C-38

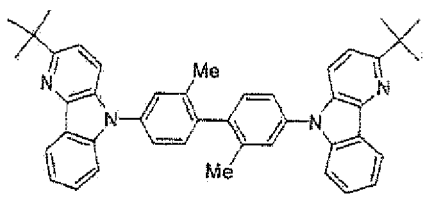

C-39

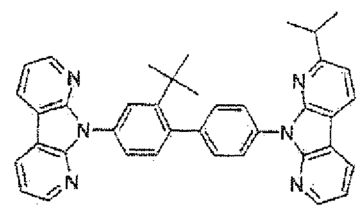

C-40

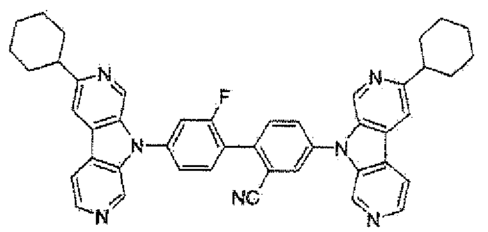

C-41

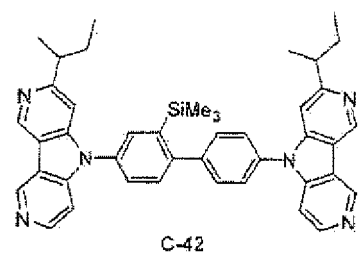

C-42

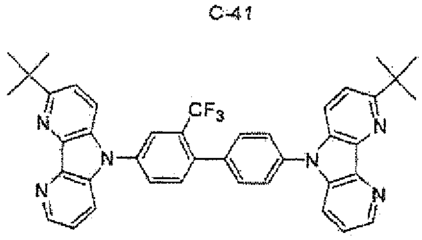

C-43

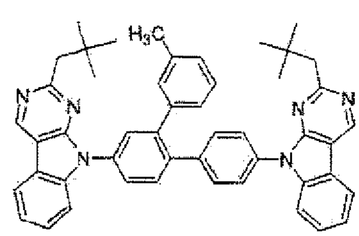

C-44

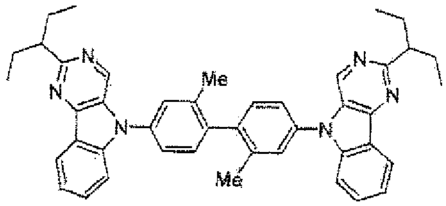

C-45

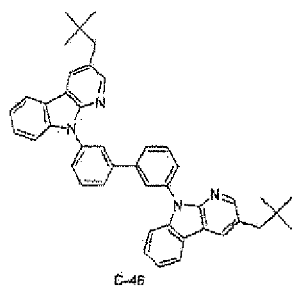

C-46

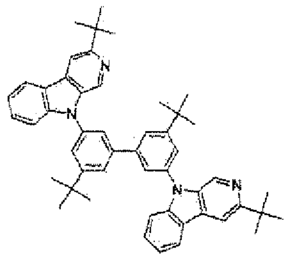

C-47

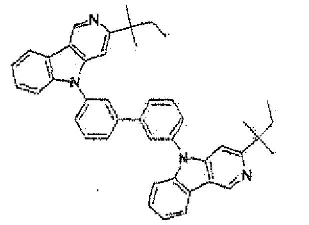

C-48

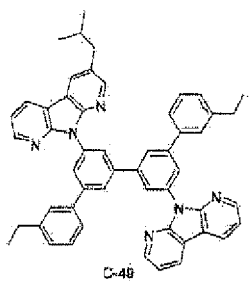

C-49

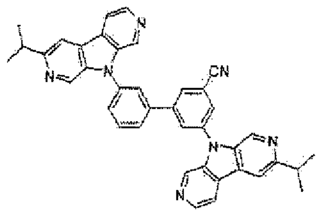

C-50

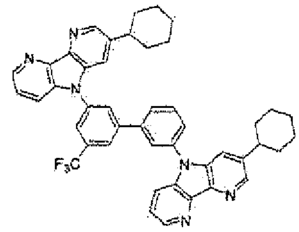

C-51

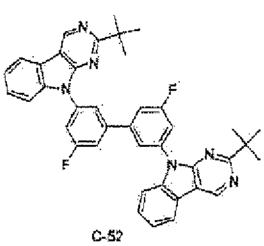

C-52

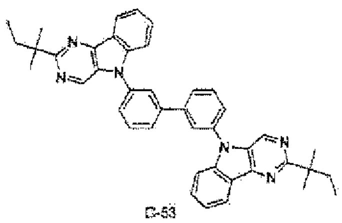

C-53

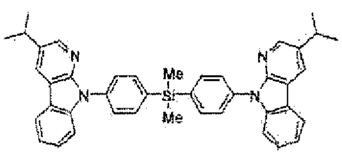

C-54

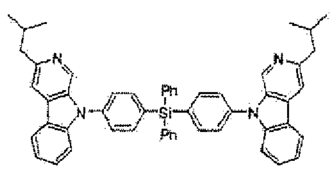

C-55

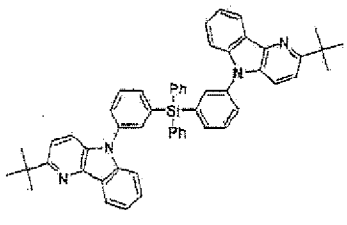

C-56

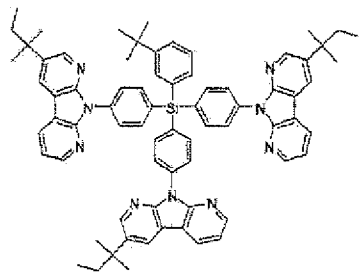

C-57

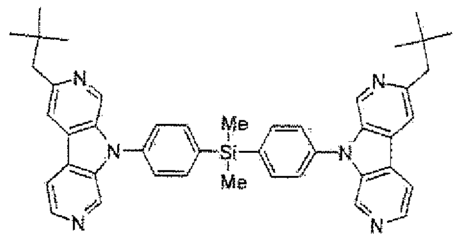

C-58

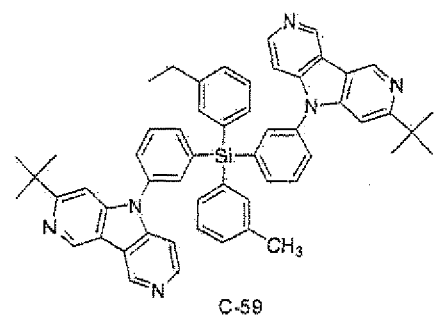

C-59

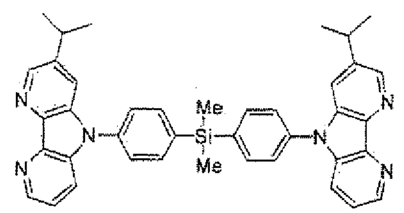

C-60

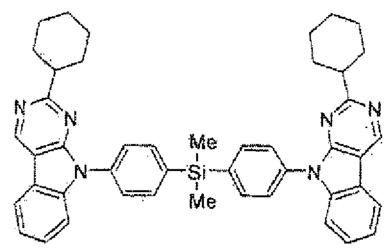

C-61

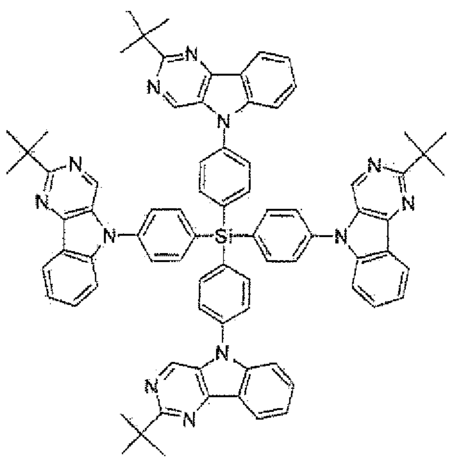

C-62

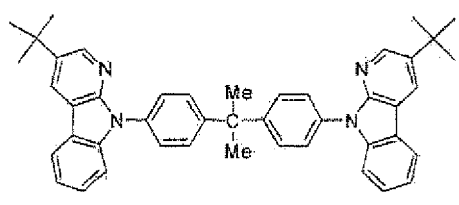

C-63

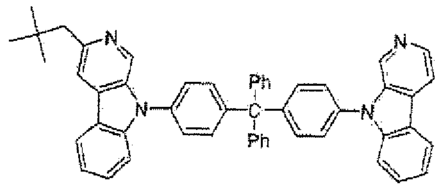

C-64

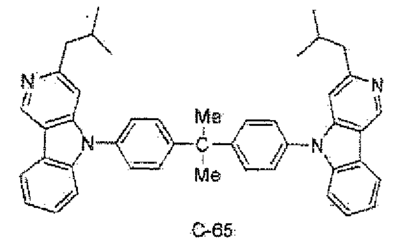

C-65

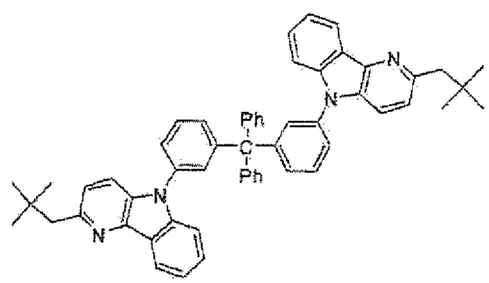

C-66

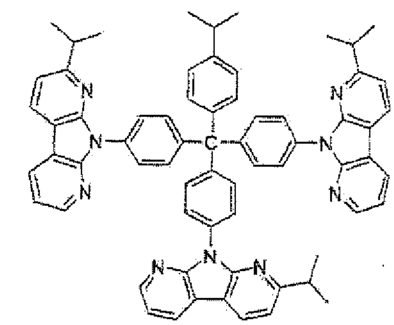

C-67

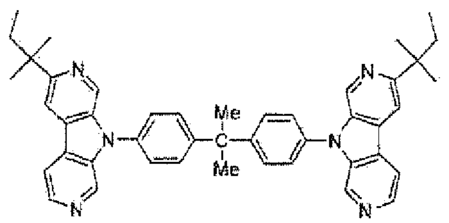

C-68

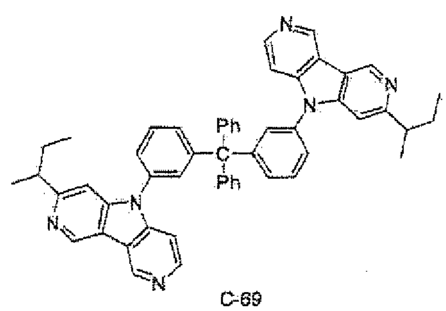

C-69

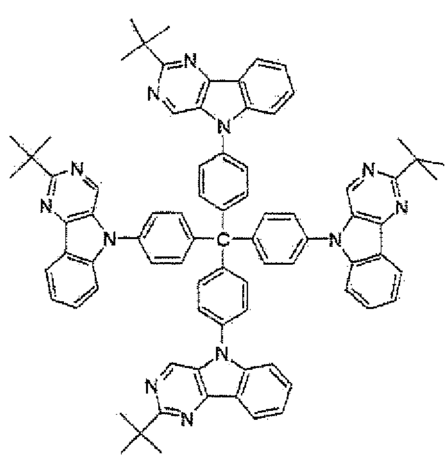

C-71

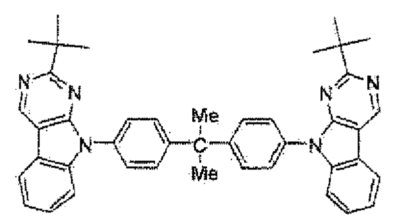

C-70

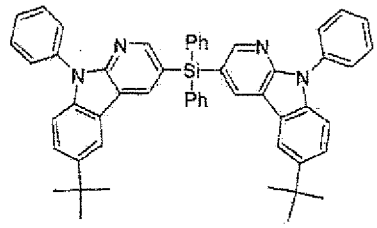

C-72

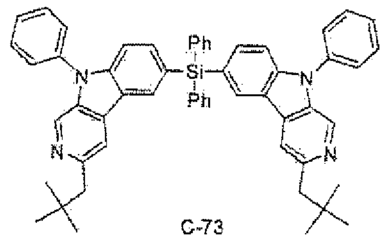

C-73

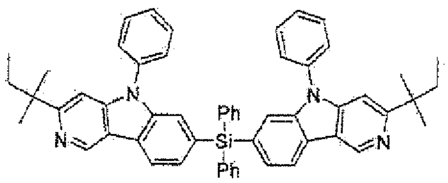

C-74

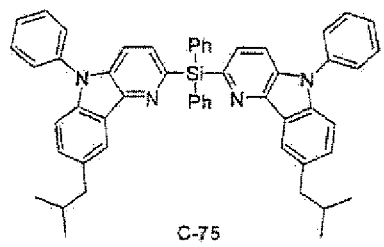

C-75

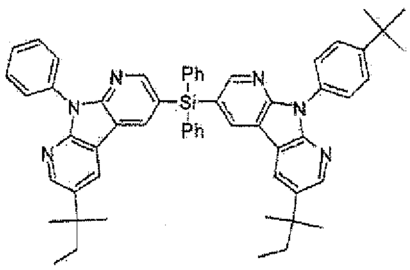

C-76

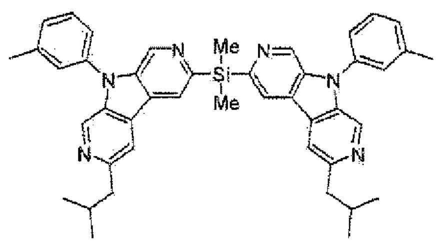

C-77

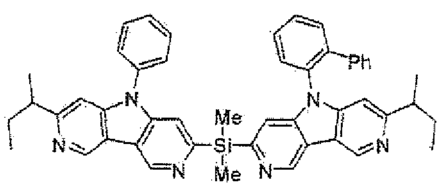

C-78

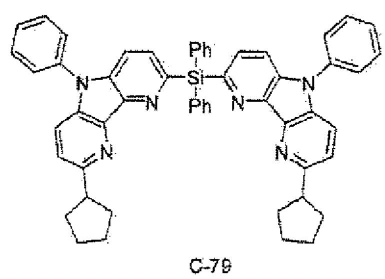

C-79

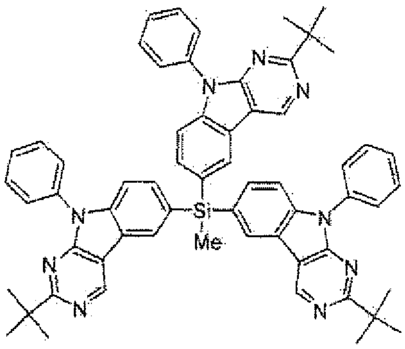

C-80

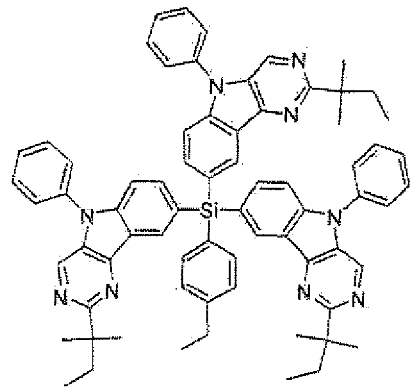

C-81

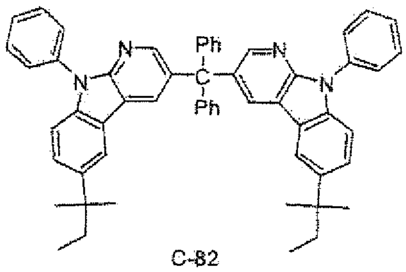

C-82

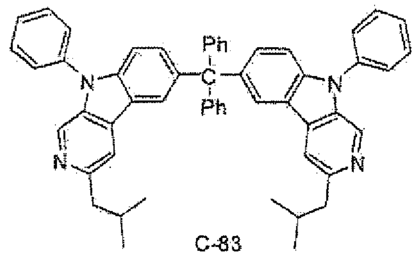

C-83

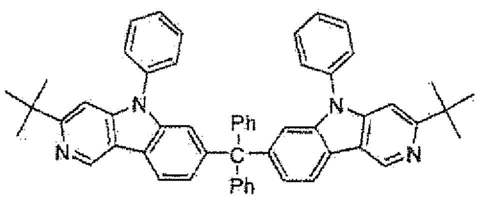

C-84

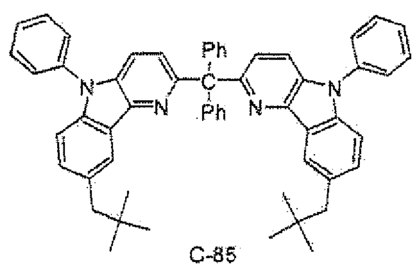

C-85

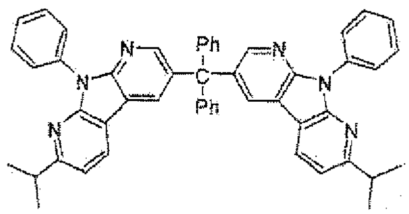

C-86

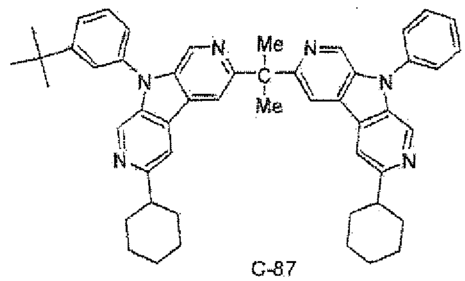

C-87

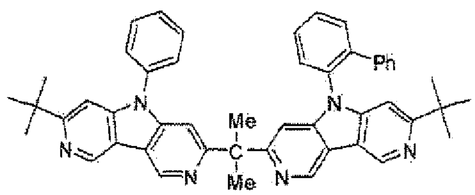

C-88

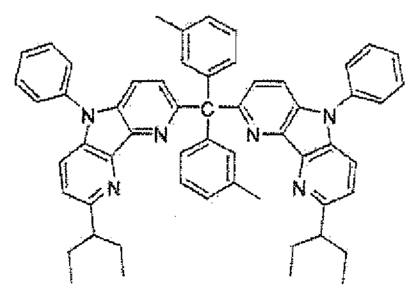

C-89

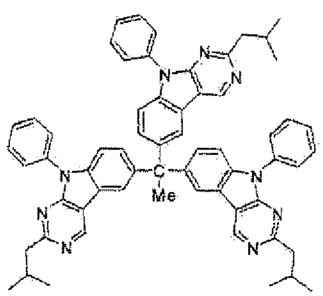

C-90

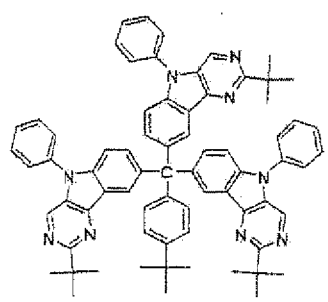

C-91

Industrial Applicability

The organic electroluminescence device of the present invention may be suitably used for display devices, displays, backlights, electrophotography, illumination light sources, recording light sources, exposure light sources, reading light sources, indicators, signboards, interiors, optical communication or the like. In particular, the organic electroluminescence device may be preferably used for devices driven in an area having high emission luminance intensity such as illumination apparatuses, display apparatuses and the like.

Although the present invention has been described with reference to detailed and specific embodiments thereof, it is obvious to those skilled in the art that various changes or modifications may be made without departing from the spirit and scope of the present invention.

This application claims priority from Japanese Patent Application (Japanese Patent Application No. 2009-201152) filed on August 31, 2009, Japanese Patent Application (Japanese Patent Application No. 2009-223452) filed on September 28, 2009, and Japanese Patent Application (Japanese Patent Application No. 2010-079922) filed on March 30, 2010, the disclosures of which are incorporated herein by reference in its entirety.

Explanation of Reference Numerals and Symbols

2··· Substrate
3··· Anode
4··· Hole injection layer
5··· Hole transporting layer
6··· Light emitting layer
7··· Hole blocking layer
8··· Electron transporting layer
9··· Cathode
10··· Organic electroluminescence device (Organic EL device)
11··· Organic layer
12··· Protective layer
14··· Adhesive layer
16··· Sealing vessel
20··· Light emission apparatus
30··· Light scattering member
30A··· Light incident surface
30B··· Light reflecting surface
32··· Microparticulate
40··· Illumination apparatus

## Claims

**1.** An organic electroluminescence device comprising at least one organic layer containing a light emitting layer comprising a light emitting material between a pair of electrodes, wherein the organic electroluminescence device comprises a compound having at least one group represented by any one of the following Formulas (7-1) to (7-10) and an iridium complex represented by the following Formula (A9-1):

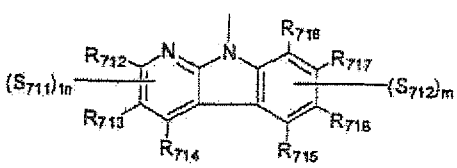
(7−1)

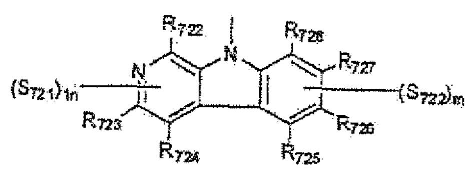
(7−2)

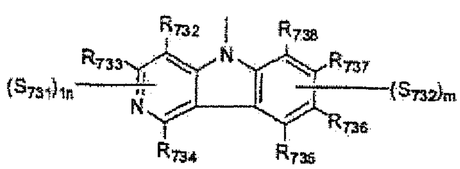
(7−3)

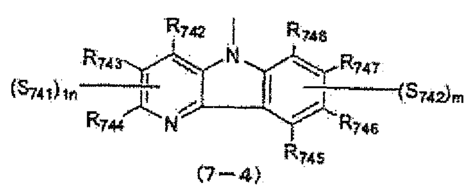
(7−4)

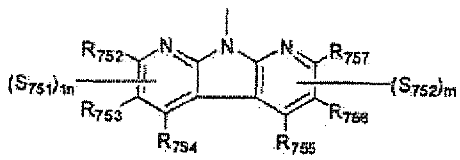

(7-5)

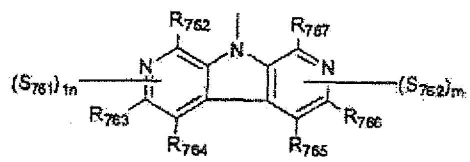

(7-6)

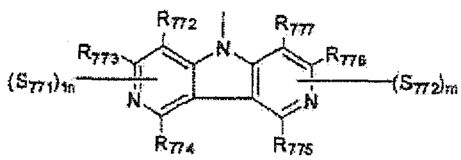

(7-7)

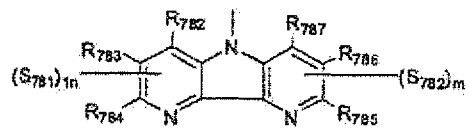

(7-8)

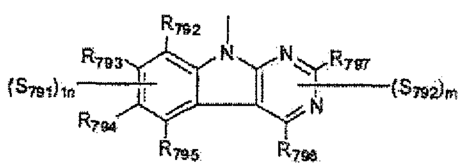

(7-9)

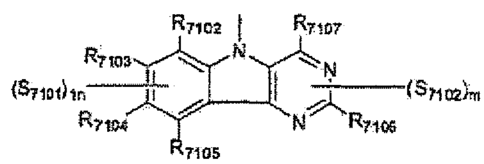

(7-10)

wherein, each of $R_{712}$ to $R_{718}$, $R_{722}$ to $R_{728}$, $R_{732}$ to $R_{738}$, $R_{742}$ to $R_{748}$, $R_{752}$ to $R_{757}$, $R_{762}$ to $R_{767}$, $R_{772}$ to $R_{777}$, $R_{782}$ to $R_{787}$, $R_{792}$ to $R_{797}$, and $R_{7102}$ to $R_{7107}$ independently represents a hydrogen atom, an alkyl group, or an alicyclic hydrocarbon group which may have an alkyl group; each of $S_{711}$ to $S_{7101}$ and $S_{712}$ to $S_{7102}$ independently represents the following substituent (S); each of $S_{711}$ to $S_{7101}$ is substituted to a carbon atom as $R_{712}$ to $R_{714}$, $R_{722}$ to $R_{724}$, $R_{732}$ to $R_{734}$, $R_{742}$ to $R_{744}$, $R_{752}$ to $R_{754}$, $R_{762}$ to $R_{764}$, $R_{772}$ to $R_{774}$, $R_{792}$ to $R_{784}$, $R_{792}$ to $R_{795}$, and $R_{7102}$ to $R_{7105}$; each of $S_{712}$ to $S_{7102}$ is substituted to a carbon atom as $R_{715}$ to $R_{718}$, $R_{725}$ to $R_{728}$, $R_{735}$ to $R_{738}$, $R_{745}$ to $R_{748}$, $R_{755}$ to $R_{757}$, $R_{765}$ to $R_{767}$, $R_{775}$ to $R_{777}$, $R_{785}$ to $R_{787}$, $R_{796}$ to $R_{797}$, and $R_{7106}$ to $R_{7107}$; ln and m represent integers of 0 to 4, and ln+m is an integer of 1 to 4:

Substituent (S)

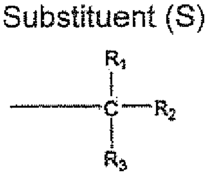

wherein, $R_1$ represents an alkyl group; $R_2$ represents a hydrogen atom or an alkyl group; $R_3$ represents a hydrogen atom or an alkyl group; and $R_1$ to $R_3$ may be linked to each other to form a ring:

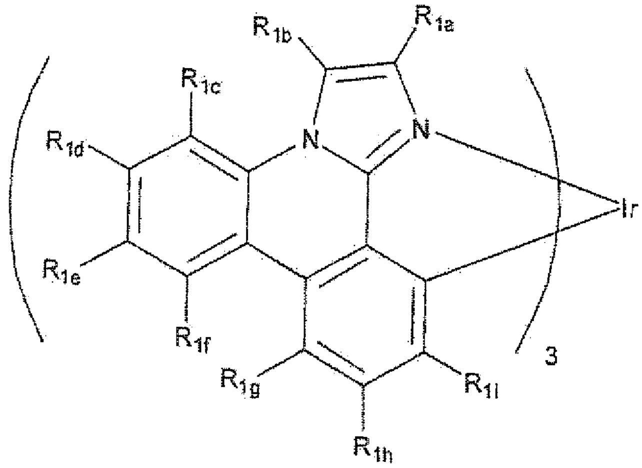

A9-1

wherein, each of $R_{1a}$ to $R_{1i}$ independently represents a hydrogen atom, an alkyl group, an alicyclic hydrocarbon group which may have an alkyl group, or a phenyl group which may have an alkyl group.

**2.** The organic electroluminescence device as claimed in claim 1, wherein the compound having at least one group represented by any one of Formulas (7-1) to (7-10) is a compound represented by the following Formula (8):

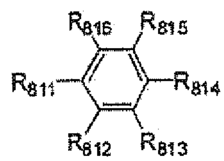

(8)

wherein, each of $R_{811}$ to $R_{816}$ independently represents a hydrogen atom, an alkyl group, an aryl group which may have an alkyl group, a fluorine group, a trifluoromethyl group, or a cyano group; and at least one of $R_{811}$ to $R_{816}$ is a group represented by any one of Formulas (7-1) to (7-10).

**3.** The organic electroluminescence device as claimed in claim 1, wherein the compound having at least one group represented by any one of Formulas (7-1) to (7-10) is a compound represented by the following Formula (9):

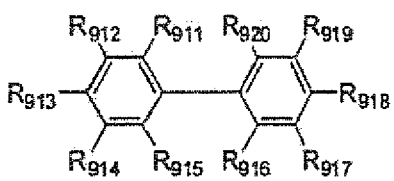

(9)

wherein, each of $R_{911}$ to $R_{920}$ independently represents a hydrogen atom, an alkyl group, an aryl group which may have an alkyl group, a fluorine group, a trifluoromethyl group. a cyano group, and a silyl group which may have an alkyl group; and at least one of $R_{911}$ to $R_{920}$ is a group represented by any one of Formula (7-1) to (7-10).

**4.** The organic electroluminescence device as claimed in claim 1, wherein the compound having at least one group represented by any one of Formulas (7-1) to (7-10) is a compound represented by the following Formula (10):

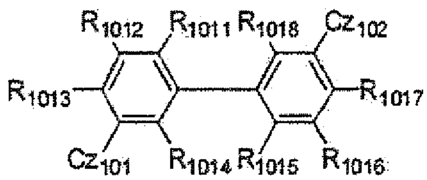

(10)

wherein, each of $R_{1011}$ to $R_{1018}$ independently represents a hydrogen atom, an alkyl group, an aryl group which may have an alkyl group, a fluorine group, a trifluoromethyl group, or a cyano group; and each of $Cz_{101}$ and $Cz_{102}$ independently represents a group represented by any one of Formula (7-1) to (7-10).

**5.** The organic electroluminescence device as claimed in claim 1, wherein the compound having at least one group represented by any one of Formulas (7-1) to (7-10) is a compound represented by the following Formula (11):

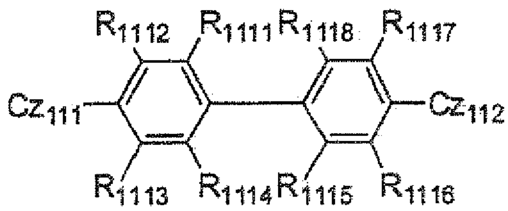

(11)

wherein, each of $R_{1111}$ to $R_{1118}$ independently represents a hydrogen atom, an alkyl group, an aryl group which may have an alkyl group, a fluorine group, a trifluoromethyl group, a cyano group, and a silyl group which may have an alkyl group; and each of $Cz_{111}$ and $Cz_{112}$ independently represents a group represented by any one of Formula (7-1) to (7-10).

**6.** The organic electroluminescence device as claimed in claim 1, wherein the compound having at least one group represented by any one of Formulas (7-1) to (7-10) is a compound represented by the following Formula (13):

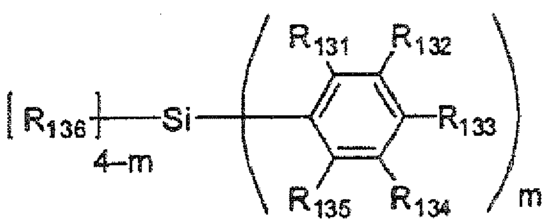

(13)

wherein, each of $R_{131}$ to $R_{135}$ independently represents a hydrogen atom and at least one of $R_{131}$ to $R_{135}$ is a group represented by any one of Formulas (7-1) to (7-10); $R_{136}$ represents a methyl group or a phenyl group which may have an alkyl group; each $R_{136}$ may be the same as or different from every other $R_{136}$; and m represents an integer of 1 to 4.

**7.** The organic electroluminescence device as claimed in claim 1, wherein the compound having at least one group represented by any one of Formulas (7-1) to (7-10) is a compound represented by the following Formula (14):

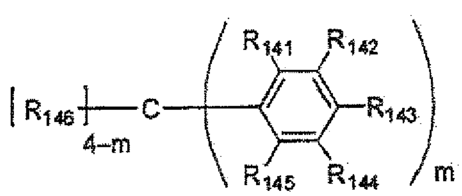

(14)

wherein, each of $R_{141}$ to $R_{145}$ independently represents a hydrogen atom, and at least one of $R_{141}$ to $R_{145}$ is a group represented by any one of Formulas (7-1) to (7-10); $R_{146}$ represents a hydrogen atom, a methyl group, or a phenyl group which may have an alkyl group, and each $R_{146}$ may be the same as or different from every other $R_{146}$; and m represents an integer of 1 to 4.

**8.** The organic electroluminescence device as claimed in claim 1, wherein the compound having at least one group represented by any one of Formulas (7-1) to (7-10) is a compound represented by the following Formula (15):

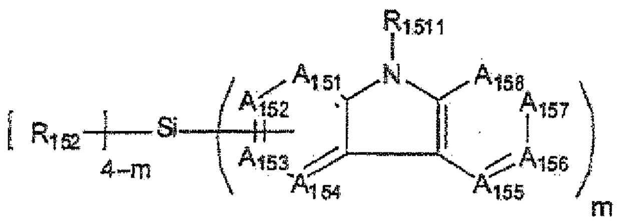
(15)

wherein, $A_{151}$ to $A_{158}$ represent an N atom or C-$R_{153}$, an alkyl group, and $R_{153}$ represents a hydrogen atom, an alkyl group, or an alicyclic hydrocarbon group which may have an alkyl group; $R_{1511}$ represents a phenyl group which may have a substituent, and the substituent which the phenyl group may have is an alkyl group or a phenyl group; each of $R_{152}$ independently represents a methyl group or a phenyl group which may have an alkyl group, and each $R_{152}$ may be the same as or different from every other $R_{152}$; m represents an integer of 1 to 4; and a silicon linking group is linked with a C atom of $A_{151}$ to $A_{158}$.

**9.** The organic electroluminescence device as claimed in claim 1, wherein the compound having at least one group represented by any one of Formulas (7-1) to (7-10) is a compound represented by the following Formula (16):

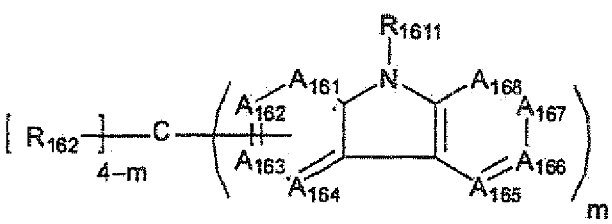
(16)

wherein, $A_{161}$ to $A_{168}$ represent an N atom or C-$R_{163}$ and $R_{163}$ represents a hydrogen atom, an alkyl group, or an alicyclic hydrocarbon group which may have an alkyl group; $R_{1611}$ represents a phenyl group which may have a substituent, and the substituent which the phenyl group may have is an alkyl group or a phenyl group; each of $R_{162}$ independently represents a methyl group or a phenyl group which may have an alkyl group; each $R_{162}$ may be the same as or different from every other $R_{162}$; m represents an integer of 1 to 4; and a carbon linking group is linked with a C atom of $A_{161}$ to $A_{168}$.

**10.** The organic electroluminescence device as claimed in any one of claims 1 to 9, wherein the substituent (S) is a group selected from the following (a) to (e), (i), (1), and (t) to (v);

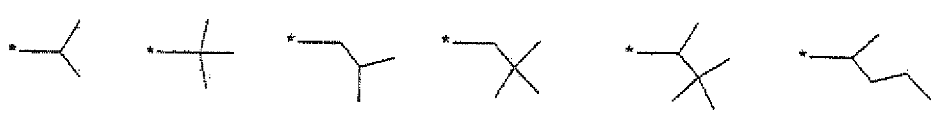
(a) (b) (c) (d) (e) (i)

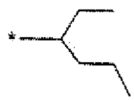 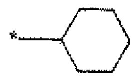 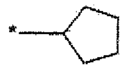 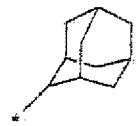

(l) (t) (u) (v)

**11.** The organic electroluminescence device as claimed in claim 10, wherein the substituent (S) is a group selected from the (a) to (e).

**12.** The organic electroluminescence device as claimed in any one of claims 1 to 11, wherein the compound having at least one group represented by any one of Formulas (7-1) to (7-10) is comprised in the light emitting layer.

**13.** The organic electroluminescence device as claimed in any one of claims 1 to 12, wherein the compound having at least one group represented by any one of Formulas (7-1) to (7-10) is comprised in a layer adjacent to the light emitting layer.

**14.** The organic electroluminescence device as claimed in any one of claims 1 to 13, wherein the iridium complex (A9-1) is comprised in the light emitting layer.

**15.** The organic electroluminescence device as claimed in any one of claims 1 to 13, wherein the compound having at least one group represented by any one of Formulas (7-1) to (7-10) and the iridium complex (A9-1) are comprised in the light emitting layer.

**16.** The organic electroluminescence device as claimed in any one of claims 1 to 15, wherein at least one of the organic layers comprising the compound having at least one group represented by any one of Formulas (7-1) to (7-10) and an organic layer comprising the iridium complex (A9-1) is formed by a wet process.

**17.** A composition comprising the compound having at least one group represented by any one of Formulas (7-1) to (7-10) as claimed in claim 1 and an iridium complex represented by (A9-1) as claimed in claim 1.

**18.** A light emitting layer comprising a compound having at least one group represented by any one of Formulas (7-1) to (7-10) as claimed in claim 1 and an iridium complex represented by (A9-1) as claimed in claim 1.

**19.** A light emission apparatus using an organic electroluminescence device as claimed in any one of claims 1 to 16.

**20.** A display apparatus using an organic electroluminescence device as claimed in any one of claims 1 to 16.

**21.** An illumination apparatus using the organic electroluminescence device as claimed in any one of claims 1 to 16.

FIG.1

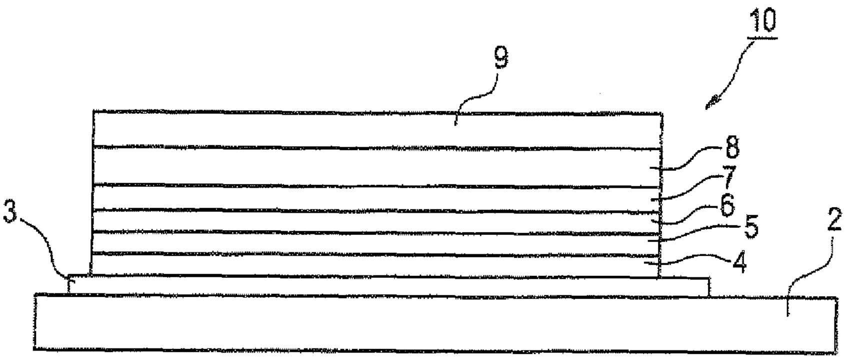


FIG.2

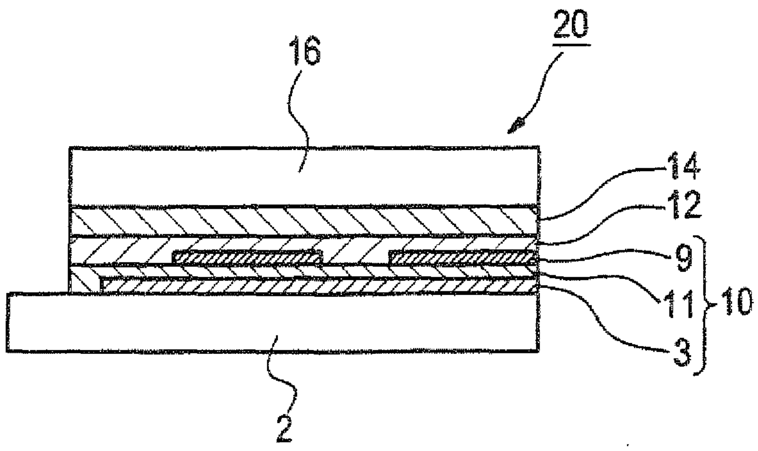


FIG.3

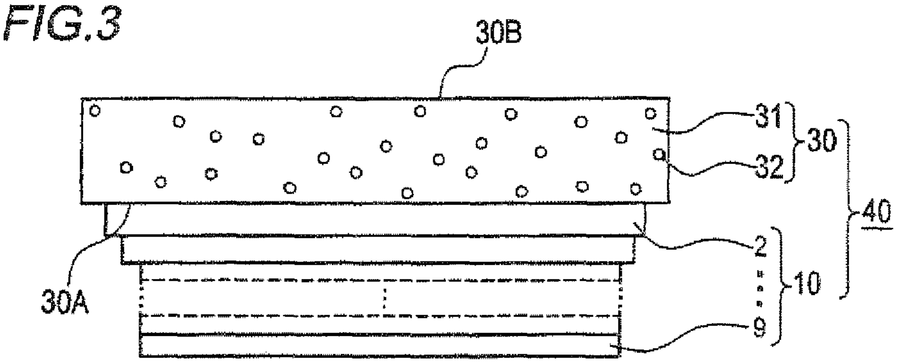

**INTERNATIONAL SEARCH REPORT**

International application No.
PCT/JP2010/064648

A. CLASSIFICATION OF SUBJECT MATTER
*H01L51/50*(2006.01)i, *C07D519/00*(2006.01)i, *C09K11/06*(2006.01)i, *C07F15/00* (2006.01)n

According to International Patent Classification (IPC) or to both national classification and IPC

B. FIELDS SEARCHED

Minimum documentation searched (classification system followed by classification symbols)
H01L51/50, C07D519/00, C09K11/06, C07F15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA, REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2008/140114 A1 (Konica Minolta Holdings, Inc.), 20 November 2008 (20.11.2008), claims; paragraphs [0045] to [0048]; paragraph [0119], compound A-97; paragraphs [0283] to [0285], [0296], [0324] to [0326], [0440], [0446]; paragraph [0452], ETL-1; paragraph [0457]; paragraph [0471] to paragraph [0472], table 1, element no.1 to 10 (Family: none) | 1-21 |
| A | JP 2009-102533 A (Konica Minolta Holdings, Inc.), 14 May 2009 (14.05.2009), claims; paragraphs [0315] to [0325] (Family: none) | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 September, 2010 (16.09.10) | 28 September, 2010 (28.09.10) |
| Name and mailing address of the ISA/ Japanese Patent Office | Authorized officer |
| Facsimile No. | Telephone No. |

**INTERNATIONAL SEARCH REPORT**

International application No.
PCT/JP2010/064648

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-311608 A (Konica Minolta Holdings, Inc.), 25 December 2008 (25.12.2008), claims; paragraphs [0188] to [0216] & WO 2008/140069 A1 | 1-21 |
| A | WO 2008/143059 A1 (Konica Minolta Holdings, Inc.), 27 November 2008 (27.11.2008), claims; paragraphs [0311] to [0317] (Family: none) | 1-21 |
| A | JP 2006-120821 A (Konica Minolta Holdings, Inc.), 11 May 2006 (11.05.2006), claims; paragraphs [0239] to [0240], [0243] to [0253] (Family: none) | 1-21 |
| A | JP 2006-120763 A (Konica Minolta Holdings, Inc.), 11 May 2006 (11.05.2006), claims; paragraphs [0086], [0195], [0212] to [0215], [0219] to [0234] (Family: none) | 1-21 |
| A | WO 2006/043440 A1 (Konica Minolta Holdings, Inc.), 27 April 2006 (27.04.2006), claims; paragraphs [0081], [0201] to [0204], [0208] to [0223] & JP 2006-542331 A | 1-21 |
| A | WO 2006/013739 A1 (Konica Minolta Holdings, Inc.), 09 February 2006 (09.02.2006), claims; paragraphs [0045], [0141], [0158] to [0161], [0165] to [0179] & JP 2006-531384 A | 1-21 |
| A | JP 2005-340123 A (Konica Minolta Holdings, Inc.), 08 December 2005 (08.12.2005), claims; paragraphs [0090], [0215] to [0217], [0232] to [0248] (Family: none) | 1-21 |
| A | WO 2004/095890 A1 (Konica Minolta Holdings, Inc.), 04 November 2004 (04.11.2004), claims; page 26, lines 13 to 19; page 48, line 21 to page 49, line 10; page 56, line 1 to page 61, line 22 & JP 2005-505744 A | 1-21 |

**INTERNATIONAL SEARCH REPORT**

International application No.
PCT/JP2010/064648

C (Continuation). DOCUMENTS CONSIDERED TO BE RELEVANT

| Category* | Citation of document, with indication, where appropriate, of the relevant passages | Relevant to claim No. |
|---|---|---|
| A | WO 2004/095889 A1 (Konica Minolta Holdings, Inc.), 04 November 2004 (04.11.2004), claims; page 83, lines 7 to 17; page 90, line 5 to page 95, line 22 & US 2005/0249970 A1 & EP 1617711 A1 & JP 2005-505736 A | 1-21 |
| A | JP 2009-094486 A (Fujifilm Corp.), 30 April 2009 (30.04.2009), claims & US 2009/0072727 A1 & EP 2039737 A2 | 1-21 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 080297033 B
- JP 2005340123 A
- JP 2006120821 A
- WO 07095118 A
- JP 2007019462 A
- US 20070190359 A
- US 20080297033 A
- JP 2008270736 A
- US 6303238 B1
- US 6097147 A
- WO 0057676 A
- WO 0070655 A
- WO 018230 A
- WO 0139234 A2
- WO 0141512 A1
- WO 0202714 A
- WO 0215645 A1
- WO 0244189 A1
- WO 0519373 A2
- JP 2001247859 A
- JP 2002302671 A
- JP 2002117978 A
- JP 2003133074 A
- JP 2002235076 A
- JP 2003123982 A
- JP 2002170684 A
- EP 1211257 A
- JP 2002226495 A
- JP 2002234894 A
- JP 2001298470 A
- JP 2002173674 A
- JP 2002203678 A
- JP 2002203679 A
- JP 2004357791 A
- JP 2006256999 A
- JP 2007084635 A
- JP 2007096259 A
- JP HEI2148687 B
- JP HEI6301355 B
- JP HEI529080 B
- JP HEI7134558 B
- JP HEI8234685 B
- JP HEI8241047 B
- JP 2784615 B
- US 5828429 A
- US 6023308 A
- JP HEI9180883 B
- JP 2004127795 A
- JP 2009201152 A
- JP 2009223452 A
- JP 2010079922 A


### Non-patent literature cited in the description


- *J. Chem. Soc., Perkin Trains.,* 1999, vol. 1, 1505-1510
- *J. Org. Chem.,* 1933, 7832-7838
- *Tetrahedron,* 1993, 49-64